# EUROPEAN PATENT SPECIFICATION

(11) **EP 2 473 528 B1**
(45) Date of publication and mention of the grant of the patent: **03.12.2014**
(21) Application number: 10747896.8
(22) Date of filing: 03.09.2010
(51) Int. Cl.: A61K 39/395

(54) **STABLE FORMULATIONS OF POLYPEPTIDES AND USES THEREOF**
STABILE FORMULIERUNGEN AUS POLYPEPTIDEN UND VERWENDUNGEN DAVON
FORMULATIONS STABLES DE POLYPEPTIDES ET LEURS UTILISATIONS

(30) Priority: 03.09.2009 US 275816 P; 18.12.2009 US 284502 P
(43) Date of publication of application: 11.07.2012
(60) Divisional application: 14171120.0
(73) Proprietor: Ablynx N.V., 9052 Ghent-Zwijnaarde (BE)
(72) Inventor: BRIGE, Ann, B-9940 Ertvelde (BE); LABEUR, Christine, B-8000 Brugge (BE); DE BRABANDERE, Veronique, B-9000 Gent (BE)
(74) Representative: Hoffmann Eitle
(86) International application number: PCT/EP2010/062975
(87) International publication number: WO 2011/026948

(56) References cited:
- WO-A1-2008/020079
- WO-A1-2008/071685
- WO-A1-2010/060768
- WO-A2-2004/041863
- WO-A2-2006/040153
- WO-A2-2006/074947
- WO-A2-2007/002261
- WO-A2-2007/104529
- WO-A2-2008/142164
- WO-A2-2010/077422

### SEQUENCE LISTING

<110> Ablynx NV
<120> STABLE FORMULATIONS OF POLYPEPTIDES AND USES THEREOF
<130> P09-023-PCT-1
<160> 6
<170> PatentIn version 3.5
<210> 1
   <211> 245
   <212> PRT
   <213> Artificial Sequence
<220>
   <223> Nanobody sequence
<400> 1
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Ser Val Phe Lys Ile Asn
               20                  25                  30
   Val Met Ala Trp Tyr Arg Gln Ala Pro Gly Lys Gly Arg Glu Leu Val
           35                  40                  45
   Ala Gly Ile Ile Ser Gly Gly Ser Thr Ser Tyr Ala Asp Ser Val Lys
       50                  55                  60
   Gly Arg Phe Thr Ile Ser Arg Asp Asn Ala Lys Asn Thr Leu Tyr Leu
   65                  70                  75                  80
   Gln Met Asn Ser Leu Arg Pro Glu Asp Thr Ala Val Tyr Tyr Cys Ala
                   85                  90                  95
   Phe Ile Thr Thr Glu Ser Asp Tyr Asp Leu Gly Arg Arg Tyr Trp Gly
               100                 105                 110
   Gln Gly Thr Leu Val Thr Val Ser Ser Gly Gly Gly Gly Ser Gly Gly
           115                 120                 125
   Gly Ser Glu Val Gln Leu Val Glu Ser Gly Gly Gly Leu Val Gln Pro
       130                 135                 140
   Gly Asn Ser Leu Arg Leu Ser Cys Ala Ala Ser Gly Phe Thr Phe Ser
   145                 150                 155                 160
   Ser Phe Gly Met Ser Trp Val Arg Gln Ala Pro Gly Lys Gly Leu Glu
                   165                 170                 175
   Trp Val Ser Ser Ile Ser Gly Ser Gly Ser Asp Thr Leu Tyr Ala Asp
               180                 185                 190
   Ser Val Lys Gly Arg Phe Thr Ile Ser Arg Asp Asn Ala Lys Thr Thr
           195                 200                 205
   Leu Tyr Leu Gln Met Asn Ser Leu Arg Pro Glu Asp Thr Ala Val Tyr
       210                 215                 220
   Tyr Cys Thr Ile Gly Gly Ser Leu Ser Arg Ser Ser Gln Gly Thr Leu
   225                 230                 235                 240
   Val Thr Val Ser Ser
                   245
<210> 2
   <211> 375
   <212> PRT
   <213> Artificial Sequence
<220>
   <223> Nanobody sequence
<400> 2
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Ser Val Phe Lys Ile Asn
               20                  25                  30
   Val Met Ala Trp Tyr Arg Gln Ala Pro Gly Lys Gly Arg Glu Leu Val
           35                  40                  45
   Ala Gly Ile Ile Ser Gly Gly Ser Thr Ser Tyr Ala Asp Ser Val Lys
       50                  55                  60
   Gly Arg Phe Thr Ile Ser Arg Asp Asn Ala Lys Asn Thr Leu Tyr Leu
   65                  70                  75                  80
   Gln Met Asn Ser Leu Arg Pro Glu Asp Thr Ala Val Tyr Tyr Cys Ala
                   85                  90                  95
   Phe Ile Thr Thr Glu Ser Asp Tyr Asp Leu Gly Arg Arg Tyr Trp Gly
               100                 105                 110
   Gln Gly Thr Leu Val Thr Val Ser Ser Gly Gly Gly Gly Ser Gly Gly
           115                 120                 125
   Gly Ser Glu Val Gln Leu Val Glu Ser Gly Gly Gly Leu Val Gln Pro
       130                 135                 140
   Gly Gly Ser Leu Arg Leu Ser Cys Ala Ala Ser Gly Ser Val Phe Lys
   145                 150                 155                 160
   Ile Asn Val Met Ala Trp Tyr Arg Gln Ala Pro Gly Lys Gly Arg Glu
                   165                 170                 175
   Leu Val Ala Gly Ile Ile Ser Gly Gly Ser Thr Ser Tyr Ala Asp Ser
               180                 185                 190
   Val Lys Gly Arg Phe Thr Ile Ser Arg Asp Asn Ala Lys Asn Thr Leu
           195                 200                 205
   Tyr Leu Gln Met Asn Ser Leu Arg Pro Glu Asp Thr Ala Val Tyr Tyr
       210                 215                 220
   Cys Ala Phe Ile Thr Thr Glu Ser Asp Tyr Asp Leu Gly Arg Arg Tyr
   225                 230                 235                 240
   Trp Gly Gln Gly Thr Leu Val Thr Val Ser Ser Gly Gly Gly Gly Ser
                   245                 250                 255
   Gly Gly Gly Ser Glu Val Gln Leu Val Glu Ser Gly Gly Gly Leu Val
               260                 265                 270
   Gln Pro Gly Asn Ser Leu Arg Leu Ser Cys Ala Ala Ser Gly Phe Thr
           275                 280                 285
   Phe Ser Ser Phe Gly Met Ser Trp Val Arg Gln Ala Pro Gly Lys Gly
       290                 295                 300
   Leu Glu Trp Val Ser Ser Ile Ser Gly Ser Gly Ser Asp Thr Leu Tyr
   305                 310                 315                 320
   Ala Asp Ser Val Lys Gly Arg Phe Thr Ile Ser Arg Asp Asn Ala Lys
                   325                 330                 335
   Thr Thr Leu Tyr Leu Gln Met Asn Ser Leu Arg Pro Glu Asp Thr Ala
               340                 345                 350
   Val Tyr Tyr Cys Thr Ile Gly Gly Ser Leu Ser Arg Ser Ser Gln Gly
           355                 360                 365
   Thr Leu Val Thr Val Ser Ser
       370                 375
<210> 3
   <211> 375
   <212> PRT
   <213> Artificial Sequence
<220>
   <223> Nanobody sequence
<400> 3
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Ser Val Phe Lys Ile Asn
               20                  25                  30
   Val Met Ala Trp Tyr Arg Gln Ala Pro Gly Lys Gly Arg Glu Leu Val
           35                  40                  45
   Ala Gly Ile Ile Ser Gly Gly Ser Thr Ser Tyr Ala Asp Ser Val Lys
       50                  55                  60
   Gly Arg Phe Thr Ile Ser Arg Asp Asn Ala Lys Asn Thr Leu Tyr Leu
   65                  70                  75                  80
   Gln Met Asn Ser Leu Arg Pro Glu Asp Thr Ala Val Tyr Tyr Cys Ala
                   85                  90                  95
   Phe Ile Thr Thr Glu Ser Asp Tyr Asp Leu Gly Arg Arg Tyr Trp Gly
               100                 105                 110
   Gln Gly Thr Leu Val Thr Val Ser Ser Gly Gly Gly Gly Ser Gly Gly
           115                 120                 125
   Gly Ser Glu Val Gln Leu Val Glu Ser Gly Gly Gly Leu Val Gln Pro
       130                 135                 140
   Gly Asn Ser Leu Arg Leu Ser Cys Ala Ala Ser Gly Phe Thr Phe Ser
   145                 150                 155                 160
   Ser Phe Gly Met Ser Trp Val Arg Gln Ala Pro Gly Lys Gly Leu Glu
                   165                 170                 175
   Trp Val Ser Ser Ile Ser Gly Ser Gly Ser Asp Thr Leu Tyr Ala Asp
               180                 185                 190
   Ser Val Lys Gly Arg Phe Thr Ile Ser Arg Asp Asn Ala Lys Thr Thr
           195                 200                 205
   Leu Tyr Leu Gln Met Asn Ser Leu Arg Pro Glu Asp Thr Ala Val Tyr
       210                 215                 220
   Tyr Cys Thr Ile Gly Gly Ser Leu Ser Arg Ser Ser Gln Gly Thr Leu
   225                 230                 235                 240
   Val Thr Val Ser Ser Gly Gly Gly Gly Ser Gly Gly Gly Ser Glu Val
   245 250 255
   Gln Leu Val Glu Ser Gly Gly Gly Leu Val Gln Pro Gly Gly Ser Leu
   260 265 270
   Arg Leu Ser Cys Ala Ala Ser Gly Ser Val Phe Lys Ile Asn Val Met
   275 280 285
   Ala Trp Tyr Arg Gln Ala Pro Gly Lys Gly Arg Glu Leu Val Ala Gly
   290 295 300
   Ile Ile Ser Gly Gly Ser Thr Ser Tyr Ala Asp Ser Val Lys Gly Arg
   305 310 315 320
   Phe Thr Ile Ser Arg Asp Asn Ala Lys Asn Thr Leu Tyr Leu Gln Met
   325 330 335
   Asn Ser Leu Arg Pro Glu Asp Thr Ala Val Tyr Tyr Cys Ala Phe Ile
   340 345 350
   Thr Thr Glu Ser Asp Tyr Asp Leu Gly Arg Arg Tyr Trp Gly Gln Gly
   355 360 365
   Thr Leu Val Thr Val Ser Ser
   370 375
<210> 4
   <211> 385
   <212> PRT
   <213> Artificial Sequence
<220>
   <223> Nanobody sequence
<400> 4
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Pro Met Gly Trp Phe Arg Gln Ala Pro Gly Lys Gly Arg Glu Phe Val
           35                  40                  45
   Ser Ser Ile Thr Gly Ser Gly Gly Ser Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Pro Glu Asp Thr Ala Val Tyr Tyr Cys
                   85                  90                  95
   Ala Ala Tyr Ile Arg Pro Asp Thr Tyr Leu Ser Arg Asp Tyr Arg Lys
               100                 105                 110
   Tyr Asp Tyr Trp Gly Gln Gly Thr Leu Val Thr Val Ser Ser Gly Gly
           115                 120                 125
   Gly Gly Ser Gly Gly Gly Ser Glu Val Gln Leu Val Glu Ser Gly Gly
       130                 135                 140
   Gly Leu Val Gln Pro Gly Asn Ser Leu Arg Leu Ser Cys Ala Ala Ser
   145                 150                 155                 160
   Gly Phe Thr Phe Ser Ser Phe Gly Met Ser Trp Val Arg Gln Ala Pro
                   165                 170                 175
   Gly Lys Gly Leu Glu Trp Val Ser Ser Ile Ser Gly Ser Gly Ser Asp
               180                 185                 190
   Thr Leu Tyr Ala Asp Ser Val Lys Gly Arg Phe Thr Ile Ser Arg Asp
           195                 200                 205
   Asn Ala Lys Thr Thr Leu Tyr Leu Gln Met Asn Ser Leu Arg Pro Glu
       210                 215                 220
   Asp Thr Ala Val Tyr Tyr Cys Thr Ile Gly Gly Ser Leu Ser Arg Ser
   225                 230                 235                 240
   Ser Gln Gly Thr Leu Val Thr Val Ser Ser Gly Gly Gly Gly Ser Gly
                   245                 250                 255
   Gly Gly Ser Glu Val Gln Leu Val Glu Ser Gly Gly Gly Leu Val Gln
               260                 265                 270
   Pro Gly Gly Ser Leu Arg Leu Ser Cys Ala Ala Ser Gly Phe Thr Phe
           275                 280                 285
   Ser Ser Tyr Pro Met Gly Trp Phe Arg Gln Ala Pro Gly Lys Gly Arg
       290                 295                 300
   Glu Phe Val Ser Ser Ile Thr Gly Ser Gly Gly Ser Thr Tyr Tyr Ala
   305                 310                 315                 320
   Asp Ser Val Lys Gly Arg Phe Thr Ile Ser Arg Asp Asn Ala Lys Asn
                   325                 330                 335
   Thr Leu Tyr Leu Gln Met Asn Ser Leu Arg Pro Glu Asp Thr Ala Val
   340 345 350
   Tyr Tyr Cys Ala Ala Tyr Ile Arg Pro Asp Thr Tyr Leu Ser Arg Asp
   355 360 365
   Tyr Arg Lys Tyr Asp Tyr Trp Gly Gln Gly Thr Leu Val Thr Val Ser
   370 375 380
   Ser
   385
<210> 5
   <211> 386
   <212> PRT
   <213> Artificial Sequence
<220>
   <223> Nanobody sequence
<400> 5
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Arg Ile Phe Ser Leu Pro
               20                  25                  30
   Ala Ser Gly Asn Ile Phe Asn Leu Leu Thr Ile Ala Trp Tyr Arg Gln
           35                  40                  45
   Ala Pro Gly Lys Gly Arg Glu Leu Val Ala Thr Ile Asn Ser Gly Ser
       50                  55                  60
   Arg Thr Tyr Tyr Ala Asp Ser Val Lys Gly Arg Phe Thr Ile Ser Arg
   65                  70                  75                  80
   Asp Asn Ser Lys Lys Thr Leu Tyr Leu Gln Met Asn Ser Leu Arg Pro
                   85                  90                  95
   Glu Asp Thr Ala Val Tyr Tyr Cys Gln Thr Ser Gly Ser Gly Ser Pro
               100                 105                 110
   Asn Phe Trp Gly Gln Gly Thr Leu Val Thr Val Ser Ser Gly Gly Gly
           115                 120                 125
   Gly Ser Gly Gly Gly Ser Glu Val Gln Leu Val Glu Ser Gly Gly Gly
       130                 135                 140
   Leu Val Gln Pro Gly Asn Ser Leu Arg Leu Ser Cys Ala Ala Ser Gly
   145                 150                 155                 160
   Phe Thr Phe Ser Ser Phe Gly Met Ser Trp Val Arg Gln Ala Pro Gly
                   165                 170                 175
   Lys Gly Leu Glu Trp Val Ser Ser Ile Ser Gly Ser Gly Ser Asp Thr
               180                 185                 190
   Leu Tyr Ala Asp Ser Val Lys Gly Arg Phe Thr Ile Ser Arg Asp Asn
           195                 200                 205
   Ala Lys Thr Thr Leu Tyr Leu Gln Met Asn Ser Leu Arg Pro Glu Asp
       210                 215                 220
   Thr Ala Val Tyr Tyr Cys Thr Ile Gly Gly Ser Leu Ser Arg Ser Ser
   225                 230                 235                 240
   Gln Gly Thr Leu Val Thr Val Ser Ser Gly Gly Gly Gly Ser Gly Gly
                   245                 250                 255
   Gly Ser Glu Val Gln Leu Leu Glu Ser Gly Gly Gly Leu Val Gln Pro
               260                 265                 270
   Gly Gly Ser Leu Arg Leu Ser Cys Ala Ala Ser Gly Arg Thr Leu Ser
           275                 280                 285
   Ser Tyr Ala Met Gly Trp Phe Arg Gln Ala Pro Gly Lys Gly Arg Glu
       290                 295                 300
   Phe Val Ser Arg Ile Ser Gln Gly Gly Thr Ala Ile Tyr Tyr Ala Asp
   305                 310                 315                 320
   Ser Val Lys Gly Arg Phe Thr Ile Ser Arg Asp Asn Ser Lys Asn Thr
                   325                 330                 335
   Leu Tyr Leu Gln Met Asn Ser Leu Arg Pro Glu Asp Thr Ala Val Tyr
               340                 345                 350
   Tyr Cys Ala Lys Asp Pro Ser Pro Tyr Tyr Arg Gly Ser Ala Tyr Leu
           355                 360                 365
   Leu Ser Gly Ser Tyr Asp Ser Trp Gly Gln Gly Thr Leu Val Thr Val
       370                 375                 380
   Ser Ser
   385
<210> 6
   <211> 386
   <212> PRT
   <213> Artificial Sequence
<220>
   <223> Nanobody sequence
<400> 6
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Arg Ile Phe Ser Leu Pro
               20                  25                  30
   Ala Ser Gly Asn Ile Phe Asn Leu Leu Thr Ile Ala Trp Tyr Arg Gln
           35                  40                  45
   Ala Pro Gly Lys Gly Arg Glu Leu Val Ala Thr Ile Asn Ser Gly Ser
       50                  55                  60
   Arg Thr Tyr Tyr Ala Asp Ser Val Lys Gly Arg Phe Thr Ile Ser Arg
   65                  70                  75                  80
   Asp Asn Ser Lys Lys Thr Leu Tyr Leu Gln Met Asn Ser Leu Arg Pro
                   85                  90                  95
   Glu Asp Thr Ala Val Tyr Tyr Cys Gln Thr Ser Gly Ser Gly Ser Pro
               100                 105                 110
   Asn Phe Trp Gly Gln Gly Thr Leu Val Thr Val Ser Ser Gly Gly Gly
           115                 120                 125
   Gly Ser Gly Gly Gly Ser Glu Val Gln Leu Val Glu Ser Gly Gly Gly
       130                 135                 140
   Leu Val Gln Pro Gly Asn Ser Leu Arg Leu Ser Cys Ala Ala Ser Gly
   145                 150                 155                 160
   Phe Thr Phe Ser Ser Phe Gly Met Ser Trp Val Arg Gln Ala Pro Gly
                   165                 170                 175
   Lys Gly Leu Glu Trp Val Ser Ser Ile Ser Gly Ser Gly Ser Asp Thr
               180                 185                 190
   Leu Tyr Ala Asp Ser Val Lys Gly Arg Phe Thr Ile Ser Arg Asp Asn
           195                 200                 205
   Ala Lys Thr Thr Leu Tyr Leu Gln Met Asn Ser Leu Arg Pro Glu Asp
       210                 215                 220
   Thr Ala Val Tyr Tyr Cys Thr Ile Gly Gly Ser Leu Ser Arg Ser Ser
   225                 230                 235                 240
   Gln Gly Thr Leu Val Thr Val Ser Ser Gly Gly Gly Gly Ser Gly Gly
                   245                 250                 255
   Gly Ser Glu Val Gln Leu Leu Glu Ser Gly Gly Gly Leu Val Gln Pro
               260                 265                 270
   Gly Gly Ser Leu Arg Leu Ser Cys Ala Ala Ser Gly Arg Thr Leu Ser
           275                 280                 285
   Ser Tyr Ala Met Gly Trp Phe Arg Gln Ala Pro Gly Lys Gly Arg Glu
       290                 295                 300
   Phe Val Ala Arg Ile Ser Gln Gly Gly Thr Ala Ile Tyr Tyr Ala Asp
   305                 310                 315                 320
   Ser Val Lys Gly Arg Phe Thr Ile Ser Arg Asp Asn Ser Lys Asn Thr
                   325                 330                 335
   Leu Tyr Leu Gln Met Asn Ser Leu Arg Pro Glu Asp Thr Ala Val Tyr
               340                 345                 350
   Tyr Cys Ala Lys Asp Pro Ser Pro Tyr Tyr Arg Gly Ser Ala Tyr Leu
           355                 360                 365
   Leu Ser Gly Ser Tyr Asp Ser Trp Gly Gln Gly Thr Leu Val Thr Val
       370                 375                 380
   Ser Ser
   385

## Description

### FIELD OF THE INVENTION

The present invention relates to formulations of single variable domains. More specifically the present invention provides formulations that contain single variable domains with a good solubility and good stability under different storage and stress conditions. The formulations of the invention are suitable for administration to human subjects.

The invention further relates to containers and pharmaceutical units comprising such formulations and to prophylactic and therapeutic uses of the formulations and pharmaceutical units of the invention.

Other aspects, embodiments, advantages and applications of the invention will become clear from the further description herein.

### BACKGROUND ART

Nanobodies (as further described herein) are characterized by formation of the antigen binding site by a single variable domain, which does not require interaction with a further domain (e.g. in the form of VH/VL interaction) for antigen recognition. Nanobodies have been described against a wide range of different targets (WO 04/062551, WO 05/044858, WO 06/040153, WO 06/122825, WO 07/104529, WO 08/020079, WO 08/074839, WO 08/071447, WO 08/074840, WO 08/074867, WO 08/077945, WO 08/101985, WO 08/142164, WO 09/068625, WO 08/142165, WO 09/068627) which could be ideal candidates for drug development. Nanobodies against IL-6R that can inhibit the IL-6/IL-6R interaction are described in WO 08/020079. Nanobodies against the p19 subunit of IL-23 that block the interaction of IL-23 with its receptor have been described in WO 09/068627. Nanobodies against RANKL that can inhibit osteoclast formation are described in WO 08/142164. The OPG/RANKL/RANK system has recently been discovered as pivotal regulatory factors in the pathogenesis of bone diseases and disorders like e.g. osteoporosis.

Proteins, such as therapeutic antibodies and Nanobodies, are often transported and/or stored for later use. It is important therefore that such proteins preserve the stability and biological activity of the protein under various conditions such as different temperature regimens and mechanical stress.

Certain prior liquid antibody preparations have shown short shelf lives and loss of biological activity of the antibodies resulting from chemical and/or physical instabilities during the transportation and storage. Chemical instability may be caused by deamidation, racemization, hydrolysis, oxidation, beta elimination or disulfide exchange, and physical instability may be caused by antibody denaturation, aggregation, precipitation or adsorption. Among those, aggregation, deamidation and oxidation are known to be the most common causes of the antibody degradation (Cleland et al., 1993, Critical Reviews in Therapeutic Drug Carrier Systems 10: 307-377). Little is known about drug formulation components that provide stable liquid formulations of Nanobodies.

There exists a need for stable liquid formulations of Nanobodies which show a good solubility of the Nanobody and which exhibit increases stability, low to undetectable levels of aggregation, low to undetectable levels of Nanobody degradation, and very little to no loss of biological activity of the Nanobody, even under different transportation and storage conditions.

### SUMMARY OF THE INVENTION

The present invention provides improved formulations (also referred to as *"formulation(s) of the invention"*) of polypeptides comprising one or more single variable domains that show good solubility of the single variable domains and retain increased stability of the single variable domains under a variety of different transportation, storage and in-use conditions. The present invention is based on the finding that the presence in the formulation of certain buffers may increase the solubility, the melting temperature and/or the stability of the single variable domains present in the formulation.

The present invention provides solubility data for formulations with polypeptides comprising one or more single variable domains (also referred to as *"polypeptide(s) of the invention"*) up to 150 mg/mL and higher. The invention further shows that such formulations can be transported, manipulated through various administration devices and retain activity, purity and potency under different stress conditions: mechanical stress conditions; storage of the formulation at various stress conditions such as different freeze/thaw cycles, at 2-8°C, at 25±5°C and at elevated temperature.

The formulation of the present invention comprises an aqueous carrier with a pH of 5.5 to 8.0 and a polypeptide comprising one or more single variable domains at a concentration of 1 mg/ml to 200 mg/ml, said formulation being formulated for administration to a human subject and said formulation further comprising:
a) A buffer at a concentration of 10mM to 100mM selected from histidine pH 6.0-6.5
   wherein said formulation has an inorganic salt concentration of 150 mM or lower and wherein the polypeptide is selected from one of SEQ ID NOS: 1 to 6

The present inventors observed that formulations that have 150 mM or less of inorganic salt show a much better stability of the single variable domains contained in the formulation. Inorganic salts frequently used in pharmaceutical formulation are NaCl and KCl. The single variable domains present in formulations containing 150 mM or less of inorganic salt have shown increased stability (e.g. less tendency to form aggregates, dimers and/or pyroglutamate, or to loose potency) at different stress storage conditions (such as e.g. during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more)), an improved melting temperature and/or an increased solubility. Preferably, the formulation contains 100 mM or less of inorganic salt, more preferably even 50 mM or less of inorganic salt. Most preferably the formulation does not contain any inorganic salt.

The polypeptide (also referred to as *"polypeptide of the invention*") comprising one or more single variable domains for use in the formulation of the invention may be therapeutic or prophylactic, and may be useful in the prevention, treatment and/or management of one or more diseases and/or disorders. In one specific aspect, the polypeptide has at least two single variable domains. In another specific aspect, the polypeptide has at least three single variable domains. Polypeptides of the invention may be selected from SEQ ID NO's: 1 to 6.

The polypeptide of the invention is present in the formulation of the present invention at a concentration of 1 to 200 mg/mL, preferably about 5 to 100 mg/mL or more, more preferably about 5 to 50 mg/mL or more, most preferably about 5 to 30 mg/mL or more, such as around 5 mg/mL, around 10 mg/mL, around 20 mg/mL, around 30 mg/mL, around 40 mg/mL, around 50 mg/mL, around 60 mg/mL, around 70 mg/mL, around 80 mg/mL, around 90 mg/mL, around 100 mg/mL, around 150 mg/mL or even more.

In an aspect of the invention, the formulation is homogeneous. In another aspect, the formulation of the invention is sterile. In addition to the polypeptide of the invention, the formulation of the present invention comprises at least an aqueous carrier (e.g. distilled water, MilliQ water or WFI) and a buffer.

The pH of the formulation of the invention should be in the range of 5.5 to 8.0, preferably the pH is around 6.0 to 7.5, more preferably around 6.2 to 7.5 or around 6.2 to 7.0. Most preferably the pH is in the range of 6.5 to 7.0, such as e.g. pH 6.5. These pH ranges have shown to provide an increased melting temperature to the polypeptides present in the formulation of the invention.

Buffers for use in the formulation of the invention are, histidine pH 6.0-6.5. Formulations comprising one of these buffers have shown a very good solubility (as defined herein) of the polypeptides of the invention, an improved melting temperature of the polypeptides present in the formulation and increased stability (e.g. less tendency to form aggregates, dimers and/or pyroglutamate, or to lose potency) at different stress storage conditions (such as e.g. during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more)). The buffer is preferably at a concentration of about 10 to 20 mM, such as 10 mM or 15 mM. In a specific aspect, the formulation of the invention comprises a histidine buffer pH 6.5 at a concentration of 15 mM or a histidine buffer pH 6.0 at a concentration of 10 mM.

Accordingly, the present invention provides stable formulations of polypeptides comprising one or more single variable domains, said formulations comprising an aqueous carrier, the polypeptide at a concentration from about 1 to 200 mg/mL or more, preferably about 5 to 100 mg/mL or more, more preferably about 5 to 50 mg/mL or more, most preferably about 5 to 30 mg/mL or more, such as around 5 mg/mL, around 10 mg/mL, around 20 mg/mL, around 30 mg/mL, around 40 mg/mL, around 50 mg/mL, around 60 mg/mL, around 70 mg/mL, around 80 mg/mL, around 90 mg/mL, around 100 mg/mL, around 150 mg/mL or even more, and a buffer such as a histidine buffer with a pH ranging from 6.0 to 7.0 at a concentration of about 10 to 20 mM.

Preferably the formulation of the invention is isotonic or slightly hypotonic and/or has an osmolality of about 290 ± 60 mOsm/kg, such as about 240 or higher, 250 or higher or 260 or higher. Isotonicity of the formulation can be further adjusted by the addition of one or more excipients and/or tonifiers.

Preferred excipients/tonifiers for use in the formulation of the present invention are saccharides and/or polyols. Accordingly, in another aspect, the formulation of the invention comprises a saccharide and/or polyol. Formulations comprising one or more saccharides and/or polyols have shown increased stability (e.g. less tendency to form aggregates, dimers and/or pyroglutamate, or to loose potency) at different stress storage conditions (such as e.g. during storage at a temperature of 37±5°C up to at least 2 weeks (preferably at least 3 weeks, at least 5 weeks, at least 8 weeks, at least 10 weeks, at least 3 months, at least 6 months, at least 1 year,1.5 year or even 2 years or more) and during mechanical stress conditions) and/or an improved melting temperature of the polypeptides present in the formulation. In a specific aspect of the invention, the excipient present in the formulation of the invention is a non-reducing sugar. In another specific aspect, the excipient present in the formulation of the invention is a disaccharide. In another specific aspect, the excipient present in the formulation of the invention is selected from sucrose, trehalose, sorbitol and mannitol. The saccharide and/or polyol is preferably present in the formulation of the invention at a concentration of about 1% to 20%, preferably about 2.5% to 15%, more preferably about 5% to 10%, such as around 5%, around 7.5%, around 8% or around 10%.

Accordingly, the present invention provides stable formulations of polypeptides comprising one or more single variable domains, said formulations comprising an aqueous carrier, the polypeptide at a concentration from about 1 to 200 mg/mL or more, preferably about 5 to 100 mg/mL or more, more preferably about 5 to 50 mg/mL or more, most preferably about 5 to 30 mg/mL or more, such as around 5 mg/mL, around 10 mg/mL, around 20 mg/mL, around 30 mg/mL, around 40 mg/mL, around 50 mg/mL, around 60 mg/mL, around 70 mg/mL, around 80 mg/mL, around 90 mg/mL, around 100 mg/mL, around 150 mg/mL or even more, and a saccharide and/or polyol at a concentration of about 1% to 20%, preferably about 2.5% to 15%, more preferably about 5% to 10%, such as around 5%, around 7.5%, around 8% or around 10%.

In another specific aspect, the formulation of the invention may comprise one or more surfactants (e.g., Tween 20, Tween 80 or a poloxamer). Formulations comprising a surfactant have shown a very good solubility (as defined herein) of the polypeptides of the invention and/or increased stability under mechanical stress. The surfactant may be present at a concentration in the range of about 0.001% to 1% (preferably between about 0.001% to 0.1%, or about 0.01% to 0.1% such as around 0.001%, around 0.005%, around 0.01%, around 0.02%, around 0.05%, around 0.08%, around 0.1%, around 0.5%, or around 1% of the formulation, preferably around 0.01%).

Accordingly, the present invention provides stable formulations of polypeptides comprising one or more single variable domains, said formulations comprising an aqueous carrier, the polypeptide at a concentration from about 1 to 200 mg/mL or more, preferably about 5 to 100 mg/mL or more, more preferably about 5 to 50 mg/mL or more, most preferably about 5 to 30 mg/mL or more, such as around 5 mg/mL, around 10 mg/mL, around 20 mg/mL, around 30 mg/mL, around 40 mg/mL, around 50 mg/mL, around 60 mg/mL, around 70 mg/mL, around 80 mg/mL, around 90 mg/mL, around 100 mg/mL, around 150 mg/mL or even more, and a surfactant (e.g., Tween 20, Tween 80 or a poloxamer) at a concentration in the range of about 0.001% to 1% (preferably between about 0.001% to 0.1%, or about 0.01% to 0.1% such as around 0.001%, around 0.005%, around 0.01%, around 0.02%, around 0.05%, around 0.08%, around 0.1%, around 0.5%, or around 1% of the formulation, preferably around 0.01%).

A preferred formulation of the invention may comprise:
a) A histidine pH 6.5 buffer at a concentration of 10mM to 100mM, such as 10mM to 20 mM;
b) Sucrose at a concentration of 1% to 10%; and
c) Tween 80 at a concentration of 0.001% to 1%.

Another preferred formulation of the invention may comprise:
a) A histidine pH 6.5 buffer at a concentration of 15 mM;
b) Sucrose at a concentration of 8%; and
c) Tween 80 at a concentration of 0.01%.

Another preferred formulation of the invention may comprise:
a) A histidine pH 6.0 buffer at a concentration of 10mM to 100mM, such as 10mM to 20 mM;
b) Sucrose at a concentration of 1% to 10%; and
c) Tween 80 at a concentration of 0.001% to 1%.

Another preferred formulation of the invention may comprise:
a) A histidine pH 6.0 buffer at a concentration of 10 mM;
b) Sucrose at a concentration of 10%; and
c) Tween 80 at a concentration of 0.005%.

The present invention provides formulations of a polypeptide comprising one or more single variable domains which exhibit high solubility of the polypeptide, little to no aggregation of the polypeptide and high stability during long periods of storage.

In one aspect, the components present in the formulations of the invention have been selected such that the polypeptides of the invention have a solubility of at least 20 mg/mL, at least 50 mg/mL, preferably at least 90 mg/mL, at least 120 mg/mL, at least 150 mg/mL or even 200 mg/mL or more.

In another aspect, the components present in the formulations of the invention have been selected such that the polypeptide present in the formulation of the invention has a melting temperature of at least 59°C or more (such as 59.5°C or more), preferably at least 60°C or more (such as 60.5°C or more), more preferably at least 61°C or more (such as 61.5°C or more) or at least 62°C or more (such as 62.5°C or more), most preferably at least 63°C or more (such as 63.5°C or more) as measured by the thermal shift assay (TSA) and/or differential scanning calorimetry (DSC).

In yet another aspect, the formulation of the present invention exhibits stability under various stress conditions such as:
- multiple (up to 10) freeze/thaw cycles;
- storage at a temperature of 2-8°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- storage at a temperature of 25±5°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- storage at a temperature of 37±5°C, up to at least 2 weeks (preferably at least 3 weeks, at least 5 weeks, at least 8 weeks, at least 10 weeks, at least 3 months, at least 6 months, at least 1 year, 1.5 year or even 2 years or more); and/or
- mechanical stress.

Mechanical stress as used in the present invention can be any form of external force applied on the formulation that may affect the stability of the polypeptide present in the formulation. Without being limiting, the mechanical stress applied to the solution can be shear stress, stir stress, shake stress, rotation stress, etc. Preferably the formulation of the invention is stable under one or more of the following forms of mechanical stress:
- shaking the formulation during 10s to 1 min;
- pushing the formulation through a needle (25G, preferably 26G, more preferably 27G, even more preferably 28G, most preferably 29G or more) with a syringe (the syringe used can be any commercially available syringe, such as e.g. a 1 mL, 2 mL, 3mL, 4 mL, 5 ml, 10 mL up to 50 mL syringe);
- rotating for two days at 10 rpm; and/or
- stirring for 1 hour at room temperature and/or 4-48 hours (such as 4-8 hours, 12 hours, 24 hours or even 48 hours) at 4°C at at least 10 rpm (such as 50 rpm, 100 rpm or more).

Preferably, the formulations of the present invention are stable under more than one (such as two, three, four, five, six or seven) of the above stress conditions, most preferably under all of the above stress conditions.

Accordingly, the polypeptide of the invention present in the formulation of the invention:
- is stable after multiple (up to 10) freeze/thaw cycles, said stability as determined by OD320/OD280 measurement, SE-HPLC, RP-HPLC, IEX-HPLC, potency assay (such as Biacore or ELISA) and/or SDS-PAGE;
- is stable during storage at a temperature of 2-8°C up to at least 2 weeks (preferably at least 3 weeks, at least 5 weeks, at least 8 weeks, at least 10 weeks, at least 3 months, at least 6 months, at least 1 year, 1.5 year or even 2 years or more), said stability as determined by OD320/OD280 measurement, SE-HPLC, RP-HPLC, IEX-HPLC, potency assay (such as Biacore or ELISA) and/or SDS-PAGE;
- is stable during storage at a temperature of 25±5°C up to at least 2 weeks (preferably at least 3 weeks, at least 5 weeks, at least 8 weeks, at least 10 weeks, at least 3 months, at least 6 months, at least 1 year, 1.5 year or even 2 years or more), said stability as determined by OD320/OD280 measurement, SE-HPLC, RP-HPLC, IEX-HPLC, potency assay (such as Biacore or ELISA) and/or SDS-PAGE;
- is stable during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more), said stability as determined by OD320/OD280 measurement, SE-HPLC, RP-HPLC, IEX-HPLC, potency assay (such as Biacore or ELISA) and/or SDS-PAGE;
- is stable when shaking the formulation during 10s to 1 min;
- is stable when pushing the formulation through a needle (25G, preferably 26G, more preferably 27G, even more preferably 28G, most preferably 29G or more) with a syringe (the syringe used can be any commercially available syringe, such as e.g. a 1 mL, 2 mL, 3mL, 4 mL, 5 mL, 10 mL, 20 mL, 30 mL, 40 mL up to 50 mL syringe);
- is stable when rotating for two days at 10 rpm; and/or
- is stable when stirring for 1 hour at room temperature and/or 4-48 hours (such as 4-8 hours, 12 hours, 24 hours or even 48 hours) at 4°C at at least 10 rpm (such as 50 rpm, 100 rpm or more).

The stability of the formulations of the present invention can be demonstrated by the fact that less than 10% of the polypeptides forms pyroglutamate at the N-terminal glutamic acid (e.g. as assessed by RP-HPLC) and/or less than 10% of the polypeptides forms dimers (e.g. as assessed by SE-HPLC) during storage under one or more of the above stress conditions. Preferably less than 10% of the polypeptides forms pyroglutamate at the N-terminal glutamic acid (e.g. as assessed by RP-HPLC) and less than 10% of the polypeptides forms dimers (e.g. as assessed by SE-HPLC) during storage under one or more of the above stress conditions.

In a specific aspect, less than 10% of the polypeptides present in the formulation of the invention forms pyroglutamate at the N-terminal glutamic acid (e.g. as assessed by RP-HPLC) during storage at a temperature of 37±5°C for up to at least 2 weeks (preferably at least 3 weeks, at least 5 weeks, at least 8 weeks, at least 10 weeks, at least 3 months, at least 6 months, at least 1 year, 1.5 year or even 2 years or more). In another specific aspect, less than 10% of the polypeptides forms dimers (e.g. as assessed by SE-HPLC) during storage at a temperature of 37±5°C for up to at least 2 weeks (preferably at least 3 weeks, at least 5 weeks, at least 8 weeks, at least 10 weeks, at least 3 months, at least 6 months, at least 1 year, 1.5 year or even 2 years or more). In yet another specific aspect, less than 10% of the polypeptides present in the formulation of the invention forms pyroglutamate at the N-terminal glutamic acid (e.g. as assessed by RP-HPLC) during storage at a temperature of 37±5°C for up to at least 2 weeks (preferably at least 3 weeks, at least 5 weeks, at least 8 weeks, at least 10 weeks, at least 3 months, at least 6 months, at least 1 year, 1.5 year or even 2 years or more) and less than 10% of the polypeptides forms dimers (e.g. as assessed by SE-HPLC) during storage at a temperature of 37±5°C for up to at least 2 weeks (preferably at least 3 weeks, at least 5 weeks, at least 8 weeks, at least 10 weeks, at least 3 months, at least 6 months, at least 1 year, 1.5 year or even 2 years or more).

Apart from this and/or in addition, the stability of the formulations of the present invention can be demonstrated by the fact that it shows only low to undetectable levels of aggregation and/or particulate formation (e.g. as assessed by SE-HPLC, subvisible particle counting, analytical ultracentrifugation, dynamic light scattering, OD320/OD280 ratio measurement and/or elastic light scattering) even during storage under one ore more of the above stress conditions. In a specific aspect, the formulations of the present invention show only low to undetectable levels of aggregation and/or particulate formation (e.g. as assessed by SE-HPLC, subvisible particle counting, analytical ultracentrifugation, dynamic light scattering and/or OD320/OD280 measurement) at a temperature of 37±5°C and/or 5±5°C for up to at least 2 weeks (preferably at least 3 weeks, at least 5 weeks, at least 8 weeks, at least 10 weeks, at least 3 months, at least 6 months, at least 1 year, 1.5 year or even 2 years or more).

Apart from this and/or in addition, the stability of the formulations of the present invention can be demonstrated by the fact that it shows only low to undetectable levels of fragmentation and/or degradation of the polypeptides (e.g. as assessed by SDS-PAGE, SE-HPLC, RP-HPLC and/or IEX-HPLC) even during storage under one or more of the above stress conditions. In a specific aspect, the formulations of the present invention show only low to undetectable levels of fragmentation and/or degradation of the polypeptides (e.g. as assessed by SDS-PAGE, SE-HPLC, RP-HPLC and/or IEX-HPLC) at a temperature of 37±5°C for up to at least 2 weeks (preferably at least 3 weeks, at least 5 weeks, at least 8 weeks, at least 10 weeks, at least 3 months, at least 6 months, at least 1 year, 1.5 year or even 2 years or more).

Apart from this and/or in addition, the stability of the formulations of the present invention can be demonstrated by the fact that it shows very little to no loss of the biological activities of the polypeptide of the invention (e.g. as assessed by ELISA and/or Biacore) even during storage under one or more of the above stress conditions. In a specific aspect, the formulations of the present invention show very little to no loss of the biological activities of the polypeptide of the invention (e.g. as assessed by ELISA and/or Biacore) at a temperature of 37±5°C for up to at least 2 weeks (preferably at least 3 weeks, at least 5 weeks, at least 8 weeks, at least 10 weeks, at least 3 months, at least 6 months, at least 1 year, 1.5 year or even 2 years or more).

More specifically, in the formulations of the present invention at least 80% (preferably at least 90%, more preferably at least 95 % or even at least 99%) of the polypeptides retain their binding activity to at least one (preferably to all) of their targets (e.g. as assessed by ELISA and/or Biacore) after storage under one or more of the above stress conditions compared to the binding activity prior to storage.

In a specific aspect, at least 80% (preferably at least 90%, more preferably at least 95 % or even at least 99%) of the polypeptides retains their binding activity (e.g. as assessed by ELISA and/or Biacore) to at least one (preferably to all) of their targets after storage at 37±5°C for up to at least 2 weeks (preferably at least 3 weeks, at least 5 weeks, at least 2 months, at least 6 months, at least 1 year, 1.5 year or even 2 years or more) compared to the binding activity prior to storage.

Accordingly the present invention provides stable formulations of polypeptides comprising one or more single variable domains, wherein:
- less than 10% of the polypeptides forms pyroglutamate at the N-terminal glutamic acid (e.g. as assessed by RP-HPLC) during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- less than 10% of the polypeptides forms dimers (e.g. as assessed by SE-HPLC) during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- at least 80% of the polypeptides retain its binding activity (e.g. as assessed by ELISA and/or Biacore) to at least one (preferably to all) of its targets after storage at 37±5 °C up to 2 weeks (preferably at least 3 weeks, at least 5 weeks, at least 2 months, at least 6 months, at least 1 year, 1.5 year or even 2 years or more) compared to the binding activity prior to storage; and/or
- the polypeptide is stable under mechanical stress.

In a preferred aspect, the formulation of the invention is a pharmaceutical formulation.

The present invention further provides methods for preparing the stable formulations of the invention. The methods of the invention may comprise the steps of concentrating a polypeptide comprising one or more single variable domains and exchanging it with the preferred buffer and/or excipient.

Also provided are containers, kits and pharmaceutical unit dosages comprising the formulations of the invention for use by, e.g., a healthcare professional. In specific embodiments, the kits or pharmaceutical unit dosages comprising the stable formulations of the invention are formulated for parenteral administration (e.g., intradermally, intramuscularly, intraperitoneally, intravenously and/or subcutaneously) of the polypeptide of the invention to a human subject. The formulations, containers, pharmaceutical unit dosages and/or kits can be used in prophylaxis and/or therapy. In a specific aspect, the formulations, containers, pharmaceutical unit dosages and/or kits are used for the prevention and/or treatment of one ore more diseases and/or disorders such as bone diseases and/or disorders (such as e.g. osteoporosis, cancer-related bone diseases, and/or bone loss associated with autoimmunity and/or viral infection) or autoimmune diseases (such as e.g. rheumatoid arthritis).

### FIGURE LEGENDS

**Figure 1****.** The 280 nm SE-HPLC chromatograms of RANKL008a formulated in phosphate (A), or histidine (B) buffers with either 50 mM NaCl, 100 mM NaCl or 10% mannitol, before and after 10 freeze/thaw cycles. A zoom on the main peak is shown as inset.
**Figure 2****.** The 280 nm RP-HPLC chromatograms of RANKL008a formulated in phosphate buffers with either 50 mM NaCl, 100 mM NaCl or 10% mannitol, before and after 10 freeze/thaw cycles. A zoom on the main peak is shown as inset.
**Figure 3****.** The 280 nm SE-HPLC chromatograms of RANKL008a formulated in phosphate buffer with either 50 mM NaCl, 100 mM NaCl or 10% mannitol, after incubation for 2 weeks at 37°C. A zoom on the main peak is shown as inset.
**Figure 4****.** Figure demonstrating the time-dependent decrease (A) and increase (B) of the surface area of, respectively, the main peak (A) and % dimers (B) observed in SE-HPLC analysis of RANKL008a formulated in different buffers and stored up to 10 weeks at 37°C.
**Figure 5****.** The 280 nm RP-HPLC chromatograms of RANKL008a formulated in phosphate buffer with either 50 mM NaCl, 100 mM NaCl or 10% mannitol, after incubation for 2 weeks at 37°C. A zoom on the main peak is shown as inset.
**Figure 6****.** Overlay of the 280 nm IEX-HPLC chromatograms of RANKL008a formulated in phosphate buffer with either 50 mM NaCl, 100 mM NaCl or 10% mannitol, after incubation for 2 weeks at 37°C. A zoom on the main peak and postpeaks is shown as inset.
**Figure 7****.** Relative amounts (%) of the main peak and the two postpeaks observed in the IEX-HPLC chromatograms of the stability samples after storage for 10 weeks at 37°C.
**Figure 8****.** Picture and visual observation of the vials after shaking. The RANKL008a sample, diluted (to 5 mg/mL) and undiluted with or without 0.01% Tween 80 was shaken strongly (10s - 1min).
**Figure 9****.** Overview of dilutions and steps made in syringeabiiity study as described in Example 1.6.
**Figure 10****.** OD 320/278 and OD 350/278 ratios (n=3) after passage/storage of RANKL008a in syringes with different diluents as described in Example 1.6.
**Figure 11****.** Relative HSA and RANKL potency of RANKL008a after dilution in different diluents and passage/storage in syringes as described in Example 1.6.
**Figure 12****.** Overview of needle/gauge size study with diluted RANKL008a as described in Example 1.7.
**Figure 13****.** Overview of further needle/gauge size study with diluted and undiluted RANKL008a as described in Example 1.7.
**Figure 14****.** Overview of the results obtained for thermal stability testing of IL6R304 (A, C, E) and IL6R305 (B, D, F) and in function of NaCl concentration (A, B), mannitol added (C, D) and buffer/excipient (E, F).
**Figure 15****.** Overview of the results obtained in thermal stability testing of IL6R304 in Tris buffer pH 7.2 or Histidine pH 6.5, with sucrose, glycine or mannitol added as excipient.
**Figure 16****.** Thermograms (obtained after subtracting the base-lines) of IL6R304 in the buffers as indicated in the graph.
**Figure 17****.** PAMAS analysis of IL6R304 formulated in PBS compared to IL6R304 formulated in PBS + 0.01% or 0.02% Tween 80. Counts of particles: >10 µm, >25 µm, >50 µm, >100 µm and >200 µm.
**Figure 18****.** SE-HPLC for the IL6R304 molecule in the presence of different concentrations of Tween 80.
**Figure 19****.** (A)-(C): Log-values of the soluble IL-6R Nanobody concentration (Y-axis) vs. PEG6000 concentration (%) (X-axis), together with the calculated solubility values. (D) represents an example of how the linear regression analysis is performed on the obtained data points to determine the intercept with the X-axis (from which the theoretical solubility at zero % PEG6000 can be deduced).
**Figure 20****.** Overlay of the SE-HPLC chromatograms of IL6R304 formulated at 10 mg/mL stored for 3 weeks at 37°C. Inset, zoom on the main peak to demonstrate the buffer-dependent differences in % aggregates.
**Figure 21****.** Figure demonstrating the buffer-dependent differences in % aggregates (peak surface area in SE-HPLC) that were observed in the stability samples of IL6R304 and IL6R305 stored for 1 week at 37°C.
**Figure 22****.** Figure demonstrating the buffer-dependent differences in % aggregates (peak surface area in SE-HPLC) that were observed in the stability samples of IL6R304 and IL6R305 stored for 3 weeks at 37°C.
**Figure 23****.** Overlays of the RP-HPLC chromatograms from IL6R304 incubated for 3 weeks at 37°C in different formulation buffers. A zoom on the main peak is shown as inset. Note the increase of two minor prepeaks and a postpeak in the IL6R304 samples stored for 3 weeks.
**Figure 24****.** Osmolality data of IL6R304 (10 mg/mL) in the 12 different buffer indicated in the graph. The horizontal bar defines the isotonic region.
**Figure 25****.** Figure demonstrating the time-dependent increase of the % oligomers/aggregates (Y-axis) observed in SE-HPLC analysis of IL6R304 stored for up to 5 weeks at 37°C (A) and 5°C (B) in the buffers indicated in the graph. The % oligomers/aggregates is expressed as the sum of the % peak surface areas of prepeak1a, prepeak 1b and prepeak 2 relative to the total peak surface area.
**Figure 26****.** (A): the % oligomers (% peak surface area) observed in the Histidine buffers after storage for 3 weeks at 37°C compared to the equivalent sample in PBS buffer. (B): time-dependent and buffer-dependent increase in the % oligomers observed in the IL-6R stability samples stored for up to 5 weeks at 37°C, at a concentration of 10 mg/mL in the buffers indicated in the graph.

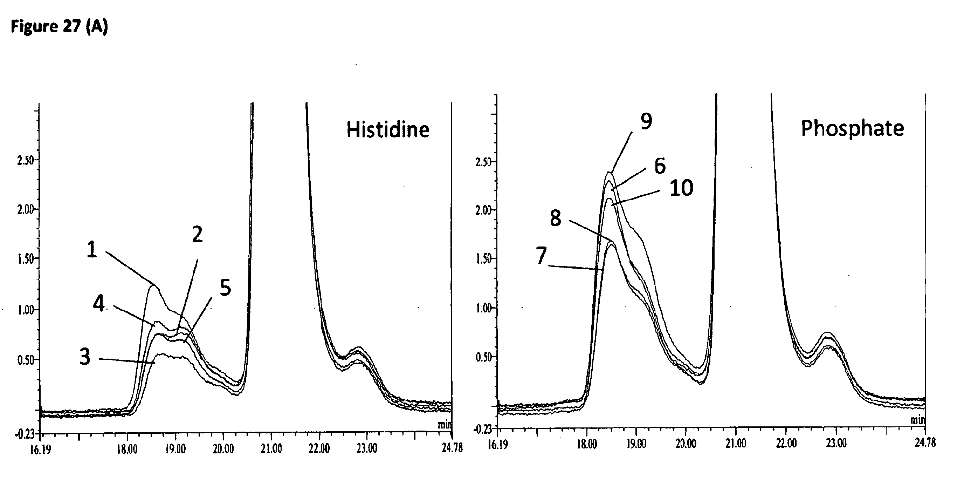

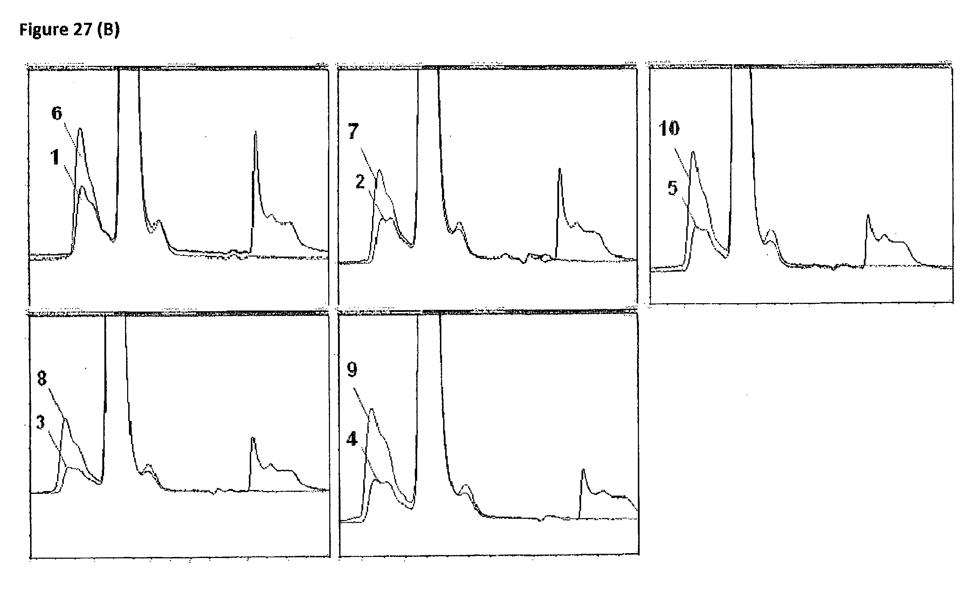

**Figure 27****.** SE-HPLC profile of IL6R304 in the different buffers as depicted in Table 21. IL6R304 was less prone to oligomerization in L-histidine buffer compared to phosphate buffer. (A) Less oligomers, which were seen as prepeaks during SE-HPLC analysis, were present in IL6R304 samples stored for 8 weeks at 37°C in L-histidine buffer (buffers 1-5) compared to phosphate buffer (buffers 6-10). The amount of oligomers was lowest in buffer 3. (B) The effect of the different excipients on oligomerization in either L-histidine versus phosphate. Note that the surface area of the postpeak was higher in phosphate compared to L-histidine.
**Figure 28****.** Kinetics of oligomer formation upon storage of IL6R304 in the different buffers. Oligomerization was significantly slower in L-histidine (buffers 1-5) compared to phosphate buffer (buffers 6-10).
**Figure 29****.** Overlay of the RP-HPLC chromatograms from IL6R304 after storage for up to 8 weeks at +37°C in 10 different formulation buffers. A zoom on the main peak and sidepeaks is shown as inset.
**Figure 30****.** Kinetics of the formation for the pyroglutamate variant of IL6R304 upon storage under stressed conditions in the different buffers. Less pyroglutamate is formed in L-histidine compared to phosphate buffer.
**Figure 31****.** Aggregation index before and after stirring of IL6R304.
**Figure 32****.** Graphical presentation of Tm values for 23IL0064 as a function of NaCl concentration (A) and of mannitol concentration (B). Tm's were obtained in the thermal shift assay at 0.1 mg/mL.
**Figure 33****.** Graphical representation of the melting temperatures measured for 231L0075 as a function of the pH, in 10 mM acetate, in 10 mM histidine, and in 10 mM phosphate buffer. The protein concentration was 0.2 mg/mL. Scanning was performed at 1°C/min, starting at 30°C.
**Figure 34****.** SE-HPLC chromatograms (OD280) of 23IL0064 in 40 mM histidine (pH 6.0) / 50 mM NaCl. The start sample was compared to samples after concentration. No aggregate formation due to concentration of the sample in the histidine buffer was observed. The SE-HPLC was run with a Phenomenex BioSep SEC S-2000 column, with D-PBS as mobile phase at 0.2 mL/min.
**Figure 35****.** Log-values of the soluble protein concentration (Y-axis) vs. PEG6000 concentration (%, X-axis). The right upper panel represents an example of how the linear regression analysis is performed on the obtained data points to determine the intercept with the X-axis (from which the theoretical solubility at zero % PEG6000 can be deduced). In the histidine buffer, the experiment was performed at two concentrations which are presented in the two bottom graphs. For these two graphs no regression was possible.
**Figure 36****.** Log-values of the soluble protein concentration (Y-axis) of p23IL0064 and 231L0075 in 10 mM phosphate buffer pH 7 with 50mM NaCl vs. PEG6000 concentration (X-axis). By regression analysis and extrapolation to a zero concentration of PEG6000, the theoretical maximum protein concentrations (apparent solubility values) were calculated and were for both proteins approximately 50 mg/mL. This number should only be used after confirmation with other techniques.
**Figure 37****.** Log-values of the soluble protein concentration (Y-axis) of p23IL0064 and p23IL0075 in 40 mM histidine pH 6.0 buffer with 50 mM NaCl vs. PEG6000 concentration (X-axis). In this graph regression was not possible since no precipitation occurred in the PEG% window explored.
**Figure 38****.** SE-HPLC chromatograms (280 nm) of 25 µg 23IL0064 non-stressed and 37°C (3w and 4w) -stressed samples in D-PBS. SE-HPLC was run on TSK-GEL G2000SWXL with D-PBS.
**Figure 39****.** RP-HPLC chromatograms (280 nm) of 25 µg 23IL0064 non-stressed and 37°C (4w) - stressed sample in D-PBS. RP-HPLC was run on Zorbax C-3 column with a water/acetonitrile 0.1%TFA gradient.
**Figure 40****.** Graphical representation of the % peak area of the different RP-HPLC peaks of 23IL0064 and product related substances after 6 weeks stress at 37°C in different formulation buffers. The first bar represents the reference sample, stored at -80°C until analysis. The pre-peak 2 was present in the start sample and did not increase during the storage at 37°C.
**Figure 41****.** Overlay of RP-HPLC chromatograms (280 nm) of 23IL0064 in 20mM Histidine pH 6.5 (conc. 22.4 mg/mL). Comparison of 6 weeks 37°C, 6 weeks 25°C, 6 weeks 4°C, and -80°C reference. RP-HPLC was run on Zorbax 300SB-C3 column with a water/acetonitrile 0.1%TFA gradient 0.3%/min) at 75°C.
**Figure 42****.** SDS-PAGE of stability samples of 23IL0064 in 20mM Histidine pH 6.5 CONC stressed for 6 weeks at 4°C, 25°C, 37°C and its -80°C Reference.
**Figure 43****.** Elastic light scattering as measured at 500nm as a function of the temperature for 23IL0075 samples with different concentrations (as indicated in the legend).
**Figure 44****.** Elastic light scattering as measured at 500nm as a function of the temperature for 250µg/mL 23IL0075 in 10mM phosphate pH 6.0. The temperature of the onset of aggregate formation is determined by means of linear fit.
**Figure 45****.** Elastic light scattering as measured at 500nm as a function of the temperature for 250µg/mL 23IL0075 in 10mM Acetate pH 6.0. The temperature of the onset of aggregate formation is determined by means of linear fit.
**Figure 46****.** Elastic light scattering as measured at 500nm as a function of the temperature for 250µg/mL 231L0075 in 10mM Histidine pH 6.0. The temperature of the onset of aggregate formation is determined by means of linear fit.
**Figure 47****.** Graphical representation of the opalescence (measured by OD500) (A) and the % oligomers (% pre-peak) detected in SE-HPLC (B) after freeze/thaw stress. The percentage of oligomers in the reference sample was approximately 0.4%. In this study, an acetate, a histidine and
   a phosphate buffer were compared, in combination with mannitol or a mixture of mannitol and glycine as excipients, and Tween 80 or poloxamer in two different concentrations as surfactants.
**Figure 48****.** Graphical representation of the opalescence (measured by OD500) (A) and the % oligomers detected in SE-HPLC (B) after shear stress. The percentage of oligomers in the reference sample was approximately 0.4%. In this study, an acetate, a histidine and a phosphate buffer were compared, in combination with mannitol or a mixture of mannitol and glycine as excipients, and Tween 80 or poloxamer in two different concentrations as surfactants.
**Figure 49****.** Graphical representation of the opalescence (measured by OD500) (A), and the % oligomers detected in SE-HPLC (B) in different histidine buffers after freeze/thaw stress. The percentage of oligomers in the reference sample was approximately 0.4%. *For one sample the condition without detergent was included.
**Figure 50****.** Graphical representation of the opalescence (measured by OD500) (A), the % oligomers detected in SE-HPLC (B), and the % activity (albumin binding) of the Nanobody measured on Biacore (C) after shear stress. The percentage of oligomers in the reference sample was approximately 0.4%. In this study no detergents were included, to mimic the situation during the final concentration step of the DSP process.
**Figure 51****.** SE-HPLC chromatograms (280nm) of 231L0075 at 25mg/mL in 10mM Histidine pH 6.0, 10% sucrose, 0.005% Tween 80: the reference sample compared to 6 weeks storage at 25°C and 37°C.

### DETAILED DESCRIPTION

Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd.Ed.), Vols. 1-3, Cold Spring Harbor Laboratory Press (1989); F. Ausubel et al, eds., "Current protocols in molecular biology", Green Publishing and Wiley Interscience, New York (1987); Lewin, "Genes II", John Wiley & Sons, New York, N.Y., (1985); Old et al., "Principles of Gene Manipulation: An Introduction to Genetic Engineering", 2nd edition, University of California Press, Berkeley, CA (1981); Roitt et al., "Immunology" (6th. Ed.), Mosby/Elsevier, Edinburgh (2001); Roitt et al., Roitt's Essential Immunology, 10th Ed. Blackwell Publishing, UK (2001); and Janeway et al., "Immunobiology" (6th Ed.), Garland Science Publishing/Churchill Livingstone, New York (2005), as well as to the general background art cited herein.

As used herein, the term "isolated" in the context of a polypeptide refers to a polypeptide which is substantially free of cellular material or contaminating proteins from the cell or tissue source from which it is derived, or substantially free of chemical precursors or other chemicals when chemically synthesized. The language "substantially free of cellular material" includes preparations of a polypeptide in which the polypeptide is separated from cellular components of the cells from which it is isolated or recombinantly produced. Thus, a polypeptide that is substantially free of cellular material includes preparations of a polypeptide having less than about 30%, 20%, 10%, or 5% (by dry weight) of heterologous protein, polypeptide, peptide, or antibody (also referred to as a "contaminating protein"). When the polypeptide is recombinantly produced, it may also be substantially free of culture medium, i.e., culture medium represents less than about 20%, 10%, or 5% of the volume of the polypeptide preparation. When the polypeptide is produced by chemical synthesis, it is preferably substantially free of chemical precursors or other chemicals, i.e., it is separated from chemical precursors or other chemicals which are involved in the synthesis of the polypeptide. Accordingly, such preparations of a polypeptide have less than about 30%, 20%, 10%, 5% (by dry weight) of chemical precursors or compounds other than the polypeptide of interest. In a specific embodiment, an "isolated" polypeptide is purified by a multi-step purification process that comprises two chromatography steps (e.g. cation exchange and anion exchange), a 100K ultrafiltration step, followed by a buffer exchange and concentration step in

### Ultrafiltration/Diafiltration mode.

As used herein, the terms "subject" and "patient" are used interchangeably. As used herein, the terms "subject" and "subjects" referto an animal, preferably a mammal including a non-primate (e.g., a cow, pig, horse, cat, dog, rat, and mouse) and a primate (e.g., a monkey, such as a cynomolgus monkey, chimpanzee, baboon and a human), and more preferably a human. In a certain embodiment, the subject is a mammal, preferably a human, with one or more diseases or disorders. In another embodiment, the subject is a mammal, preferably a human, at risk of developing one or more diseases and/or disorders.

The terms "stability" and "stable" as used herein in the context of a formulation comprising a polypeptide comprising one or more single variable domains refer to the resistance of the polypeptide in the formulation to aggregation, to the formation of degradation products and/or to the formation of fragmentation products under given transportation and/or storage conditions. Apart from this and/or in addition, the "stable" formulations of the invention retain biological activity under given transportation and/or storage conditions. The stability of said polypeptide can be assessed by degrees of aggregation, degradation and/or fragmentation (as measured e.g. by SE-HPLC, RP-HPLC, IEX-HPLC, subvisible particle counting, analytical ultracentrifugation, dynamic light scattering, OD320/OD280 ratio measurement, elastic light scattering, etc.), and/or by % of biological activity (as measured e.g. by ELISA, Biacore, etc.) compared to a reference formulation. For example, a reference formulation may be a reference standard frozen at -20°C or below -65°C (such as e.g. - 80°C) consisting of the same polypeptide at the same concentration in D-PBS or consisting of the same polypeptide at the same concentration and in the same buffer as the stressed samples but without applying the stress conditions, which reference formulation regularly gives a single peak by SE-HPLC, RP-HPLC and/or IEX-HPLC and/or keeps its biological activity in Biacore and/or ELISA.

"Solubility" is often described as the maximum achievable protein concentration whereby all of the protein remains in solution. At this concentration the protein should still be monomeric and free of aggregates. For determining protein solubility only a limited number of (mostly empirical) techniques are currently available. A first and popular technique consists of concentrating the sample by using centrifugal ultrafiltration up to the point where an opalescent solution is formed. Subsequently, the insoluble fraction is removed and the protein content of the supernatant is measured. Centrifugal concentrating devices such as for example Vivaspin concentrators with a molecular weight cut-off of 5 kDa can be used but require reasonable amounts of protein. Solubility can also be monitored using an inert macromolecule such as polyethylene glycol (PEG; Mr > 6,000), which precipitates proteins primarily through an excluded volume effect, a process that can be generally understood in terms of a simple colloidal phase separation. A logarithmic linear relationship between protein solubility and weight percent polyethylene glycol can be obtained, and from this plot the intercept yields the solubility value. The term "good solubility" of the polypeptide of the invention, as used herein, means that no or little precipitation is observed of the polypeptide of the invention during downstream processing (DSP) and/or during storage for a short or longer time at 5° or -20°C at concentrations ranging from 20-200 mg/mL or more. The formation of precipitates (oligomers or other particulates) can be measured e.g. by SE-HPLC, OD320/OD280 ratio measurement and/or elastic light scattering. Preferably, the polypeptides present in the formulations of the present invention have a solubility of at least 20 mg/mL, at least 30 mg/mL, at least 40 mg/mL, at least 50 mg/mL, at least 60 mg/mL, at least 65 mg/mL, at least 70 mg/mL, at least 80 mg/mL, at least 90 mg/mL, at least 100 mg/mL, at least 110 mg/mL, at least 120 mg/mL, at least 130 mg/mL, at least 140 mg/mL, at least 150 mg/mL, at least 200 mg/mL or even more. Preferably, the OD320/OD280 ratio of the formulations of the present invention is 0.05 or lower, such as 0.01 or lower or 0.005 or lower. The scattering in the formulation of the present invention should be within detection limit and preferably lower than 1000 abs, such as 750 abs or lower or 500 abs or lower.

The phrase "low to undetectable levels of aggregation" as used herein refers to samples containing no more than 5%, no more than 4%, no more than 3%, no more than 2%, no more than 1% or no more than 0.5% aggregation by weight of protein. Unless explicitly referred to differently, aggregation as used in the present invention means the development of high molecular weight aggregates, i.e. aggregates with an apparent molecular weight of more/higher than the apparent molecular weight observed in SE-HPLC analysis for dimers of the polypeptide of the invention (such as e.g. 44kDa as observed for SEQ ID NO: 4; 36-38 kDa as observed for SEQ ID NO's 1-3; and 36kDa as observed for SEQ ID NO: 5 in SE-HPLC) in comparison with molecular weight markers. Aggregation can be assessed by various methods known in the art. Without being limiting, examples include high performance size exclusion chromatography (SE-HPLC), subvisible particle counting, analytical ultracentrifugation (AUC), dynamic light scattering (DLS), static light scattering (SLS), elastic light scattering, OD320/OD280 measurement, Fourier Transform infrared Spectroscopy (FTIR), circular dichroism (CD), urea-induced protein unfolding techniques, intrinsic tryptophan fluorescence and/or differential scanning calorimetry techniques.

The term "low to undetectable levels of fragmentation and/or degradation" as used herein refers to samples containing equal to or more than 80%, 85%, 90%, 95%, 98% or 99% of the total protein, for example, in a single peak as determined by SE-HPLC, RP-HPLC and/or IEX-HPLC, representing the non-degraded polypeptide, and containing no other single peaks having more than 5%, more than 4%, more than 3%, more than 2%, more than 1%, or more than 0.5% of the total protein in each.

The term "very little to no loss of the biological activities" as used herein refers to single variable domain activities, including but not limited to, specific binding abilities of the single variable domain to the target of interest as measured by various immunological assays, including, but not limited to ELISAs and/or by Surface Plasmon Resonance (Biacore). In one embodiment, the single variable domains of the formulations of the invention retain at least 50%, preferably at least 55%, 60%, 65%, 70%, 75%, 80%, 85%, 90%, 95%, 98% or even 99% or more of the ability to specifically bind to an antigen as compared to a reference formulation, as measured by an immunological assay known to one of skill in the art or described herein. For example, an ELISA based assay (e.g. as described in the Example section) may be used to compare the ability of the single variable domain to specifically bind to its target. A "reference formulation" as used herein refers to a formulation that is frozen at a temperature of -20±5°C or at below -64°C (such as e.g. at -80°C) consisting of the same single variable domain at the same concentration in D-PBS or consisting of the same single variable domains at the same concentration in the same buffer/excipients as the stressed samples but without applying the stress conditions, which reference formulation regularly gives a single peak by SE-HPLC, RP-HPLC and/or IEX-HPLC and/or keeps its biological activity in Biacore and/or ELISA.

The phrase "pharmaceutically acceptable" as used herein means approved by a regulatory agency of the Federal or a state government, or listed in the U.S. Pharmacopeia, European Pharmacopoeia or other generally recognized pharmacopeia for use in animals, and more particularly in humans. In this sense, it should be compatible with the other ingredients of the formulation and not eliciting an unacceptable deleterious effect in the subject.

According to the European Pharmacopoeia, a solution is considered isotonic if it has an osmolality of 290 ± 30 mOsm/kg. Osmolality measurements were therefore performed on the different formulations used in the stability studies. Isotonicity can be measured by, for example, a vapor pressure or ice-freezing type osmometer.

As used herein, the term "effective amount" refers to the amount of an agent (e.g. a prophylactic or therapeutic agent) which is sufficient to reduce and/or ameliorate the severity and/or duration of one or more diseases and/or disorders.

The term "polyol" as used herein refers to sugars that contains many hydroxyl (-OH) groups compared to a normal saccharide. Polyols include alcohols and carbohydrates such as mannitol, sorbitol, maltitol, xylitol, isomalt, erythritol, lactitol, sucrose, glucose, galactose, fructose, fucose, ribose, lactose, maltose and cellubiose.

As used herein, the terms "therapeutic agent" and "therapeutic agents" refer to any agent(s) which can be used in the prevention, treatment and/or management of one ore more diseases and/or disorders. In the context of the present invention, the term "therapeutic agent" refers to a polypeptide comprising one or more single variable domains. In certain other embodiments, the term "therapeutic agent" refers to an agent other than the polypeptide of the invention which might be used in the formulation.

As used herein, the term "therapeutically effective amount" refers to the amount of a therapeutic agent (e.g. a polypeptide comprising one or more single variable domains), that is sufficient to reduce the severity of one or more diseases and/or disorders.

The term "excipient" as used herein refers to an inert substance which is commonly used as a diluent, vehicle, preservative, binder or stabilizing agent for drugs which imparts a beneficial physical property to a formulation, such as increased protein stability, increased protein solubility, and/or decreased viscosity. Examples of excipients include, but are not limited to, proteins (e.g., serum albumin), amino acids (e.g., aspartic acid, glutamic acid, lysine, arginine, glycine), surfactants (e.g., SDS, Tween 20, Tween 80, poloxamers, polysorbate and nonionic surfactants), saccharides (e.g., glucose, sucrose, maltose and trehalose), polyols (e.g., mannitol and sorbitol), fatty acids and phospholipids (e.g., alkyl sulfonates and caprylate). For additional information regarding excipients, see Remington's Pharmaceutical Sciences (by Joseph P. Remington, 18th ed., Mack Publishing Co., Easton, PA), which is incorporated herein in its entirety.

The term "variable domain" or "immunoglobulin variable domain" refers to the part or domain of an immunoglobulin molecule or antibody which is partially or fully responsible for antigen binding. The term "single variable domain" or "immunoglobulin single variable domain" (both terms are used interchangeably), defines molecules wherein the antigen binding site is present on, and formed by, a single immunoglobulin domain. This sets single variable domains apart from "conventional" immunoglobulins or their fragments, wherein two immunoglobulin domains, in particular two "variable domains" interact to form an antigen binding site. Typically, in conventional immunoglobulins, a heavy chain variable domain (VH) and a light chain variable domain (VL) interact to form an antigen binding site. In this case, the complementarity determining regions (CDRs) of both VH and VL will contribute to the antigen binding site, i.e. a total of 6 CDRs will be involved in antigen binding site formation.

In contrast, the binding site of a single variable domain is formed by a single VH or VL domain. Hence, the antigen binding site of a single variable domain is formed by no more than three CDRs. The term "single variable domain" does comprise fragments of conventional immunoglobulins wherein the antigen binding site is formed by a single variable domain.

Generally, single variable domains will be amino acid sequences that essentially consist of 4 framework regions (FR1 to FR4 respectively) and 3 complementarity determining regions (CDR1 to CDR3 respectively); or any suitable fragment of such an amino acid sequence (which will then usually contain at least some of the amino acid residues that form at least one of the CDR's). Such single variable domains and fragments are most preferably such that they comprise an immunoglobulin fold or are capable for forming, under suitable conditions, an immunoglobulin fold. As such, the single variable domain may for example comprise a light chain variable domain sequence (e.g. a V_{L} sequence) or a suitable fragment thereof; or a heavy chain variable domain sequence (e.g. a V_{H} sequence or V_{HH} sequence) or a suitable fragment thereof; as long as it is capable of forming a single antigen binding unit (i.e. a functional antigen binding unit that essentially consists of the single variable domain, such that the single antigen binding domain does not need to interact with another variable domain to form a functional antigen binding unit, as is for example the case for the variable domains that are present in for example conventional antibodies and scFv fragments that need to interact with another variable domain - e.g. through a V_{H}/V_{L} interaction - to form a functional antigen binding domain).

In one aspect of the invention, the single variable domains are light chain variable domain sequences (e.g. a V_{L} sequence), or heavy chain variable domain sequences (e.g. a V_{H} sequence); more specifically, the single variable domains can be heavy chain variable domain sequences that are derived from a conventional four-chain antibody or heavy chain variable domain sequences that are derived from a heavy chain antibody.

The single variable domain may be a domain antibody (or an amino acid sequence that is suitable for use as a domain antibody), a single domain antibody (or an amino acid sequence that is suitable for use as a single domain antibody), a "dAb" (or an amino acid sequence that is suitable for use as a dAb) or a Nanobody^{®} (as defined herein, and including but not limited to a V_{HH} sequence) *[Note: Nanobody^{®} and Nanobodies^{®} are registered trademarks of Ablynx N.V.];* other single variable domains, or any suitable fragment of any one thereof. For a general description of (single) domain antibodies, reference is also made to the prior art cited herein, as well as to EP 0 368 684. For the term "dAb's", reference is for example made to Ward et al. 1989 (Nature 341 (6242): 544-546), to Holt et al. 2003 (Trends Biotechnol. 21(11): 484-490); as well as to for example WO 04/068820, WO 06/030220, WO 06/003388 and other published patent applications of Domantis Ltd. It should also be noted that, although less preferred in the context of the present invention because they are not of mammalian origin, single variable domains can be derived from certain species of shark (for example, the so-called "IgNAR domains", see for example WO 05/18629).

In particular, the polypeptides of the invention may comprise one or more Nanobodies or a suitable fragment thereof. For a further description of V_{HH}'s and Nanobodies, reference is made to the review article by Muyldermans 2001 (Reviews in Molecular Biotechnology 74: 277-302); as well as to the following patent applications, which are mentioned as general background art: WO 94/04678, WO 95/04079 and WO 96/34103 of the Vrije Universiteit Brussel; WO 94/25591, WO 99/37681, WO 00/40968, WO 00/43507, WO 00/65057, WO 01/40310, WO 01/44301, EP 1134 231 and WO 02/48193 of Unilever; WO 97/49805, WO 01/21817, WO 03/035694, WO 03/054016 and WO 03/055527 of the Vlaams instituut voor Biotechnologie (VIB); WO 03/050531 of Algonomics N.V. and Ablynx N.V.; WO 01/90190 by the National Research Council of Canada; WO 03/025020 (= EP 1 433 793) by the Institute of Antibodies; as well as WO 04/041867, WO 04/041862, WO 04/041865, WO 04/041863, WO 04/062551, WO 05/044858, WO 06/40153, WO 06/079372, WO 06/122786, WO 06/122787 and WO 06/122825, by Ablynx N.V. and the further published patent applications by Ablynx N.V. Reference is also made to the further prior art mentioned in these applications, and in particular to the list of references mentioned on pages 41-43 of the International application WO 06/040153, which list and references are incorporated herein by reference. As described in these references, Nanobodies (in particular V_{HH} sequences and partially humanized Nanobodies) can in particular be characterized by the presence of one or more *"Hallmark residues"* in one or more of the framework sequences. A further description of the Nanobodies, including humanization and/or camelization of Nanobodies, as well as other modifications, parts or fragments, derivatives or "Nanobody fusions", multivalent constructs (including some non-limiting examples of linker sequences) and different modifications to increase the half-life of the Nanobodies and their preparations can be found e.g. in WO 08/101985 and WO 08/142164.

The total number of amino acid residues in a Nanobody can be in the region of 110-120, is preferably 112-115, and is most preferably 113. It should however be noted that parts, fragments, analogs or derivatives (as further described herein) of a Nanobody are not particularly limited as to their length and/or size, as long as such parts, fragments, analogs or derivatives meet the further requirements outlined herein and are also preferably suitable for the purposes described herein.

Thus, in the meaning of the present invention, the term "single variable domain" comprises polypeptides which are derived from a non-human source, preferably a camelid, preferably a camelid heavy chain antibody. They may be humanized, as previously described. Moreover, the term comprises polypeptides derived from non-camelid sources, e.g. mouse or human, which have been "camelized", as previously described.

The term "single variable domain" also encompasses variable domains of different origin, comprising mouse, rat, rabbit, donkey, human and camelid variable domains; as well as fully human, humanized or chimeric variable domains. For example, the invention comprises camelid variable domains and humanized camelid variable domains, or camelized variable domains, e.g. camelized dAb as described by Ward et al (see for example WO 94/04678 and Davies and Riechmann (1994, FEBS Lett. 339(3): 285-290) and (1996, Protein Eng.9(6): 531-537)). Moreover, the invention comprises fused variable domains, e.g. multivalent and/ or multispecific constructs (for multivalent and multispecific polypeptides containing one or more V_{HH} domains and their preparation, reference is also made to Conrath et al. 2001 (J. Biol. Chem. 276: 7346-7350) as well as to for example WO 96/34103 and WO 99/23221).

Unless indicated otherwise, the term "immunoglobulin sequence" -whether used herein to refer to a heavy chain antibody or to a conventional 4-chain antibody - is used as a general term to include both the full-size antibody, the individual chains thereof, as well as all parts, domains or fragments thereof (including but not limited to antigen-binding domains or fragments such as V_{HH} domains or V_{H}/V_{L} domains, respectively). The terms antigen-binding molecules or antigen-binding protein are used interchangeably with immunoglobulin sequence, and include Nanobodies.

The single variable domains provided by the invention are preferably in essentially isolated form (as defined herein), or form part of a polypeptide of the invention (as defined herein), which may comprise or essentially consist of one or more single variable domains and which may optionally further comprise one or more further amino acid sequences (all optionally linked via one or more suitable linkers). For example, and without limitation, the one or more single variable domains may be used as a binding unit in such a polypeptide, which may optionally contain one or more further amino acid sequences that can serve as a binding unit (i.e. against one or more other targets), so as to provide a monovalent, multivalent or multispecific polypeptide of the invention, respectively as e.g. described in WO 08/101985, WO 08/142164, WO 09/068625, WO 09/068627 and WO 08/020079. Such a protein or polypeptide may also be in essentially isolated form (as defined herein) and the methods of the present invention for the expression and/or production of single variable domains equally apply to polypeptides comprising one or more single variable domains.

According to the invention, the term "single variable domain" may comprise constructs comprising two or more antigen binding units in the form of single variable domain, as outlined above. For example, two (or more) variable domains with the same or different antigen specificity can be linked to form e.g. a bivalent, trivalent or multivalent construct. By combining variable domains of two or more specificities, bispecific, trispecific etc. constructs can be formed. For example, a variable domain according to the invention may comprise two variable domains directed against target A, and one variable domain against target B. Such constructs and modifications thereof, which the skilled person can readily envisage, are all encompassed by the term variable domain as used herein and are also referred to as *"polypeptide of the invention"* or *"polypeptides of the invention".*

As further described in paragraph m) on page 53 of WO 08/020079, an amino acid sequence (such as a Nanobody, an antibody, a polypeptide of the invention, or generally an antigen binding protein or polypeptide or a fragment thereof) that can (specifically) bind to, that has affinity for and/or that has specificity for a specific antigenic determinant, epitope, antigen or protein (or for at least one part, fragment or epitope thereof) is said to be *"against"* or *"directed against"* said antigenic determinant, epitope, antigen or protein.

The polypeptide comprising one or more single variable domains for use in the formulation of the invention may be therapeutic or prophylactic, and may be useful in the treatment and/or management of one or more diseases. In one specific aspect, the polypeptide has at least two single variable domains. In another specific aspect, the polypeptide has at least three single variable domains. Preferably, the polypeptide comprises at least one single variable domain directed against HSA. In another specific aspect, the polypeptide comprises at least a single variable domain against RANKL. In another specific aspect, the polypeptide comprises at least a single variable domain against IL-6R. In another specific aspect, the polypeptide comprises at least a single variable domain against IL-23. More preferably, the polypeptide is directed against and/or specifically binds RANKL and HSA, IL-6R and HSA and/or IL-23 and HSA. In yet another aspect, polypeptide comprises at least a single variable domain against RANKL and at least a single variable domain against HSA. In yet another aspect, polypeptide comprises at least a single variable domain against IL-6R and at least a single variable domain against HSA. In yet another aspect, polypeptide comprises at least a single variable domain against IL-23 and at least a single variable domain against HSA. In yet another aspect, polypeptide comprises at least two single variable domains against RANKL and at least a single variable domain against HSA. In yet another aspect, polypeptide comprises at least two single variable domains against IL-6R and at least a single variable domain against HSA. In yet another aspect, polypeptide comprises at least two single variable domains against IL-23 and at least a single variable domain against HSA. Polypeptides of the invention are selected from SEQ ID NO's: 1 to 6.

The concentration of polypeptide of the invention present in the formulation is from 1 to 200 mg/mL such as about 1 mg/mL, about 2 mg/mL, about 5 mg/mL, about 10 mg/mL, about 15 mg/mL or about 20 mg/mL, about 30 mg/mL, about 40 mg/mL, about 50 mg/mL, about 60 mg/mL, about 65 mg/mL, about 70 mg/mL, about 80 mg/mL, about 90 mg/mL or about 100 mg/mL or more. In certain embodiments, the concentration of polypeptide of the invention can be 110 mg/mL or more, 120 mg/mL or more, 130 mg/mL or more, 140 mg/mL or more, 150 mg/mL or more or even 200 mg/mL or more. In a specific aspect, a formulation of the invention comprises about 10 mg/mL of polypeptide of the invention.

The formulation of the invention comprises an aqueous carrier having a pH of 5.5 to 8.0 and a polypeptide as defined above (*"polypeptide of the invention."*) comprising one or more single variable domains at a concentration of 1 mg/mL to 200 mg/mL, said formulation being formulated for administration to a human subject, wherein said formulation further comprises:
a) A buffer at a concentration of 10mM to 100mM selected from histidine pH 6.0-6.5 ; and
wherein said formulation has an inorganic salt concentration of 150 mM or lower.

The stable formulations of the present invention comprise polypeptides of the invention that have a good solubility and a high stability even during transportation and/or long periods of storage and that exhibit little to no aggregation. In addition to the polypeptide of the invention, the formulations of the present invention comprise at least an aqueous carrier and a buffer. The carrier used in the formulation of the invention should be a liquid carrier. Preferably the carrier is an aqueous carrier such as e.g. distilled water, MilliQ water or Water for Injection (WFI).

The formulation should not contain inorganic salt at a concentration of more than 150 mM. Without being limiting, inorganic salts for use in the formulation of the invention can be selected from NaCl and KCl. Accordingly the formulation of the invention has an inorganic salt concentration of 150 mM or lower, preferably 120 mM or lower, or 100 mM or lower, more preferably 90 mM or lower, 80 mM or lower, 75 mM or lower, such as 50 mM or lower or even 40 mM or lower, 25 mM or lower, 10 mM or lower or 5 mM or lower. Most preferably, the formulation does not contain any inorganic salt.

The pH of the formulation of the invention generally should not be equal to the isoelectric point of the particular polypeptide of the invention present in the formulation and may range from 5.5 to about 8.0, or from about 6.0 to about 7.5, preferably from about 6.2 to 7.5, from about 6.5 to 7.5, most preferably from about 6.5 to 7.0. In a specific aspect, the formulation of the invention has a pH of about 6.5. In another specific aspect, the formulation of the invention has a pH of about 7.0. In yet another specific aspect, the formulation of the invention has a pH of about 6.0.

The formulation is buffered by a buffer selected from the group consisting of histidine pH 6.0-6.5.

The concentration of the buffer present in the formulation of the invention may range from 10 mM to 100 mM, 10 mM to 50 mM, 10 mM to 25 mM, 10 mM to 20 mM. In a specific aspect, the concentration of buffer in the formulations of the invention is 10 mM, 15 mM, 20 mM, 25 mM, 50 mM, 75 mM, or 100 mM. Preferably, the concentration is between 10 and 20 mM, such as 10 mM or 15 mM.

Any form of histidine suitable for formulation and parenteral administration may be used in the formulation of the invention. The purity of histidine should be at least 98%, at least 99%, or at least 99.5%. In a specific aspect, a formulation of the invention comprises 15 mM histidine buffer pH 6.5. In another specific aspect, a formulation of the invention comprises 10 mM histidine buffer pH 6.0.

In addition to histidine, hepes buffers pH7.0 may be used in the formulations of the present invention. Any form of hepes suitable for formulation and parenteral administration may be used in the formulation of the invention. The purity of hepes should be at least 98%, at least 99%, or at least 99.5%. In a specific aspect, a formulation of the invention comprises 15 mM hepes buffer pH7.0.

It will be understood by one skilled in the art that the formulation of the invention may be isotonic or slightly hypotonic with human blood, i.e. the formulation of the invention has essentially the same or a slightly lower osmotic pressure as human blood. Such isotonic or slightly hypotonic formulation generally has an osmotic pressure from about 240 mOSm/kg to about 320 mOSm/kg, such as about 240 mOSm/kg or higher, 250 mOSm/kg or higher or 260 mOSm/kg or higher.

Tonicity of a formulation is adjusted by the use of tonicity modifiers. "Tonicity modifiers" are those pharmaceutically acceptable inert substances that can be added to the formulation to provide an isotonicity of the formulation. A preferred tonicity modifier in the formulation of the invention are excipients. Preferred excipients for use in the formulation of the invention may be selected from sugars, polyols and surfactants.

Accordingly, in another aspect, the formulation of the invention comprises an excipient. Preferred excipients include polyols and/or sugars. The polyol and/or sugar may be a monosaccharide such as glucose or mannose, or a polysaccharide including disaccharides such as (without being limiting) sucrose and lactose, as well as sugar derivatives including sugar alcohols and sugar acids. Polyols and sugar alcohols include (without being limiting) mannitol, xylitol, erythritol, threitol, sorbitol and glycerol. A non-limiting example of a sugar acid is L-gluconate. Other exemplary sugars include (without being iimiting) trehalose, glycine, maltose, raffinose, etc. The concentration of the excipient may range from about 1% to 10% (w:v), preferably from about 2.5% to 10% (w:v), more preferably from about 5% to 10% (w:v), such as e.g. 5% (w:v), 7.5% (w:v), 8% or 10% (w:v). Throughout the present invention the concentration of the excipient will be given as % (w:v). In a preferred aspect, the formulation comprises sucrose, preferably at a concentration of about 5% to 10% (w:v), such as about 8% (w:v).

In another aspect, the formulation of the invention comprises a surfactant. A surfactant refers to a surface-active agent comprising a hydrophobic portion and a hydrophilic portion. In a preferred aspect, the surfactant is non-ionic. Certain exemplary non-ionic surfactants include (without being limiting) PEG8000, and polysorbate, including without being limiting, polysorbate 80 (Tween 80) and polysorbate 20 (Tween 20), Triton X-100, polyoxypropylene-polyoxyethylene esters (Pluronic^{®}), and NP-40. In a specific aspect, the surfactant is selected from Tween 20, Tween 80 or a poloxamer. The concentration of the surfactant may range from about 0.001% to 1% (v:v) (preferably from about 0.001% to 0.1% (v:v), or 0.01% to 0.1% (v:v) such as 0.001% (v:v), 0.005% (v:v), 0.01% (v:v), 0.02% (v:v), 0.05% (v:v), 0.08% (v:v), 0.1% (v:v), 0.5% (v:v), or 1% (v:v) of the formulation, preferably 0.01% (v:v)). Throughout the present invention the concentration of the surfactant will be given as % (v:v). In a specific embodiment, the surfactant is Tween 20 or Tween 80, which is at a concentration of 0.001% (v:v), 0.005% (v:v), 0.01% (v:v), 0.02% (v:v), 0.05% (v:v), 0.08% (v:v), 0.1% (v:v), 0.5% (v:v) or 1% (v:v) of the formulation, preferably 0.01% (v:v).

In a preferred aspect, the formulation of the present invention comprises an aqueous carrier having a pH of 5.5 to 8.0 and a polypeptide as defined above (*"polypeptide of the invention"*) comprising one or more single variable domains at a concentration of 1 mg/mL to 200 mg/mL, said formulation being formulated for administration to a human subject and said formulation further comprises at least two components selected from:
a) A buffer at a concentration of 10mM to 100mM selected from the group consisting of histidine pH 6.0-6.5, hepes pH 7.0-8.0, MES pH 6.0, succinate pH 6.0-6.5 and acetate pH 5.5-6.0;
b) An excipient at a concentration of 1% to 20%;
c) A surfactant at a concentration of 0.001% to 1% selected from Tween 80, Tween 20 or poloxamers;
wherein said formulation has an inorganic salt concentration of 150 mM or lower.

In one aspect, in addition to the polypeptide of the invention, the formulation of the present invention comprises at least an aqueous carrier having a pH of 5.5 to 8.0, a buffer selected from histidine pH 6.0-6.5, and an excipient. Most preferably the buffer is histidine pH 6.5 or histidine pH 6.0. The buffer preferably has a concentration ranging from, 10 mM to 50 mM, 10 mM to 25 mM, 10 mM to 20 mM. In a specific aspect, the concentration of buffer in the formulations of the invention is 10 mM, 15 mM, 20 mM, 25 mM, 50 mM, 75 mM, or 100 mM. In a preferred aspect, the concentration is between 10 and 20 mM, such as 10 mM or 15 mM. In a specific aspect, a formulation of the invention comprises 15 mM histidine buffer pH 6.5. in another specific aspect, a formulation of the invention comprises 10 mM histidine buffer pH 6.0. Preferred excipients are polyols such as mannitol, sorbitol, etc., saccharides such as e.g. sucrose, mannose, trehalose, etc. The concentration of the excipient may range from about 1% to 20%, preferably from about 2.5% to 15%, more preferably from about 5% to 10%, such as e.g. 5%, 7.5%, 8% or 10%. In a preferred aspect, the formulation comprises sucrose, preferably at a concentration of about 5% to 10%, such as about 8% or about 10%. Accordingly, a preferred formulation of the invention may comprise 15mM histidine pH 6.5 and 8% sucrose. Another preferred formulation of the invention may comprise 10mM histidine pH 6.0 and 10% sucrose.

In another aspect, in addition to the polypeptide of the invention, the formulation of the present invention may comprise at least an aqueous carrier having a pH of 5.5 to 8.0, a buffer selected from histidine phH 6.0-6.5 and a surfactant. Most preferably the buffer is histidine pH 6.5 or histidine pH 6.0. The buffer preferably has a concentration ranging from, 10 mM to 50 mM, 10 mM to 25 mM, 10 mM to 20 mM. In a specific aspect, the concentration of buffer in the formulation of the invention is 10 mM, 15 mM, 20 mM, 25 mM, 50 mM, 75 mM, or 100 mM. In a preferred aspect, the concentration is between 10 and 20 mM, such as 10 mM or 15 mM. In a specific aspect, a formulation of the invention comprises 15 mM histidine buffer pH 6.5. In another specific aspect, a formulation of the invention comprises 10 mM histidine buffer pH 6.0.The surfactant may be selected from Tween 20, Tween 80 or poloxamers. The concentration of the surfactant may range from about 0.001% to 1% (preferably from about 0.001% to 0.1%, or 0.01% to 0.1% such as 0.001%, 0.005%, 0.01%, 0.02%, 0.05%, 0.08%, 0.1%, 0.5%, or 1% of the formulation, preferably 0.01%). In a specific embodiment, the surfactant is Tween 20 or Tween 80, which is at a concentration of 0.001%, 0.005%, 0.01%, 0.02%, 0.05%, 0.08%, 0.1%, 0.5% or 1% of the formulation, such as e.g. 0.01% Tween 80 or 0.005% Tween 80. Accordingly, a preferred formulation of the invention may comprise 15mM histidine pH 6.5 and 0.01% Tween 80. Another preferred formulation of the invention may comprise 10mM histidine pH 6.0 and 0.005% Tween 80.

In yet another aspect, in addition to the polypeptide of the invention, the formulation of the present invention may comprise at least an aqueous carrier having a pH of 5.5 to 8.0, an excipient and a surfactant. Preferred excipients are polyols such as mannitol, sorbitol, etc., saccharides such as e.g. sucrose, mannose, trehalose, etc. The concentration of the excipient may range from about 1% to 20%, preferably from about 2.5% to 15%, more preferably from about 5% to 10%, such as e.g. 5%, 7.5%, 8% or 10%. In a preferred aspect, the formulation comprises sucrose, preferably at a concentration of about 5% to 10%, such as about 8% or 10%. The surfactant may be selected from Tween 20, Tween 80 or a poloxamer. The concentration of the surfactant may range from about 0.001% to 1% (preferably from about 0.001% to 0.1%, or 0.01% to 10.1% such as 0.001%, 0.005%, 0.01%, 0.02%, 0.05%, 0.08%, 0.1%, 0.5%, or 1% of the formulation, preferably 0.01%). In a specific embodiment, the surfactant is Tween 20 or Tween 80, which is at a concentration of 0.001%, 0.005%, 0.01%, 0.02%, 0.05%, 0.08%, 0.1%, 0.5% or 1% of the formulation, such as e.g. 0.01% Tween 80 or 0.005% Tween 80. Accordingly, a preferred formulation of the invention may comprise 8% sucrose and 0.01% Tween 80. Another preferred formulation of the invention may comprise 10% sucrose and 0.005% Tween 80.

Accordingly, a formulation of the invention may, in addition to the polypeptide of the invention, comprise for example:
a) A buffer selected from a histidine buffer pH 6.5, histidine buffer pH 6.0 and hepes buffer pH 7.0 at a concentration of 10mM to 100mM; and
b) An excipient selected from sucrose, sorbitol, trehalose and mannitol at a concentration of 1% to 20%.
   or
a) A buffer selected from a histidine buffer pH 6.5, histidine buffer pH 6.0 and hepes buffer pH 7.0 at a concentration of 10mM to 100mM; and
c) A surfactant selected from Tween 80, Tween 20 or a poloxamer at a concentration of 0.001% to 1%.
   or
b) An excipient selected from sucrose, sorbitol, trehalose and mannitol at a concentration of 1% to 20%; and
c) A surfactant selected from Tween 80, Tween 20 or a poloxamer at a concentration of 0.001% to 1%.
   or
a) A buffer selected from a histidine buffer pH 6.5, and hepes buffer pH 7.0 at a concentration of 15 mM; and
b) An excipient selected from sucrose, sorbitol, trehalose and mannitol at a concentration of 1% to 20%.
   or
a) A buffer selected from a histidine buffer pH 6.5 and hepes buffer pH 7.0 at a concentration of 15 mM; and
c) A surfactant selected from Tween 80, Tween 20 or a poloxamer at a concentration of 0.001% to 1%.
   or
a) A buffer selected from a histidine buffer pH 6.5 and hepes buffer pH 7.0 at a concentration of 10mM to 100mM; and
b) An excipient selected from sucrose, sorbitol, trehalose and mannitol at a concentration of 8%.
   or
b) An excipient selected from sucrose, sorbitol, trehalose and mannitol at a concentration of 8%; and
c) A surfactant selected from Tween 80, Tween 20 or a poloxamer at a concentration of 0.001% to 1%.
   or
a) A buffer selected from a histidine buffer pH 6.5 and hepes buffer pH 7.0 at a concentration of 10mM to 100mM; and
c) A surfactant selected from Tween 80, Tween 20 or a poloxamer at a concentration of 0.01%.
   or
b) An excipient selected from sucrose, sorbitol, trehalose and mannitol at a concentration of 1% to 20%; and
c) A surfactant selected from Tween 80, Tween 20 or a poloxamer at a concentration of 0.01%.
   or
a) A buffer selected from a histidine buffer pH 6.0 at a concentration of 10 mM; and
b) An excipient selected from sucrose, sorbitol, trehalose and mannitol at a concentration of 1% to 20%.
   or
a) A buffer selected from a histidine buffer pH 6.0 at a concentration of 10 mM; and
c) A surfactant selected from Tween 80, Tween 20 or a poloxamer at a concentration of 0.001% to 1%.
   or
a) A buffer selected from a histidine buffer pH 6.0 at a concentration of 10mM to 100mM; and
b) An excipient selected from sucrose, sorbitol, trehalose and mannitol at a concentration of 10%.
   or
b) An excipient selected from sucrose, sorbitol, trehalose and mannitol at a concentration of 10%; and
c) A surfactant selected from Tween 80, Tween 20 or a poloxamer at a concentration of 0.001% to 1%.
   or
a) A buffer selected from a histidine buffer pH 6.0 at a concentration of 10mM to 100mM; and
c) A surfactant selected from Tween 80, Tween 20 or a poloxamer at a concentration of 0.005%.
   or
b) An excipient selected from sucrose, sorbitol, trehalose and mannitol at a concentration of 1% to 20%; and
c) A surfactant selected from Tween 80, Tween 20 or a poloxamer at a concentration of 0.005%.
   or
a) Histidine buffer pH 6.5 or pH 6.0 at a concentration of 10mM to 100mM; and
b) Sucrose at a concentration of 1% to 20%.
   or
a) Histidine buffer pH 6.5 or pH 6.0 at a concentration of 10mM to 100mM; and
c) Tween 80 at a concentration of 0.001% to 1%.
   or
b) Sucrose at a concentration of 1% to 20%; and
c) Tween 80 at a concentration of 0.001% to 1%.
   or
a) 15 mM histidine buffer pH 6.5; and
b) 8% sucrose.
   or
a) 15 mM histidine buffer pH 6.5; and
c) 0.01% Tween 80.
   or
b) 8% sucrose; and
c) 0.01% Tween 80.
   or
a) 10 mM histidine buffer pH 6.0; and
b) 10% sucrose.
   or
a) 10 mM histidine buffer pH 6.0; and
c) 0.005% Tween 80.
   or
b) 10% sucrose; and
c) 0.005% Tween 80.

The formulation of the invention comprises a polypeptide selected from SEQ ID NO's: 1 to 6. Accordingly the formulation of the invention may comprise:
a) 15 mM histidine buffer pH 6.5;
b) 8% sucrose; and
d) A polypeptide selected from SEQ ID NO's: 1 to 6 (e.g. at a concentration of 10 mg/ml). or
a) 15 mM histidine buffer pH 6.5;
c) 0.01% Tween 80; and
d) A polypeptide selected from SEQ ID NO's: 1 to 6 (e.g. at a concentration of 10 mg/ml). or
b) 8% sucrose;
c) 0.01% Tween 80; and
d) A polypeptide selected from SEQ ID NO's: 1 to 6 (e.g. at a concentration of 10 mg/ml). or
a) 10 mM histidine buffer pH 6.0;
b) 10% sucrose; and
c) A polypeptide selected from SEQ ID NO's: 1 to 6 (e.g. at a concentration of 10 mg/ml). or
a) 10 mM histidine buffer pH 6.0;
c) 0.005% Tween 80; and
d) A polypeptide selected from SEQ ID NO's: 1 to 6 (e.g. at a concentration of 10 mg/ml). or
b) 10% sucrose;
c) 0.005% Tween 80; and
d) A polypeptide selected from SEQ ID NO's: 1 to 6 (e.g. at a concentration of 10 mg/ml).

The formulation of the present invention comprises an aqueous carrier having a pH of 5.5 to 8.0 and a polypeptide as defined above (*"polypeptide of the invention"*) comprising one or more single variable domains at a concentration of 1 mg/mL to 200 mg/mL, said formulation being formulated for administration to a human subject and comprises the components selected from:
a) A buffer at a concentration of 10mM to 100mM selected from histidine pH 6.0-6.5
wherein said formulation has an inorganic salt concentration of 150 mM or lower.

Accordingly in addition to the polypeptide of the invention, the formulations of the present invention may comprise at least an aqueous carrier having a pH of 5.5 to 8.0, a buffer selected from the group consisting of histidine pH 6.0-6.5, an excipient and a surfactant. Most preferably the buffer is histidine pH 6.5 or histidine pH 6.0. The buffer preferably has a concentration ranging from 10 mM to 50 mM, 10 mM to 25 mM, 10 mM to 20 mM. In a specific aspect, the concentration of buffer in the formulations of the invention is 10 mM, 15 mM, 20 mM, 25 mM, 50 mM, 75 mM, or 100 mM. In a preferred aspect, the concentration is between 10 and 20 mM, such as 10 mM or 15 mM. In a specific aspect, a formulation of the invention comprises 15 mM histidine buffer pH 6.5. In another specific aspect, a formulation of the invention comprises 10 mM histidine buffer pH 6.0. Preferred excipients are polyols such as mannitol, sorbitol, etc., saccharides such as e.g. sucrose, mannose, trehalose, etc. The concentration of the excipient may range from about 1% to 20%, preferably from about 2.5% to 15%, more preferably from about 5% to 10%, such as e.g. 5%, 7.5%, 8% or 10%. In a preferred aspect, the formulation comprises sucrose, preferably at a concentration of about 5% to 10%, such as about 8% or 10%. The surfactant may be selected from Tween 20, Tween 80 or a poloxamer. The concentration of the surfactant may range from about 0.001% to 1% (preferably from about 0.001% to 0.1%, or 0.01% to 0.1% such as 0.001%, 0.005%, 0.01%, 0.02%, 0.05%, 0.08%, 0.1%, 0.5%, or 1% of the formulation, preferably 0.01% or 0.005%). In a specific embodiment, the surfactant is Tween 20 or Tween 80, which is at a concentration of 0.001%, 0.005%, 0.01%, 0.02%, 0.05%, 0.08%, 0.1%, 0.5% or 1% of the formulation, such as e.g. 0.01% Tween 80 or 0.005% Tween 80. Accordingly, a preferred formulation of the invention may comprise 15 mM histidine pH 6.5, 8% sucrose and 0.01% Tween 80. Another preferred formulation of the invention may comprise 10 mM histidine pH 6.0, 10% sucrose and 0.005% Tween 80.

Accordingly, a formulation of the invention may, in addition to the polypeptide of the invention, comprise for example:
a) A buffer selected from a histidine buffer pH 6.5, a histidine buffer pH 6.0 at a concentration of 10mM to 100mM;
b) An excipient selected from sucrose, sorbitol, trehalose and mannitol at a concentration of 1% to 20%; and
c) A surfactant selected from Tween 80, Tween 20 or a poloxamer at a concentration of 0.001% to 1%.
   or
a) a histidine buffer pH 6.5 at a concentration of 15 m M;
b) An excipient selected from sucrose, sorbitol, trehalose and mannitol at a concentration of 1% to 20%; and
c) A surfactant selected from Tween 80, Tween 20 or a poloxamer at a concentration of 0.001% to 1%.
   or
a) a histidine buffer pH 6.5 at a concentration of 10mM to 100mM;
b) An excipient selected from sucrose, sorbitol, trehalose and mannitol at a concentration of 8%; and
c) A surfactant selected from Tween 80, Tween 20 or a poloxamer at a concentration of 0.001% to 1%.
   or
a) a histidine buffer pH 6.5 at a concentration of 10mM to 100mM;
b) An excipient selected from sucrose, sorbitol, trehalose and mannitol at a concentration of 1% to 20%; and
c) A surfactant selected from Tween 80, Tween 20 or a poloxamer at a concentration of 0.01%.
   or
a) a histidine buffer pH 6.5 at a concentration of 15 mM;
b) An excipient selected from sucrose, sorbitol, trehalose and mannitol at a concentration of 8%; and
c) A surfactant selected from Tween 80, Tween 20 or a poloxamer at a concentration of 0.001% to 1%.
   or
a) a histidine buffer pH 6.5 at a concentration of 10mM to 100mM;
b) An excipient selected from sucrose, sorbitol, trehalose and mannitol at a concentration of 8%; and
c) A surfactant selected from Tween 80, Tween 20 or a poloxamer at a concentration of 0.01%.
   or
a) a histidine buffer pH 6.5 at a concentration of 15 mM;
b) An excipient selected from sucrose, sorbitol, trehalose and mannitol at a concentration of 1% to 20%; and
c) A surfactant selected from Tween 80, Tween 20 or a poloxamer at a concentration of 0.01%.
   or
a) A buffer selected from a histidine buffer pH 6.0 at a concentration of 10 mM;
b) An excipient selected from sucrose, sorbitol, trehalose and mannitol at a concentration of 1% to 20%; and
c) A surfactant selected from Tween 80, Tween 20 or a poloxamer at a concentration of 0.001% to 1%.
   or
a) A buffer selected from a histidine buffer pH 6.0 at a concentration of 10mM to 100mM;
b) An excipient selected from sucrose, sorbitol, trehalose and mannitol at a concentration of 10%; and
c) A surfactant selected from Tween 80, Tween 20 or a poioxamer at a concentration of 0.001% to 1%.
   or
a) A buffer selected from a histidine buffer pH 6.0 at a concentration of 10mM to 100mM;
b) An excipient selected from sucrose, sorbitol, trehalose and mannitol at a concentration of 1% to 20%; and
c) A surfactant selected from Tween 80, Tween 20 or a poloxamer at a concentration of 0.005%.
   or
a) A buffer selected from a histidine buffer pH 6.0 at a concentration of 10 mM;
b) An excipient selected from sucrose, sorbitol, trehalose and mannitol at a concentration of 10%; and
c) A surfactant selected from Tween 80, Tween 20 or a poloxamer at a concentration of 0.001% to 1%.
   or
a) A buffer selected from a histidine buffer pH 6.0 at a concentration of 10mM to 100mM;
b) An excipient selected from sucrose, sorbitol, trehalose and mannitol at a concentration of 10%; and
c) A surfactant selected from Tween 80, Tween 20 or a poloxamer at a concentration of 0.005%.
   or
a) A buffer selected from a histidine buffer pH 6.0 at a concentration of 15 mM;
b) An excipient selected from sucrose, sorbitol, trehalose and mannitol at a concentration of 1% to 20%; and
c) A surfactant selected from Tween 80, Tween 20 or a poloxamer at a concentration of 0.005%.
   or
a) Histidine buffer pH 6.5 at a concentration of 10mM to 100mM;
b) Sucrose at a concentration of 1% to 20%; and
c) Tween 80 at a concentration of 0.001% to 1%.
   or
a) 15 mM histidine buffer pH 6.5;
b) 8% sucrose; and
c) 0.01% Tween 80.
   or
a) Histidine buffer pH 6.0 at a concentration of 10mM to 100mM;
b) Sucrose at a concentration of 1% to 20%; and
c) Tween 80 at a concentration of 0.001% to 1%.
   or
a) 10 mM histidine buffer pH 6.0;
b) 10% sucrose; and
c) 0.005% Tween 80.

The formulation of the invention comprises a polypeptide selected from SEQ ID NO's: 1 to 6. Accordingly the formulation of the invention may comprise:
a) 15 mM histidine buffer pH 6.5;
b) 8% sucrose;
c) 0.01% Tween 80; and
d) A polypeptide selected from SEQ ID NO's: 1 to 6 (e.g. at a concentration of 10 mg/ml).
   or
a) 10 mM histidine buffer pH 6.0;
b) 10% sucrose;
c) 0.005% Tween 80; and
d) A polypeptide selected from SEQ ID NO's: 1 to 6 (e.g. at a concentration of 10 mg/ml).

The components present in the formulations of the invention have been selected such that the polypeptides of the invention have a good solubility (as defined herein). Preferably, the polypeptides present in the formulations of the present invention have a solubility of at least 0.7 mM, at least 0.8 mM, at least 0.9 mM, at least 1.0 mM, at least 1.1 mM, at least 1.2 mM, at least 1.3 mM, at least 1.4 mM, at least 1.5 mM, at least 1.6 mM, at least 1.7 mM, at least 1.8 mM, at least 1.9 mM, at least 2.0 mM, at least 2.1 mM, at least 2.2 mM, at least 2.3 mM, at least 2.4 mM, at least 2.5 mM, at least 2.6 mM, at least 2.7 mM, at least 2.8 mM, at least 2.9 mM, at least 3.0 mM, at least 3.2 mM, at least 3.4 mM, at least 3.6 mM or more and/or at least 20 mg/ml, at least 30 mg/mL, at least 40 mg/mL, at least 50 mg/mL, at least 60 mg/mL, at least 65 mg/mL, at least 70 mg/mL, at least 80 mg/mL, at least 90 mg/mL, at least 100 mg/mL, at least 110 mg/mL, at least 120 mg/mL, at least 130 mg/mL, at least 140 mg/mL, at least 150 mg/mL or even 200 mg/mL or more as determined by the PEG exclusion method or by centrifugal ultrafiltration. A very good solubility of the polypeptides of the invention has been obtained with a formulation comprising a histidine buffer pH 6.5 or with a formulation comprising Tween 80. Accordingly, the present invention relates to a formulation as claimed comprising an aqueous carrier and a polypeptide comprising one or more single variable domains, said formulation being formulated for administration to a human subject, wherein said formulation further comprises at least one of:
a) A histidine buffer pH 6.5 at a concentration of 10mM to 100mM (preferably 10mM to 50mM, more preferably 10 to 20 mM, such as 15 mM);
c) Tween 80 at a concentration of 0.001% to 1% (preferably from about 0.001% to 0.1%, or 0.01% to 0.1% such as 0.001%, 0.005%, 0.01%, 0.02%, 0.05%, 0.08%, 0.1%, 0.5%, or 1% of the formulation, preferably 0.01%);
wherein said formulation has an inorganic salt concentration of 150 mM or lower; and wherein the solubility of the polypeptide is at least at least 20 mg/mL, at least 50 mg/mL, preferably at least 90 mg/mL, at least 120 mg/mL, at least 150 mg/mL or even 200 mg/mL or more as determined by the PEG exclusion method or by centrifugal ultrafiltration. In a preferred aspect, the formulation comprises a histidine buffer pH 6.5 at a concentration of 10mM to 100mM (preferably 10mM to 50mM, more preferably 10 to 20 mM, such as 15 mM) and Tween 80 at a concentration of 0.001% to 1% (preferably from about 0.001% to 0.1%, or 0.01% to 0.1% such as 0.001%, 0.005%, 0.01%, 0.02%, 0.05%, 0.08%, 0.1%, 0.5%, or 1% of the formulation, preferably 0.01%).

Apart from this and/or in addition, the polypeptides of the invention present in the formulation of the invention should preferably have a melting temperature of at least 59°C or more (such as 59.5°C or more), preferably at least 60°C or more (such as 60.5°C or more), more preferably at least 61°C or more (such as 61.5°C or more) or at least 62°C or more (such as 62.5°C or more), most preferably at least 63°C or more (such as 63.5°C or more) as measured by the thermal shift assay (TSA) and/or differential scanning calorimetry (DSC).

Without being limiting, melting point determination can be done by the fluorescence-based thermal shift assay which is based on the fact that upon thermal unfolding the hydrophobic regions of proteins, usually hidden in the core of the protein fold, become accessible for binding to a hydrophobic fluorescent dye. The fluorescence emission of this dye is quenched in aqueous solution, whereas upon binding to the hydrophobic patches of an unfolded protein a sharp increase in the fluorescence yield of the probe is observed. Temperature induced unfolding is typically a two-state process with a sharp transition between the folded and unfolded state, where the melting temperature (Tm) is defined as the temperature at which half of the protein is in the unfolded state, i.e. the first derivative of the fluorescence signal upon gradual heating of the sample is plotted and the observed peak (or peaks when multiple domains and/or variants of the same domain are present) represents the melting temperature. The thermal shift assay can be performed in a typical real-time PCR instrument where melting curves can be recorded accurately in high-throughput mode with only small quantities of protein required.

During a differential scanning calorimetry experiment the sample is heated at a constant rate in an adiabatic environment (ΔT = 0). The energy required to keep the temperature difference between a reference and the sample cell at zero is measured and yields the heat capacity as a function of temperature (Cp(T)). The temperature corresponding to the maximum heat capacity represents the melting temperature (Tₘ). If the temperature dependent unfolding process is reversible other thermodynamic parameters such as the unfolding enthalpy (ΔH_{unfolding}) can be determined.

Increased melting temperatures have been observed for the polypeptides of the invention when present in a formulation with a pH of about 6.0 to 8.0, preferably 6.2 to 7.5, more preferably 6.5 to 7.5, most preferably 6.5-7.0. Increased melting temperature have also been obtained for the polypeptides of the invention when present in a formulation that comprise hepes pH 7.0, histidine pH 6.0-6.5, MES pH 6.0 or acetate pH 6.0, or a formulation that comprises an excipient, preferably a saccharides and/or polyol such as mannitol, trehalose, sorbitol or sucrose. Accordingly, also disclosed herein is a formulation comprising an aqueous carrier at a pH of 6.0 to 8.0 and a polypeptide comprising one or more single variable domains, said formulation being formulated for administration to a human subject, wherein said formulation further comprises at least one of:
a) A buffer at a concentration of 10mM to 100mM (preferably 10mM to 50mM, more preferably 10 to 20 mM, such as 15 mM or 10 mM) selected from the group consisting of histidine pH 6.0-6.5, hepes pH 7.0-8.0, MES pH 6.0, succinate pH 6.0-6.5 and acetate pH 5.5-6.0;
b) An excipient, preferably a saccharide and/or polyol such as mannitol, sorbitol, trehalose or sucrose at a concentration of 1% to 20% (preferably 2.5% to 15%, more preferably 5% to 10%, such as 5%, 7.5%, 8% or 10%).
wherein said formulation has an inorganic salt concentration of 150 mM or lower; and wherein the melting temperature of the polypeptide of the invention is at least 59°C or more (such as 59.5°C or more), preferably at least 60°C or more (such as 60.5°C or more), more preferably at least 61°C or more (such as 61.5°C or more) or at least 62°C or more (such as 62.5°C or more), most preferably at least 63°C or more (such as 63.5°C or more) as measured by the thermal shift assay (TSA) and/or differential scanning calorimetry (DSC).

In a preferred aspect, the formulation comprises a buffer at a concentration of 10mM to 100mM (preferably 10mM to 50mM, more preferably 10 to 20 mM, such as 15 mM) selected from the group consisting of histidine pH 6.0-6.5, hepes pH 7.0-8.0, MES pH 6.0, succinate pH 6.0-6.5 and acetate pH 5.5-6.0, and an excipient, preferably a saccharide and/or polyol such as mannitol, sorbitol, trehalose or sucrose at a concentration of 1% to 20% (preferably 2.5% to 15%, more preferably 5% to 10%, such as 5%, 7.5%, 8% or 10%).

Apart from this and/or in addition, the formulation of the present invention exhibits stability under at least one or more of the following stress conditions:
- multiple (up to 10) freeze/thaw cycles;
- storage at a temperature of 2-8°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- storage at a temperature of 25±5°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- mechanical stress.

Preferably the formulation of the invention is stable under one or more of the following forms of mechanical stress:
- shaking the formulation during 10s to 1 min;
- pushing the formulation through a needle (25G, preferably 26G, more preferably 27G, even more preferably 28G, most preferably 29G or more) with a syringe (the syringe used can be any commercially available syringe, such as e.g. a 1 mL, 2 mL, 3mL, 4 mL, 5 mL, 10 mL, 20 mL, 30 mL, 40 mL up to 50 mL syringe);
- rotating for two days at 10 rpm; and/or
- stirring for 1 hour at room temperature and/or 4-48 hours at 4°C at at least 10 rpm (such as 50 rpm, 100 rpm or more).

Preferably, the formulation of the present invention is stable under more than one of the above stress conditions, such as at least two, at least three, at least four, at least five, at least six, at least seven or most preferably under all of the above stress conditions.

In one aspect, the formulation of the invention exhibits stability under one or more forms of mechanical and/or shear stress. Mechanical stress as used in the present invention can be any form of external force applied on the formulation that may affect the stability of the polypeptide present in the formulation. Without being limiting, the mechanical stress applied to the solution include shear stress, stir stress, shake stress, rotation stress, etc. The formulation of the invention may for example be shaken during at least 10s, 20s, 30s, 40s, 50s up to 1 minute or more. The formulation of the invention may be pushed through a syringe (the syringe used can be any commercially available syringe, such as e.g. a 1 mL, 2 mL, 3mL, 4 mL, 5 mL, 10 mL, 20 mL, 30 mL, 40 mL up to 50 mL syringe) with needle once, twice, three times, four times, five times up to 10 times or more. Preferably the needle has a size of 25G (such as 26G, 27G, 28G, 29G, 30G) or more. More preferably the size of the needles is 27G or more. In addition or alternatively, the formulation of the invention may be rotated for 1 hour, 2 hours, 6 hours, 12 hours, 1 day up to two days or more at 10 rpm. The formulation of the invention may be stirred for 1 hour at room temperature and/or 4 hours, 8 hours, 12 hours, 24 hours or even 48 hours or more at 2-8°C. The speed of rotation is preferably above 10 rpm, such as e.g. 50 rpm, 100 rpm or more.

The stability of the formulation under mechanical stress can be assessed e.g. by visual inspection of the formulation or by measurement of the OD320/OD280 ratio. Preferably the OD320/OD280 ratio is 0.05 or lower, such as 0.01 or 0.005.

A good stability of the polypeptides of the invention under mechanical stress has been obtained with a formulation comprising an excipient, preferably a saccharide and/or polyol such as mannitol or sucrose or comprising Tween 80. Accordingly, also disclosed herein is a formulation comprising an aqueous carrier and a polypeptide comprising one or more single variable domains, said formulation being formulated for administration to a human subject, wherein said formulation further comprises at least one of:
b) An excipient, preferably a saccharide and/or polyol such as mannitol or sucrose at a concentration of 1% to 20% (preferably 2.5% to 10%, more preferably 5% to 10%, such as 5%, 7.5%, 8% or 10%);
c) A surfactant at a concentration of 0.001% to 1% (preferably from about 0.001% to 0.1%, or 0.01% to 0.1% such as 0.001%, 0.005%, 0.01%, 0.02%, 0.05%, 0.08%, 0.1%, 0.5%, or 1% of the formulation, preferably 0.01%) selected from Tween 20, Tween 80 or a poloxamer,
wherein said formulation has an inorganic salt concentration of 150 mM or lower; and
wherein the polypeptide is stable under mechanical stress as determined by OD320/OD280 ratio measurement. In a preferred aspect, the formulation comprises an excipient, preferably a saccharide and/or polyol such as mannitol or sucrose at a concentration of 1% to 20% (preferably 2.5% to 15%, more preferably 5% to 10%, such as 5%, 7.5%, 8% or 10%) and a surfactant at a concentration of 0.001% to 1% (preferably about 0.001% to 0.1%, or 0.01% to 0.1% such as 0.001%, 0.005%, 0.01%, 0.02%, 0.05%, 0.08%, 0.1%, 0.5%, or 1% of the formulation, preferably 0.01% or 0.005%) selected from Tween 20 and Tween 80. A preferred formulation comprises 8% sucrose and 0.01% Tween 80. Another preferred formulation comprises 10% sucrose and 0.005% Tween 80.

In a specific aspect, the formulation of the invention comprises an excipient, preferably a saccharide and/or polyol such as mannitol or sucrose at a concentration of 1% to 20% (preferably 2.5% to 15%, more preferably 5% to 10%, such as 5%, 7.5%, 8% or 10%) and/or Tween 20, Tween 80 or a poloxamer (at a concentration ranging from about 0.001% to 0.1%, or 0.01% to 0.1% such as 0.001%, 0.005%, 0.01%, 0.02%, 0.05%, 0.08%, 0.1%, 0.5%, or 1% of the formulation, preferably 0.01%) and is characterized that the polypeptides present in the formulation are stable under mechanical stress.

In a preferred aspect, the formulation of the invention comprises an excipient, preferably a saccharide and/or polyol such as mannitol or sucrose at a concentration of 1% to 20% (preferably 2.5% to 15%, more preferably 5% to 10%, such as 5%, 7.5%, 8% or 10%) and/or Tween 80 (at a concentration of 0.001%, 0.005%, 0.01%, 0.02%, 0.05%, 0.08%, 0.1%, 0.5%, or 1% of the formulation, preferably 0.01% or 0.005%) and is characterized that the polypeptides present in the formulation of the invention are stable when shaking the formulation during at least 10s, 20s, 30s, 40s, 50s up to 1 minute of more. In another preferred aspect, the formulation of the invention comprises an excipient, preferably a saccharide and/or polyol such as mannitol or sucrose at a concentration of 1% to 20% (preferably 2.5% to 15%, more preferably 5% to 10%, such as 5%, 7.5%, 8% or 10%) and/or Tween 80 (at a concentration of 0.001%, 0.005%, 0.01%, 0.02%, 0.05%, 0.08%, 0.1%, 0.5%, or 1% of the formulation, preferably 0.01% or 0.005%) and is characterized that the polypeptides present in the formulation of the invention are stable when pushing the formulation through a syringe (the syringe used can be any commercially available syringe, such as e.g. a 1 mL, 2 mL, 3mL, 4 mL, 5 mL, 10 mL, 20 mL, 30 mL, 40 mL up to 50 mL syringe) with needle size of 25G or more (such as 26G, 27G, 28G, 29G, 30G or more, preferably 27G or more) once, twice, three times, four times, five times up to 10 times or more. In another preferred aspect, the formulation of the invention comprises an excipient, preferably a saccharide and/or polyol such as mannitol or sucrose at a concentration of 1% to 20% (preferably 2.5% to 15%, more preferably 5% to 10%, such as 5%, 7.5%, 8% or 10%) and/or Tween 80 (at a concentration of 0.001%, 0.005%, 0.01%, 0.02%, 0.05%, 0.08%, 0.1%, 0.5%, or 1% of the formulation, preferably 0.01% or 0.005%) and is characterized that the polypeptides present in the formulation of the invention are stable when rotating the formulation for 1 hour, 2 hours, 6 hours, 12 hours, 1 day up to two days or more at 10 rpm. In another preferred aspect, the formulation of the invention comprises an excipient, preferably a saccharide and/or polyol such as mannitol or sucrose at a concentration of 1% to 20% (preferably 2.5% to 15%, more preferably 5% to 10%, such as 5%, 7.5%, 8% or 10%) and/or Tween 80 (at a concentration of 0.001%, 0.005%, 0.01%, 0.02%, 0.05%, 0.08%, 0.1%, 0.5%, or 1% of the formulation, preferably 0.01% or 0.005%) and is characterized that the polypeptides present in the formulation of the invention are stable when stirring the formulation for 1 hour at room temperature and/or 4 hours, 8 hours, 12 hours, 24 hours or even 48 hours or more at 2-8°C at at least 10 rpm (such as 50 rpm, 100 rpm or more).

A "freeze/thaw cycle" or "F/T cycle" is defined as the freezing of a sample in a freezer (-20±5°C) or ultrafreezer (below -64°C (such as e.g. at -80°C)) until solid, followed by thawing at room temperature until all ice crystals have visually disappeared. In one aspect, the formulation of the invention exhibits stability under multiple (up to 10) freeze/thaw cycles. The formulation of the invention may exhibit stability under at least 1, at least 2, at least 3, at least 5 to up to at least 10 freeze/thaw cycles, such as e.g. 10 cycles at -20°C, 2 cycles at -80°C + 1 cycle at -20°C or 2 cycles at-80°C + 6 cycles at -20°C.

In yet another aspect, the formulation of the invention exhibits stability when stored at a temperature of 5±5°C. The formulation of the invention may exhibit stability when stored at a temperature of 5±5°C for at least 2 weeks, 3 weeks, 4 weeks, 8 weeks, 10 weeks, up to 3 months, 6 months, 11 months, 1 year, 1.5 year or even 2 years and more.

in yet another aspect, the formulation of the invention exhibits stability when stored at a temperature of 25±5°C. The formulation of the invention may exhibit stability when stored at a temperature of 25±5°C for at least 2 weeks, 3 weeks, 4 weeks, at least 5 weeks, at least 8 weeks, at least 10 weeks, at least 3 months, at least 6 months, at least 1 year, 1.5 year or even 2 years or more.

In yet another aspect, the formulation of the invention exhibits stability when stored at a temperature of 37±5°C. The formulation of the invention may exhibit stability when stored at a temperature of 37±5°C for at least 2 weeks, 3 weeks, 4 weeks, at least 5 weeks, at least 8 weeks, at least 10 weeks, at least 3 months, at least 6 months, at least 1 year, 1.5 year or even 2 years or more.

As is known to one skilled in the art, the temperatures indicated in this text can be subject to normal variations.

Preferably, in those formulations that are stable under one or more of the above stress conditions:
- less than 10% (more preferably less than 5%, even more preferably less than 3%, most preferably less than 1%) of the polypeptide of the invention forms pyroglutamate at the N-terminal glutamic acid (e.g. as assessed by RP-HPLC) during storage under (one of the above) stress conditions, such as e.g. at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- less than 10% (more preferably less than 5%, even more preferably less than 3%, most preferably less than 1%) of the polypeptide of the invention forms dimers (e.g. as assessed by SE-HPLC) during storage under (one of the above) stress conditions, such as e.g. at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- at least 80% (at least 85%, at least 90%, at least 95%, at least 98%, at least 99%, or at least 99.5%) of the polypeptides of the invention retain their binding activity (e.g. as assessed by ELISA and/or Biacore) to at least one of their (preferably to all of their) targets after storage under (one of the above) stress conditions, such as e.g. at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more) compared to the binding activity prior to the stress condition.

Stability of the formulations can be assessed by various analytical and/or immunological methods known in the art. The protein content of the polypeptides of the invention can, for example be detected by spectrophotometrical methods.

SDS-PAGE allows the visualization of the polypeptides in a given sample via direct staining. SDS-PAGE is used to separate proteins according to their size. Both reducing and non-reducing SDS-PAGE analysis can be performed.

The molecular size distribution and the relative amounts of polypeptide of the invention and protein impurities can be determined by Size Exclusion High Performance Liquid Chromatography (SE-HPLC). The relative amount of a specific protein impurity, expressed as area %, can be calculated by dividing the peak area corresponding to the impurity by the total integrated area. SE-HPLC methods are known to the skilled person and are also described in the Example section.

Reversed Phase High Performance Liquid chromatography (RP-HPLC) separates molecules with respect to differences in hydrophobicity and is based on the reversible interaction between the molecule and the hydrophobic stationary phase. In this assay a Zorbax 300SB-C3 column (Agilent Technologies, Palo Alto, US) can be used. The relative amount of a specific protein impurity, expressed as area %, can be calculated by dividing the peak area corresponding to the impurity by the total integrated area. RP-HPLC methods are known to the skilled person and are also described in the Example section.

Polypeptides of the invention and their charge variants can be separated by Ion Exchange High Performance Liquid Chromatography (IEX-HPLC). Also potential impurities can be detected with this method. The relative amount of a specific protein impurity, expressed as area %, can be calculated by dividing the peak area corresponding to the impurity by the total integrated area. IEX-HPLC methods are known to the skilled person and are also described in the Example section.

The polypeptides present in the formulation of the invention preferably do not form pyroglutamate at the N-terminal glutamic acid. The formation of pyroglutamate in the sample can e.g. be measured by RP-HPLC. For example, analysis by RP-HPLC of a formulation containing SEQ ID NO: 4 after storage for 10 weeks at a temperature of 37°C, showed the formation of pyroglutamate as a separate peak at 18-19 minutes. Preferably in the formulation of the invention, less than 10% (more preferably less than 5%, even more preferably less than 3%, most preferably less than 1%) of the polypeptides form pyroglutamate at the N-terminal glutamic acid (e.g. as assessed by RP-HPLC) under one or more of the above stress conditions.

Little to no pyroglutamate formation of the polypeptides of the invention has been observed in formulations with a pH of 7.0 or lower, preferably 6.5 or lower,.such as 6.5, 6.0 or 5.5, in e.g. histidine buffers and acetate buffers. Accordingly, also disclosed herein is a formulation comprising an aqueous carrier at a pH of 7.0 or lower and a polypeptide comprising one or more single variable domains, said formulation being formulated for administration to a human subject, wherein said formulation further comprises:
a) A buffer at a concentration of 10mM to 100mM (preferably 10mM to 50mM, more preferably 10 to 20 mM, such as 15 mM or 10 mM) selected from the group consisting of histidine pH 6.0-6.5 and acetate pH 5.5-6.0,
wherein said formulation has an inorganic salt concentration of 150 mM or lower; and
wherein less than 10% (preferably less than 8%, more preferably less than 7%, most preferably less than 5%) of the polypeptides forms pyroglutamate at the N-terminal glutamic acid during one or more of the above stress conditions (such as during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more)), the % of pyroglutamate as measured by RP-HPLC. In a preferred aspect, the formulation comprises a histidine buffer pH 6.5 at 15 mM. In another preferred aspect, the formulation comprises a histidine buffer pH 6.0 at 10 mM.

The polypeptides present in the formulation of the invention also preferably do not form dimers. The formation of dimers in the sample can e.g. be measured by SE-HPLC. For example, analysis in SE-HPLC of a formulation containing SEQ ID NO: 4 after storage for 10 weeks at a temperature of 37°C, showed the formation of a separate peak eluting at an apparent molecular weight of 44 kDa in comparison with molecular weight markers, while the monomeric polypeptide eluted between the 44 and 17 kDa molecular weight markers. This separate peak at 44 kDa represented a dimeric form of SEQ ID NO: 4. Preferably in the formulation of the invention, less than 10% (more preferably less than 5%, even more preferably less than 3%, most preferably less than 1%) of the polypeptides forms dimers (e.g. as assessed by SE-HPLC) during storage under one or more of the above stress conditions.

Little to no dimer formation of the polypeptides of the invention has been observed in formulations with a histidine buffer or an acetate buffer and in formulations that comprise an excipient, preferably a saccharide and/or polyol such as mannitol, trehalose, sorbitol or sucrose. Accordingly, also disclosed herein is a formulation comprising an aqueous carrier and a polypeptide comprising one or more single variable domains, said formulation being formulated for administration to a human subject, wherein said formulation further comprises at least one of:
a) A buffer at a concentration of 10mM to 100mM (preferably 10mM to 50mM, more preferably 10 to 20 mM, such as 15 mM or 10 mM) selected from the group consisting of histidine pH 6.0-6.5 and acetate pH 5.5-6.0;
b) An excipient, preferably a saccharide, a non-reducing sugar and/or polyol such as mannitol, trehalose, sorbitol or sucrose at a concentration of 1% to 20% (preferably 2.5% to 15%, more preferably 5% to 10%, such as 5%, 7.5%, 8% or 10%),
wherein said formulation has an inorganic salt concentration of 150 mM or lower; and wherein less than 10% (preferably less than 8%, more preferably less than 7%, most preferably less than 5%) of the polypeptides forms dimers during one or more of the above stress conditions (such as during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more)), the % of dimers as measured by SE-HPLC. In a preferred aspect, the formulation comprises a histidine buffer pH 6.5 at 15 mM. In another preferred aspect, the formulation comprises a histidine buffer pH 6.0 at 10 mM. In a preferred aspect, the formulation comprises a buffer at a concentration of 10mM to 100mM (preferably 10mM to 50mM, more preferably 10 to 20 mM, such as 15 mM or 10 mM) selected from the group consisting of histidine pH 6.0-6.5 and acetate pH 5.5-6.0 and an excipient, preferably a saccharide, non-reducing sugar and/or polyol such as mannitol, trehalose, sorbitol or sucrose at a concentration of 1% to 20% (preferably 2.5% to 15%, more preferably 5% to 10%, such as 5%, 7.5%, 8% or 10%), such as e.g. 15 mM histidine pH 6.5 and 8% sucrose; or 10 mM histidine pH 6.0 and 10% sucrose.

Preferably in the formulation of the invention, less than 10% (more preferably less than 5%, even more preferably less than 3%, most preferably less than 1%) of the polypeptides forms pyroglutamate at the N-terminal glutamic acid (e.g. as assessed by RP-HPLC) and less than 10% (more preferably less than 5%, even more preferably less than 3%, most preferably less than 1%) of the polypeptides forms dimers (e.g. as assessed by SE-HPLC) during storage under one or more of the above stress conditions.

Apart from this and/or in addition, the formulation of the present invention should show only low to undetectable levels of aggregation even during storage under one or more of the above stress conditions. For example, in the formulation of the invention, no more than 5%, no more than 4%, no more than 3%, no more than 2%, no more than 1%, and most preferably no more than 0.5% of the polypeptides forms an aggregate after storage under one or more of the above stress conditions.

Aggregation as used in the present invention means the development of high molecular weight aggregates, i.e. aggregates with an apparent molecular weight in SE-HPLC analysis of more/higher than the molecular weight observed for dimers. As described above, 44 kDa is the apparent molecular weight observed in SE-HPLC analysis for dimers of SEQ ID NO: 4, 36- 38 kDa is the apparent molecular weight observed in SE-HPLC analysis for dimers of SEQ ID NO's: 1-3 and 36 kDa is the apparent molecular weight observed in SE-HPLC analysis for dimers of SEQ ID NO: 5. Aggregation can be assessed by various methods known in the art. Without being limiting, examples include SE-HPLC, analytical ultracentrifugation, dynamic light scattering, subvisible particle counting and OD320/OD280 measurement.

In an analytical ultracentrifuge, a sample being spun can be monitored in real time through an optical detection system, using ultraviolet light absorption and/or interference optical refractive index sensitive system. This allows the operator to observe the evolution of the sample concentration versus the axis of rotation profile as a result of the applied centrifugal field. With modern instrumentation, these observations are electronically digitized and stored for further mathematical analysis. Two kinds of experiments are commonly performed on these instruments: sedimentation velocity experiments and sedimentation equilibrium experiments.

Sedimentation velocity experiments aim to interpret the entire time-course of sedimentation, and report on the shape and molar mass of the dissolved macromolecules, as well as their size-distribution (Perez-Ramirez and Steckert (2005) Therapeutic Proteins: Methods and Protocols. C.M. Smales and D.C. James, Eds. Vol. 308: 301-318. Humana Press Inc, Totowa, NJ, US.). The size resolution of this method scales approximately with the square of the particle radii, and by adjusting the rotor speed of the experiment size-ranges from 100 Da to 10 GDa can be covered. Sedimentation velocity experiments can also be used to study reversible chemical equilibria between macromolecular species, by either monitoring the number and molar mass of macromolecular complexes, by gaining information about the complex composition from multi-signal analysis exploiting differences in each components spectroscopic signal, or by following the composition dependence of the sedimentation rates of the macromolecular system, as described in Gilbert-Jenkins theory.

Sedimentation equilibrium experiments are concerned only with the final steady-state of the experiment, where sedimentation is balanced by diffusion opposing the concentration gradients, resulting in a time-independent concentration profile. Sedimentation equilibrium distributions in the centrifugal field are characterized by Boltzmann distributions. This experiment is insensitive to the shape of the macromolecule, and directly reports on the molar mass of the macromolecules and, for chemically reacting mixtures, on chemical equilibrium constants.

The kinds of information that can be obtained from an analytical ultracentrifuge include the gross shape of macromolecules, the conformational changes in macromolecules, and size distributions of macromolecular samples. For macromolecules, such as proteins, that exist in chemical equilibrium with different non-covalent complexes, the number and subunit stoichiometry of the complexes and equilibrium constant constants can be studied. (see also Scott D.J., Harding S.E. and Rowe A.J. Analytical Ultracentrifugation Techniques and Methods, RSC Publishing)

Dynamic light scattering (also known as Photon Correlation Spectroscopy or quasi-elastic light scattering) is a technique in physics, which can be used to determine the size distribution profile of small particles in solution. When a beam of light passes through a colloidal dispersion, the particles or droplets scatter some of the light in all directions. When the particles are very small compared with the wavelength of the light, the intensity of the scattered light is uniform in all directions (Rayleigh scattering); for larger particles (above approximately 250nm diameter), the intensity is angle dependent (Mie scattering). If the light is coherent and monochromatic, as from a laser for example, it is possible to observe time-dependent fluctuations in the scattered intensity using a suitable detector such as a photomultiplier capable of operating in photon counting mode.

These fluctuations arise from the fact that the particles are small enough to undergo random thermal (Brownian) motion and the distance between them is therefore constantly varying. Constructive and destructive interference of light scattered by neighbouring particles within the illuminated zone gives rise to the intensity fluctuation at the detector plane which, as it arises from particle motion, contains information about this motion. Analysis of the time dependence of the intensity fluctuation can therefore yield the diffusion coefficient of the particles from which, via the Stokes Einstein equation, knowing the viscosity of the medium, the hydrodynamic radius or diameter of the particles can be calculated. (see also Berne B. J. and Pecora R. Dynamic Light Scattering With Applications to Chemistry, Biology and Physics, Dover Publications)

Aggregation can also be measured by the PAMAS SVSS-C (Small Volume Syringe System-C) instrument (PArtikelMess - und AnalyseSysteme GMBH), which is a particle size distribution analyzer for low viscous fluids. It uses the principle of light obscuration to detect sub-visible particles in the size range 1 µm - 200 µm. The validation criteria/specified limits of the European Pharmacopoeia (EP<2.9.19 Particulate Contamination: sub-visible particles) for small and large volume parenterals are defined by the *total counts per container:*
- For particles > 10 µm, no more than 6000 counts per container
- For particles > 25 µm, no more than 600 counts per container

The OD320/OD280 ratio is also a measure for turbidity or the presence of particulates in the sample. In a preferred aspect, the OD320/OD280 ratio of the formulation of the invention should be 0.05 or lower, preferably 0.01 or lower, such as 0.005 or lower.

The tendency for aggregate formation of a polypeptide in a certain formulation can also be measured by elastic light scattering. Elastic light scattering can be measured in a spectrofluorometer (e.g. excitation and emission wavelength 500 nm) by temperature-induced denaturation as measured e.g. at an angle of 90°. Preferably the maximum scatter will stay within the absorption detection limit. The scatter should be 1000 abs. or lower, preferably 750 abs or lower, such as 500 abs or lower.

No particulate formation has been observed in formulations comprising a histidine buffer pH 6.0-6.5 under different stress conditions (such as e.g. storage at a temperature of 5±5°C up to up to at least 2 weeks (preferably at least 3 weeks, at least 5 weeks, at least 8 weeks, at least 10 weeks, at least 3 months, at least 6 months, at least 1 year, 1.5 year or even 2 years or more) or storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more)). Accordingly, the present invention relates to a formulation comprising an aqueous carrier and a polypeptide comprising one or more single variable domains, said formulation being formulated for administration to a human subject, wherein said formulation further comprises:
a) A histidine buffer pH 6.0-6.5 at a concentration of 10mM to 100mM (preferably 10mM to 50mM, more preferably 10 to 20 mM, such as 15 mM),
wherein said formulation has an inorganic salt concentration of 150 mM or lower; and wherein no particulates are present under one or more of the above stress conditions (e.g. when stored at a temperature of 5±5°C up to up to at least 2 weeks (preferably at least 3 weeks, at least 5 weeks, at least 8 weeks, at least 10 weeks, at least 3 months, at least 6 months, at least 1 year, 1.5 year or even 2 years or more) or when stored at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more)) as measured by OD320/OD280 ratio measurement and/or elastic light scattering. In a preferred aspect the formulation comprises a histidine buffer pH 6.5 at 15 mM. In another preferred aspect the formulation comprises a histidine buffer pH 6.0 at 10 mM.

Apart from this and/or in addition, the formulation of the present invention shows only low to undetectable levels of fragmentation and/or degradation even during storage under one ore more of the above stress conditions. Fragmentation and degradation can be measured e.g. by SE-HPLC and/or RP-HPLC. For example, analysis in SE-HPLC of a formulation containing SEQ ID NO: 4 after storage for 10 weeks at a temperature of 37°C, showed the formation of some minor postpeaks, representing degradation products of SEQ ID NO: 4. For example, analysis by RP-HPLC of a formulation containing SEQ ID NO: 4 after storage for 10 weeks at a temperature of 37°C, showed the formation of some minor peaks at 8-9 minutes, representing degradation products. Preferably in the formulation of the invention, no more than 5%, no more than 4%, no more than 3%, no more than 2%, no more than 1%, no more than 0.5%, no more than 0.1%, no more than 0.05%, and most preferably no more than 0.01% of the polypeptides shows degradation and/or fragmentation after storage under one or more of the above stress conditions.

The techniques of static light scattering (SLS), tangential flow filtration (TFF), Fourier Transform Infrared Spectroscopy (FTIR), circular dichroism (CD), urea-induced protein unfolding techniques, intrinsic tryptophan fluorescence and/or 1-anilino-8-naphthalenesulfonic acid (ANS) protein binding can also be used to assess the physical properties and stability of polypeptides.

Apart from this and/or in addition, the formulation of the present invention shows very little to no loss of potency and/or biological activity of their polypeptides, even during storage under one ore more of the above stress conditions.

The potency and/or biological activity of a biological describes the specific ability or capacity of said biological to achieve a defined biological effect. The potency and biological activities of the polypeptides of the invention can be assessed by various assays including any suitable in vitro assay, cell-based assay, in vivo assay and/or animal model known per se, or any combination thereof, depending on the specific disease or disorder involved. Suitable in vitro assays will be clear to the skilled person, and for example include ELISA; FACS binding assay; Biacore; competition binding assay (AlphaScreen^{®}, Perkin Elmer, Massachusetts, USA; FMAT); TRAP assay (osteoclast differentiation assay; Rissanen et al. 2005, J. Bone Miner. Res. 20, Suppl. 1: S256); NF-kappaB reporter gene assay (Mizukami et al. 2002, Mol. Cell. Biol. 22: 992-1000). For example, SEQ ID NO: 4 interacts with RANKL and blocks the interaction of this ligand with RANK, thereby preventing signalization through this receptor. SEQ ID NO's: 1 to 3 interact with IL-6R and block the interaction of this receptor with IL-6. SEQ ID NO's: 5 and 6 interact with IL-23 and block the interaction of this ligand with its receptor. The potency of SEQ ID NO's: 1 to 6 for blocking the respective ligand/receptor interaction can be determined, e.g. by ELISA, Biacore, AlphaScreen^{®}.

For example, in one embodiment, Biacore kinetic analysis uses Surface Plasmon Resonance (SPR) technology to monitor macromolecular interactions in real time and is used to determine the binding on and off rates of polypeptides of the formulation of the invention to their target. Biacore kinetic analysis comprises analyzing the binding and dissociation of the target from chips with immobilized polypeptides of the invention on their surface. A typical Biacore kinetic study involves the injection of 250 µL of polypeptide reagent at varying concentration in HBS buffer containing 0.005% Tween 20 over a sensor chip surface, onto which has been immobilized the antigen. In the BIAcore 3000 system, the ligand is immobilized on carboxymethylated dextran over a gold surface, while the second partner (analyte) is captured as it flows over the immobilized ligand surface. The immobilized ligands are remarkably resilient and maintain their biological activity. The bound analytes can be stripped from the immobilized ligand without affecting its activity to allow many cycles of binding and regeneration on the same immobilized surface. Interaction is detected in real time via SPR and at high sensitivity. Because the same affinity may reflect different on-rates and off-rates, this instrument excels over most other affinity measuring methods in that it measures on-rates (ka) and off-rates (kd). Concentration determination experiments are also feasible.

The formulation of the present invention exhibits almost no loss in biological activities of the polypeptide during the prolonged storage under the conditions described above, as assessed by various immunological assays including, for example, enzyme-linked immunosorbent assay (ELISA) and Surface Plasmon Resonance to measure the ability of the polypeptide to specifically bind to an antigen. The polypeptides present in the formulation of the present invention retain, even under the above defined stress conditions (such as storage under certain temperature stress for defined periods) more than 80%, more than 85%, more than 90%, more than 95%, more than 98%, more than 99%, or more than 99.5% of their initial biological activities (e.g., the ability to bind to RANKL, IL-6R, IL-23 and/or HSA) of the polypeptides prior to the storage. In some embodiments, the polypeptides in the formulation of the invention retain under the above defined stress conditions at least 80%, at least 85%, at least 90%, at least 95%, at least 98%, at least 99%, or at least 99.5% of the biological activity (e.g., the ability to bind to RANKL, IL-6R, IL-23 and/or HSA) compared to the polypeptides present in a reference formulation prior to the storage.

In one embodiment, the polypeptides of the invention binds HSA. In the formulations of the present invention, at least 80% (preferably at least 85%, at least 90%, at least 95%, at least 98%, at least 99%, or at least 99.5%) of said polypeptides retain their binding activity to HSA under one or more of the above stress conditions (such as storage under certain temperature stress for defined periods) compared to the binding activity prior to the stress condition. Without being limiting, the binding of the polypeptides to HSA can be determined e.g. by ELISA and/or Biacore.

In another embodiment, the polypeptides of the invention bind RANKL. in the formulation of the present invention at least 80% (at least 85%, at least 90%, at least 95%, at least 98%, at least 99%, or at least 99.5%) of said polypeptides retain their binding activity to RANKL after storage under one or more of the above stress conditions compared to the binding activity prior to storage.

In another embodiment, the polypeptides of the invention bind IL-6R. In the formulation of the present invention at least 80% (at least 85%, at least 90%, at least 95%, at least 98%, at least 99%, or at least 99.5%) of said polypeptides retain their binding activity to IL-6R after storage under one or more of the above stress conditions compared to the binding activity prior to storage.

In another embodiment, the polypeptides of the invention bind IL-23. In the formulation of the present invention at least 80% (at least 85%, at least 90%, at least 95%, at least 98%, at least 99%, or at least 99.5%) of said polypeptides retain their binding activity to IL-23 after storage under one or more of the above stress conditions compared to the binding activity prior to storage.

In a preferred aspect, at least 80% (at least 85%, at least 90%, at least 95%, at least 98%, at least 99%, or at least 99.5%) of the polypeptides present in the formulation of the invention retain their binding activity to all of their targets (such as e.g. RANKL and HSA, IL-6R and HSA or IL-23 and HSA) after storage under one or more of the above stress conditions compared to the binding activity prior to storage.

Suitable animal models for determining the potency and/or biological activity of the polypeptides present in the formulations of the invention will be clear to the skilled person and will depend on the intended disease and/or disorder to be prevented and/or treated by the polypeptide of the invention. Suitable animal models for testing the potency and/or biological activity of SEQ ID NO's: 1 to 6 are e.g. described in WO 08/020079, WO 09/068627 and WO 08/142164.

Little to no loss of potency of the polypeptides of the invention has been observed in formulations with a histidine buffer and in formulations that comprise an excipient, preferably a saccharide, non-reducing sugar and/or polyol such as mannitol, sorbitol, trehalose or sucrose. Accordingly, the present invention relates to a formulation as claimed comprising an aqueous carrier and a polypeptide comprising one or more single variable domains, said formulation being formulated for administration to a human subject, wherein said formulation further comprises at least one of:
a) A histidine buffer at a concentration of 10mM to 100mM (preferably 10mM to 50mM, more preferably 10 to 20 mM, such as 15 mM or 10 mM);
b) An excipient, preferably a saccharide, non-reducing sugar and/or polyol such as mannitol, sorbitol, trehalose or sucrose at a concentration of 1% to 20% (preferably 2.5% to 15%, more preferably 5% to 10%, such as 5%, 7.5%, 8% or 10%),
wherein said formulation has an inorganic salt concentration of 150 mM or lower; and
wherein at least 80% (preferably at least 85%, at least 90%, at least 95%, at least 98%, at least 99%, or at least 99.5%) of the polypeptides retain their binding activity to at least one (preferably to all) of their targets under one or more of the above stress conditions (such as during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more)) compared to the binding activity prior to the stress conditions, said binding activity as measured by ELISA and/or Biacore. In a preferred aspect, the formulation comprises a histidine buffer at a concentration of 10mM to 100mM (preferably 10mM to 50mM, more preferably 10 to 20 mM, such as 15 mM or 10 mM) and an excipient, preferably a saccharide, non-reducing sugar and/or polyol such as mannitol, sorbitol, trehalose or sucrose at a concentration of 1% to 20% (preferably 2.5% to 15%, more preferably 5% to 10%, such as 5%, 7.5%, 8% or 10%), such as e.g. 15 mM histidine pH 6.5 and 8% sucrose; or 10 mM histidine pH 6.0 and 10% sucrose.

Accordingly, in the stable formulations of the present invention preferably:
- the polypeptide of the invention has a solubility of at least 20 mg/mL, at least 50 mg/mL, preferably at least 90 mg/mL, at least 120 mg/mL, at least 150 mg/mL or even 200 mg/mL or more) (e.g. as assessed by PEG exclusion method or by centrifugal ultrafiltration);
- the polypeptide of the invention has a melting temperature of at least 59°C or more (such as 59.5°C or more), preferably at least 60°C or more (such as 60.5°C or more), more preferably at least 61°C or more (such as 61.5°C or more) or at least 62°C or more (such as 62.5°C or more), most preferably at least 63°C or more (such as 63.5°C or more) (e.g. as assessed by TSA or DSC);
- less than 10% (more preferably less than 5%, even more preferably less than 3%, most preferably less than 1%) of the polypeptide of the invention forms pyroglutamate at the N-terminal glutamic acid (e.g. as assessed by RP-HPLC) during storage under one or more (of the above) stress conditions, such as e.g. at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- less than 10% (more preferably less than 5%, even more preferably less than 3%, most preferably less than 1%) of the polypeptide of the invention forms dimers (e.g. as assessed by SE-HPLC) during storage under one or more (of the above) stress conditions, such as e.g. at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more);
- at least 80% (at least 85%, at least 90%, at least 95%, at least 98%, at least 99%, or at least 99.5%) of the polypeptides of the invention retain their binding activity (e.g. as assessed by ELISA and/or Biacore) to at least one (preferably to all) of their targets after storage under one or more (of the above) stress conditions, such as e.g. at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more) compared to the binding activity prior to the stress condition; and/or
- the polypeptide of the invention is stable under one or more of the following mechanical stress conditions:
   ∘ shaking the formulation during 10s to 1 min;
   ∘ pushing the formulation through a needle (25G, preferably 26G, more preferably 27G, even more preferably 28G, most preferably 29G or more) with a syringe (the syringe used can be any commercially available syringe, such as e.g. a 1 mL, 2 mL, 3mL, 4 mL, 5 mL, 10 mL, 20 mL, 30 mL, 40 mL up to 50 mL syringe);
   o rotating for two days at 10 rpm; and/or
   o stirring for 1 hour at room temperature and/or 4-48 hours at 4°C at at least 10 rpm (such as 50 rpm, 100 rpm or more).

An example of a preferred formulation of the invention with these characteristics comprises 10 mg/mL of the polypeptide of the invention, 15 mM histidine pH 6.5, 8% sucrose and 0.01% Tween 80. Another example of a preferred formulation of the invention with these characteristics comprises 10 mg/mL of the polypeptide of the invention, 10 mM histidine pH 6.0, 10% sucrose and 0.005% Tween 80.

General methods for producing the single variable domains and/or polypeptides present in the formulation of the invention are known to the skilled person and/or have been described in the art. The single variable domains and/or polypeptides can be produced in any host known to the skilled person. For example but without being limiting, the single variable domains and/or polypeptides can be produced in prokaryotic hosts among which E. coli or eukaryotic hosts, for example eukaryotic host selected from insect cells, mammalian cells, and lower eukaryotic hosts comprising yeasts such as Pichia, Hansenula, Saccharomyces, Kluyveromyces, Candida, Torulopsis, Torulaspora, Schizosaccharomyces, Citeromyces, Pachysolen, Debaromyces, Metschunikowia, Rhodosporidium, Leucosporidium, Botryoascus, Sporidiobolus, Endomycopsis, preferably Pichia pastoris. Production of Nanobodies in prokaryotes and lower eukaryotic hosts such as *Pichia pastoris* has e.g. been described in WO 94/04678, WO 94/25591 and WO 08/142164. The contents of these applications are explicitly referred to in the connection with general culturing techniques and methods, including suitable media and conditions. The skilled person can also devise suitable genetic constructs for expression of the polypeptides of the invention in different hosts on the basis of the present application and common general knowledge. The present invention also relates to conditions and genetic constructs described in the art, for example the general culturing methods, plasmids, promoters and leader sequences described in WO 94/25591, WO 08/020079, Gasser et al. 2006 (Biotechnol. Bioeng. 94: 535); Gasser et al. 2007 (Appl. Environ. Microbiol. 73: 6499); or Damasceno et al. 2007 (Microbiol. Biotechnol. 74: 381).

More particularly, the method for the expression and/or production of a polypeptide comprising one or more single variable domains at least comprising the steps of:
a) cultivating a host or host cell (as defined herein) under conditions that are such that said host or host cell will multiply;
b) maintaining said host or host cell under conditions that are such that said host or host cell expresses and/or produces the polypeptide;
c) isolating and/or purifying the secreted polypeptide from the medium.

To produce/obtain expression of the polypeptide, the transformed host cell or transformed host organism may generally be kept, maintained and/or cultured under conditions such that the (desired) polypeptide is expressed/produced. Suitable conditions will be clear to the skilled person and will usually depend upon the host cell/host organism used, as well as on the regulatory elements that control the expression of the (relevant) nucleotide sequence. Again, reference is made to the handbooks and patent applications mentioned above.

Generally, suitable conditions may include the use of a suitable medium, the presence of a suitable source of food and/or suitable nutrients, the use of a suitable temperature, and optionally the presence of a suitable inducing factor or compound (e.g. when the nucleotide sequences of the invention are under the control of an inducible promoter); all of which may be selected by the skilled person. Again, under such conditions, the amino acid sequences of the invention may be expressed in a constitutive manner, in a transient manner, or only when suitably induced.

The polypeptide of the invention may then be isolated from the host cell/host organism and/or from the medium in which said host cell or host organism was cultivated, using protein isolation and/or purification techniques known per se, such as (preparative) chromatography and/or electrophoresis techniques, differential precipitation techniques, affinity techniques (e.g. using a specific, cleavable amino acid sequence fused with the polypeptide of the invention) and/or preparative immunological techniques (i.e. using antibodies against the polypeptide to be isolated).

In the present invention, the host can be removed from the culture medium by routine means. For example, the host can be removed by centrifugation or filtration. The solution obtained by removal of the host from the culture medium is also referred to as culture supernatant, or clarified culture supernatant. The polypeptides of the invention can be purified from the culture supernatant by standard methods. Standard methods include, but are not limited to chromatographic methods, including size exclusion chromatography, hydrophobic interaction chromatography, ion exchange chromatography, and affinity chromatography. These methods can be performed alone or in combination with other purification methods, e.g. precipitation or gel electrophoresis. The skilled person can devise suitable combinations of purification methods for the polypeptides of the invention on the basis of common general knowledge. For specific examples the art cited herein is referred to.

In one exemplary embodiment, the polypeptides of the invention can be purified from culture supernatant by a combination of affinity chromatography on Protein A, ion exchange chromatography and size exclusion chromatography. Reference to any "step of purification", includes, but is not limited to these particular methods.

More specifically, the polypeptides of the invention can be purified from culture supernatant using a process wherein the clarified supernatant (obtained by centrifugation) is captured on any combination of columns selected from (without being limiting) affinity chromatography resin such as Protein A resin, Cation Exchange Chromatography (CIEC) or an Anion Exchange Chromatography (AIEC) using for example Poros 50HS (POROS), SOURCE 30S or SOURCE 15S (GE Healthcare), SP Sepharose (GE Healthcare), Capto S (GE Healthcare), Capto MMC (GE Healthcare) or Poros 50HQ (POROS), SOURCE 30Q or SOURCE 15Q (GE Healthcare), Q Sepharose (GE Healthcare), Capto Q and DEAE Sepharose (GE Healthcare), Size exclusion chromatography (SE-HPLC) using for example Superdex 75 or Superdex 200 (GE Healthcare), hydrophobic interaction chromatography (HIC) using for example octyl, butyl sepharose or equivalents, optionally also including a tangential flow filtration (TFF) step. Any combination of columns can be used for the purification of the polypeptides of the invention, such as e.g. Protein A resin followed by Cation Exchange Chromatography or two Cation Exchange Chromatography steps.

The present invention also provides methods for preparing the stable formulations of the invention comprising the polypeptides of the invention. More particularly, the present invention provides methods for preparing stable formulations of such polypeptides, said methods comprising concentrating a fraction containing the purified polypeptide to the final polypeptide concentration using e.g. a semipermeable membrane with an appropriate molecular weight (MW) cutoff (e.g. a 5 kD cutoff for single variable domains; a 10 kD cutoff for bivalent polypeptides comprising two single variable domains; or a 15 kD cutoff for trivalent polypeptides comprising three single variable domains) and diafiltering and/or ultrafiltering to buffer exchange and further concentrate the polypeptide fraction into the formulation buffer using the same membrane. As extensively described above, the formulation buffer of the present invention may further comprise at least one of:
a) A buffer at a concentration of 10mM to 100mM selected from the group consisting of hepes pH 7.0-8.0, histidine pH 6.0-6.5, MES pH 6.0 and acetate pH 5.5-6.0;
b) An excipient at a concentration of 1% to 20%;
c) A surfactant at a concentration of 0.001% to 1% selected from Tween 80, Tween 20 or a poloxamer.

The pH of the formulation may range from about 5.5 to about 8.0, or may range from about 6.0 to about 7.5, preferably from about 6.2 to 7.5, from about 6.2 to 7.0, most preferably from about 6.5 to 7.0.

Surfactant (e.g. Tween 20, Tween 80 or poloxamer) will be added after the final diafiltration/ultrafiltration step at a concentration in the range of about 0% to 1%, preferably 0.001% to 0.1%, or 0.01% to 0.1% such as 0.001%, 0.005%, 0.01%, 0.02%, 0.05%, 0.08%, 0.1%, 0.5%, or 1% of the formulation, preferably 0.01% or 0.005%.

The formulation of the present invention may be sterilized by various sterilization methods, including sterile filtration, radiation, etc. In a specific embodiment, the polypeptide formulation is filter-sterilized with a presterilized 0.2 micron filter.

Preferably, the formulation of the present invention is supplied in a hermetically sealed container. Liquid formulations may comprise a quantity between 1 mL and 20 mL, preferably about 1mL, 2 mL, 3 mL, 4 mL, 5 mL, 6 mL, 7 mL, 8 mL, 9 mL, 10 mL, 15 mL, or 20 mL.

The formulation of the present invention can be prepared as unit dosage forms by preparing a vial containing an aliquot of the formulation for a one time use. For example, a unit dosage of liquid formulation per vial may contain 1 mL, 2 mL, 3 mL, 4 mL, 5 mL, 6 mL, 7 mL, 8 mL, 9 mL, 10 mL, 15 mL, or 20 mL of the formulation. The pharmaceutical unit dosage forms can be made suitable for any form of delivery of the polypeptide of the invention including (without being limiting) parenteral delivery, topical delivery, pulmonary delivery, intranasal delivery, vaginal delivery, enteral delivery, rectal delivery, oral delivery and/or sublingual delivery. In one aspect, the present invention relates to a pharmaceutical unit dosage form suitable for parenteral (such as e.g. intravenous, intraarterial, intramuscular, intracerebral, intraosseous, intradermal, intrathecal, intraperitoneal, subcutaneous, etc.) administration to a subject, comprising a formulation of the invention in a suitable container. In another preferred aspect, the subject is a human. In a specific embodiment, the formulations of the present invention are formulated into single dose vials as a sterile liquid that contains 10 mg/mL of one of SEQ ID NO's: 1 to 6, 15 mM histidine buffer at pH 6.5, 8% sucrose and 0.01% Tween 80. In another specific embodiment, the formulations of the present invention are formulated into single dose vials as a sterile liquid that contains 10 mg/mL of one of SEQ ID NO's: 1 to 6, 10 mM histidine buffer at pH 6.0, 10% sucrose and 0.005% Tween 80.

The amount of a formulation of the present invention which will be effective in the prevention, treatment and/or management of a certain disease or disorder can be determined by standard clinical techniques well-known in the art or described herein. The precise dose to be employed in the formulation will also depend on the route of administration, and should be decided according to the judgment of the practitioner and each patient's circumstances. Effective doses may be extrapolated from dose-response curves derived from in vitro or animal model test systems. For formulations of the polypeptide, encompassed by the invention, the dosage administered to a patient may further be calculated using the patient's weight in kilograms (kg) multiplied by the dose to be administered in mg/kg.

The required volume (in mL) to be given is then determined by taking the mg dose required divided by the concentration of the polypeptide formulation. The final calculated required volume will be obtained by pooling the contents of as many vials as are necessary into syringe(s) to administer the polypeptide formulation of the invention.

The present invention also encompasses a finished packaged and labelled pharmaceutical product. This article of manufacture or kit includes the appropriate unit dosage form in an appropriate vessel or container such as a glass vial or other container that is hermetically sealed. In one embodiment, the unit dosage form is suitable for intravenous, intramuscular, intranasal, oral, topical or subcutaneous delivery. Thus, the invention encompasses formulations, preferably sterile, suitable for each delivery route. In the case of dosage forms suitable for parenteral administration (such as e.g. subcutaneous administration) the active ingredient, e.g., polypeptide of the invention, is sterile and suitable for administration as a particulate free solution.

As with any pharmaceutical product, the packaging material and container are designed to protect the stability of the product during storage and shipment. Further, the products of the invention include instructions for use or other informational material that advise the physician, technician or patient on how to appropriately prevent or treat the disease or disorder in question. In other words, the article of manufacture includes instruction means indicating or suggesting a dosing regimen including, but not limited to, actual doses, monitoring procedures, and other monitoring information.

Specifically, the invention provides an article of manufacture comprising packaging material, such as a box, bottle, tube, vial, container, sprayer, insufflator, intravenous (i.v.) bag, envelope and the like; and at least one unit dosage form of a pharmaceutical agent contained within said packaging material, wherein said pharmaceutical agent comprises the formulation containing the polypeptide. The packaging material includes instruction means which indicate that said polypeptide can be used to prevent, treat and/or manage one or more symptoms associated with the disease or disorder by administering specific doses and using specific dosing regimens as described herein.

The invention also provides an article of manufacture comprising packaging material, such as a box, bottle, tube, vial, container, sprayer, insufflator, intravenous (i.v.) bag, envelope and the like; and at least one unit dosage form of each pharmaceutical agent contained within said packaging material, wherein one pharmaceutical agent comprises a formulation containing the polypeptide of interest, and wherein said packaging material includes instruction means which indicate that said agents can be used to prevent, treat and/or manage the disease or disorder by administering specific doses and using specific dosing regimens as described herein.

The invention also provides an article of manufacture comprising packaging material, such as a box, bottle, tube, vial, container, sprayer, insufflator, intravenous (i.v.) bag, envelope and the like; and at least one unit dosage form of each pharmaceutical agent contained within said packaging material, wherein one pharmaceutical agent comprises a formulation containing the polypeptide, and wherein said packaging material includes instruction means which indicate that said agents can be used to prevent, treat and/or manage one or more symptoms associated with the disease or disorder by administering specific doses and using specific dosing regimens as described herein.

The formulations, containers, pharmaceutical unit dosages and kits of the present invention may be administered to a subject to prevent, treat and/or manage a specific disease and/or disorder. In a specific aspect, the formulations, containers, pharmaceutical unit dosages and kits of the present invention are administered to a subject to prevent, treat and/or manage a disease and/or disorder associated with or characterized by aberrant expression and/or activity of a certain target or one or more symptoms thereof. In another specific aspect, the formulations, containers, pharmaceutical unit dosages and kits of the present invention are administered to a subject to prevent, treat and/or manage diseases and/or disorders associated with aberrant expression and/or activity of RANKL, diseases and/or disorders associated with overexpression of IL-6, or diseases and disorders associated with heterodimeric cytokines and their receptors or one or more symptoms thereof.

Diseases and disorders associated with aberrant expression and/or activity of RANKL are for example bone diseases and disorders, and include (without being limiting) the following diseases and disorders: Osteoporosis (McClung 2006, Current Osteoporosis Reports 4: 28-33), including, but not limited to, primary osteoporosis, endocrine osteoporosis (including, but not limited to, hyperthyroidism, hyperparathyroidism (Anandarajah and Schwarz 2006, J. Cell Biochem. 97: 226-232), Cushing's syndrome, and acromegaly), hereditary and congenital forms of osteoporosis (including, but not limited to, osteogenesis imperfecta, homocystinuria, Menkes' syndrome, Riley-Day syndrome), osteoporosis due to immobilization of extremities, glucocorticoid-induced osteoporosis (Locklin et al. 2001, Bone 28 (Suppl.): S80; McClung 2006, Current Osteoporosis Reports 4: 28-33; Anandarajah and Schwarz 2006, J. Cell Biochem. 97: 226-232) and post-menopausal osteoporosis (McClung 2006, Current Osteoporosis Reports 4: 28-33); (Juvenile or Familial) Paget's disease (Cundy et al. 2002, Hum. Mol. Genet. 11: 2119-2127; Whyte et al. 2002, J. Bone Miner. Res. 17: 26-29; Whyte et al. 2002, N. Engl. J. Med. 347: 175-184; Johnson-Pais et al. 2003, J. Bone Miner Res. 18: 376-380; Anandarajah and Schwarz 2006, J. Cell Biochem. 97: 226-232; Anandarajah and Schwarz 2006, J. Cell Biochem. 97: 226-232); Osteomyelitis, i.e., an infectious lesion in bone, leading to bone loss; Hypercalcemia (Anandarajah and Schwarz 2006, J. Cell Biochem. 97: 226-232), including, but not limited to, hypercalcemia resulting from solid tumors (including, but not limited to, breast, lung and kidney) and hematologic malignancies (including, but not limited to, multiple myeloma (Sordillo and Pearse 2003, Cancer 97 (3 Suppl): 802-812; Vanderkerken et al. 2003, Cancer Res. 63: 287-289), lymphoma and leukemia), idiopathic hypercalcemia, and hypercalcemia associated with hyperthyroidism and renal function disorders; Bone loss, including but not limited to, osteopenia following surgery, osteopenia induced by steroid administration, osteopenia associated with disorders of the small and large intestine, and osteopenia associated with chronic hepatic and renal diseases; Osteonecrosis, i.e., bone cell death, including, but not limited to, osteonecrosis associated with traumatic injury, osteonecrosis associated with Gaucher's disease, osteonecrosis associated with sickle cell anemia, osteonecrosis associated with systemic lupus erythematosus, osteonecrosis associated with rheumatoid arthritis, osteonecrosis associated with periodontal disease, osteonecrosis associated with osteolytic metastasis, and osteonecrosis associated with other condition; Bone loss associated with arthritic disorders such as psoriatic arthritis, rheumatoid arthritis, loss of cartilage and joint erosion associated with rheumatoid arthritis (Bezerra et al. 2005, Brazilian Journal of Medical and Biological Research 38: 161-170; Anandarajah and Schwarz 2006, J. Cell Biochem. 97: 226-232); Arthritis (Bezerra et al. 2005, Brazilian Journal of Medical and Biological Research 38: 161-170), including inflammatory arthritis (McClung 2006, Current Osteoporosis Reports 4: 28-33), Collagen-induced arthritis (Bezerra et al. 2005, Brazilian Journal of Medical and Biological Research 38: 161-170); Periprosthetic osteolysis (McClung 2006, Current Osteoporosis Reports 4: 28-33; Anandarajah and Schwarz 2006, J. Cell Biochem. 97: 226-232); Cancer-related bone disease (McClung 2006, Current Osteoporosis Reports 4: 28-33); Bone loss associated with aromatase inhibitor therapy (Lewiecki 2006, Expert Opin. Biol. Ther. 6: 1041-1050); Bone loss associated with androgen deprivation therapy (Lewiecki 2006, Expert Opin. Biol. Ther. 6: 1041-1050); Bone loss associated bone metastasis; Bone loss associated with diseases having immune system involvement, such as adult and childhood leukaemias, cancer metastasis, autoimmunity, and various viral infections (Holstead Jones et al. 2002, Ann. Rheum. Dis. 61 (Suppl II): ii32-ii39); Osteopenic disorders such as adult and childhood leukaemia (Oliveri et al. 1999, Henry Ford Hosp. Med. 39:45-48); chronic infections such as hepatitis C or HIV (Stellon et al. 1985, Gastroenterology 89: 1078-1083); autoimmune disorders such as diabetes mellitus (Piepkorn et al. 1997, Horm. Metab. Res. 29: 584-91), and lupus erythematosus (Seitz et al. 1985, Ann. Rheum Dis. 44: 438-445); allergic diseases such as asthma (Ebeling et al. 1998, J. Bone Min. Res. 13: 1283-1289); lytic bone metastases in multiple cancers such as breast cancer (Coleman 1998, Curr. Opin. Oncol. 10 (Suppl 1): 7-13); Prostate cancer; Myeloma bone disease (Anandarajah and Schwarz 2006, J. Cell Biochem. 97: 226-232); Periodontal infections (Anandarajah and Schwarz 2006, J. Cell Biochem. 97: 226-232); Expansile skeletal hyperphosphatasia (Anandarajah and Schwarz 2006, J. Cell Biochem. 97: 226-232); Bone metastases (Lewiecki 2006, Expert Opin. Biol. Ther. 6: 1041-1050; Anandarajah and Schwarz 2006, J. Cell Biochem. 97: 226-232).

Also provided by the present invention is the prevention and/or treatment with the formulations, containers, pharmaceutical unit dosages and kits of the invention of other diseases and disorders associated with an imbalance in the RANKL/RANK/OPG pathway. Such diseases and disorders include but are not limited to osteoporosis, inflammatory conditions, autoimmune conditions, asthma, rheumatoid arthritis, multiple sclerosis, Multiple myeloma (Sordillo and Pearse 2003, Cancer 97 (3 Suppl): 802-812; Vanderkerken et al. 2003, Cancer Res. 63: 287-289); Vascular diseases (Anandarajah and Schwarz 2006, J. Cell Biochem. 97: 226-232) and Cardiovascular disease (Lewiecki 2006, Expert Opin. Biol. Ther. 6: 1041-1050).

Also provided by the present invention is the prevention and/or treatment with the formulations, containers, pharmaceutical unit dosages and kits of the invention of diseases and disorders associated with osteopetrosis such as osteopetrosis tarda, osteopetrosis congenita and marble bone disease.

Disease and disorders caused by aberrant expression and or activity, such as excessive IL-6 production or signaling include sepsis (Starnes et al., 1999) and various forms of cancer such as multiple myeloma disease (MM), renal cell carcinoma (RCC), plasma cell leukaemia (Klein et al., 1991), lymphoma, B-lymphoproliferative disorder (BLPD) and prostate cancer. Non-limiting examples of other diseases caused by aberrant expression and/or activity, such as excessive IL-6 production or signalling include bone resorption (osteoporosis) (Roodman et al., 1992; Jilka et al., 1992), cachexia (Strassman et al., 1992), psoriasis, mesangial proliferative glomerulonephritis, Kaposi's sarcoma, AIDS-related lymphoma (Emilie et al., 1994), inflammatory diseases and disorder such as rheumatoid arthritis, systemic onset juvenile idiopathic arthritis, hypergammaglobulinemia (Grau et al., 1990), Crohn's disease, ulcerative colitis, systemic lupus erythematosus (SLE), multiple sclerosis, Castleman's disease, IgM gammopathy, cardiac myxoma, asthma (in particular allergic asthma) and autoimmune insulin-dependent diabetes mellitus (Campbell et al., 1991).

Diseases and disorders associated with heterodimeric cytokines and their receptors include inflammation and inflammatory disorders such as bowel diseases (colitis, Crohn's disease, IBD), infectious diseases, psoriasis, cancer, autoimmune diseases (such as MS), carcoidis, transplant rejection, cystic fibrosis, asthma, chronic obstructive pulmonary disease, rheumatoid arthritis, viral infection, common variable immunodeficiency.

The formulations, containers, pharmaceutical unit dosages and kits of the present invention may also be advantageously utilized in combination with one or more other therapies (e.g., one or more other prophylactic or therapeutic agents), preferably therapies useful in the prevention, treatment and/or management of the (same or another) disease or disorder. When one or more other therapies (e.g., prophylactic or therapeutic agents) are used, they can be administered separately, in any appropriate form and by any suitable route. Therapeutic or prophylactic agents include, but are not limited to, small molecules, synthetic drugs, peptides, polypeptides, proteins, nucieic acids (e.g., DNA and RNA nucleotides including, but not limited to, antisense nucleotide sequences, triple helices, RNAi, and nucleotide sequences encoding biologically active proteins, polypeptides or peptides), antibodies, other single variable domains, synthetic or natural inorganic molecules, mimetic agents, and synthetic or natural organic molecules. Any therapy (e.g., prophylactic or therapeutic agents) which is known to be useful, or which has been used or is currently being used for the prevention, treatment and/or management of one or more symptoms associated with a specific disease or disorder, can be used in combination with the formulations of the present invention in accordance with the invention described herein.

A formulation of the invention may be administered to a mammal, preferably a human, concurrently with one or more other therapies (e.g., one or more other prophylactic or therapeutic agents). The term "concurrently" is not limited to the administration of prophylactic or therapeutic agents/therapies at exactly the same time, but rather it is meant that the formulation of the invention and the other agent/therapy are administered to a mammal in a sequence and within a time interval such that the polypeptide contained in the formulation can act together with the other agent/therapy to provide an increased benefit than if they were administered otherwise. For example, the formulation of the invention and the one or more other prophylactic or therapeutic agents may be administered at the same time or sequentially in any order at different points in time; however, if not administered at the same time, they should be administered sufficiently close in time so as to provide the desired therapeutic or prophylactic effect.

When used in combination with other therapies (e.g., prophylactic and/or therapeutic agents), the formulations of the invention and the other therapy can act additively or synergistically. The invention contemplates administration of a formulation of the invention in combination with other therapies (e.g., prophylactic or therapeutic agents) by the same or different routes of administration, e.g., oral and parenteral.

Various delivery systems are known and can be used to administer the formulation of the present invention. Methods of administering formulations of the present invention include, but are not limited to, parenteral administration (e.g., intradermal, intramuscular, intraperitoneal, intravenous and, preferably subcutaneous), epidural administration, topical administration, and mucosal administration (e.g., intranasal and oral routes). In a specific embodiment, liquid formulations of the present invention are administered parenteral.

The invention will now be further described by means of the following non-limiting preferred aspects and examples:

### EXAMPLES

### Example 1: Formulation and stability studies with RANKL008a

### Example 1.1: Materials and methods used in the study

### 1.1.1 Single variable domains

RANKL008a (SEQ ID NO: 4; EVQLVESGGGLVQPGGSLRLSCAASGFTFSSYPMGWFRQAPGKGREFVS SITGSGGSTYYADSVKGRFTISRDNAKNTLYLQMNSLRPEDTAVYYCAAYIRPDTYLSRDYRKYDYWGQGTLVTVSS GGGGSGGGSEVQLVESGGGLVQPGNSLRLSCAASGFTFSSFGMSWVRQAPGKGLEWVSSISGSGSDTLYADSVKG RFTISRDNAKTTLYLQMNSLRPEDTAVYYCTIGGSLSRSSQGTLVTVSSGGGGSGGGSEVQLVESGGGLVQPGGSLR LSCAASGFTFSSYPMGWFRQAPGKGREFVSSITGSGGSTYYADSVKGRFTISRDNAKNTLYLQMNSLRPEDTAVYYC AAYIRPDTYLSRDYRKYDYWGQGTLVTVSS) has been described as SEQ ID NO: 759 in WO 2008/142164. RANKL008a is a trivalent bispecific Nanobody consisting of three humanized variable domains of a heavy-chain Ilama antibody, of which two identical subunits are specific for binding to RANKL while the remaining subunit binds to HSA. The subunits are fused head-to-tail with a G/S linker in the following format: RANKL13H5-9GS-Alb8-9GS-RANKL13H5.

RANKL008a was expressed in Pichia pastoris and purified on SP Sepharose as a capturing step and a Q filter as a polishing step or on SP Sepharose as a capturing step and Capto MMC as a polishing step or alternatively by using a ProtA capture step followed by and SP Sepharose polishing step. Concentration of the Nanobody and buffer switch to PBS, 10 mM phosphate + 100 mM NaCl, 10 mM phosphate + 5% mannitol or 10 mM phosphate + 115 mM NaCl or others buffers was done via UF/DF or by dialysis. A final filtration on a 0.22 µm filter was performed. The different batches of RANKL008a ranged in concentration from 143 to 62.8 mg/mL. Most batches were prepared at concentrations of about 60-85 mg/mL.

### 1.1.2 Other reagents

All reagents used in the study are standard chemical reagents of highest purity. The complete composition of D-PBS was 137 mM NaCl, 2.7 mM KCl, 10 mM Sodium Phosphate dibasic, 2 mM Potassium Phosphate monobasic.

### 1.1.3 Equipment and columns for testing

HPLC experiments were carried out on an Agilent 1200 series instrument from Agilent Technologies (Palo Alto, USA). The columns used were:
- RP-HPLC: Zorbax 300SB-C3 5-micron;
- SE-HPLC: TSKgel G2000SW_{XL} (Tosoh Bioscience, Japan);
- IEX-HPLC: Dionex ProPac _{W}CX-10 column;

The concentration of the purified RANKL008a batches was determined by spectroscopy at 280 nm either using a NanoDrop ND-1000 (Thermoscientific), or a Uvikon 943 Spectrophotometer (Kontron Instruments) or an Eppendorf Biophotometer 613.

Particle size distribution was measured on a PAMAS SVSS-C particle counter (PArtikelMess-und AnalyseSysteme GMBH).

For potency measurements a Biacore 3000 (GE Healthcare) was used. Elisa based potency assays were performed using standard laboratory equipment and plate reader instrumentation.

Osmolality measurement was done with an osmometer Model 3320 from Advanced instruments.

### 1.1.4 Inhibition ELISA for RANKL potency measurement

RANKL008a interacts with human (soluble) receptor activator of nuclear factor-kappa B ligand (RANKL) and blocks the interaction of this ligand with its human receptor activator of nuclear factor-kappaB (RANK), thereby preventing signalization through this receptor. The potency of RANKL008a was measured by an ELISA-based inhibition assay that allows assessment of the relative potency of the RANKL binding moieties of an unknown batch of RANKL008a relative to that of a reference batch.

For the reference, the control and the test samples, different dilutions of Nanobodies were prepared. These dilutions were pre-incubated with a constant amount of 5 ng/mL soluble RANKL and a constant amount of 200 ng/mL RANK-Fc. Subsequently, this mixture was transferred to a microtiter plate coated with a non-blocking anti-Fc Nanobody. After washing, residual bound RANKL was detected with a polyclonal biotinylated anti-human RANKL antibody, followed by hors radish peroxidase (HRP)-labeled streptavidin detection.

The relative potency of the test samples compared to the reference samples was analysed by use of PLA 2.0 Software (Stegmann Systems).

### 1.1.5 ELISA for HSA blinding

The relative potency of the HSA binding moiety in RANKL008a was measured by an ELISA. Briefly, HSA was coated onto a plastic multiwell Maxisorp ELISA plate by adsorption. After blocking excess binding sites on the plates with Superblock T20, a dilution series of references, control and test samples was applied on the plate. Bound Nanobody was subsequently detected using a bivalent anti-Nanobody Nanobody, directly conjugated to horseradish peroxidase (HRP).

The relative potency of the test samples compared to the reference samples was analysed by use of PLA 2.0 Software (Stegmann Systems).

### 1.1.6 Purity assay of RANKL008a by Size Exclusion High Performance Liquid Chromatography (SE-HPLC)

The SE-HPLC assay consisted of a pre-packed silica gel TSKgel G2000SW_{XL} column, a mobile phase consisting of KCl, NaCl and phosphate buffer pH 7.2 (D-PBS) and UV detection at 280 nm. The relative amount of the specific protein, variant, or impurities expressed as area %, was calculated by dividing the peak area corresponding to the specific protein or to any protein impurity by the total area of all integrated peaks.

### 1.1.7 Purity assay of RANKL008a by Reverses Phase High Performance Liquid Chromatography (RP-HPLC)

In the RP-HPLC assay a Zorbax 300SB-C3 column (Agilent Technologies, Palo Alto, US) was used. The relative amount of a specific protein impurity was determined by measuring the light absorbance of the components eluting from the RP-HPLC column. The relative amount of the specific protein, variant, or impurities expressed as area %, was calculated by dividing the peak area corresponding to the specific protein or to any protein impurity by the total area of all integrated peaks.

### 1.1.8 Purity assay of RANKL008a by Ion Exchange High Performance Liquid Chromatography (IEX-HPLC)

The IEX-HPLC assay combined the use of a pre-packed Dionex ProPac WCX-10 weak cation exchange column, a mobile phase consisting of citrate buffer pH 5.5 and UV detection at 280 nm. After loading the protein(s) on the column, bound materials were eluted by a sodium chloride gradient. The relative amount of the specific protein, variant, or impurities expressed as area %, was calculated by dividing the peak area corresponding to the specific protein or to any protein impurity by the total area of all integrated peaks.

### 1.1.9 Relative potency determination on Biacore

RANKL or HSA was immobilized on the Biacore chip (amine coupling using the Biacore amine coupling kit). After a preconditioning step of 5 injections of RANKL008a, all samples were diluted to 2.5 nM in triplicate and analyzed on the chip. Slopes were determined using the general fit method and the linear fit model (BIAevaluation software). To determine the initial binding rate (IBR), the slope between 5s and 30s was selected. The values of these slopes were transferred in excel and the percentage activity/potency compared to the RANKL008a reference material was determined. Biacore potency is thus expressed as relative potency compared to the reference materials.

### Example 1.2: Stability of the Nanobody in different buffers after different freeze/thaw cycles

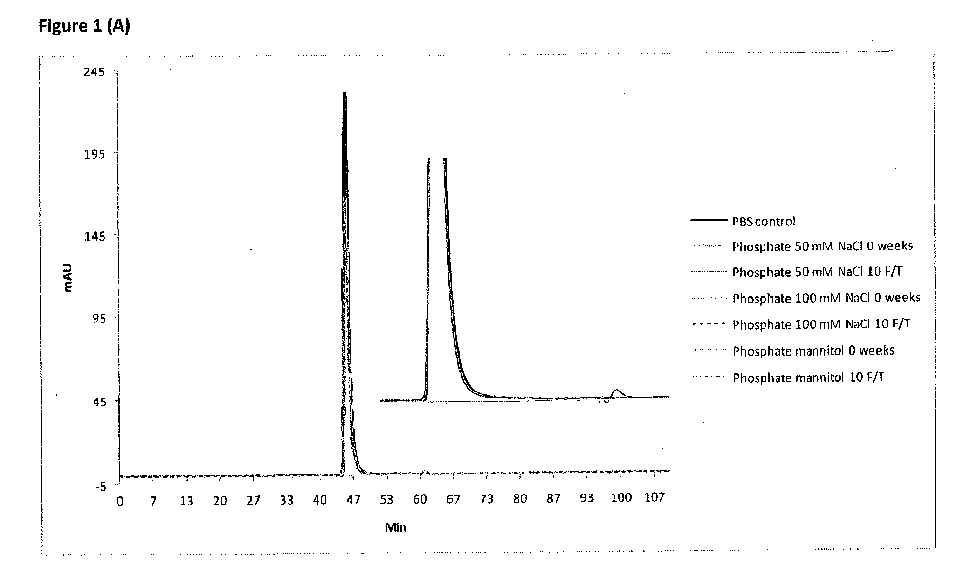

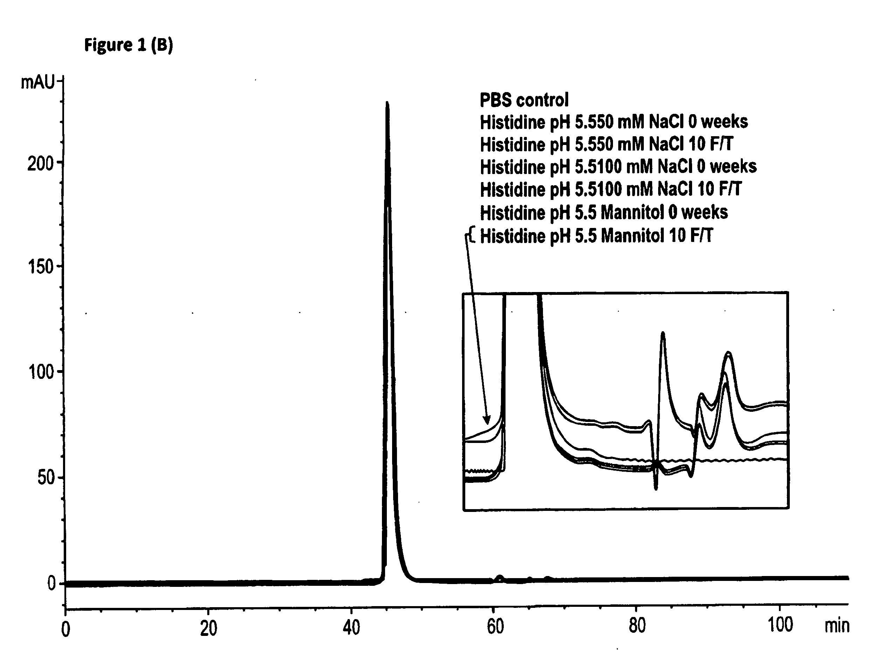

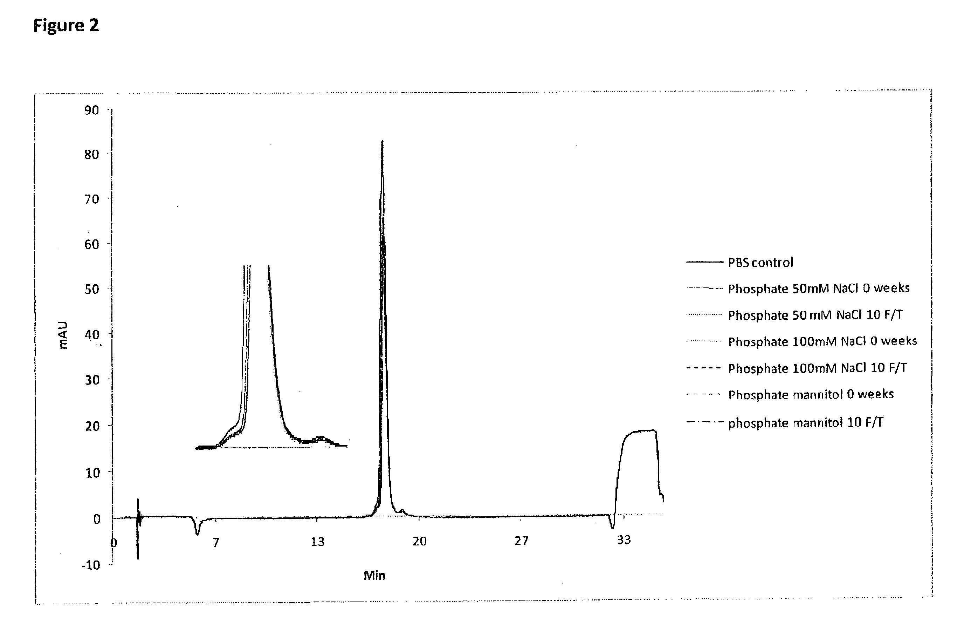

A freeze/thaw stability study was performed to determine the effect of repetitive freeze and thawing on the recovery, physical stability and chemical stability of RANKL008a. Aliquots of batch RANKL008a formulated at ∼60-85 mg/mL in the buffers 1-12 given in Table 1 were subjected to 10 freeze/thaw (F/T) cycles at -20°C. One F/T cycle is defined by freezing the sample for 1 hour in a freezer at -20°C followed by thawing at room temperature for 30 minutes. The stressed samples were compared with reference material (stored at 4°C) using SE-HPLC (Figure 1 (A); representative figure of the experiments performed in phosphate buffer), RP-HPLC (Figure 2; representative figure of the experiments performed in phosphate buffer) and the ELISA potency assays (Table 2). All other data of the freeze thaw experiments demonstrate similar patterns as given in Figures 1 and 2 (except Figure 1 (B) see below).

Subjecting RANKL008a to 10 F/T cycles had no significant effect on its stability: the SE-HPLC- and RP-HPLC profiles were comparable between the reference batches and material subjected to multiple freeze/thaw cycles. There was no decrease in the total surface area and no new peaks were being formed, except in 20 mM L-histidine, pH 5.5 + 10% mannitol and 20 mM L-histidine, pH 6 + 10% mannitol, where the main peak had a very small shoulder in the SE-HPLC chromatograms (Figure 1(B) showing the data at pH 5.5 and where the minor shoulder on the main peak is indicated by an arrow). In Table 3 and Table 4 data are included for the integration of the different peaks (expressed as % surface area) in the SE-HPLC and RP-HPLC analysis respectively. These data demonstrate that no changes occur in the profiles after 10 freeze thaw cycles.

Analysis by the ELISA potency assays indicated no loss of activity after repetitive freezing and thawing in all formulations, except in 20 mM Histidine, pH 5.5 + 10% mannitol where there appeared to be a lower RANKL and HSA binding potency.

### Example 1.3: Stability of the Nanobody in different buffers when stored at 37 °C up to 10 weeks

RANKL008a was formulated in different buffers at ∼60-85 mg/mL (buffers 1-12 given in Table 1). The stability of the different samples was assessed in accelerated stress conditions at 37°C±3°C. Samples were taken after 2, 3, 5 and 10 weeks storage at this temperature and were analyzed using SE-HPLC, RP-HPLC and IEX-HPLC. Biacore was performed on the samples stored for 10 weeks to evaluate loss in potency.

### 1.3.1 SE-HPLC analysis

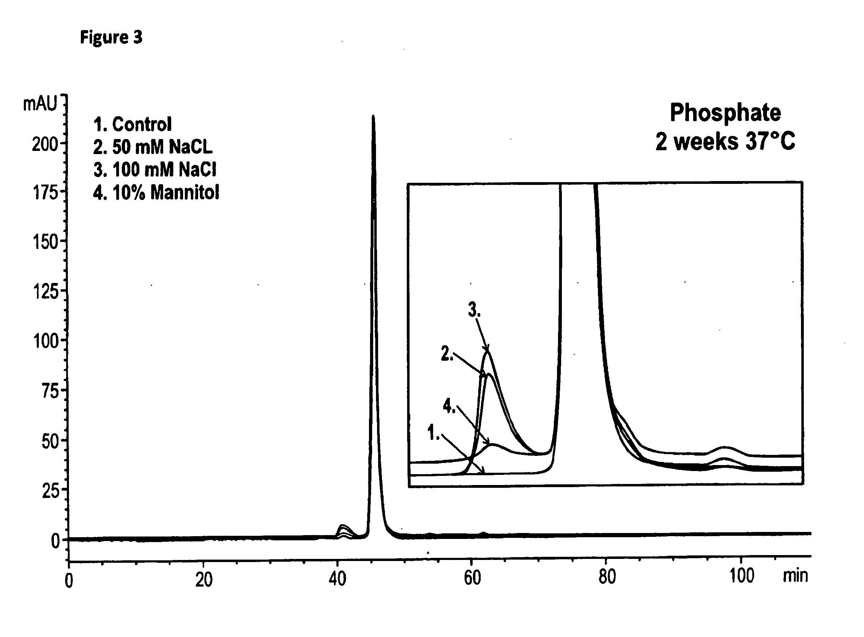

The results of the analysis of a sample by SE-HPLC is given in Figure 3 where an example is shown for the sample stored during two weeks at 37°C in the presence of 50 or 100 mM salt or 10% mannitol phosphate buffer. Storage at 37°C resulted in the formation of a clear prepeak eluting at about 40 minutes and some minor postpeaks close to the main peak; at the 60 minutes elution time (see insert in Figure 3) some degradation fragments could be observed. In Table 3 the integration data for all samples analysed is summarized for the different peaks observed (except peaks after 60 minutes elution time). The peak area of the prepeak increased over time but was reduced by the addition of mannitol to the buffer (Table 3). The postpeaks after 60 minutes elution time corresponded to degradation products (due to remaining proteolytic activity in sample). The relative area (%) of these peaks increased only slightly, implying that degradation was restricted to a minimum.

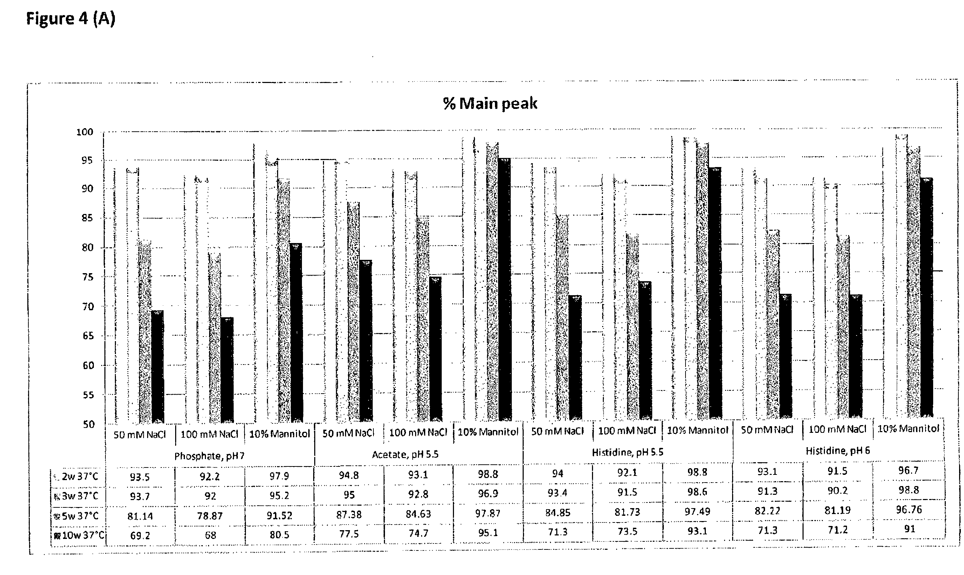

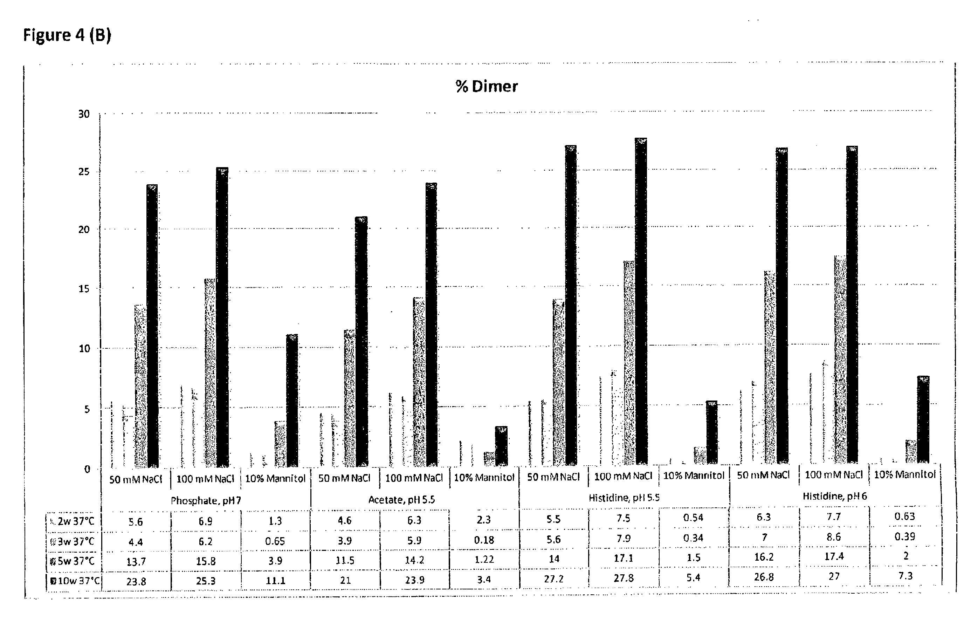

The prepeak represented the dimeric form of RANKL008a. The peak surface area of the prepeak increased with storage time (Table 3) and was accompanied by a concomitant decrease in surface area of the main peak (Table 3). The propensity to form dimers was significantly lower in the formulations containing 10% mannitol, which seemed to have a positive effect in suppressing the dimerization process. Note the significant lower amounts of dimers observed in the Acetate and Histidine buffers (pH 5.5) containing 10% mannitol (Table 1 and Figure 4). Figure 4(A) summarizes the % surface area for the main peak in the different buffers and at different time points when stored at 37°C. Figure 4(B) summarizes the data for the % prepeak (dimer).

### 1.3.2 RP-HPLC analysis

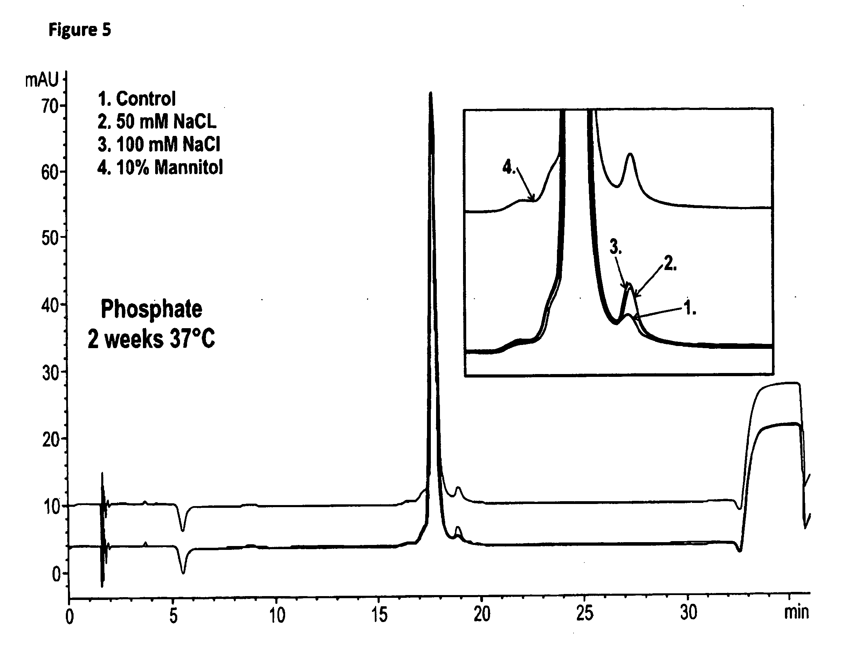

A representative RP-HPLC is given in Figure 5 where the RP-HPLC chromatogram of a RANKL008a sample stored in phosphate buffer with different concentrations salt or 10% mannitol is represented. The RP-HPLC profiles of RANKL008a formulated in the 12 different buffers were comparable to this Figure. In Table 4 integration data for the different peaks detected is summarized. In Figure 5 the inset shows a zoom on the main peak where the two pre-peaks can be discriminated, while the post peak that is fully base line resolved from the main peak is the pyro-glutamate variant of the RANKL008a where the N-terminal glutamic acid has been converted to the pyroglutamate form.

There were two differences between the RP-HPLC profiles of the reference batch and the storage samples. Firstly, the pyroglutamate peak increased with increased incubation time and was more apparent in phosphate buffer at pH 7 than at pH 5.5 or pH 6. Secondly, a prepeak was being formed in function of storage time. The surface area of this peak was higher in the phosphate buffer.

There were no differences in the RP-HPLC profiles of the samples without or with mannitol.

### 1.3.3 IEX-HPLC analysis

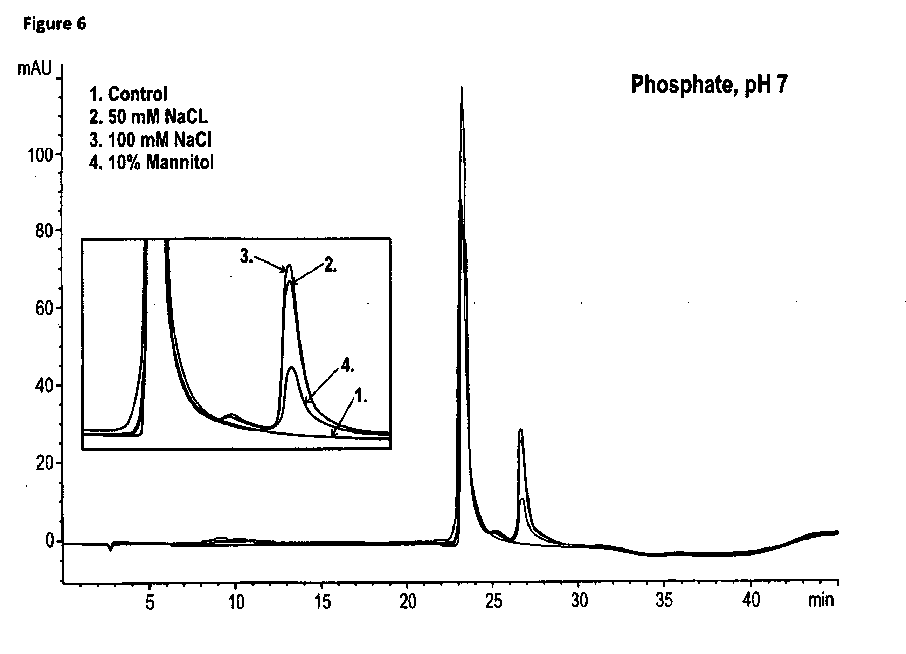

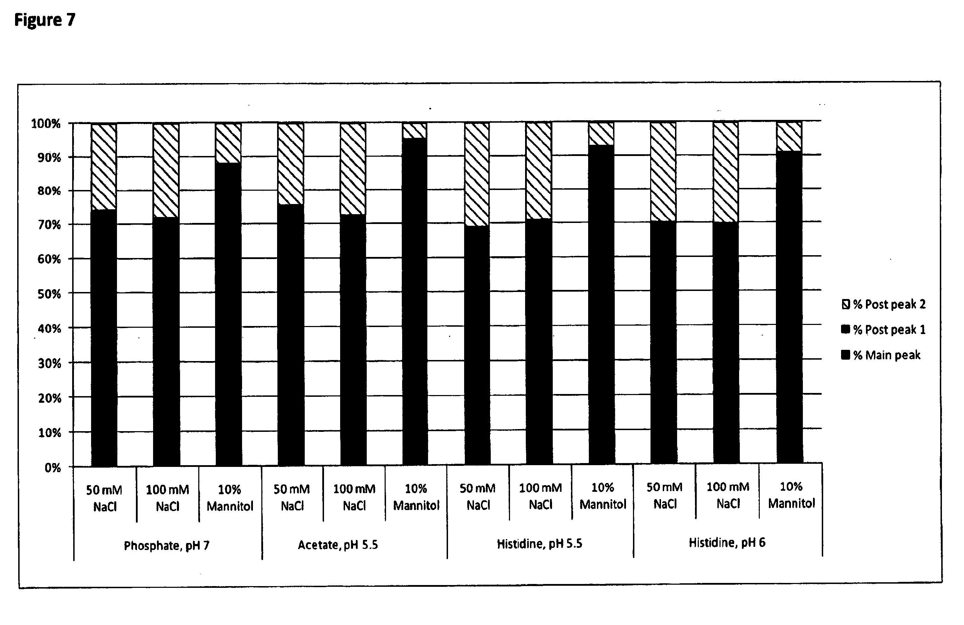

A representative IEX-HPLC chromatogram of the RANKL008a stored for 2 weeks in phosphate buffer with different salt concentrations or 10% mannitol is depicted in Figure 6. The inset shows a zoom in on the main peak where a minor post peak 1 and a more significant post-peak 2 is observed. Results of the analysis of the different samples by IEX-HPLC are given in Table 5 and Figure 7.

The first postpeak constituted maximally 4.5% of the total peak surface area. The surface area of this peak was the highest in phosphate buffer and the lowest or even absent in the mannitol-containing buffers. The peak area of the second peak on the other hand was substantial, yet significantly lower in the buffers containing 10% mannitol (Figure 7). The material eluting in the post-peak was collected by fraction collection and re-chromatographed on the SE-HPLC column described above. This post-peak 2 elutes in the SE-HPLC chromatogram at the dimer position demonstrating that i) this dimer does not dissociate under these conditions and that ii) this dimer elutes later on the IEX-HPLC column. Therefore we can conclude that the post-peak 2 is the dimerized form of the RANKL008a.

### 1.3.4 Biacore potency analysis of the RANKL008a stored at 37°C

The RANKL and HSA binding of RANKL008a in stability samples stored for 10 weeks at 37 °C was compared with the activity of the unstressed reference batch using Biacore analysis. The relative potencies are given in Table 6 and are expressed as % activity compared to reference batch.

After 10 weeks of storage at 37°C the relative potency of RANKL008a for binding RANKL had dropped to 70-80% in the different buffers (Table 6). In histidine, pH 6 + 10% mannitol, the activity remained the highest (87.4%). The higher the NaCl concentration in the buffer, the lower the relative potency in the sample (compare the values obtained in buffers with 50 mM NaCl and 100 mM NaCl in Table 6).

The relative potency for HSA binding had dropped more compared to the activity for RANKL binding after 10 weeks storage at 37°C. This decrease in activity however was less significant in the mannitol-containing buffers than in the NaCl-containing buffers. As observed for RANKL binding, the percentage activity on HSA decreased with increasing concentrations of NaCl in the different buffers.

### Example 1.4: Osmolality measurement for the Nanobodies in the different buffers

Osmolality measurements were performed on the different formulations used in the stability studies:

| | |
|---|---|
| RANKL008a in 10 mM Phosphate/10% mannitol: | 635 mOsm/kg |
| RANKL008a in 10 mM Acetate/10% mannitol: | 643 mOsm/kg |
| RANKL008a in 20 mM L-histidine pH 5.5/10% mannitol: | 712 mOsm/kg |
| RANKL008a in 20 mM L-histidine pH 6.0/10% mannitol: | 667 mOsm/kg |
| RANKL008a in 10mM acetate buffer pH 5.5/ 100mM NaCl: | 272 mOsm/kg |

RANKL008a in 10mM phosphate/5% mannitol: 389 mOsm/kg
Formulations containing 10% mannitol were hypertonic.

### Example 1.5: Stability of the Nanobodies during mechanical stress

Mechanical stress experiments were performed on RANKL008a (62.2 mg/mL) in 10 mM phosphate buffer pH 7.0 with 115 mM NaCl. The RANKL008a sample was diluted (in the 10 mM phosphate buffer pH 7.0 with 115 mM) or undiluted with and without 0.01% Tween 80. The samples were shaken, stirred, rotated and pushed through a needle with a syringe (the syringe used can be any commercially available syringe, such as e.g. a 1 mL, 2 mL, 3mL, 4 mL, 5 mL, 10 mL, 20 mL, 30 mL, 40 mL up to 50 mL syringe) as follows:
- Diluted to 5 mg/mL or undiluted and shaken (10s - 1 min);
- Pushed through a syringe (3mL) with needle 25G (undiluted) (10x);
- Rotated (10 rpm) on an end over end mixer for 2 days at room temperature (undiluted)
- Stirred 1 hour at room temperature for 2 days at 5°C (diluted to 5 mg/mL)
The different samples were compared visually for any differences in appearance.

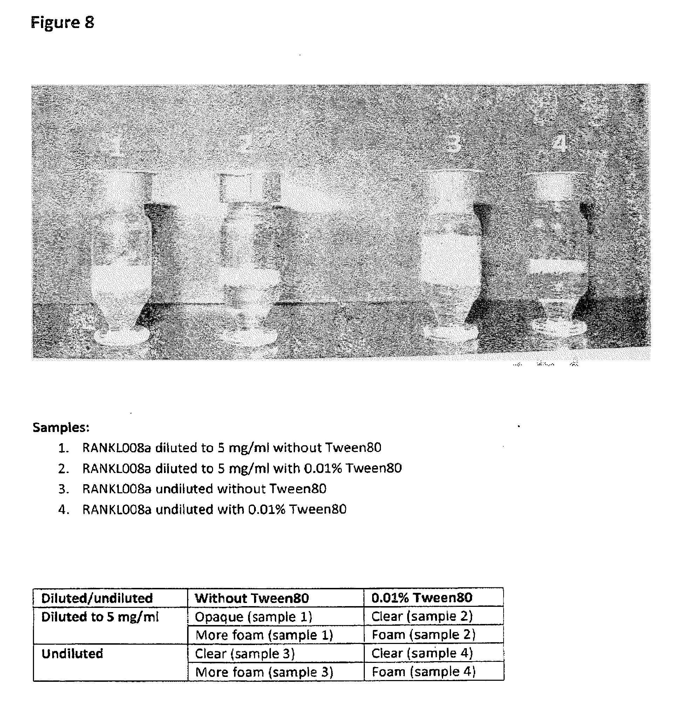

Strong shaking for a short time (10s) caused strong foaming of the samples in the absence of Tween 80, the diluted sample got very opaque (Figure 8) while this was less pronounced for the undiluted sample. In the presence of the Tween 80 this opacity was not observed.

The undiluted RANKL008a sample with and without 0.01% Tween 80 was pushed 10 times through a needle (25G) with a 3 mL syringe. The sample without Tween 80 got opaque, there was also formation of foam and tiny air bubbles were visible when tapping the vial, in the vial with 0.01% Tween 80 opacity was limited.

The undiluted RANKL008a sample with or without 0.01% Tween 80 was rotated for 2 days at 10 rpm. Both samples stayed clear.

The diluted (to 5 mg/mL) RANKL008a sample with or without 0.01% Tween 80 was stirred for 1 hour at room temperature and further for 2 days at 5°C. The visual observations are as follows: stirring during 1h at room temperature induced an opacity which was not observed in the presence of 0.01% Tween 80. After stirring 1 hour at room temperature, the sample without Tween 80 was slightly opaque while the sample with Tween 80 stayed clear. After 2 days stirring at 5°C, both sample were opaque but the opacity in the sample without Tween 80 was higher.

With the addition of 0.01% Tween 80, the RANKL008a sample was less or not opaque after mechanical stress and there was less foam, formation. This indicates that the sample is less susceptible to denaturation at the air-water interface if Tween 80 is added.

### Example 1.6: Syringeability of the different Nanobody formulations

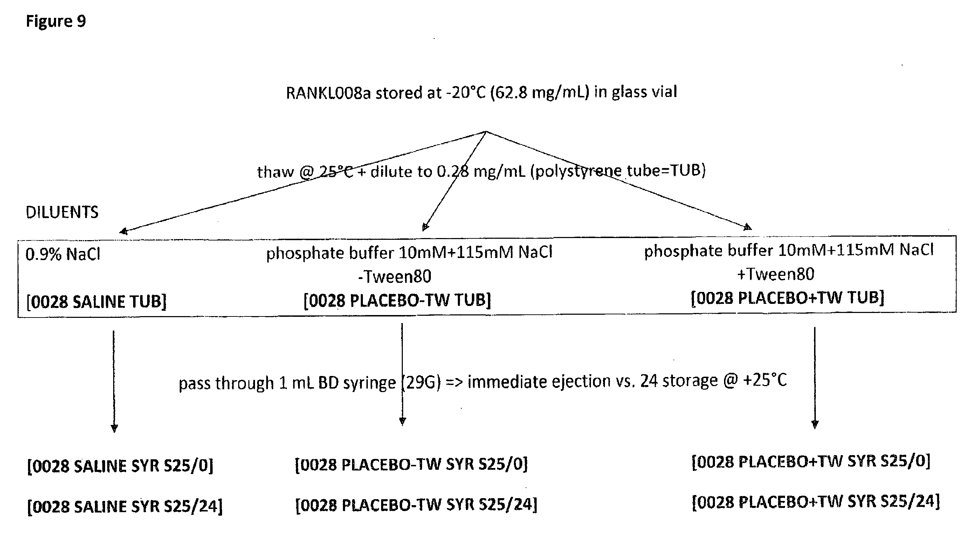

The effect of using different diluents - i.e. saline solution, phosphate buffer without Tween 80 or phosphate buffer with Tween 80 - on content, visual appearance and potency of RANKL008a at low concentration (0.28 mg/mL) was determined after passage through different syringes and needles. RANKL008a was diluted in different diluents followed by passage or 24h storage in syringes (Becton Dickinson) (Figure 9). Figure 9 contains the legends to the different samples generated during this experiment where the following codes are applied:
S25/0: storage at 25°C for 0 minute
S25/24: storage at 25°C for 24h
-TW: buffer minus Tween 80
+TW: buffer + Tween 80
PLACEBO refers to the following buffer: 10 mM Na₂HPO₄ pH 7.0 + 115 mM NaCl
TUB: sample stored in a polystyrene tube

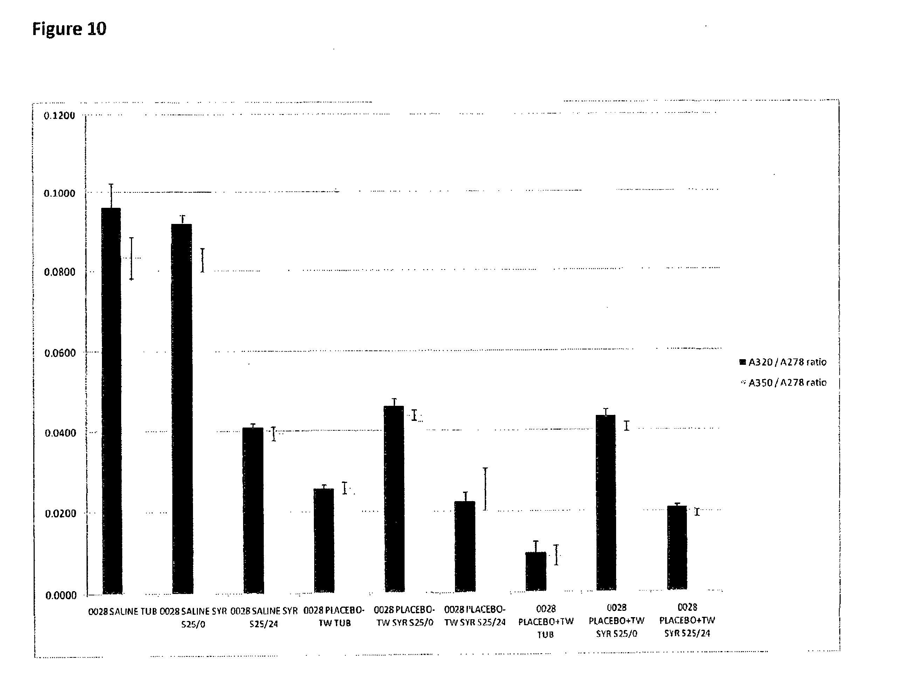

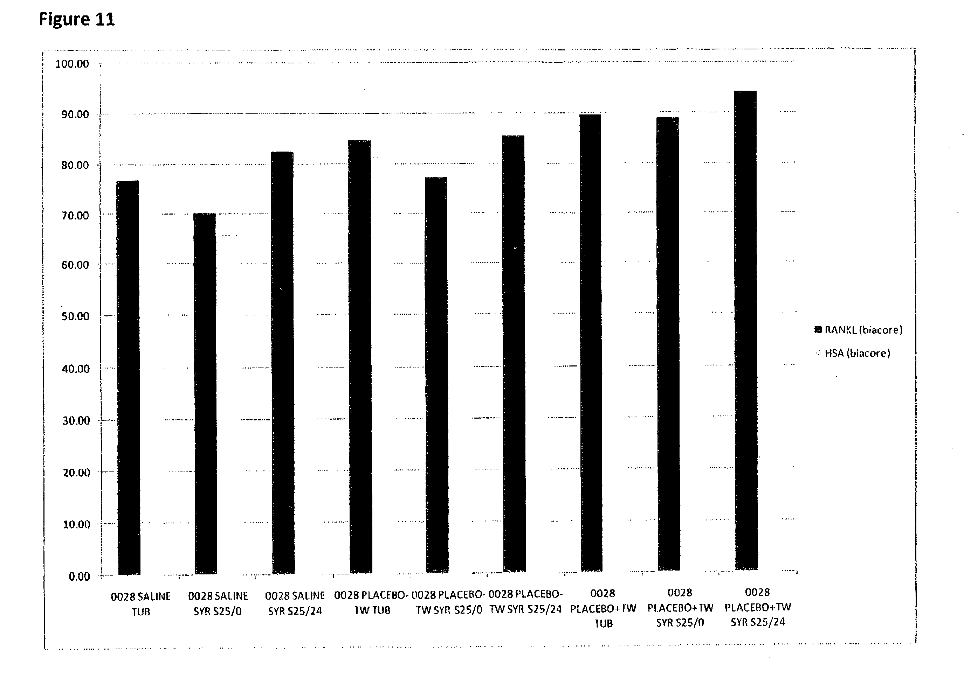

Visual inspection and content determination of RANKL008a after dilution in different diluents and passage/storage in syringes is given in Table 7. Data on turbidity measurement at 320 and 350 nm are given in Figure 10. The relative HSA and RANKL potency of RANKL008a after dilution in different diluents and passage/storage in syringes is shown in Figure 11.

Passage through a syringe slightly increased turbidity when using 10 mM Na₂HPO₄, 115 mM NaCl (pH 7.0). Dilution in saline solution caused a drop in RANKL/HSA binding activity of 18-34.0%. A similar effect was observed using 10 mM Na₂HPO₄, 115 mM NaCl (pH 7.0) without Tween 80, i.e. a drop of 15-27%. In contrast, dilution in 10 mM Na₂HPO₄, 115 mM NaCl (pH 7.0) with Tween 80 did not appear to have a dramatic effect confirming the beneficial role of Tween 80 in the buffer.

### Example 1.7: Stability of Nanobody formulations during syringe passage with different needle size

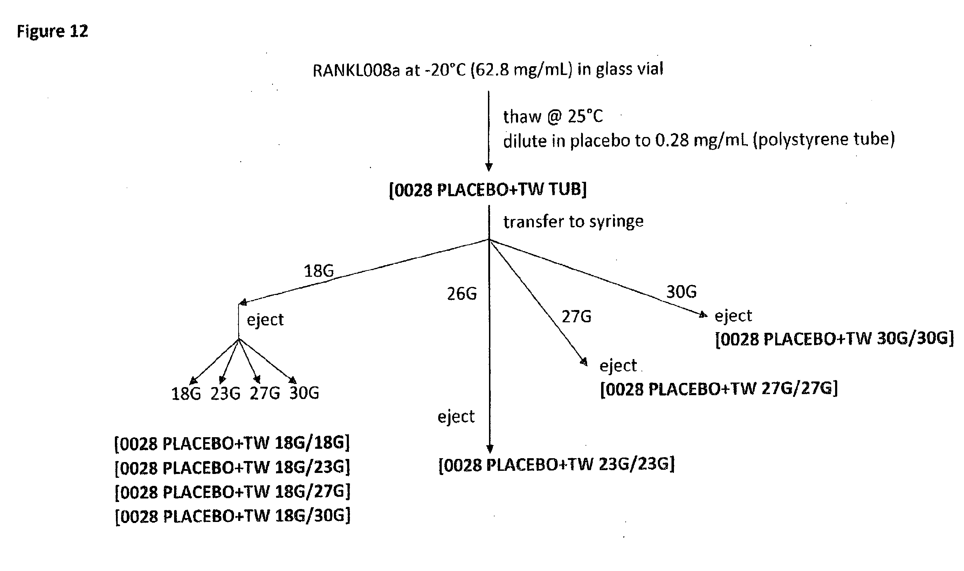

The effect of syringe passage on visual appearance of the RANKL008a using different needle sizes and needle size combinations was evaluated. RANKL008a was diluted in an Eppendorf tube (TUB) in 10 mM Na₂HPO₄, 115 mM NaCl, 0.01% Tween 80 (v:v), (pH 7.0) to a final concentration of 0.28 mg/mL followed by single passage through a 1 mL Becton Dickinson syringe equipped with different needles (i.e. Terumo 18G, 23G, 27G and 30G) (Figure 12). In this Figure and Table 8 the following codes apply:
+TW: buffer + Tween 80
PLACEBO refers to the following buffer: 10 mM Na₂HPO₄ pH 7.0 + 115 mM NaCl
TUB: sample stored in a polystyrene tube
18G/18G: sample drawn up with a 18G needle and expelled through a 18G needle
18G/27G: drawn up with a 18G needle and expelled through a 27G needle
All other coding is similar to the two examples given above.

Turbidity was determined by visual inspection and by measurement of the absorption at wavelengths of 320 nm, 340 nm, 350 nm and/or 500 nm, and determining the ratio of the obtained value over the absorption at A278 nm (mostly 320/278 and 350/278). A ratio of >0.05 was considered significant. Visual inspection, content and turbidity of RANKL008a before (TUB) and after passage through syringes with different needle size is shown in Table 8.

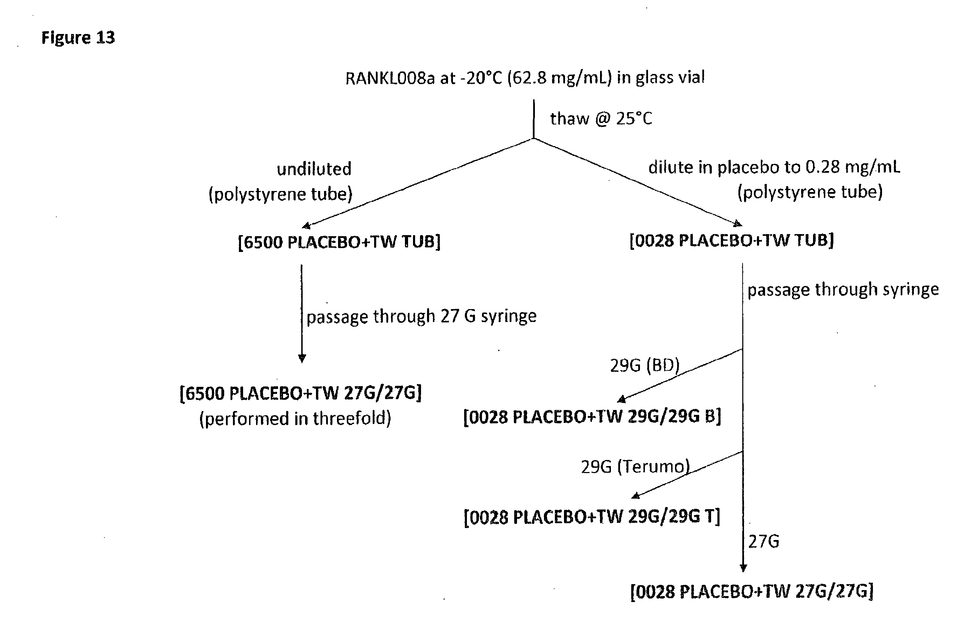

In a further experiment, RANKL008a was subjected to single passage through a 1 mL syringe equipped with different needle sizes (i.e. 27G and 29G) both undiluted (65 mg/mL) and diluted in 10 mM Na₂HPO₄, 115 mM NaCl, 0.01% Tween 80 (v:v), (pH 7.0) to a final concentration of 0.28 mg/mL (Figure 13). Visual inspection, content and turbidity of RANKL008a before (TUB) and after passage through syringes with different needle size is shown in Table 9.

In Table 9 and Figure 13 the following codes are used:
+TW: buffer + Tween 80
PLACEBO refers to the following buffer: 10 mM Na₂HPO₄ pH 7.0 + 115 mM NaCl
TUB: sample stored in a polystyrene tube
27G/27G: sample drawn up with a 27G needle and expelled through a 27G needle
29G/29G: drawn up with a 29G needle and expelled through a 29G needle
T: Terumo needle, B Becton Dickinson needle
0028 refers to concentration at 0.28 mg/mL, 6500 to about 65 mg/mL

Different combinations of needle sizes did not have a significant effect on RANKL008a recovery or sample turbidity both at low (0.28 mg/mL) and high (62.8 mg/mL) concentration (up to gauge sizes 29 and 27 respectively). In both experiments turbidity values were low (<0.05).

### Example 2: Formulation and stability studies with Nanobodies that bind IL-6R

### Example 2.1: Materials and methods used in the study

### 2.1.1 Single variable domains

Three Nanobodies that were used in this study have been described in PCT application No. PCT/EP2010/054764 to Ablynx N.V.. IL6R304 is a bispecific Nanobody consisting of two humanized variable domains of a heavy-chain Ilama antibody, one binding to IL-6R, the other one (Alb8) binding to HSA. The trivalent bispecific Nanobodies IL6R305 and IL6R306 consist of two identical subunits that are specific for IL-6R while the third subunit binds to HSA. The build-up of the subunits differs in IL6R305 and IL6R306 (see Table C-27 of PCT/EP2010/054764). The subunits in all three Nanobodies are fused head to-tail with a 9G/S linker. The sequences and characteristics of the three Nanobodies are given in Table 10.

The Nanobodies were expressed in Pichia pastoris. Concentration of the Nanobody and buffer switch to PBS or other formulation buffer was done via UF/DF (Sartorius Hydrosart Sartocon Slice 200, 10 kDa). A final filtration was carried out at 0.22 µm. An overview of the different IL-6R Nanobody batches is given in Table 11.

Unstressed samples in PBS or other formulations were used as reference material for analyzing the storage stability samples.

### 2.1.2 Other reagents

Reagents used in the study are given in Table 12. The complete composition of D-PBS was 137 mM NaCl, 2.7 mM KCl, 10 mM Sodium Phosphate dibasic, 2 mM Potassium Phosphate monobasic.

### 2.1.3 Equipment and methods for measurements

HPLC experiments were carried out on an Agilent 1200 series instrument from Agilent Technologies (Palo Alto, USA) or on a Dionex Ultimate 3000 instrument. The columns used were:
- RP-HPLC: Zorbax 300SB-C3 5-micron, 4.6 X 150 mm (Agilent, Cat. No. 883995-909) or Zorbax 300SB-C8 5-micron, 4.6 X 150 mm (Agilent, Cat. No. 883995-906);
- SE-HPLC: Phenomenex BioSep SEC S2000 (00H-2145-KD)

Concentration determinations of the Nanobodies were done with NanoDrop ND-1000 (Thermoscientific), with a Uvikon 943 Spectrophotometer (Kontron Instruments) or with an Eppendorf Biophotometer 6131 at 280 nm.

Particle size distribution was measured on a PAMAS SVSS-C particle counter (PArtikelMess- und AnalyseSysteme GMBH).

Osmolality measurement was done with an osmometer Model 3320 from Advanced instruments.

For measurement of binding activity Biacore 3000 (GE Healthcare) was used.

The thermal shift assay (TSA) was performed on a LightCycler480 Q-PCR device (Roche).

For determination of the Tm, an automated VP-capillary Differential Scanning Calorimeter (DSC, MicroCal) was used.

### 2.1.4 Purity assay of the IL-6R Nanobodies by Size Exclusion High Performance Liquid

### Chromatography (SE-HPLC)

The SE-HPLC assay consisted of a pre-packed Phenomenex BioSep SEC S2000 column, a mobile phase consisting of KCl, NaCl and phosphate buffer pH 7.2 (D-PBS) and UV detection at 280 nm. The relative amount of specific protein impurity was expressed as area %, and was calculated by dividing the peak area corresponding to the protein impurity by the total integrated area.

The method can resolve and quantify the relative amounts of intact material and product related impurities such as aggregates and degradation fragments.

### 2.1.5 Purity assay of the IL-6R Nanobodies by Reverses Phase High Performance Liquid

### Chromatography (RP-HPLC)

In the RP-HPLC assay a Zorbax 300SB-C3 or Zorbax 300SB-C8 column (Agilent Technologies, Palo Alto, US) at elevated temperatures were used. With the C3 column, mobile phase A consisted of 0.1% TFA and mobile phase B consisted of 0.1% TFA in ACN/isopropanol. With the C8 column, mobile phase A consisted of 0.1% TFA and mobile phase B consisted of 0.1% TFA in 1-propanol. The relative amount of a specific protein impurity was determined by measuring the light absorbance (280 nm) of the components eluting from the RP-HPLC column. The relative amount of a specific protein impurity, expressed as area %, was calculated by dividing the peak area corresponding to the impurity by the total integrated area.

### 2.1.6 Measurement of particle size distribution (PAMAS)

The measurements on the PAMAS SVSS-C particle counter were performed as follows: 100 µl sample was diluted 1/10 in 1 mL MilliQ water and 10 consecutive measurements were performed in all 16 channels (diameter set 1, 2, 3, 4, 5, 6, 7, 8, 9, 10, 15, 25, 50, 100, 150 and 200 µm). For calculation of the average value, the first 2 measurements were excluded and the dilution factor was taken into account. The results are given as cumulative data (total particle counts >x µm) or differential data (total particle counts between diameter x and y µm). Only the cumulative data are presented.

### 2.1.7 Affinity measurement on Biacore

A chip was first immobilized with HSA (amine coupling using the Biacore amine coupling kit). After a preconditioning step of 5 injections of the Nanobody, all samples were diluted to 2.5 nM in triplicate and analyzed on the chip. Quality control of the chips using the reference sample was included in the experiment to detect any loss of activity or decrease in response (deterioration of the chip). Slopes were determined using the general fit method and the linear fit model (BIAevaluation software). To determine the initial binding rate (IBR), the slope between 5s and 30s was selected. The values of these slopes were transferred in excel and the percentage activity compared to the reference was determined.

### 2.1.8 ELISA based potency assay for HSA blinding

Human Serum Albumin (HSA) was immobilized onto a multiwell Maxisorp ELISA plate by adsorption. After blocking excess binding sites on the plates with Superblock T20 (PBS) blocking buffer, a dilution series of test and reference samples was applied on the plate. Bound Nanobody was subsequently detected using a bivalent anti-Nanobody Nanobody directly conjugated to horseradish peroxidase (HRP). In the presence of H₂O₂ HRP catalyzes a chemical reaction with Tetramethylbenzidine (es TMB) which results in the formation of a color. The reaction was stopped by adding 1N HCl. The optical density of the color was measured at 450 nm.

### 2.1.9 ELIAS based potency assay for IL-6R binding

For the reference, control and test samples, different dilutions of the Nanobodies were prepared. These dilutions were pre-incubated with a constant amount of 100 ng/mL IL-6, foliowed by the addition of 4 ng/mL soluble IL-6R. Subsequently, this mixture was transferred to a microtiter plate coated with a non neutralizing anti-IL-6R Nanobody. After washing, residual bound IL-6 was detected with biotinylated anti-human IL-6 monoclonal antibody, followed by HRP-labeled streptavidin detection. In the presence of H₂O₂ HRP catalyzes a chemical reaction with Tetramethylbenzidine (es TMB) which results in the formation of a color. The reaction was stopped by adding 1N HCl. The optical density of the color was measured at 450 nm. The relative potency of the test samples compared to the reference sample was analyzed by use of PLA 2.0 Software.

### 2.1.10 Capillary Isoelectric Focusing (cIEF)

Capillary isoelectric Focusing (clEF) is an analysis/separation technique that differentiates proteins with respect to charge, i.e., it separates proteins according to their isoelectric points (pl). The separation principle is similar to gel-based/flatbed IEF but differs mainly in its format, that is, the separation takes place in an open tube of narrow format (capillary) eliminating the need for any anticonvective matrix support. Also, cIEF is a fully automated instrument with online detection and data acquisition. A drawback of traditional clEF in a conventional CE instrument is that the focused (stationary) zones must be mobilized past the single-point detection area in order to record the signal. During mobilization the zones may become broadened with contaminant loss of resolution and decreased detectability. Moreover, the analysis time and risk of protein aggregation/precipitation will increase. By imaging clEF the focusing process is followed in real-time over the whole-column/capillary by a CCD camera excluding the mobilization step. As soon as the focusing process is completed the analysis run is finished.

### Example 2.2: Tm determination

The melting temperature (Tm) in different buffers was determined using the fluorescence- based thermal shift assay (TSA, for IL6R304 and IL6R305) and by differential scanning calorimetry (DSC, for IL6R304).

### 2.2.1 Thermal shift assay

The thermal shift assay or TSA can be performed in 96-well plate in a Q-PCR device to evaluate the effect of buffer couple, ionic strength, pH and excipients on the thermal stability of proteins. The assay results in a Tm value that is indicative for the thermal stability in the tested buffers. Briefly, the assay follows the signal changes of a fluorescence dye, such as Sypro Orange, while the protein undergoes thermal unfolding. When Sypro Orange is added to a properly folded protein solution, it is exposed in an aqueous environment and its fluorescence signal is quenched. When the temperature rises, the protein undergoes thermal unfolding and exposes its hydrophobic core region. Sypro Orange then binds to the hydrophobic regions, unquenches which results in the increase of the fluorescence signal.

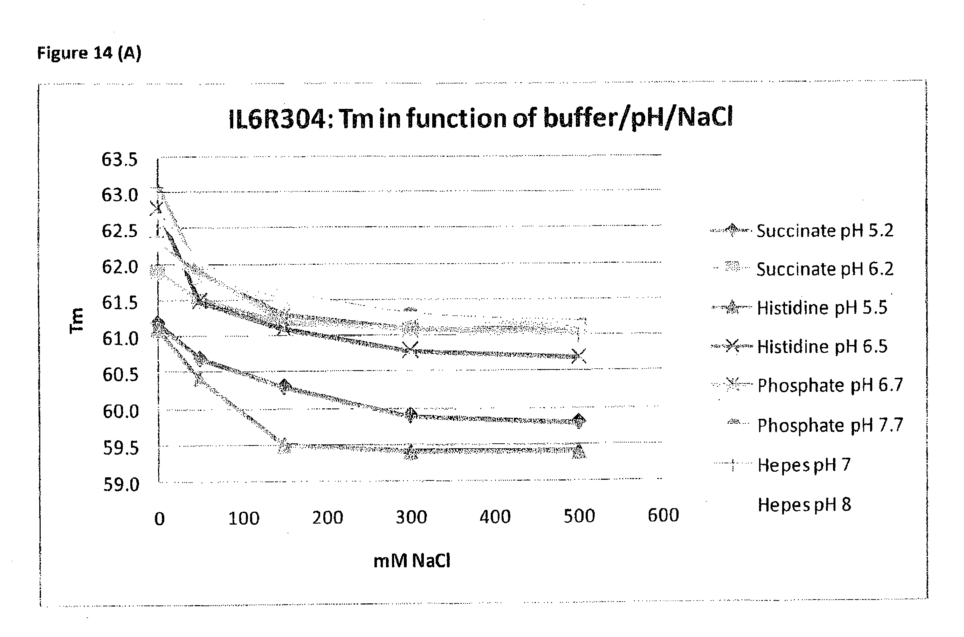

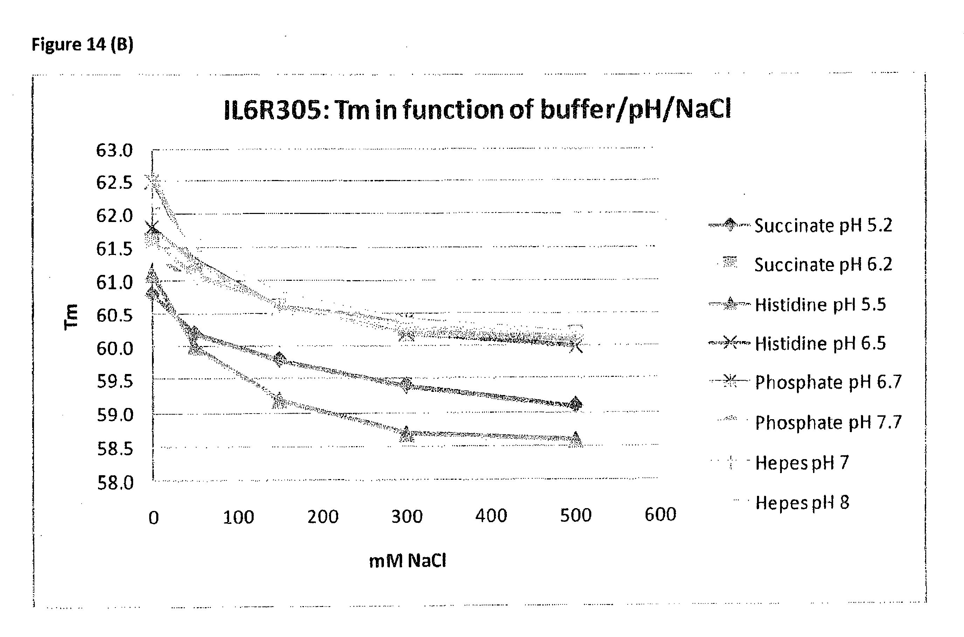

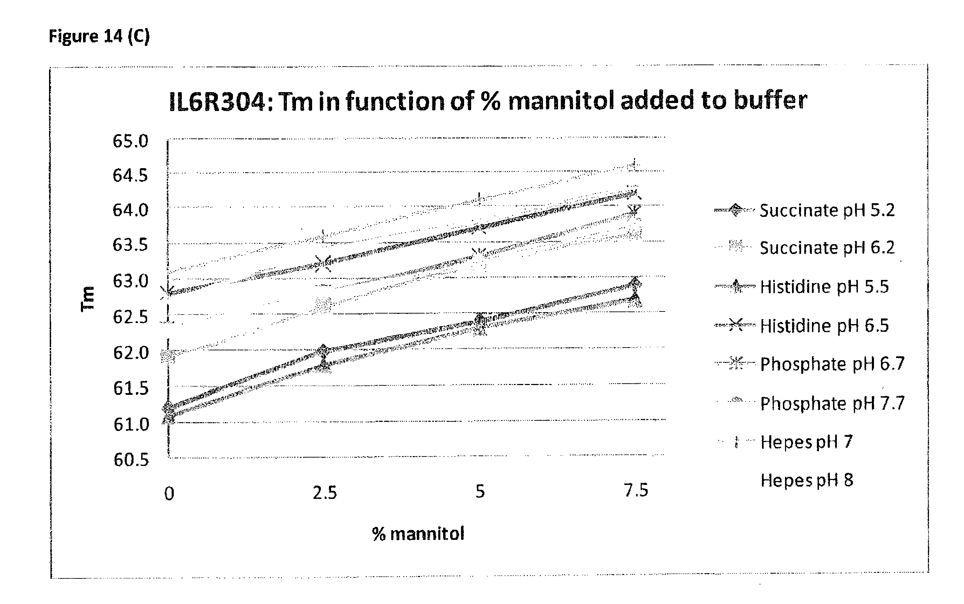

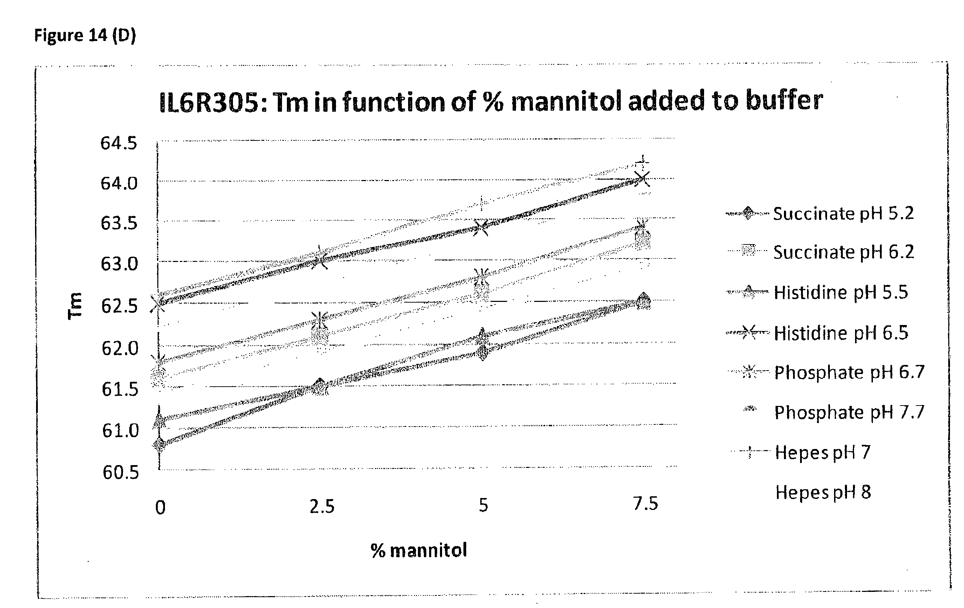

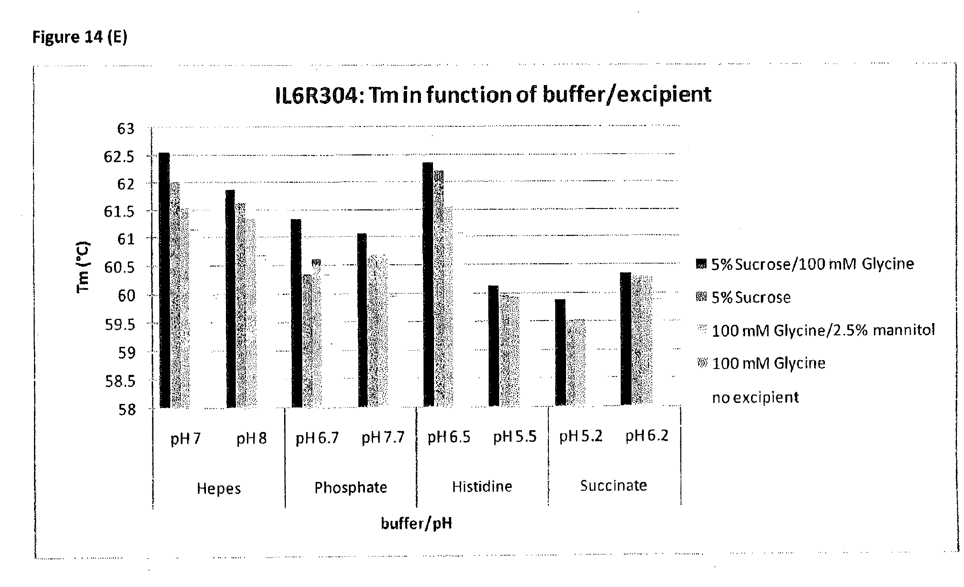

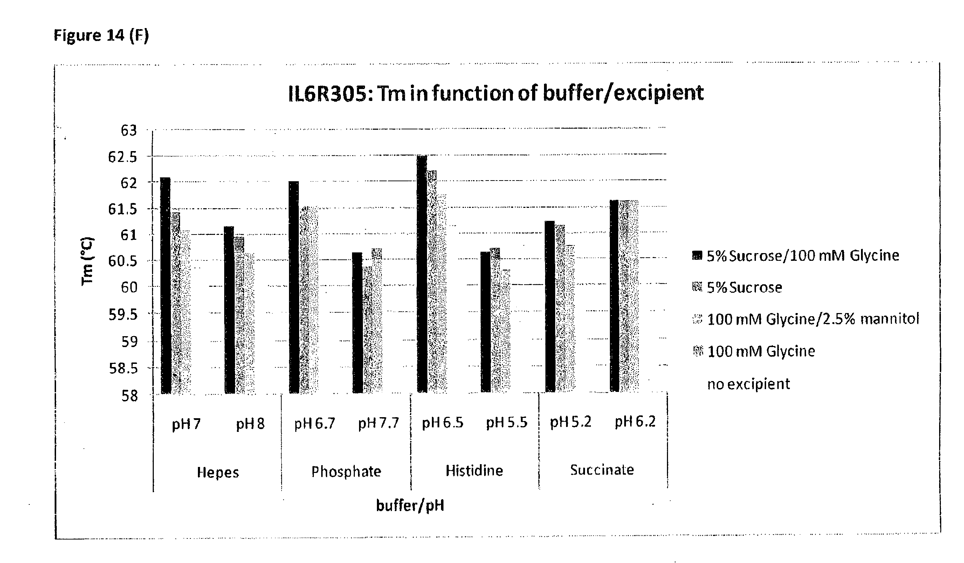

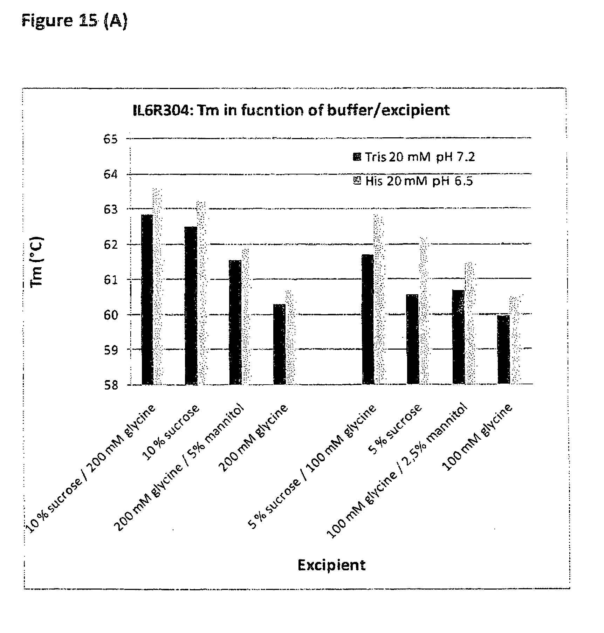

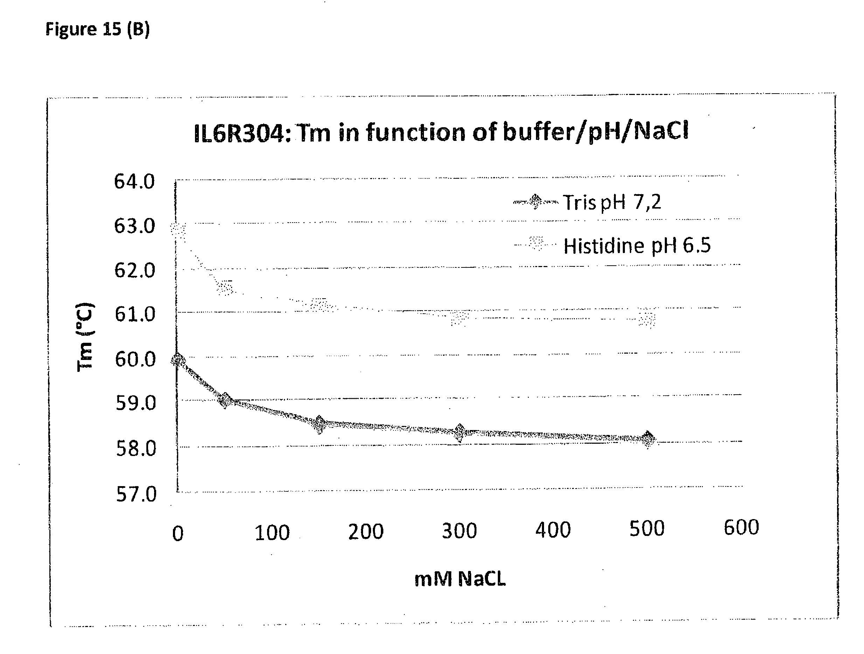

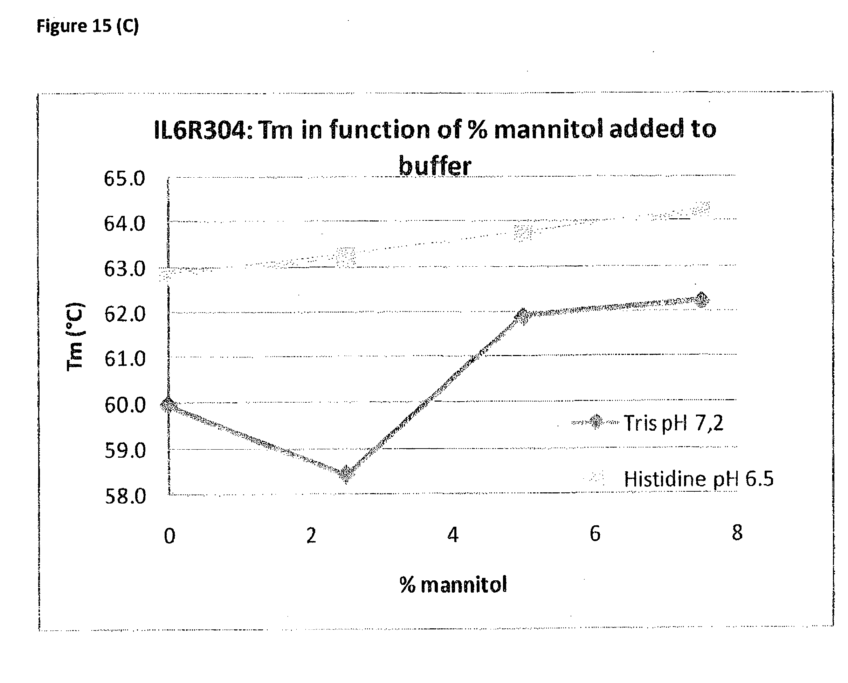

In a first experiment, the Tm was assessed for IL6R304 and IL6R305 in different buffers, excipients and combinations thereof using the TSA assay. The obtained Tm values are displayed graphically in Figure 14 and Figure 15.

In all conditions tested, the Tm values were slightly higher for IL6R304 than for IL6R305. The buffers and excipients tested had a similar effect on the Tm values of IL6R304 and IL6R305:

Effect of buffer pH: the highest melting points were obtained in Hepes buffer (pH 7 and pH 8) and L-histidine pH 6.5, followed by phosphate buffer (pH 6.7 and 7.7), Tris pH 7.2 and succinate buffer pH 6.2. The lowest melting points were obtained in PBS (58.82°C for IL6R304) and in the buffers with the lowest pH, i.e. succinate pH 5.2 and L-histidine pH 5.5. The higher melting point in L-histidine pH 6.5 correlates well with the higher solubility of IL6R304 in this buffer (see Example 2.3).

Effect of [NaCl] concentration: the highest melting temperatures were measured when no sodium chloride was added to the buffers. Tm values decreased gradually with increasing NaCl concentration; the effect plateaus at 300 mM NaCl.

Effect of the excipients mannitol, sucrose and glycine: all excipients tested appeared to have a stabilizing effect on IL6R304 and IL6R305, since the melting temperatures increased with increasing excipient concentration. The highest Tm values were obtained in buffers containing 7.5% mannitol or 5% sucrose.

In summary, IL6R304 and IL6R305 seemed to be more stable in buffers that had a neutral pH and which contained a low NaCl concentration and significant amounts of mannitol or sucrose. This is represented schematically in Table 13. L-histidine pH 6.5 appeared to be a good buffer to take forward in further stability testing and formulation work. Another important argument for proceeding with a L-histidine buffer is that the solubility of IL6R304 was shown to increase dramatically in this buffer compared to PBS (see Example 2.3). Buffers containing high NaCl concentration should be avoided, while Hepes buffers at neutral pH could preferably be used.

### 2.2.2 Differential scanning calorimetry

To identify other suitable buffers to be used in purification protocols, i.e. in which the protein displays acceptable stability, differential scanning calorimetry (DSC) was used to determine the melting temperature of IL6R304 in different candidate buffers.

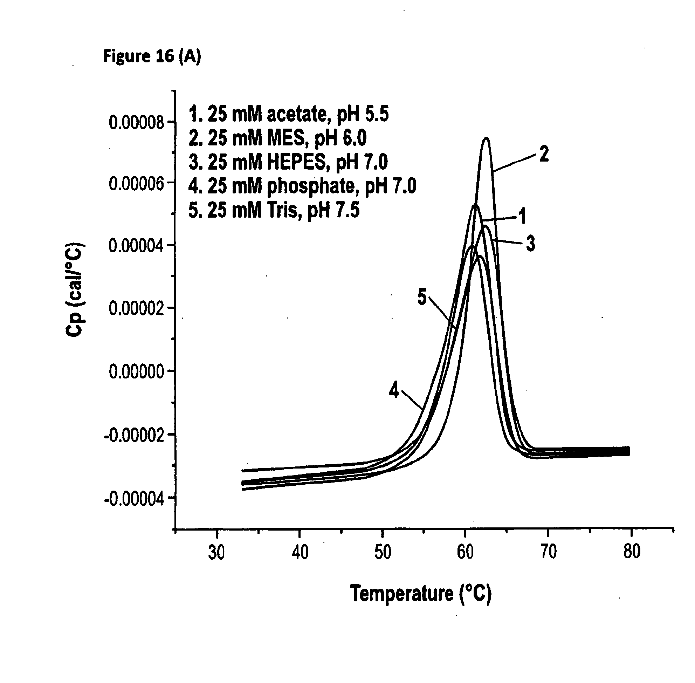

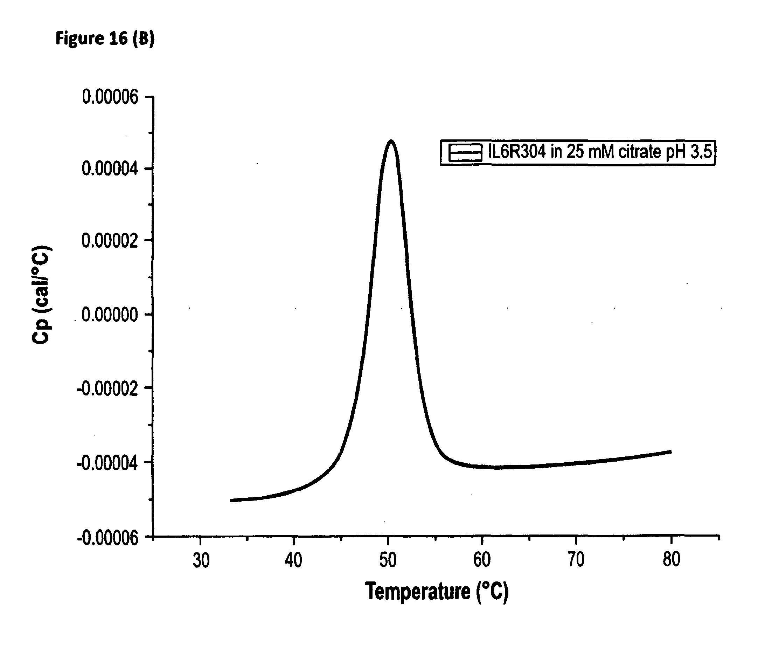

Figure 16 shows the results of a DSC experiment performed on IL6R304 formulated in different buffers. The highest melting temperatures were observed in MES (pH 6.0) and Hepes (pH 7.0), whereas acetate, Tris-HCl and phosphate indicate a slightly lower thermal stability. The Tm value obtained in citrate (pH 3.5) was on average 10°C lower than in the other buffers. All heat capacity (Cp) melting peaks were sharp and rather symmetrical in all buffers. The restoration of a base-line after the transition indicated that no precipitation had occurred.

An overview of all Tm values obtained in the DSC experiments is shown in Table 14. From these results we can conclude that adding more NaCl lead to a gradual decrease in melting temperature, as was also observed in the TSA.

### 2.2.3 TSA experiment using experimental design

A second TSA experiment was performed on IL6R304 using experimental design (DOE). The DOE consisted of different steps. The outcome of each step was used to define the DOE for the next step. Briefly, the experimental set-ups and obtained results were the following:
- Step 1: L-histidine, succinate, phosphate and Tris were tested at different pH (6-7) and buffer strength (10 - 40 mM). The most promising formulation was found to be low ionic strength L-histidine and Phosphate buffers with a pH of 7 and 6, respectively.
- Step 2: the effect of adding NaCl and different excipients to 15 mM L-histidine pH 6.5 and 7 and 15 mM Phosphate pH 6 and 6.5 was tested. One representative of three excipient families was included: mannitol (polyol), sucrose (non-reducing sugar) and arginine (amino acid). It was concluded that Arginine and/or NaCl decrease the Tm of IL6R304. A maximal melting temperature was reached in sucrose or a mix of sucrose/mannitol (10% in total). Furthermore, a higher Tm was obtained in L-histidine than in phosphate. For both buffers, best results were obtained at pH 6.5.
- Step 3: other representatives, i.e. sorbitol, xylitol, ribitol, trehalose and glycine, from the three excipient families were tested in 15 mM L-histidine and 15 mM phosphate, both at pH 6.5. Again, the Tm of IL6R304 was higher in L-histidine buffer than in phosphate for all excipients and excipient combinations. The best buffer formulation contained 10% sorbitol or trehalose.

### (Table 15)

In a fourth step, the outcome of the statistical analysis of the DOE was confirmed by a repeat of the TSA and by differential scanning calorimetry.

### Example 2.3: Solubility of the Nanobodies in different buffers

### 2.3.1 Solubility in PBS and the effect of Tween 80

During downstream processing and storage of the IL6R304, IL6R305 and IL6R306 Nanobodies in D-PBS buffer, precipitation occurred. Precipitates were already formed during storage overnight at 5°C or -20°C, even in samples that were filtered (0.22 µm) before storage. This not only resulted in significant product loss, but also made it inconvenient to perform subsequent experiments since filtration steps needed to be incorporated constantly. From these observations it was clear that PBS is not a suitable formulation buffer for any of the IL-6R Nanobodies and that alternative storage buffers needed to be identified. In an initial experiment, it was assessed whether Tween 80 could prevent this precipitation (aggregation) from occurring.

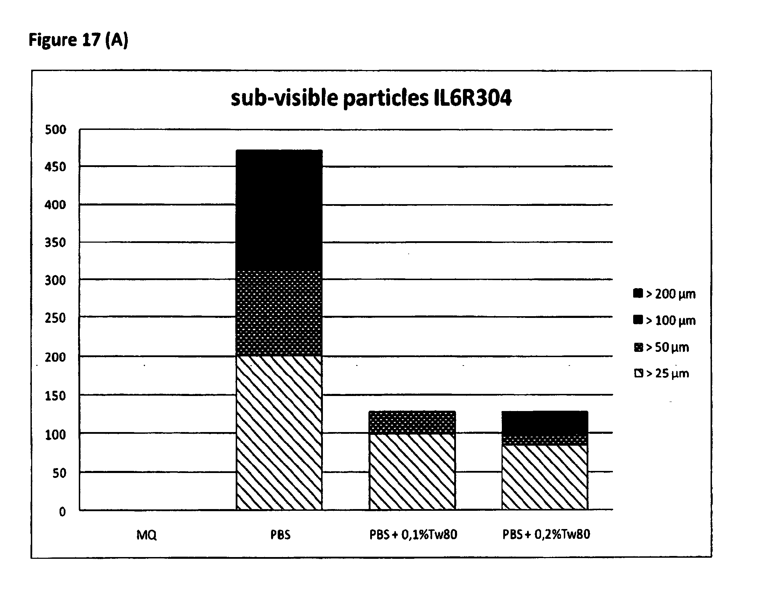

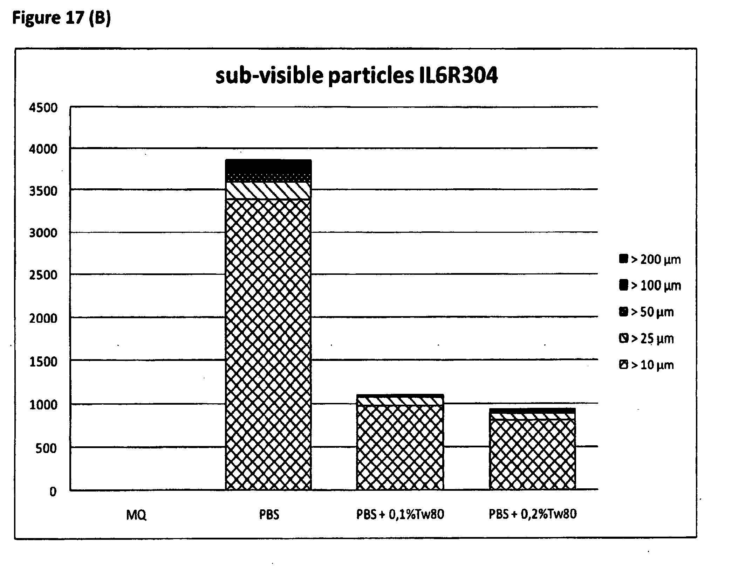

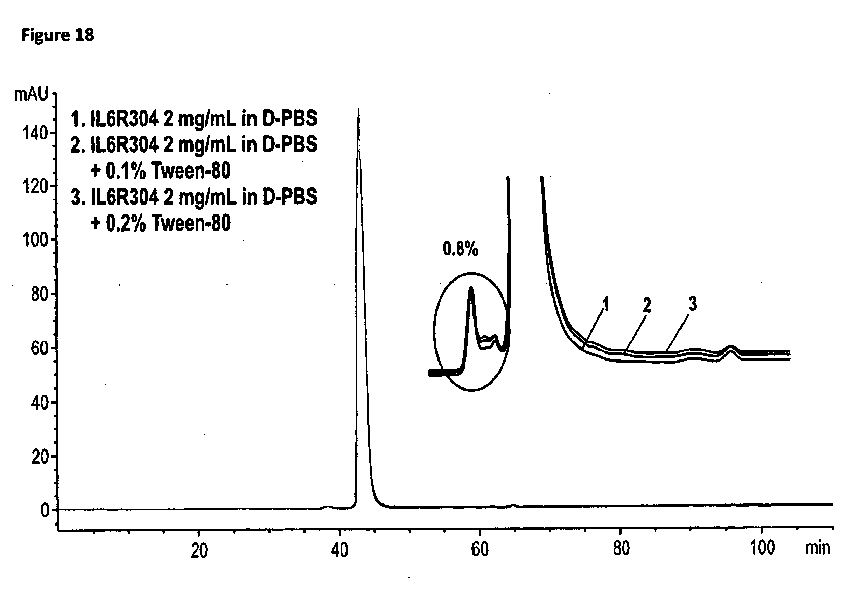

Briefly, IL6R304 (P#051108nr1) was diluted to 2 mg/mL in PBS buffer, PBS buffer + 0.1% (v:v) Tween 80 or PBS buffer + 0.2% (v:v) Tween 80. The three samples were stored for 4 days at 5°C and subsequently analyzed for visible particulates (appearance testing by visual inspection; Table 16), sub-visible particle counts (PAMAS; Figure 17), via UV spectroscopy (A320/A280 ratio, i.e. measure for the presence of particulates; Table 16) and SE-HPLC (Figure 18). Significantly more and larger particles were present in the IL6R304 sample formulated in PBS compared to the IL6R304 sample formulated in PBS + Tween 80.

### 2.3.2 Concentration experiments to determine the solubility of IL6R304 and IL6R305

IL6R304 and IL6R305, both formulated in PBS, were concentrated stepwise with a Vivascience concentrator (Vivaspin 500 5,000 MWCO, 500 µl concentrator). During the concentration experiment, the retentate was mixed gently regularly and was analyzed visually for particulates/precipitation. The presence of insoluble aggregates in the retentate was verified by checking the protein concentration before and after centrifugation at maximum speed. Based on the results from the thermal shift assay (see Example 2.2.1), the solubility of IL6R304 was also analyzed in 20 mM Histidine, pH 6.5.

In conclusion, the solubility of IL6R304 and IL6R305 in PBS was limited, with estimated values of 20 mg/mL and 15 mg/mL, respectively (concentration at which protein precipitation occurred). No precipitation of IL6R304 was observed at a concentration between 20-90 mg/mL in L-histidine buffer suggesting that the solubility can be increased significantly by changing the formulation buffer. Note that no significant protein loss has been observed.

### 2.3.3 Determination of the theoretical solubility

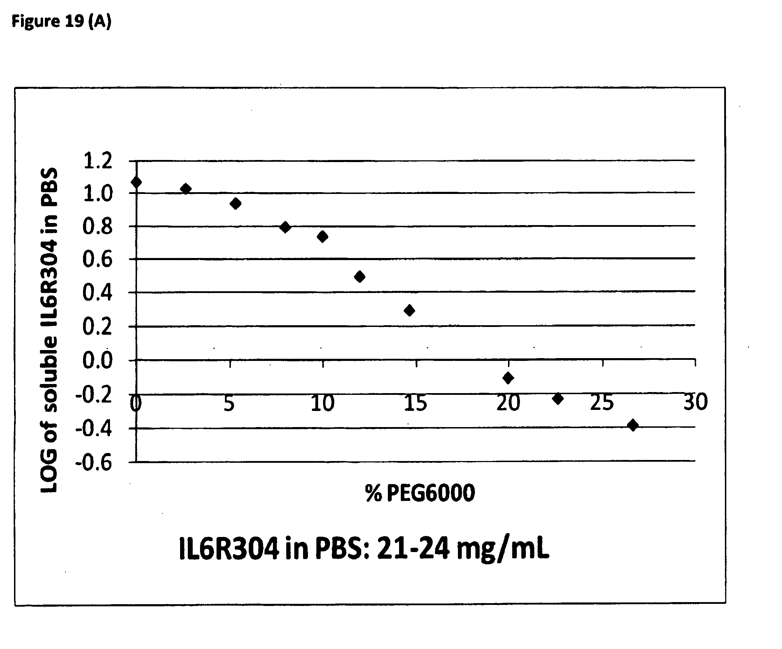

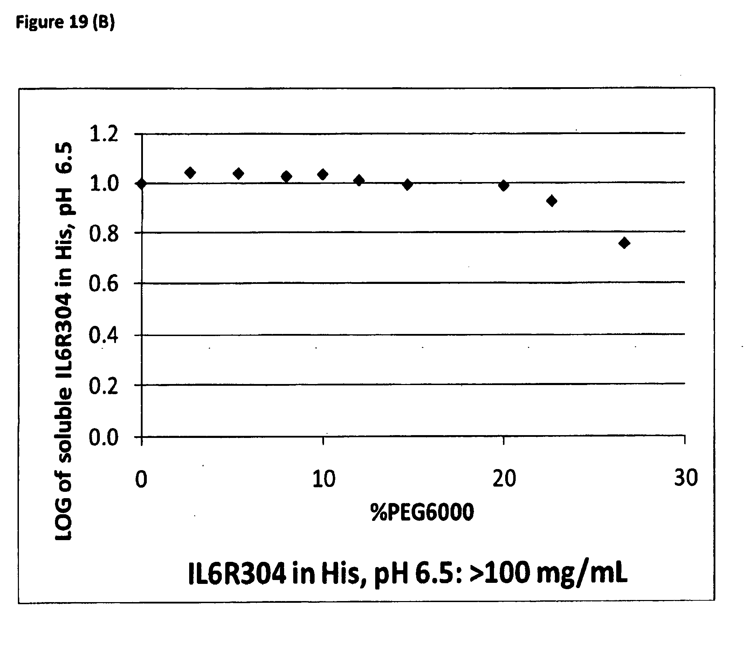

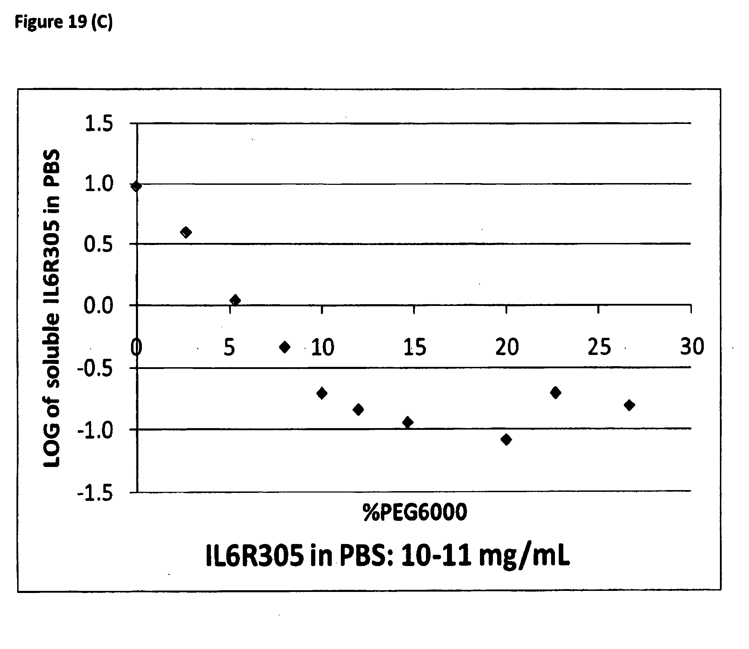

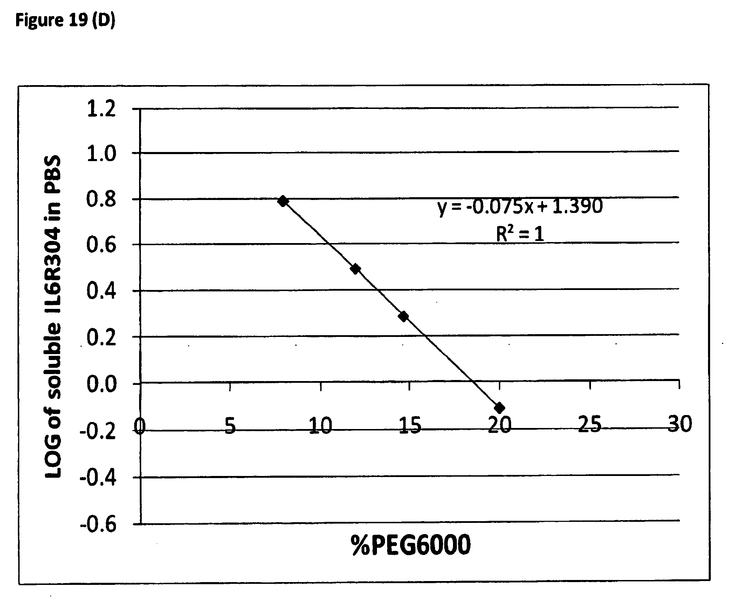

The theoretical solubility of IL6R304 and IL6R305 was determined using the PEG exclusion method in PBS buffer (IL6R304, IL6R305) or in 20 mM L-histidine, pH 6.5 (IL6R304). Briefly, a concentrated Nanobody solution (30-80 mg/mL) in the respective buffer was incubated for 15 minutes at room temperature in the presence of increasing concentrations of PEG6000. After centrifugation at 20000 x *g* for 3 minutes, log values of the [soluble protein concentration] were plotted versus PEG6000 concentration (Figure 19). By regression analysis and extrapolation to a zero concentration of PEG6000, the theoretical maximum protein concentration (and thus solubility values) could be obtained for the Nanobodies in the buffers tested. The obtained solubility values correlated well with the values obtained experimentally using the stepwise concentration experiments with the Vivaspin centrifugal concentrators. When comparing the solubility of IL6R304 in PBS and L-histidine pH 6.5, it could be concluded that the solubility of IL6R304 increased significantly in the L-histidine buffer.

### Example 2.4: Storage stability study of the Nanobodies at 37 °C

An initial storage stability study was performed to get a general understanding of the stability of the IL-6R Nanobodies and to determine if adding mannitol in the formulation buffer has a beneficial effect in minimizing the formation of potential dimers, as was observed for RANKL008a (see Example 1.3).

The three IL-6R Nanobodies were formulated in different buffers (Table 17) at a concentration of 10 mg/mL (IL6R304), 7.1 mg/mL (IL6R305) and 10.3 mg/mL (IL6R306). The stability of the different samples was assessed in accelerated stress conditions at 37°C. Samples were analyzed after 1 week using SE-HPLC and RP-HPLC. Selected samples of IL6R304 and IL6R305 were also analyzed after 3 weeks of storage.

### 2.4.1 SE-HPLC analysis

For all three IL-6R Nanobodies, prolonged storage at 37°C resulted in the formation of prepeaks and some minor postpeaks. The postpeaks probably corresponded to degradation products (due to remaining proteolytic activity in sample). The surface area of these postpeaks remained very low, suggesting only minimal degradation after 3 weeks at 37°C.

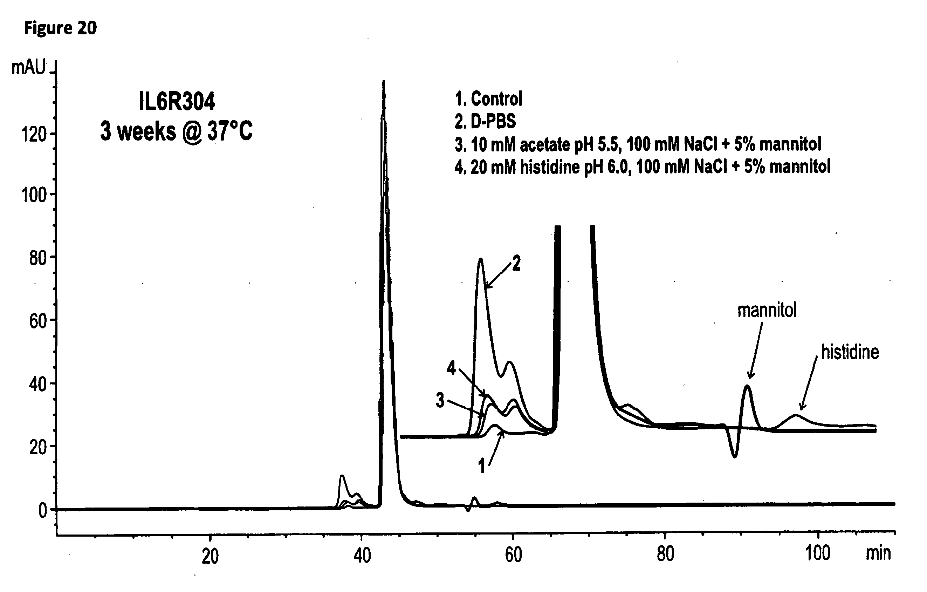

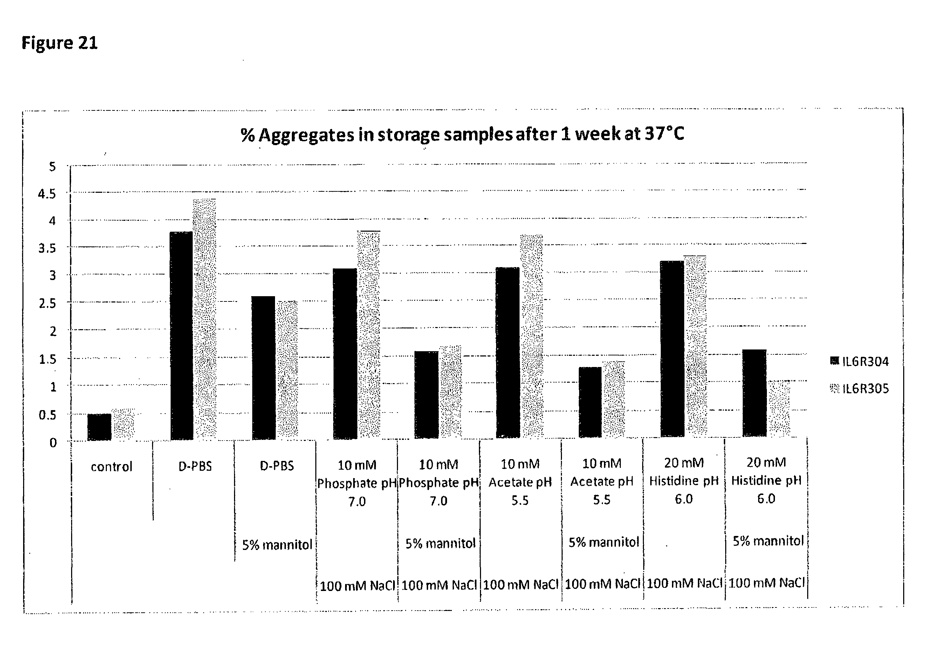

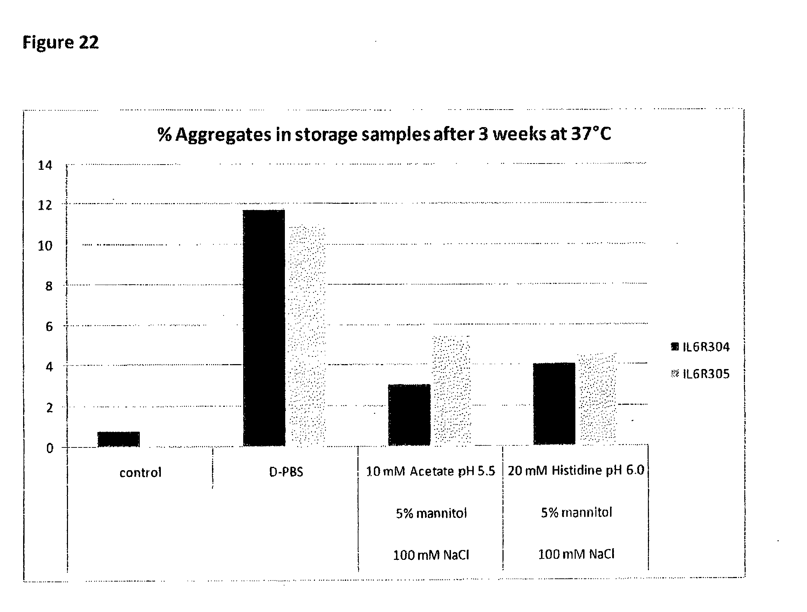

All three IL-6R Nanobodies had a strong tendency to form dimers/oligomers (aggregates), which were visible as prepeak(s) in the chromatograms of the SE-HPLC analysis. An example chromatogram is shown in Figure 20. The peak area of the prepeak increased significantly over time (represented as % aggregates in Figure 21 and Figure 22) and was accompanied by a concomitant decrease in surface area of the main peak. The propensity to form dimmers/oligomers appeared to be somewhat higher for IL6R305 than for IL6R304. Also, more dimmers/oligomers were being formed in PBS compared to the other buffers that were tested. Importantly, the lowest amounts of oligomers were observed in the mannitol-containing formulations.

### 2.4.2 RP-HPLC analysis

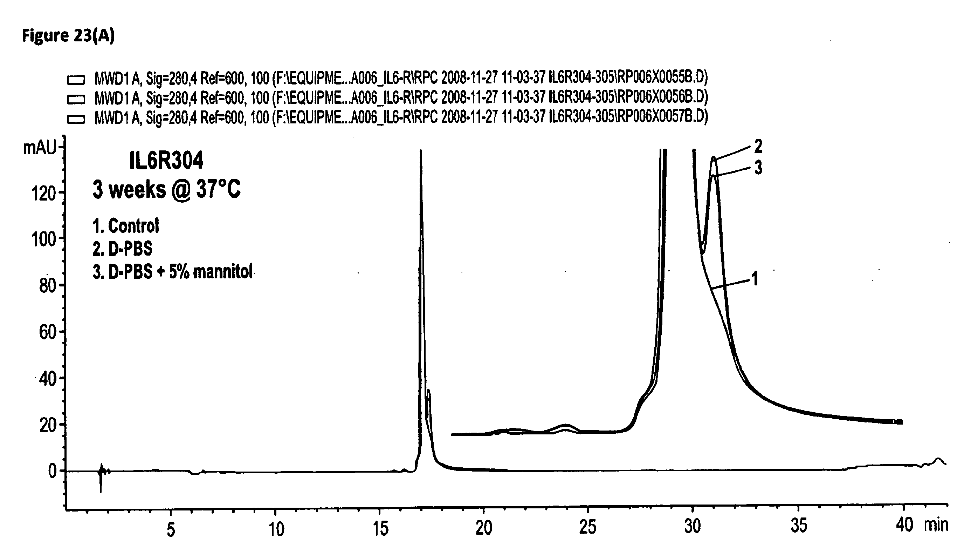

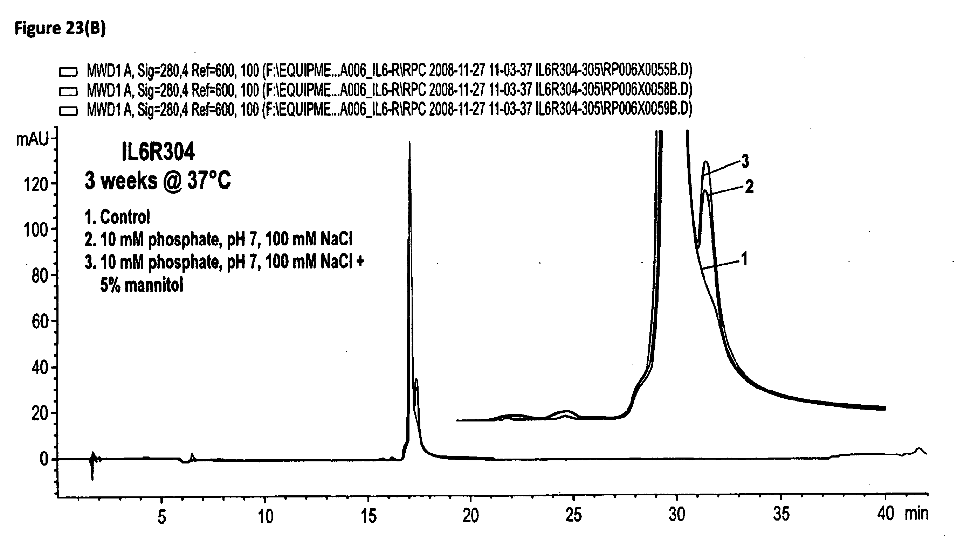

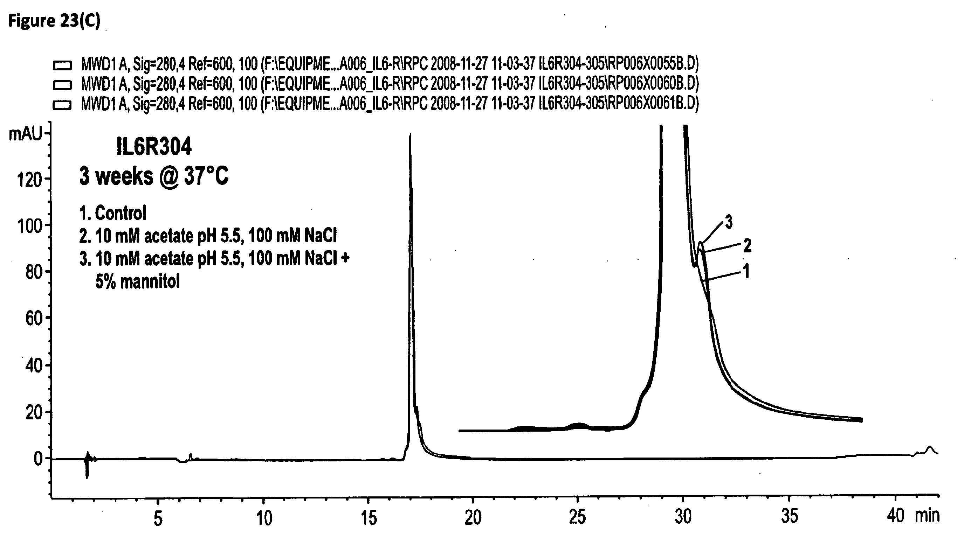

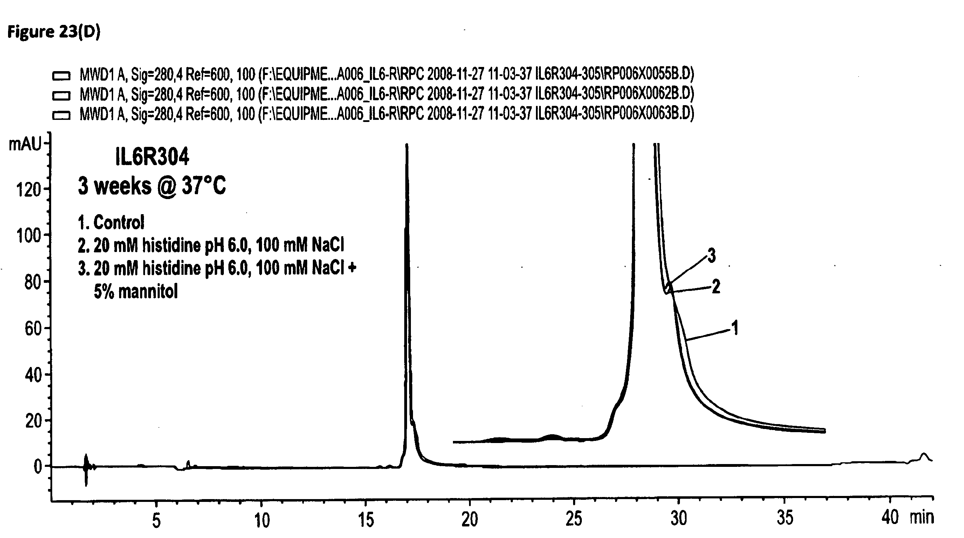

The RP-HPLC profile of IL6R304 at time point 0 weeks included a main peak with a shoulder eluting before the main material and a postpeak that was not well resolved from the main peak. This postpeak most likely corresponded to the pyroglutamate-containing variant of IL6R304. The surface area of this peak increased with storage time and was highest in PBS and phosphate buffer compared to the acetate and histidine buffers. After 3 weeks of storage, the surface areas of two thus far unidentified prepeaks increased (Figure 23).

The RP-HPLC profile of IL6R305 at time point 0 weeks included a main peak with some minor prepeaks. The resolving power of the RP-HPLC method used was insufficient to separate the pyroglutamate-containing variant from the main material.

### Example 2.5: Osmolality measurement

According to the European Pharmacopoeia, a solution is considered isotonic if it has an osmolality of 290 ± 30 mOsm/kg. Osmolality measurements on 20 mM L-histidine pH 6.5 containing different concentrations of excipients were therefore performed to define the range of excipient concentration that would be acceptable for an isotonic liquid formulation of IL6R304.

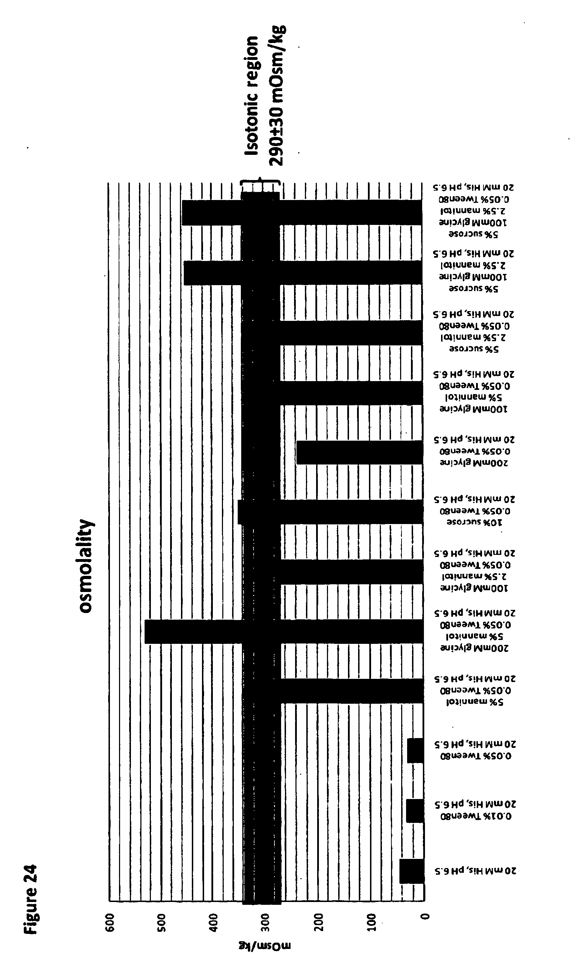

IL6R304 (10 mg/mL) was formulated in the different buffers of Table 18. The results from osmolality measurement of these formulations are shown in Figure 24.

### Example 2.6: Storage stability study of IL6R304 at 5 °C and 37 °C

**An** overview **of the** different formulation buffers and methods used in stability testing of IL6R304 batch P#051108nr1 is given in Table 18 and Table 19, respectively. Because IL6R304 was found to be prone to aggregation and precipitation, Tween 80 was added to most formulations.

### 2.6.1 Appearance and OD280

No turbidity was observed in the samples stored for 5 weeks at 5°C, indicating that 20 mM L-histidine pH 6.5 is a much better storage buffer for IL6R304 than PBS.

After storage for 1 week at 37°C, a slight turbidity was observed in all 12 samples. In the IL6R304 samples in buffers 1, 2, 3, 7, 8 and 9 more opalescence was observed compared to IL6R304 in the buffers 4, 5, 6, 10, 11 and 12, which all contained mannitol. After storage for 2 weeks at 37°C, slightly more turbidity was observed compared to the 1 week samples. However, the trend observed for the 1 week samples continued: the IL6R304 samples in buffers 1, 2, 3, 7, 8 and 9 showed more opalescence compared to the mannitol-containing buffers. After storage for 5 weeks at 37°C, turbidity was stili present and slightly less in the samples containing mannitol. However, it seemed like the turbidity had not increased compared to the 2 weeks samples.

Despite the opacity observed in the stressed samples, the protein concentration in the samples has not decreased significantly (data not shown) although there was a slight trend to a lower concentration due to a higher turbidity. Also, the OD320/OD280 ratio, which is a measure for turbidity or the presence of particulates, was <0.05 in all buffer conditions. In fact, the ratio was 2-10 fold lower than observed in the unstressed sample in PBS, again showing that the L-histidine pH 6.5 buffer has a stabilizing effect on IL6R304.

### 2.6.2 SE-HPLC analysis

Samples of the reference material (0 weeks) and samples stored for up to 6 months at 5°C and 37°C were analyzed using SE-HPLC.

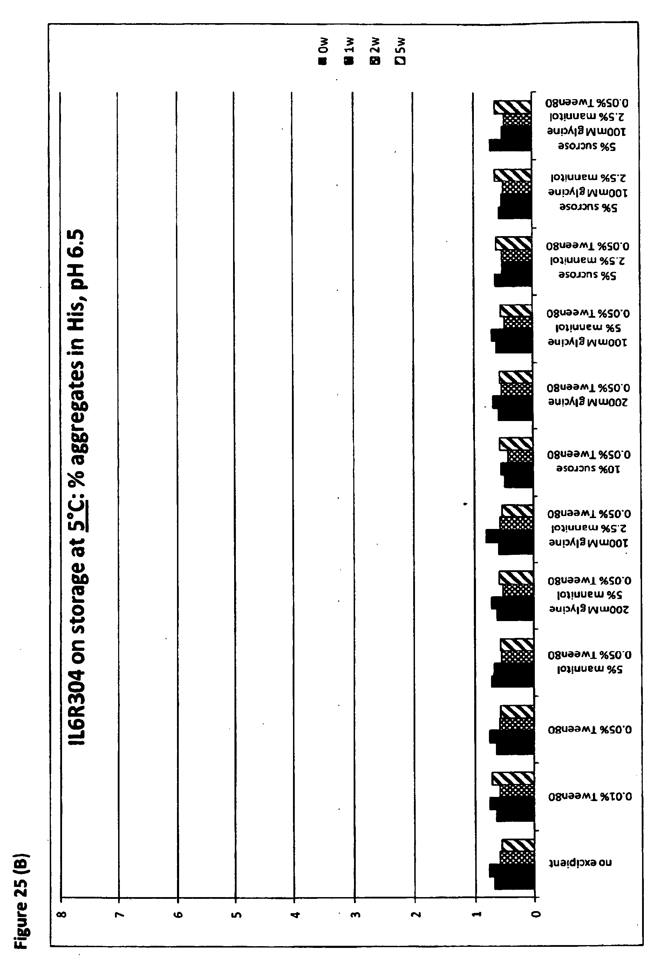

No differences were observed between the SE-HPLC profiles of the reference samples (at 0 weeks) and the samples stored for up to 5 weeks at 5°C. In addition, there were no significant differences between the different buffers. The small amounts of aggregates already present in the start material were not increasing with prolonged storage time (Figure 25(B)), indicating that the Histidine buffer had a stabilizing effect on ILR304, even in the absence of excipients such as Tween 80, mannitol or sucrose. Note that IL6R304 formed aggregates when stored for a short time (hours- days) at 5°C in D-PBS buffer.

SE-HPLC analysis of the samples stored for 6 months at 5°C also did not show increase in area % of the pre-peaks, meaning that no oligomers were formed under these storage conditions, not even in the formulation containing only 20 mM L-histidine, pH 6.5 i.e. without Tween-80 or any excipient (data not shown).

Prolonged storage at 37°C resulted in the formation of prepeaks and some minor postpeaks. The postpeaks probably corresponded to degradation products (due to remaining proteolytic activity in sample). The relative area (%) of these peaks increased only slightly, implying that degradation was restricted to a minimum. The other peaks visible in the chromatograms were background peaks arising from the buffer components.

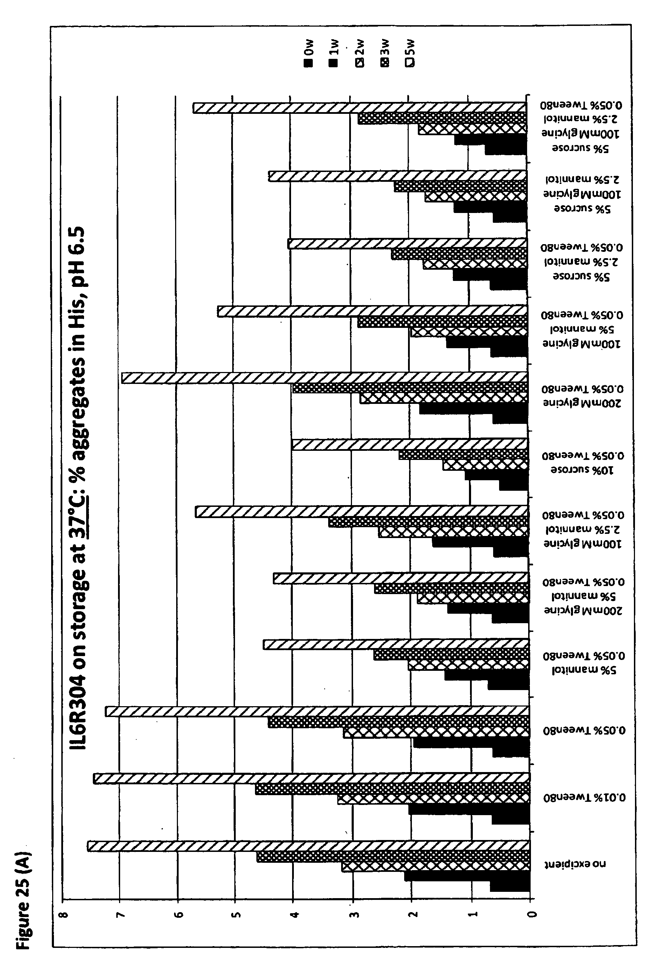

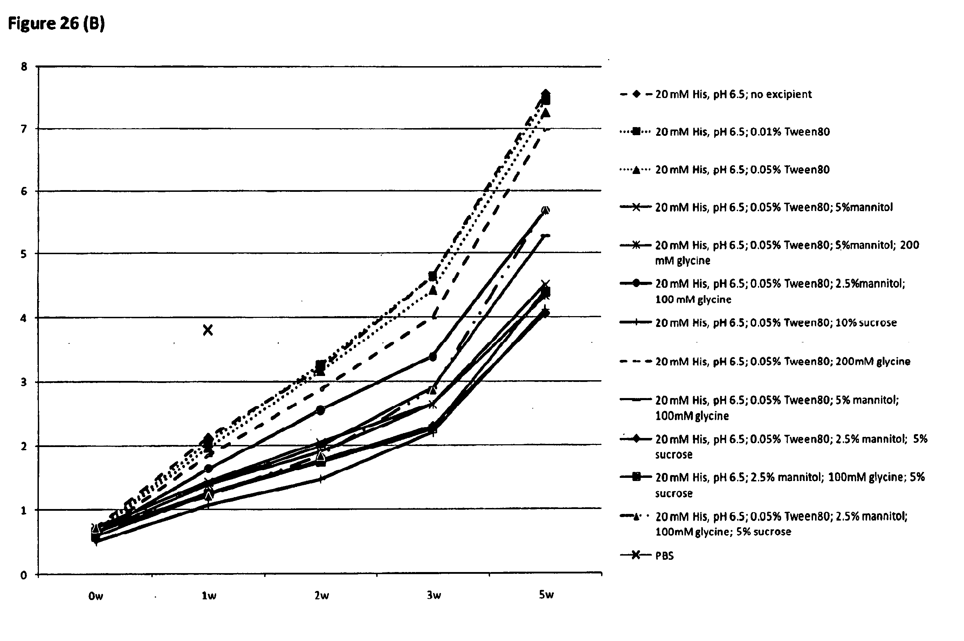

The peak area of the prepeaks increased significantly over time (Figure 25 (A) and Figure 26(B)). Given the relative position of the prepeaks to the main peak, the prepeaks most likely represented dimeric or oligomeric forms (aggregates) of IL6R304. The peak surface area of the prepeak increased with storage time and was accompanied by a concomitant decrease in surface area of the main peak.

An important observation was that the propensity to form dimers/oligomers was buffer-dependent: the propensity to oligomerize was significantly lower in the mannitol- and sucrose-containing formulations. Glycine appeared not to have such a positive effect in preventing the oligomerization process. Tween 80 had no inhibitory effect on the formation of oligomers.

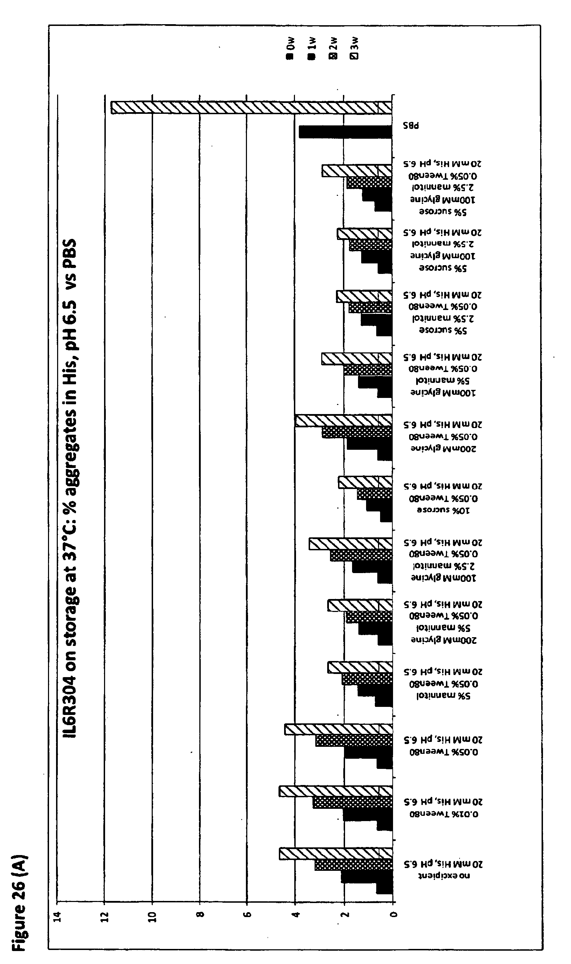

Importantly, the % oligomers observed in all 12 L-histidine buffers after storage for 3 weeks at 37°C was significantly lower than the equivalent sample in D-PBS buffer, i.e. 2.2-4.6% in L-histidine, pH 6.5 compared to 11.7% PBS (Figure 26(A)). This buffer-dependent effect on the physical stability of IL6R304 correlated very well with the buffer-dependent differences observed in thermal stability testing of IL6R304 (Example 2.2): the melting temperature of IL6R304 was found to be only 58.8°C in PBS but is 62.8°C in 20 mM L-histidine, pH 6.5. Increasing the intrinsic stability of IL6R304 by changing the formulation buffer from PBS to L-histidine proved to have a clear beneficial effect on its stability upon storage.

In the samples stored for 6 months at 37°C, the lowest % of oligomers was found in the formulation containing 10% sucrose, again corroborating the stabilizing effect of sucrose on IL6R304 (Table 20).

### 2.6.3 RP-HPLC analysis

Samples of the reference material (0 weeks) and samples stored for up to 5 weeks at 5°C and 37°C were analyzed using RP-HPLC.

The RP-HPLC profiles at time point 0 weeks included a main peak, two pre-peaks and a badly resolved postpeak. This post-peak most probably corresponded to the pyroglutamate-containing variant of IL6R304.

The RP-HPLC profiles of the reference batch and the stability samples stored for up to 5 weeks at 5°C were found to be comparable.

In the stability samples stored at 37°C, the peak surface area of the pyroglutamate peak increased with storage time while the surface area of the main peak decreased. The total area remained unchanged.

After 5 weeks of storage at 37°C, the surface area of the two prepeaks had increased in all buffer conditions. The identity of these variants is unknown at the moment, but could correspond to degradation fragments.

There were no significant buffer-dependent differences in the RP-HPLC profiles of the different samples suggesting that chemical modifications, such as pyroglutamate formation and oxidation which are typically detected by RP-HPLC, were limited and at present unaffected by the buffer.

### Example 2.7: Storage stability study of IL6R304 at -70°C, -20°C, 5 °C, 25°C and 37 °C

IL6R304 was formulated at 10 mg/mL in the 10 different buffers shown in Table 21, stored at **-**70°C, -20°C, +5°C and +37°C for 8 weeks and for 1 week +25°C. Stability samples were analyzed using SE-HPLC, RP-HPLC, OD280 and visual inspection. Selected samples were also analyzed using Biacore (HSA binding) and potency assays (HSA and IL-6R).

### 2.7.1. Storage for 8 weeks at -70°C, -20°C, 5°C and 1 week at 25°C

IL6R304 was shown to be stable after storage for 8 weeks at -**70°C**, -20°C, 5°C and for 1 week at 25°C in all 10 buffers tested. No significant differences were observed in potency, turbidity, SE-HPLC and RP-HPLC profiles between the reference material and the 10 different storage samples.

### 2.7.2. Storage for 8 weeks at 37°C

### Appearance and OD280

Compared to the samples stored at -70°C, -20°C and 5°C, turbidity was observed in the samples stored at 37°C. The absorbance values at 350 nm had increased accordingly to >0.01 AU in most buffers, although the A350/A280 ratio was still <0.05 in all buffer conditions. Despite the opacity observed in the stressed samples, the protein concentration in the samples had not decreased significantly.

### SE-HPLC

Prolonged storage at 37°C resulted in the time-dependent formation of a postpeak and prepeak. The postpeak has a retention time between 22 and 23 minutes and most likely corresponded to IL6R304 degradation fragments. The surface area of this peak however remained low (approximately 2%), suggesting only minimal degradation after 8 weeks at 37°C. The other postpeaks visible in the chromatograms were background peaks arising from the buffer components.

The SE-HPLC profile of IL6R304 at time point 0 weeks included a main peak and two minor prepeaks, which were not completely baseline-resolved. The surface area of the prepeaks increased over time and was accompanied by a concomitant decrease in surface area of the main peak. Given the relative position and heterogeneity of the prepeaks, they most likely represented dimeric and/or oligomeric forms of IL6R304. Because of this heterogeneity and the decreasing resolution between the prepeaks over time, the peaks were for simplicity integrated as a single peak.

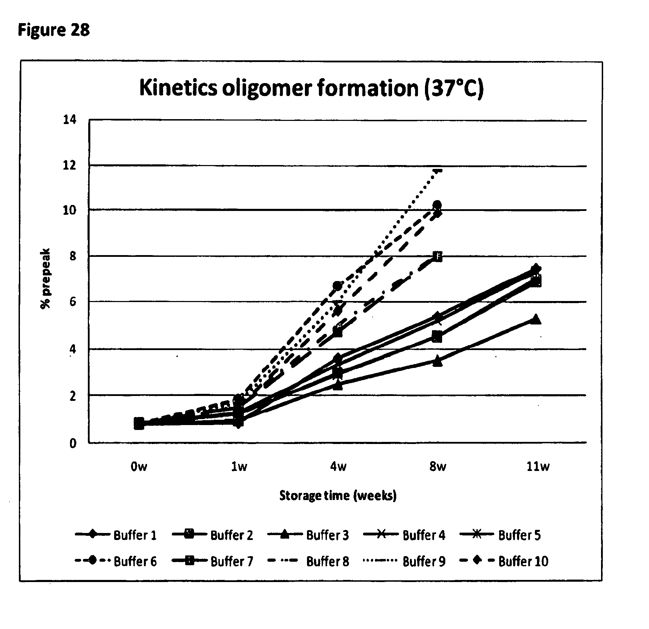

An important observation was that the propensity to form dimers/oligomers was buffer-dependent: about 2-fold less oligomers were being formed in L-histidine buffer compared to phosphate buffer (Figure 27, Figure 28). The lowest amount of oligomers was observed in the trehalose-containing formulation, followed by the sucrose-containing formulation. Overall, after storage of IL6R304 at 37°C for several weeks, the amount of oligomers present in these buffers was significantly less than observed previously in D-PBS or in L-histidine pH 6.5 devoid of any excipient.

The presence of a non-reducing sugar suppressed the extent of IL6R304 oligomerization considerably.

### RP-HPLC

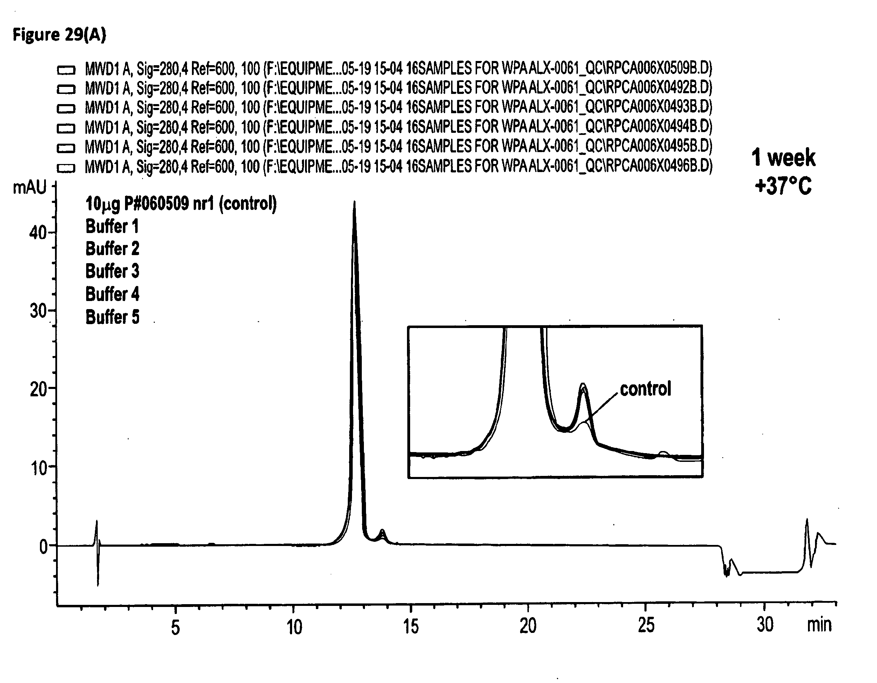

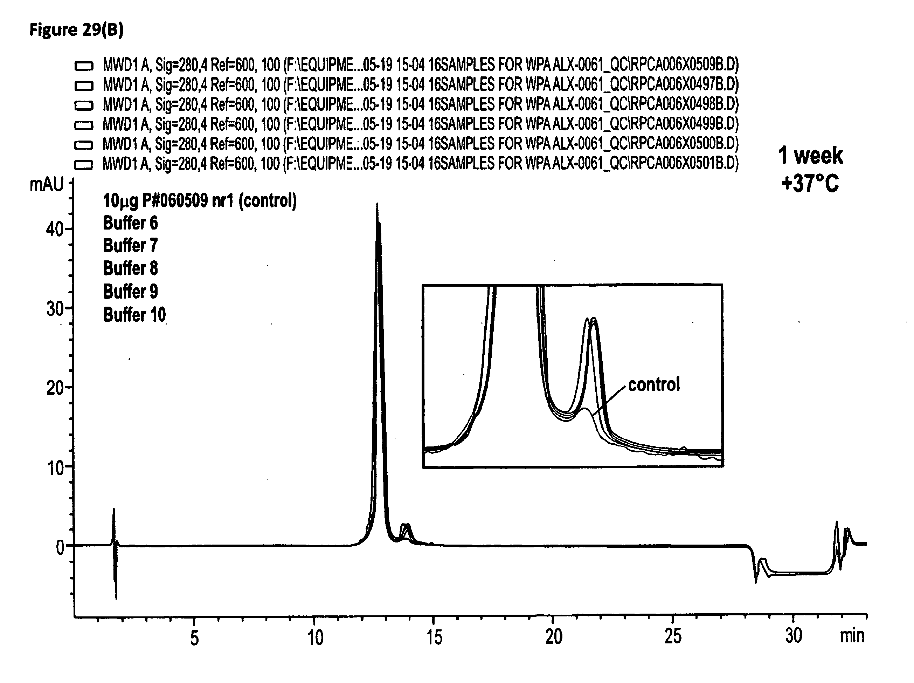

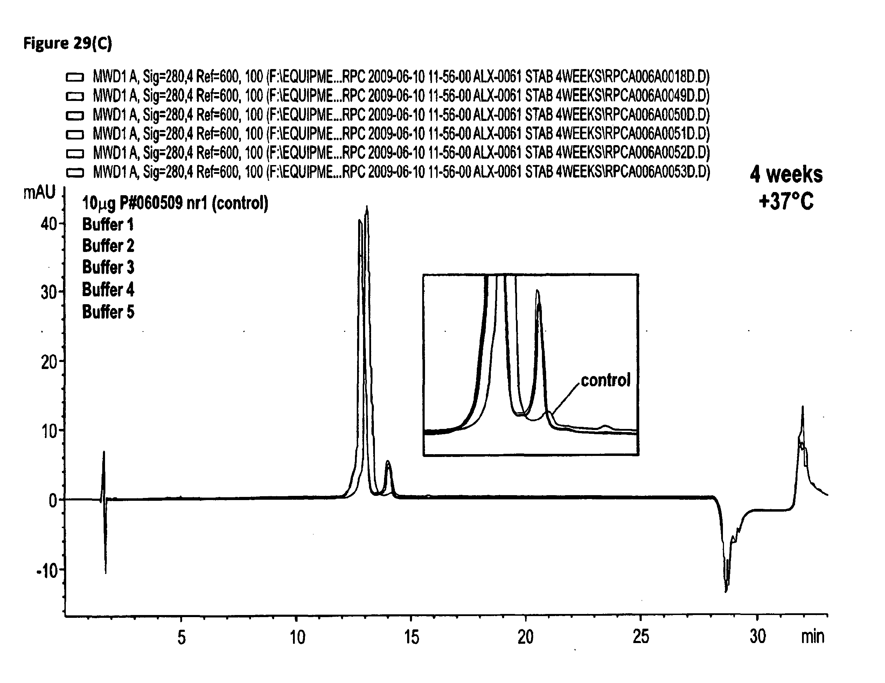

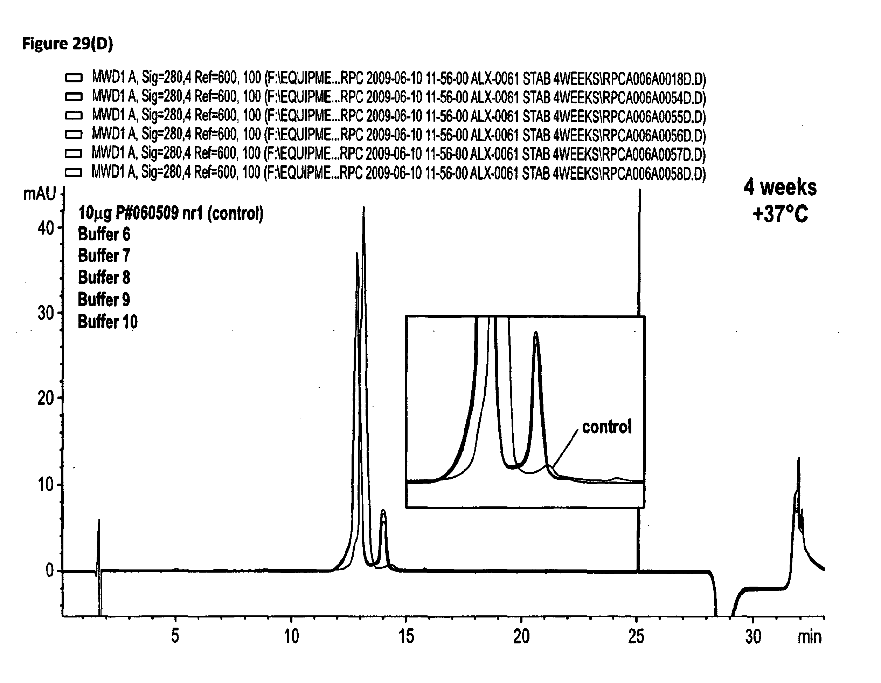

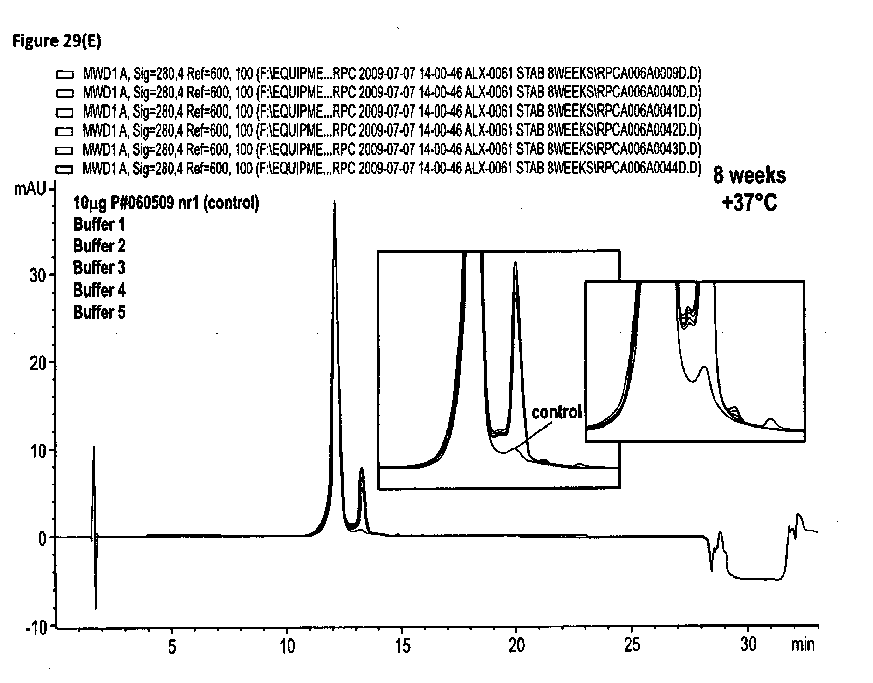

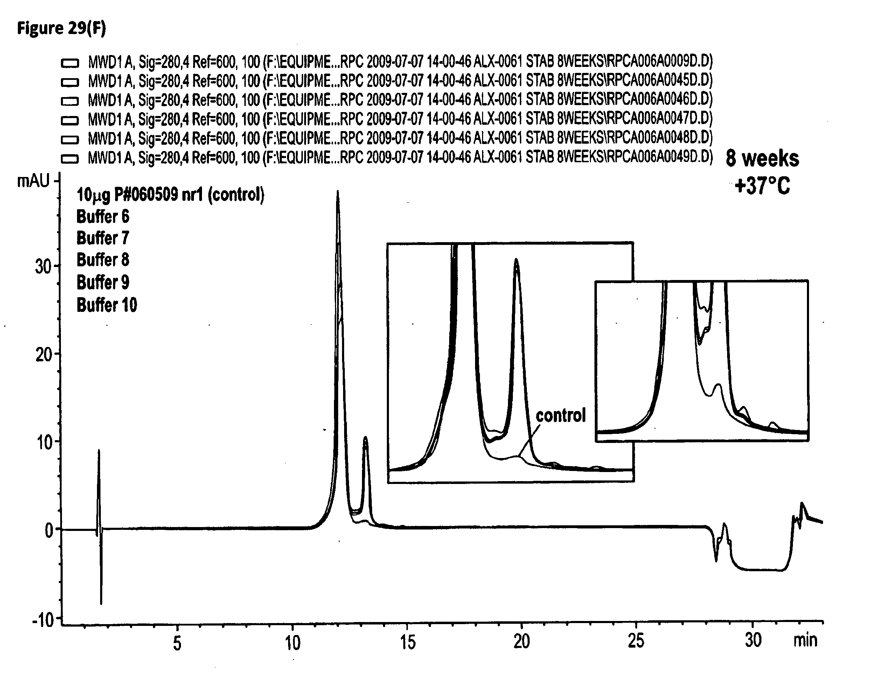

The RP-HPLC chromatograms from the ILR304 stability samples stored for up to 8 weeks at 37°C are shown in Figure 29.

The RP-HPLC profile of ILR304 at time point 0 weeks included a main peak, with 2 badly resolved shoulders eluting before the main material, a first postpeak corresponding to the pyroglutamate-containing variant of ILR304 and a second postpeak, corresponding to the ILR304 variant missing one disulphide bridge. The identity of both variants has been confirmed by LC-MS.

After storage during 1 week, the surface area of the second postpeak had decreased to a relative area % of 0, most likely due to spontaneous oxidation into the correctly folded molecule.

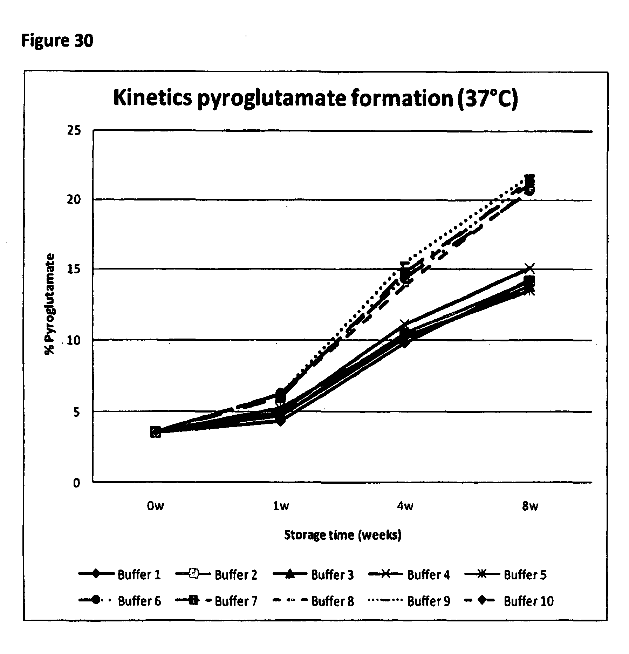

The surface area of the pyroglutamate peak increased with storage time while the surface area of the main peak decreased. The total surface area was not changing significantly over time or among the different buffers. Figure 30 clearly demonstrates that the kinetics of pyroglutamate formation was different in L-histidine, pH 6.5 versus phosphate, pH 6.5. At all time points, less pyroglutamate was present in the L-histidine buffer. On the other hand, there was no correlation between the type of excipient present in the buffer and the amount of pyroglutamate being observed.

After storage for 8 weeks, two new postpeaks were being formed. The first postpeak was situated between the main peak and the pyroglutamate peak, while the second postpeak eluted just after the pyroglutamate peak. The identity of these variants is currently not known.

### Capillary isoelectric focusing (cIEF)

clEF integration data of IL6R304 stored for 8 weeks at 37 °C in the different buffers are shown in Table 22. The samples formulated in 15 mM L-histidine, pH 6.5 contain less charge variants compared to the phosphate buffer.

### Potency assay and Biacore

The potency of the samples stored for 8 weeks at 37°C in buffers 1-5 was determined relative to an unstressed reference batch using the HSA-binding ELISA and the inhibition ELISA for IL-6R as described in Examples 2.1.8 and 2.1.9 (Table 23). The HSA binding functionality of the samples stored in buffers 1-10 was also analyzed using Biacore (Table 24). Samples formulated in the same buffers and stored at -70°C were included as the reference molecules.

Whereas the potency assays showed comparable HSA and IL-6R binding potencies between the stability samples and the reference material, Biacore analysis demonstrated some differences in HSA binding activities.

Overall, the activities of the samples formulated in phosphate buffer (buffers 6-10) were lower than in L-histidine (buffers 1-5). A functionality loss of approximately 16% was observed in the buffers containing a combination of sucrose and glycine (buffer 4 and 9). The combination of sucrose and mannitol (buffer 5 and 10) showed no loss of functionality of IL6R304 in the L-histidine buffer, while a decrease of 10% was observed in the phosphate buffer. Formulations containing either mannitol, sucrose or trehalose showed an activity between 90 and 100% after storage for 8 weeks at 37°C.

### General conclusion about the storage stability study at 37°C

Storage for up to 8 weeks of IL6R304 in different formulation buffers under temperature stress conditions (37°C) resulted in the following observations:
- The propensity of IL6R304 to form oligomers was dependent on the buffer and excipient: about 2-fold less oligomers were being formed in L-histidine buffer compared to phosphate buffer, while the presence of a non-reducing sugar suppressed the extent of IL6R304 oligomerization even further;
- The chemical stability of IL6R304 was better in L-histidine buffer compared to phosphate buffer;
- The HSA binding activity was maintained longer in L-histidine buffer compared to phosphate buffer.

### Example 2.8: Stability under stir stress

IL6R304 was formulated at 1 mg/mL in the 10 different buffers shown in Table 21. Aliquots of 5 mL were stirred at maximum speed for up to 24 hours at 2-8°C. Samples were analyzed after 2, 4 and 24 hours of stirring.

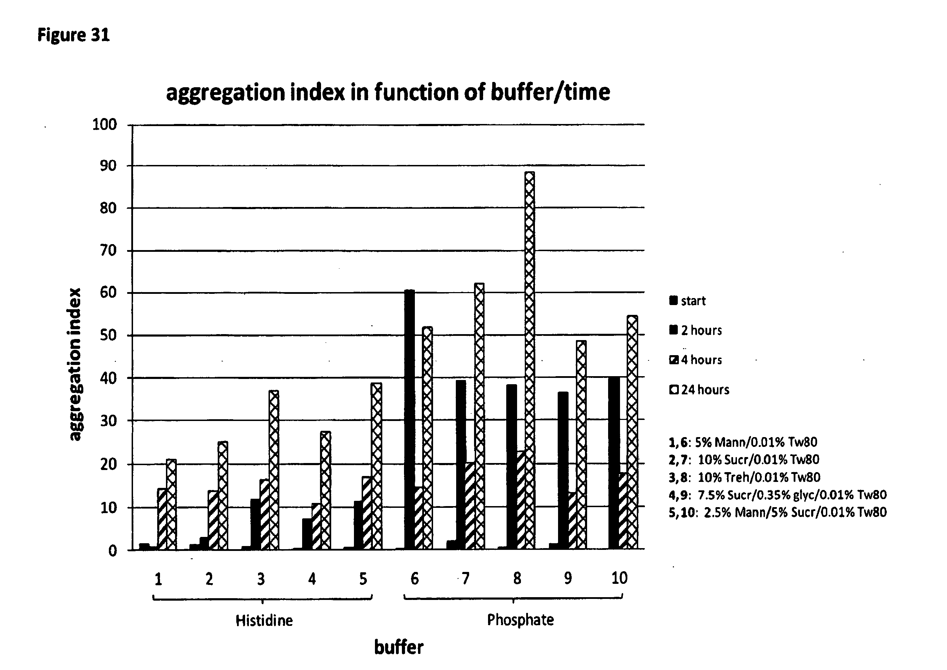

All solutions remained clear after 2 hours of stirring (Table 25). An increase in turbidity was observed in six out of ten buffers after 4 hours. The highest opalescence was present in buffer 10. Overall, the increase in turbidity was more pronounced in the phosphate buffers (buffer 6-10). These observations were confirmed after determination of the aggregation index, defined as 100 * OD350 / (OD280 - OD350) (Figure 31). No soluble aggregates were observed during SE-HPLC of the different samples.

In conclusion, the stir stress data suggest a somewhat better stir stress stability of IL6R304 in L-histidine, pH 6.5 compared to phosphate buffer, pH 6.5. No significant differences were observed between the various excipients, although a slightly higher turbidity was observed in the presence of 10 % trehalose and 2.5% mannitol/5% sucrose.

### Example 2.9: Long-term stability study at -70 °C, +5°C and +25°C

IL6R304 batch CMC-D-0048, formulated in 15 mM L-Histidine, 8% sucrose, 0.01% Tween80 (pH6.5) at 10.52 mg/mL, was stored for 6 months at -**70°C**, +5°C and +25°C. Samples were analysed after 3 and 6 months of storage by visual inspection (appearance), A280 (content), SEC-HPLC, clEF, RP-HPLC and potency assays (1L6R inhibition assay and HSA binding assay). The results are summarized in Table 42, Table 43 and Table 44 for storage at -70°C, +5°C, and +25°C, respectively.

There were no significant changes in appearance, content, potency, clEF and HPLC profiles between the control sample (timepoint 0 months) and all test samples stored at -70°C or 5°C indicating that IL6R304 is stable for at least 6 months under these conditions. Regarding the sample stored at +25°C, the following observations were made when comparing the results of the stressed samples and the control sample:
- SE-HPLC: there is a small, yet gradual increase in the surface area of the pre peak (oligomers) and post peak (degradation fragments).
- clEF: a post peak is being formed which is believed to correspond to the pyroglutamate variant.
- RP-HPLC: three new peaks are being formed, i.e. a pre peak, most likely corresponding to degradation fragments that are present in the samples (see also SEC-HPLC data) and two yet unidentified post peaks. The surface area of pre peak 2 and post peak 2 (pyroglutamate) are gradually increasing with prolonged incubation time.
- No potency loss is observed in the samples stored for up to 6 months at +25°C.

### Example 3: Formulation and stability studies with Nanobodies that bind IL23

### Example 3.1: Materials and methods used in the study

### 3.1.1 Single variable domains

23IL0064 (SEQ ID NO: 5; EVQLLESGGGLVQPGGSLRLSCAASGRIFSLPASGNIFNLLTIAWYRQAPGKG RELVATINSGSRTYYADSVKGRFTISRDNSKKCLYLQMNSLRPEDTAVYYCQTSGSGSPNFWGQGTLVTVSSGGGGS GGGSEVQLVESGGGLVQPGNSLRLSCAASGFTFSSFGMSWVRQAPGKGLEWVSSISGSGSDTLYADSVKGRFTISR DNAKTTLYLQMNSLRPEDTAVYYCTIGGSLSRSSQGTLVTVSSGGGGSGGGSEVQLLESGGGLVQPGGSLRLSCAAS GRTLSSYAMGWFRQAPGKGREFVSRISQGGTAIYYADSVKGRFTISRDNSKNTLYLQMNSLRPEDTAVYYCAKDPS PYYRGSAYLLSGSYDSWGQGTLVTVSS) has been described as SEQ ID NO: 2616 in WO 2009/068627. 23IL0064 consists of three humanized variable domains of a heavy-chain Ilama antibody: 119A3v16 and 81A12v4, binding different epitopes of IL23 p19, and the ALB8 binding HSA. 23IL0075 (SEQ ID NO: 6; EVQLLESGGGLVQPGGSLRLSCAASGRIFSLPASGNIFNLLTIAWYRQAPGKGRELVATINSGSRTYYADSVK GRFTISRDNSKKTLYLQMNSLRPEDTAVYYCQTSGSGSPNFWGQGTLVTVSSGGGGSGGGSEVQLVESGGGLVQP GNSLRLSCAASGFTFSSFGMSWVRQAPGKGLEWVSSISGSGSDTLYADSVKGRFTISRDNAKTTLYLQMNSLRPED TAVYYCTIGGSLSRSSQGTLVTVSSGGGGSGGGSEVQLLESGGGLVQPGGSLRLSCAASGRTLSSYAMGWFRQAPG KGREFVARISQGGTAIYYADSVKGRFTISRDNSKNTLYLQMNSLRPEDTAVYYCAKDPSPYYRGSAYLLSGSYDSWG QGTLVTVSS) has been described as SEQ ID NO: 2622 in WO 2009/068627. 23IL0075 consists of three humanized variable domains of a heavy-chain Ilama antibody: 119A3v16 and 81A22v5, binding different epitopes of IL23 p19, and the ALB8 binding HSA. The subunits in both Nanobodies are fused head-to-tail with a 9G/S linker.

231L0064 (3.79 mg/mL in D-PBS) and 23IL0075 (4.21 mg/mL in D-PBS) were expressed in Pichia pastoris. After clarifying the fermentation broth via a centrifugation step, followed by a TFF step, the Nanobodies were captured on MabCapture A (Poros), followed by an elution at pH 2.6 using 100mM glycine. A buffer switch to 1/10 D-PBS was performed, and the Nanobodies were further polished on Poros 50HS (Poros). Finally, a treatment with 50 mM OGP for LPS-removal was performed, followed by a final size exclusion step using Superdex 75 pg (GE Healthcare).

Unstressed samples in D-PBS or other formulations were used as reference material for analyzing the storage stability samples.

### 3.1.2 Other critical reagents

Reagents used in the study are given in Table 26. The complete composition of D-PBS was 137 mM NaCl, 2.7 mM KCl, 10 mM Sodium Phosphate dibasic, 2 mM Potassium Phosphate monobasic.

### 3.1.3 Equipment and methods for measurements

HPLC experiments were carried out on an Agilent 1200 series instrument from Agilent Technologies (Palo Alto, USA). The columns used were:
- RP-HPLC: Zorbax 300SB-C3 5-micron, 4.6 X 150 mm (Agilent, Cat. No. 883995-909) and Zorbax 300SB-C8 5-micron, 4.6 X 150 mm (Agilent, Cat. No. 883995-906)
- SE-HPLC: TSKgel G2000SW_{XL} (Tosoh Bioscience, Japan; Part#08540)
- IEX-HPLC:: ProPac WCX-10, 4x250 mm, 10 µm (Dionex)

Concentration determinations of the Nanobodies was done with NanoDrop ND-1000 (Thermoscientific), with a Uvikon 943 Spectrophotometer (Kontron Instruments) or an Eppendorf Biophotometer 6131 at 280 nm.

Particle size distribution was measured on a PAMAS SVSS-C particle counter (PArtikelMess- und AnalyseSysteme GMBH).

Osmolality measurement was done with an osmometer Model 3320 from Advanced instruments.

The thermal shift assay was performed on a LightCycler480 Q-PCR device (Roche).

For determination of the Tm, an automated VP-capillary Differential Scanning Calorimeter (DSC, MicroCal) was used.

Elastic light scattering was measured in a Jasco Spectrofluorometer (FP-6500).

### 3.1.4 Purity assay of the Nanobodies by Size Exclusion High Performance Liquid Chromatography (SE-HPLC)

The 5E-HPLC assay consisted of a pre-packed silica gel TSKgel G2000SW_{XL} column equipped with a guard column pre-column filter, a mobile phase consisting of KCl, NaCl and phosphate buffer pH 7.2 (D-PBS) and UV detection at 280 nm. The relative amount of specific protein impurity was expressed as area %, and was calculated by dividing the peak area corresponding to the specific protein impurity by the total integrated area.

### 3.1.5 Purity assay of the Nanobodies by Reverses Phase High Performance Liquid Chromatography (RP-HPLC)

In the RP-HPLC assay a Zorbax 300SB-C3 column or Zorbax 300SB-C8 column (Agilent Technologies, Palo Alto, US) was used. The relative amount of a specific protein impurity was determined by measuring the light absorbance of the components eluting from the RP-HPLC column. The relative amount of a specific protein impurity, expressed as area %, was calculated by dividing the peak area corresponding to the impurity by the total integrated area.

### 3.1.6 Purity assay of the Nanobodies by Ion Exchange High Performance Liquid Chromatography (IEX-HPLC)

The IEX-HPLC assay combined the use of a pre-packed Dionex ProPac WCX-10 weak cation exchange column, a mobile phase consisting of citrate buffer pH5.5 and UV detection at 280 nm. After loading the protein(s) on the column, bound materials were eluted by a sodium chloride gradient. The relative amount of the specific protein, variant, or impurities expressed as area %, was calculated by dividing the peak area corresponding to the specific protein or to any protein impurity by the total area of all integrated peaks.

### 3.1.7 Measurement of particle size distribution (PAMAS)

The measurements on the PAMAS SVSS-C particle counter were performed as follows: 100 µl sample was diluted 1/10 in 1 mL MilliQ water and 10 consecutive measurements were performed in all 16 channels (diameter set 1, 2, 3, 4, 5, 6, 7, 8, 9, 10, 15, 25, 50, 100,150 and 200 µm). For calculation of the average value, the first 2 measurements were excluded and the dilution factor was taken into account. The results are given as cumulative data (total particle counts >x µm) or differential data (total particle counts between diameter x and y µm). Only the cumulative data are presented.

### 3.1.8 Capillary Isoelectric Focusing (cIEF)

Capillary Isoelectric Focusing (clEF) is an analysis/separation technique that differentiates proteins with respect to charge, i.e., it separates proteins according to their isoelectric points (pl). The separation principle is similar to gel-based/flatbed IEF but differs mainly in its format, that is, the separation takes place in an open tube of narrow format (capillary) eliminating the need for any anticonvective matrix support. Also, clEF is a fully automated instrument with online detection and data acquisition. A drawback of traditional cIEF in a conventional CE instrument is that the focused (stationary) zones must be mobilized past the single-point detection area in order to record the signal. During mobilization the zones may become broadened with contaminant loss of resolution and decreased detectability. Moreover, the analysis time and risk of protein aggregation/precipitation will increase. By imaging clEF the focusing process is followed in real-time over the whole-column/capillary by a CCD camera excluding the mobilization step. As soon as the focusing process is completed the analysis run is finished.

### Example 3.2: Melting temperature of the Nanobodies

The measurement of the melting temperature of a protein in different buffers is a faster way to screen for buffers in which the protein has the highest physical stability. It is generally accepted that this will be predictive for the long term stability at lower temperatures. The melting temperature can be determined using many different techniques.

### 3.2.1 Melting temperature of 23IL0064 in different formulation buffers measured by Thermal shift assay

We used the thermal shift assay (TSA), which measures the change in fluorescence intensity when the Sypro orange binds the hydrophobic parts of the protein that is undergoing thermal unfolding. The thermal shift assay was performed on the LightCycler480 Q-PCR device (Roche) making use of 96-well plates.

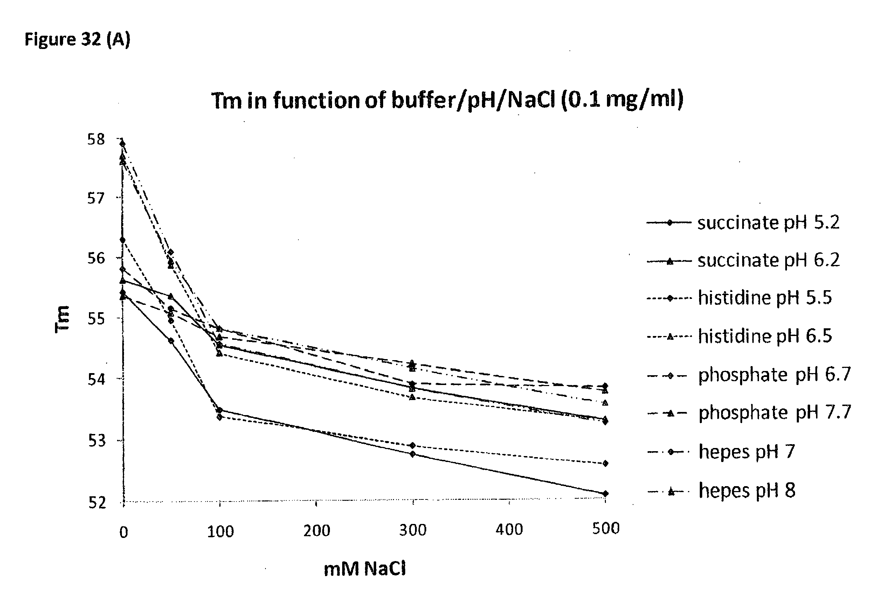

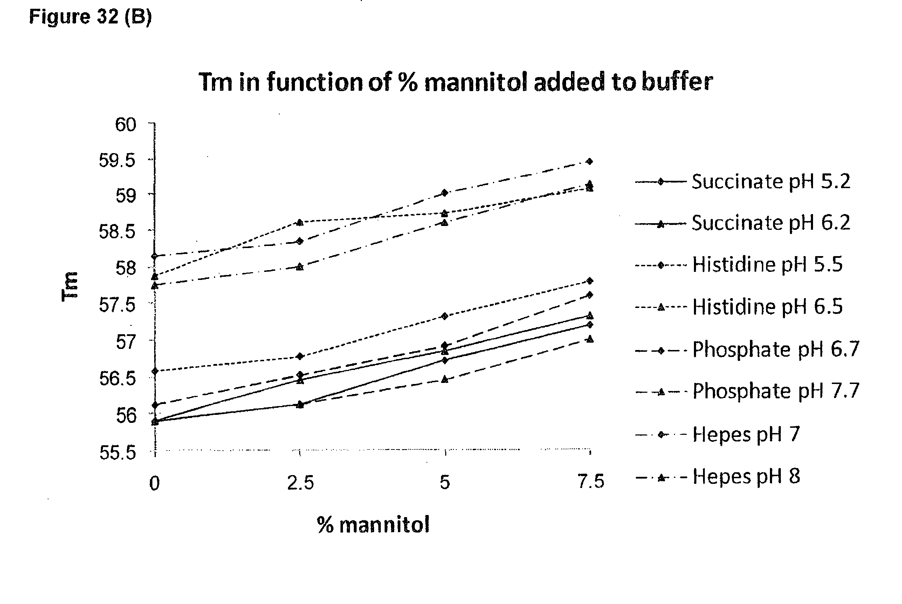

In a first study, the effect of buffer couple, ionic strength and pH on the thermodynamic stability of 231L0064 was evaluated in a thermal shift analysis experiment. The tested buffers used are presented in Table 28. Each buffer was tested at a pH interval spanning 1 pH unit around their pKa. An overview of the results is presented in Figure 32. The melting temperatures decreased with increasing salt concentration. Further pH 5.2 and pH 5.5 seemed less favorable compared to pH 6.2 and above. The best buffers were Hepes (pH 7 and 8) and Histidine pH 6.5. In all buffers, the addition of mannitol slightly increased the melting temperatures.

### 3.2.2 Melting temperature of 231L0064 and 23IL0075 in different formulation buffers measured by Thermal shift assay and differential scanning calorimetry (DSC)

23IL0064 and 23IL0075 were analyzed in parallel in different formulation and purification buffers by the thermal shift assay (TSA) (Tables 34) and DSC (Table 33). For both molecules and in all tested buffers (acetate, MES, Hepes, TRIS, and Histidine), the melting temperatures decreased with increasing NaCl concentration, and the melting temperatures were highest in the presence of mannitol,

### 3.2.3 Melting temperature of 23IL0075 in a wide range of formulation buffers measured by Thermal shift assay

The melting temperature for 23IL0075 in another range of buffers and a lower pH range was screened by TSA. For this, a design of experiments was set up to investigate the Tm of 23IL0075 varying the following parameters: pH range between 5 and 6, buffer concentration between 10 and 50 mM, and 4 different buffers: histidine, acetate, phosphate and succinate. A full factorial design + central composite design was performed, in total 22 different buffers were tested in 84 experiments. The experiments were divided over two 96-well plates, on the second plate the pH range was expanded to 4.8 and 6.2, and the buffer concentration to 7 and 65 mM. The phosphate buffer was only tested at pH 6 and 6.2. From the experimental results, for each buffer a model was built that was used to predict Tm's as a function of the buffer concentration and the pH. The results are shown in Table 35.

Succinate clearly had overall the lowest predicted Tm values. For acetate and histidine at low concentrations and high pH the largest predicted Tm values were obtained. In fact the model showed for all buffers highest predicted Tm values for lowest buffer concentrations. According to the Design-Expert Numerical Optimization of the model the best buffer was histidine buffer pH 6.2 (desirability 1), followed closely by acetate pH 6 (desirability 0.96) and phosphate pH 6 (desirability 0.90).

### 3.2.4 Confirmation of Tm by differential scanning calorimetry

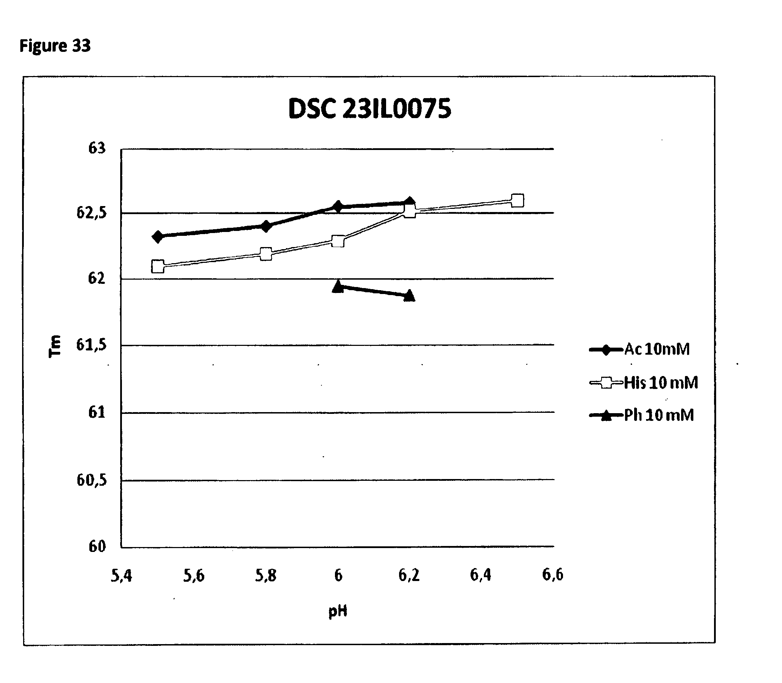

We confirmed the Tm determination by differential scanning calorimetry in the buffers selected in Example 3.2.3. The results are shown in the graph in Figure 33. The trends were identical to what was observed in the TSA though the absolute values of the Tm were higher: around 62°C in DSC compared to around 59 °C in the TSA.

### 3.2.5 Melting temperature of 23IL0075 in a range of formulation buffers to explore a wide range of excipients measured by Thermal shift assay

A second design of experiments (DOE) was prepared to study the influence of some combinations of excipients on the thermodynamic stability of 231L0075. The combination of a sugar or a polyol (mannitol, sucrose or sorbitol), with an amino acid (Glycine or arginine/glutamic acid mixture), and a non-ionic detergent (Tween 80, Tween 20 or P-F68) were studied. These combinations were tested in three buffers (10mM Acetate pH 5.5, 10mM Histidine pH 6.0 and 10mM Phosphate pH 6.0) (see Table 35). Based on the measured melting temperatures, the optimal buffer compositions were calculated (see Table 36). Highest melting temperatures were predicted for the histidine buffer, the lowest melting temperatures for the phosphate buffer. For all three buffers sucrose came out as the best excipient, the mixture of arginine and glutamic acid as the worst. Mannitol and sorbitol, alone or in combination with glycine were also good as excipient, though always gave a little bit lower Tm than sucrose. Glycine alone was not so efficient.

### Example 3.3: Solubility of the Nanobodies

### 3.3.1 Concentration experiments to determine the solubility of 23IL0064

The IL23 Nanobody 231L0064, respectively in D-PBS buffer, in NaPhosphate 10 mM pH 7 with 50 mM NaCl, and in L-histidine 40 mM pH 6 with 50 mM NaCl, was concentrated stepwise with a Vivaspin concentrator (Vivascience 5,000 MWCO 0.5-mL and 5-mL concentrators). During the concentration experiment, the retentate was regularly gently mixed and the concentration determined by OD280 measurements. The presence of insoluble aggregates in the retentate was verified by checking the protein concentration before and after centrifugation at maximum speed in a benchtop Eppendorf centrifuge. In all three buffers a concentration of 50 mg/mL could be reached without visual precipitation. Then the protein solution was transferred from the 5 mL to the 0.5 mL Vivaspin^{®} concentrator and concentrated at a much higher centrifugal force (15000xg instead of 1500xg). This caused rapid further concentration, and local concentrations close to the membrane were even much higher than the average concentration measured after recovery of the retentate. In Table 27, the final concentrations achieved are summarized, obtained after pipetting up and down the retentate, and centrifugation to remove precipitate.

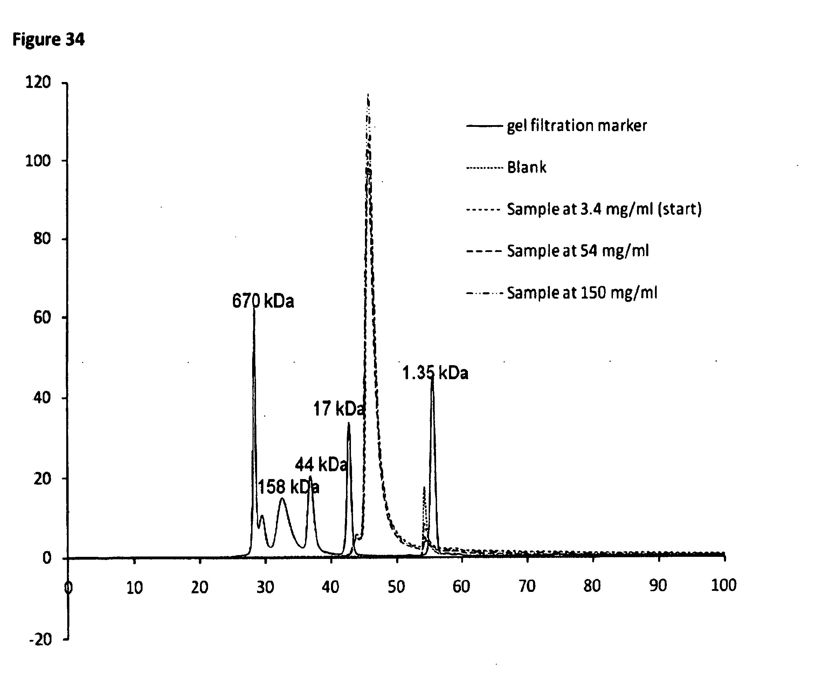

All samples were also analyzed by SE-HPLC. Concentrating in D-PBS buffer resulted in an increase in % pre-peak. In the 10mM phosphate buffer and in the 40mM Histidine buffer, both with 50mM NaCl, the SE-HPLC profile remained exactly the same at 83 and 150 mg/mL compared to the starting material (Figure 34). A generic SE-HPLC method was used on a Phenomenex BioSep SEC S-2000 column, with D-PBS as mobile phase at 0.2 mL/min.

A sample of the concentrated 23IL0064 protein solutions was diluted to 55 mg/mL using the respective buffers for further use in the PEG precipitation method.

### 3.3.2 Determination of the theoretical solubility of 23IL0064

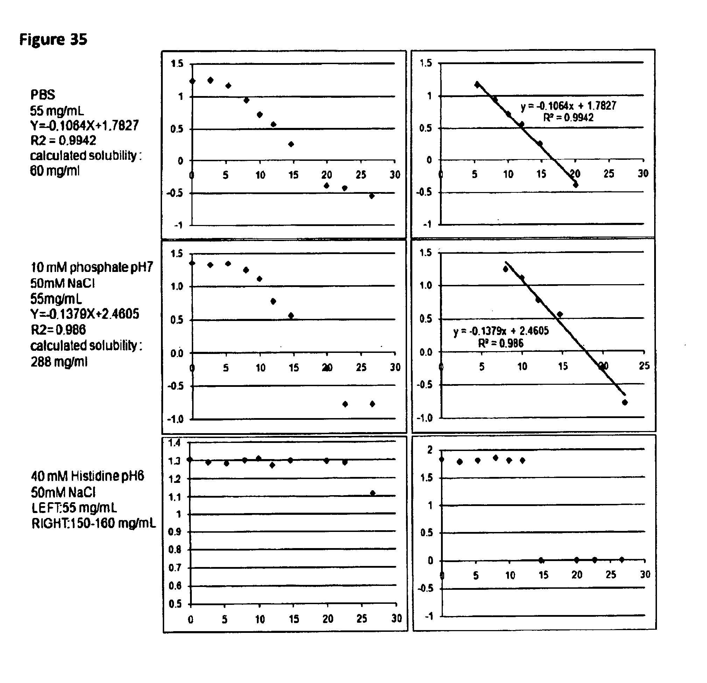

The theoretical solubility of 23IL0064 was determined using the PEG exclusion method in D-PBS buffer, in NaPhosphate 10 mM pH 7, NaCl 50 mM, and in L-histidine 40 mM pH 6, NaCl 50 mM. Briefly, a concentrated Nanobody solution (55 mg/mL) in the respective buffer was incubated for 15 minutes at room temperature in the presence of increasing concentrations of PEG6000. After centrifugation at 20000 x g for 3 minutes, log values of the [soluble protein concentration] were plotted versus PEG6000 concentration (Figure 35). By regression analysis and extrapolation to a zero concentration of PEG6000, the theoretical maximum protein concentration (and thus solubility values) could be obtained for the Nanobody in the buffers tested.

When extrapolating from the regression plots to a zero concentration of PEG6000, a theoretical solubility value of 60 mg/mL and of 288 mg/mL was calculated for 23IL0064 in D-PBS and in 10mM phosphate buffer /50mM NaCl respectively. 23IL0064 showed extremely good solubility in the histidine buffer: in the experiment starting from 55 mg/mL only at a PEG concentration of 27% the protein started to precipitate slightly. Therefore the experiment was repeated with the protein dissolved at 150 mg/mL. There only at 10% PEG precipitation occurred, but then no volume was left for OD measurements. So the solubility was actually too high to obtain a value in this assay.

In conclusion highest solubility was obtained in 40 mM Histidine pH 6 with 50 mM NaCl. In phosphate buffer with 50mM NaCl, the solubility was better than in D-PBS (with 137 mM NaCl).

### 3.3.3 Determination of the theoretical solubility of 231L0064 and 23IL0075

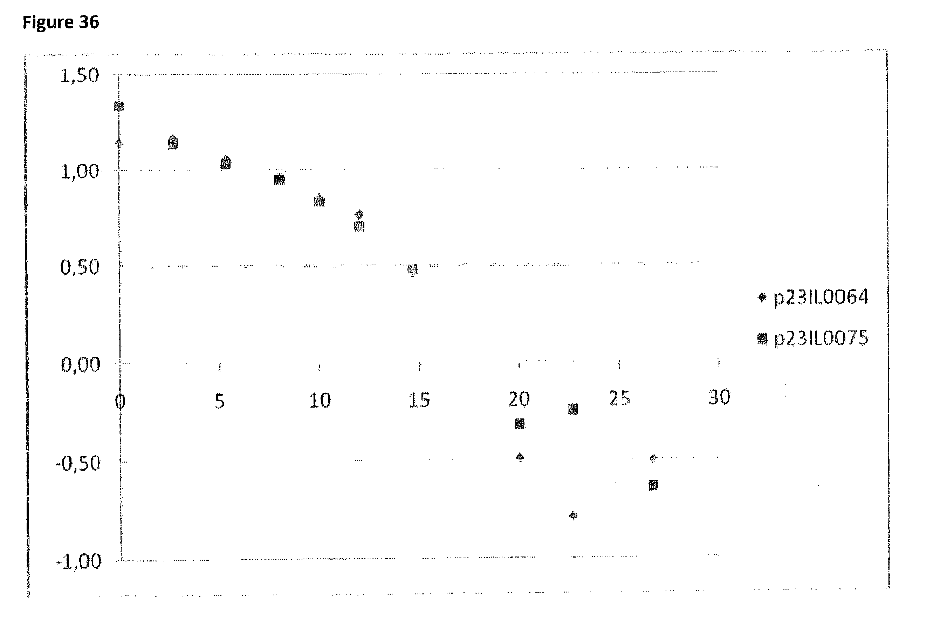

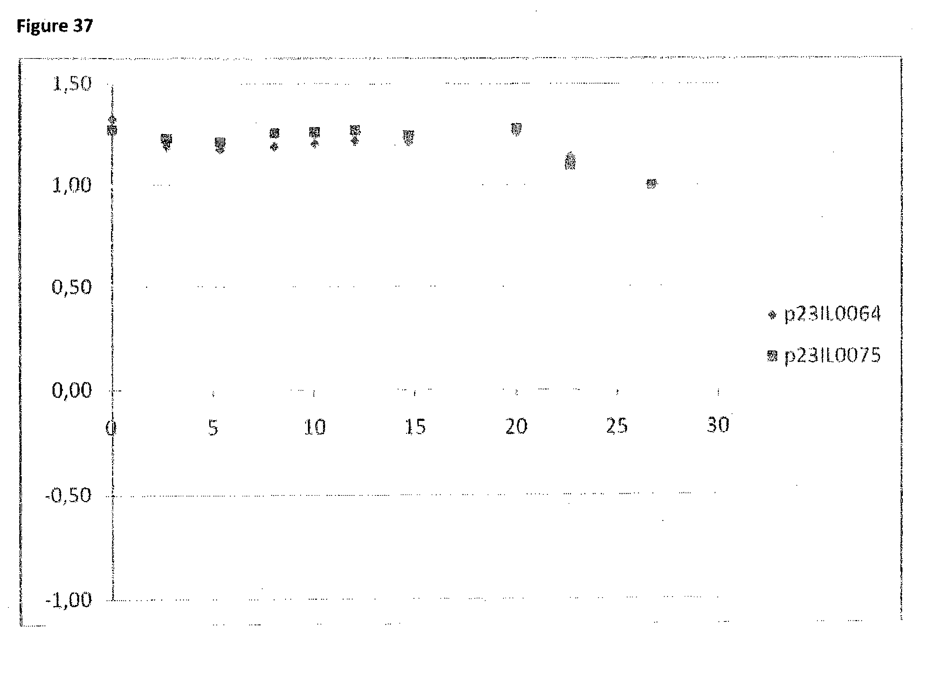

The theoretical solubility was determined for 23IL0064 and 23IL0075 using the PEG exclusion method in NaPhosphate 10 mM pH7, NaCl 50 mM, and in L-histidine 40 mM pH 6, NaCl 50 mM (the same buffers as used in the previous solubility study for 231L0064 described above). The graphs (Figure 36 and Figure 37) representing the protein concentration in the supernatant as a function of the % PEG concentration were very similar as obtained in the previous experiment. Again the apparent solubility in histidine was higher than in the phosphate buffer, and could not be calculated due to minimal precipitation under the protein (5 mg/mL) and PEG concentrations (26.7%) used. The calculated solubility in phosphate buffer pH 7 for 23IL0064 and 23IL0075 were lower than in the previous experiment, i.e. 55 and 42 mg/mL respectively, while we observed up to 288 mg/mL in the earlier experiment. We must stress though that these experiments were performed pipetting extremely low volumes of highly viscous solutions, and therefore the absolute numbers of solubility should be confirmed with other techniques.

### Example 3.4: Stressed stability studies for 231L0064 in D-PBS

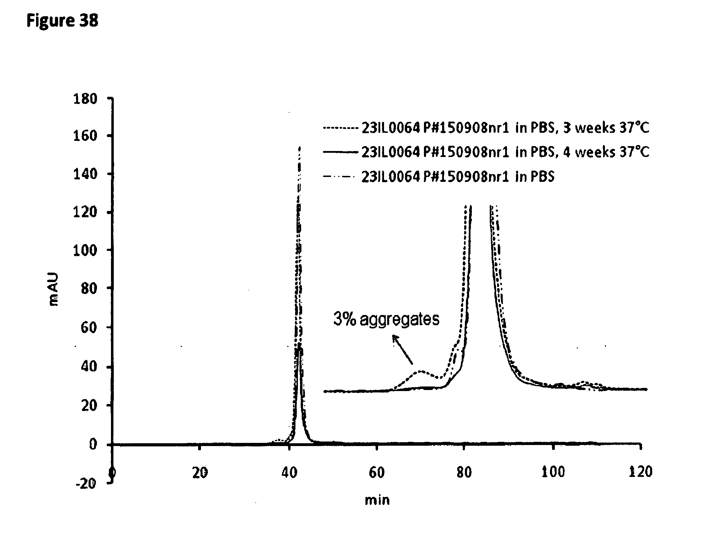

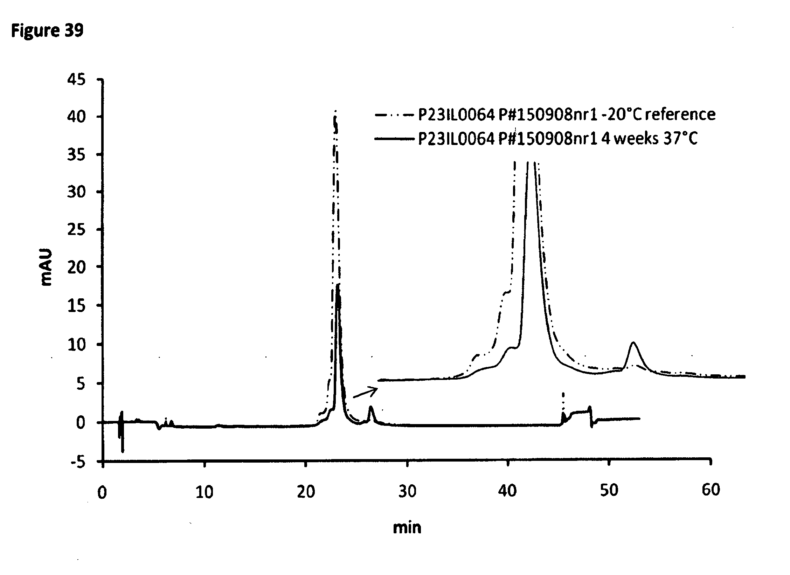

An initial 37°C stressed stability study was performed in D-PBS. The original batch was sterilized through a 0.22 µm filter and 500 µl was stored at 37°C in 1.5 mL-eppendorf vials for each time point (4, 8, 12, 16, 20 and 24 weeks). Additionally approximately 9x 100 µl was stored at -20°C (reference). A first sample was already analyzed after 3 weeks using SE-HPLC and a pre-peak of aggregates was detected (3%) (Figure 38). After 4 weeks at 37°C, the total peak area on SE-HPLC and on RP-HPLC was reduced to only half of the reference sample (Figures 38 and 39). We therefore decided to prematurely terminate the stability study. In some of the remaining samples the content was still measured by OD280 after centrifugation. The loss of material in the 4w-37°C-sample through precipitation (all samples were centrifuged before analysis) was confirmed in the additional 3 samples stressed for 6 weeks at 37°C: in 2 of the 3 samples half of the material was lost (Table 29).

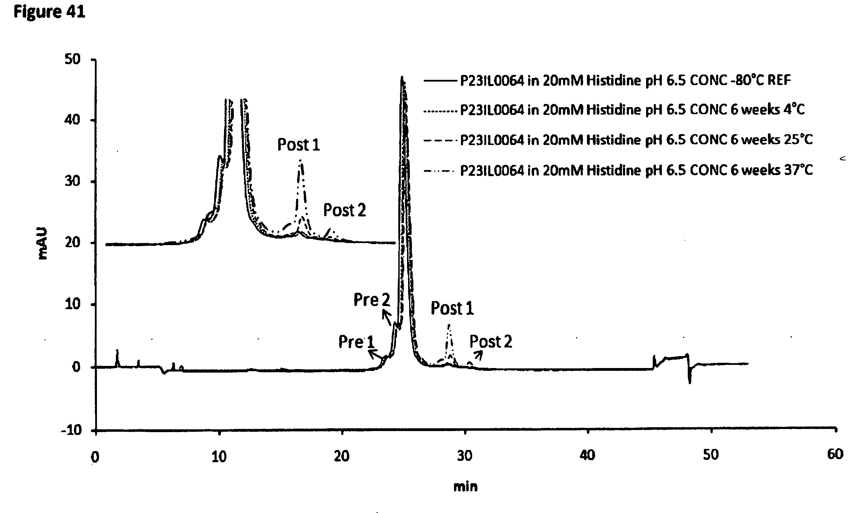

It can be concluded that 23IL0064 in D-PBS easily formed aggregates which precipitate following storage for 4 weeks on at 37°C. In the RP-HPLC analysis of the 37°C-stressed sample also 12% post-peak was observed corresponding to N-terminal pyro-glutamate formation (Figure 41).

### Example 3.5: Stressed Stability Study for 231L0064 in histidine buffer

From the results described in previous Examples on the solubility, the thermal shift assay, and the 37°C-stressed stability in D-PBS for 23IL0064, it was concluded that phosphate was not the optimal buffer for formulation of 23IL0064. In the TSA described in Example 3.2.2, we explored some potential formulation buffers and the highest Tm's were obtained in histidine pH 6.5, Hepes pH 7, and Hepes pH 8. In the solubility experiment (see Example 3.3) the solubility in a 40 mM histidine pH 6, with 50 mM NaCl was very high. We therefore decided to test the storage stability in histidine buffer. In Table 30 a list of tested formulation buffers is given. The goal of this set-up was to compare histidine pH 6.5 with histidine pH 6, investigate the influence of some commonly used excipients, and the influence of a higher concentration on the stability (difference between 5 mg/mL and 22 mg/mL). One sample in Hepes pH 8 was also included, only to be tested after 3 weeks at 37°C.

For this study, 3.2 mL of the original batch was dialyzed to the 20mM Hepes buffer pH 8 and approximately 65 mL (approx. 246g) was dialyzed to the 20mM Histidine buffer pH 6.5. The excipients were added in concentrated solutions (2x), and the sample at pH 6 was prepared by adding HCl. The samples were then concentrated to approximately 5 mg/mL, sterilized through a 0.22 µm filter and aliquoted in 1.5 mL-eppendorfs (500µL/eppendorf) for storage under the different conditions.

### 3.5.1 Stability during freeze thaw

One sample of each of above formulations in histidine was subjected to 10 freeze/thaw cycles. The samples were analyzed by RP-HPLC and OD280 content. No difference with the reference sample (one freeze/thaw) was observed.

### 3.5.2 Shear stress

Two samples of each of above formulations in histidine were subjected to shear stress. The test was conducted in a cold room (4-8°C) in small glass tubes with 300 µl of protein solution, stirred through a magnetic bar for 4 and 8 hours at a medium rotation speed.

In all samples stressed with 4 and 8 hours of shearing clear opalescence was present. This was quantified by OD280 content analysis after centrifugation of the samples. Analysis by SE-HPLC did not reveal any aggregates. RP-HPLC analysis after 4 hours of shear stress showed no degradation, but after 8 hours of stress some increase of the non-resolved pre-peak appeared, especially in the concentrated sample. Also on SDS-PAGE generally no degradation was detected. In Table 31 a crude ranking based on the opalescence and material loss in the content analysis is given.

### 3.5.3 Storage at 4°C, 25°C and 37°C for 6 weeks

The first analysis was performed after 2.5 weeks at 25°C and 37°C storage. The samples were analyzed on RP-HPLC (see also Table 32), SE-HPLC, SDS-PAGE and OD 280/350. Very little degradation was observed after 2.5 weeks (data not shown). The results after 6 weeks of storage at 37°C are discussed below (for the sample in Hepes pH 8, the 2.5 weeks 37°C results are discussed).

### RP-HPLC analysis

RP-HPLC analysis was performed mainly to detect chemical degradation. Stress at 25°C and at 37°C typically caused increase of the post-peak corresponding to the N-terminal pyroglutamate. This post-peak increased less in the histidine buffer pH 6 than in pH 6.5, and fastest in the Hepes buffer pH 8: e.g. after 2.5 weeks at 37°C there was 11% pyroglutamate post peak in Hepes pH 8, 7% in histidine pH 6.5, and 5% in histidine pH 6 (data not shown).

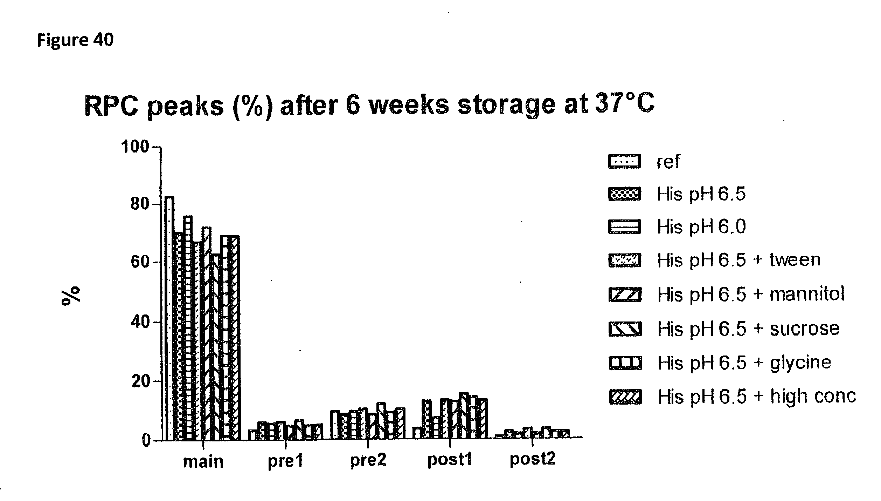

Further a second unknown post-peak appeared. In Figure 40 an overview of the integration data is given. In Figure 41 an overlay between the chromatograms obtained for the different storage temperatures of 231L0064 in histidine buffer pH 6.5 at 22mg/mL is given.

### SE-HPLC analysis

For the samples at 5 mg/mL stressed for 6 weeks at 37°C, small amounts of aggregates (between 0.5 and 1%) were observed. The peaks were integrated. The separated pre- and postpeaks were never higher than 1 percent.

For the sample at 22.4 mg/mL however, 3% aggregates were detected in the sample stored at 37°C.

### SDS-PAGE analysis

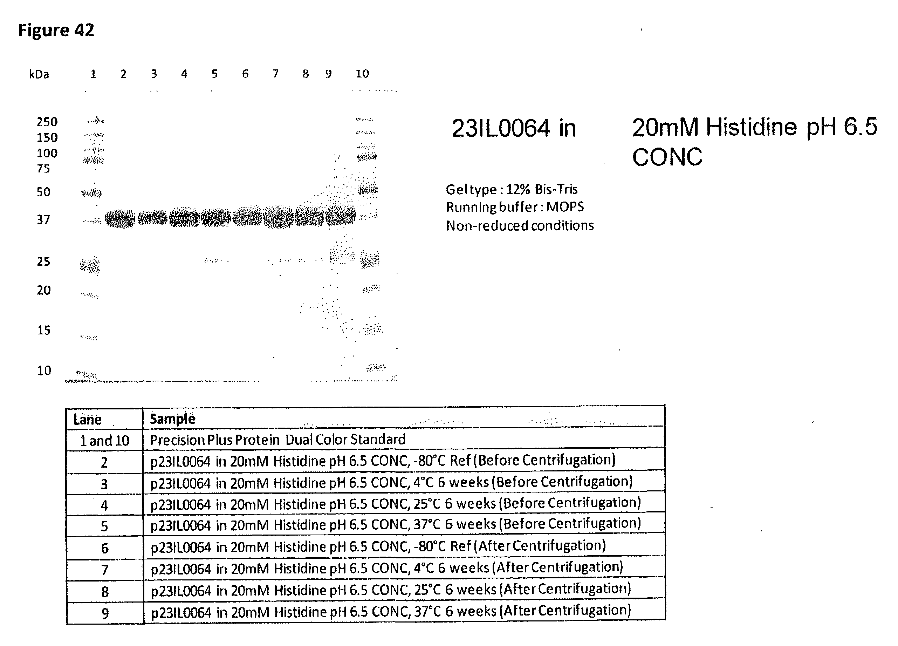

Analysis by SDS-PAGE showed little degradation. For the samples stressed at 37°C for 6 weeks, a slight increase in intensity of a degradation band at a Mw of approximately 27 kDa was observed and some thin bands under the main band were present (Figure 42). No difference between the different formulation buffers was observed.

### Analysis of OD280, OD350 and subvisible particles

All samples in the storage stability study were further analyzed for their Nanobody content (by OD280), for their opacity (by OD350), and to detect subvisible particles (by PAMAS). Very similar results were obtained between the reference and the different temperature storage samples (data not shown).

### Example 3.6: Elastic light scattering

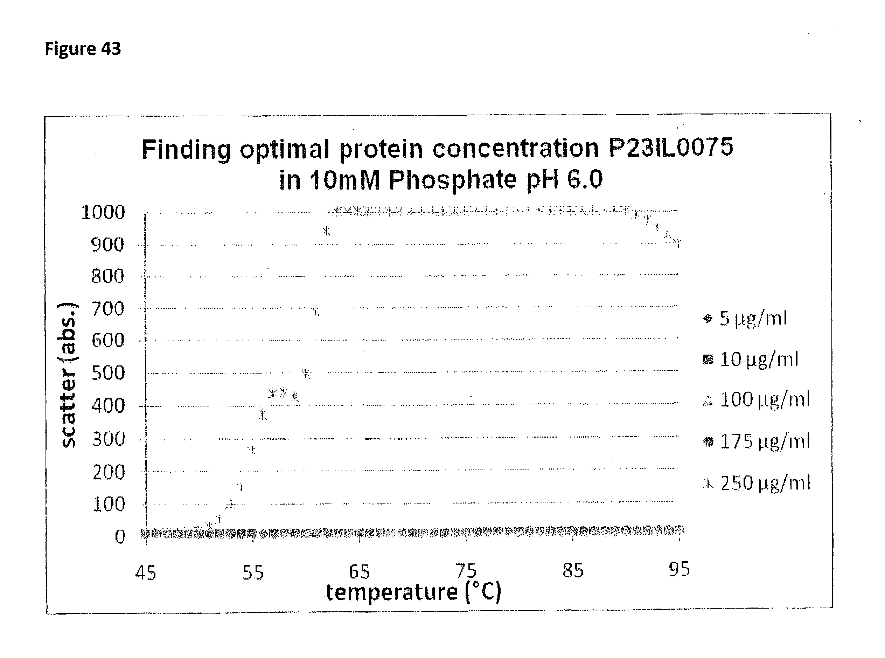

The tendency for aggregate formation of 23IL0075 in the different formulation buffers was determined using elastic light scattering measured at an angle of 90° by temperature-induced denaturation as measured in the Jasco Spectrofluorometer (excitation and emission wavelength 500 nm). First we looked for the optimal protein concentration, using the 10 mM phosphate buffer pH 6.0. At concentrations of 175µg/mL 23IL0075 or lower no increase in scatter intensity was seen in the tested temperature interval: (45-95°C). Only at 250µg/mL scatter was observed. The curve seemed to display two transitions, which could indicate the formation of two different types of aggregates (see Figure 43).

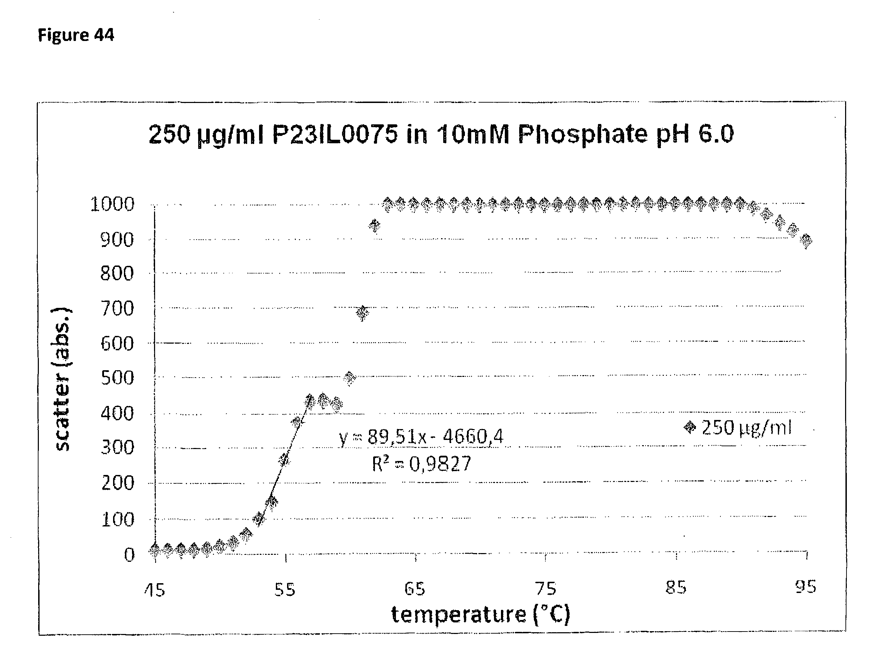

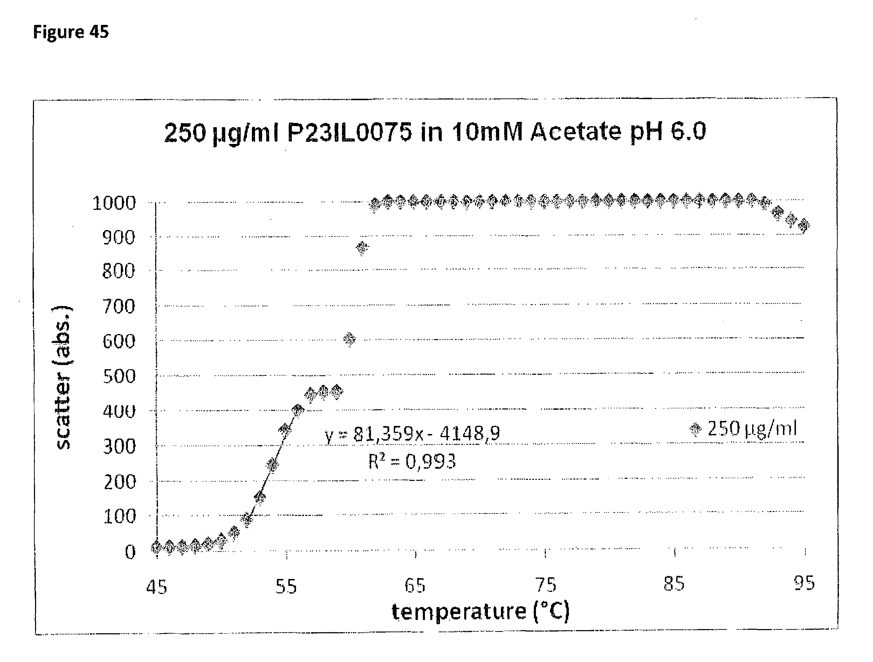

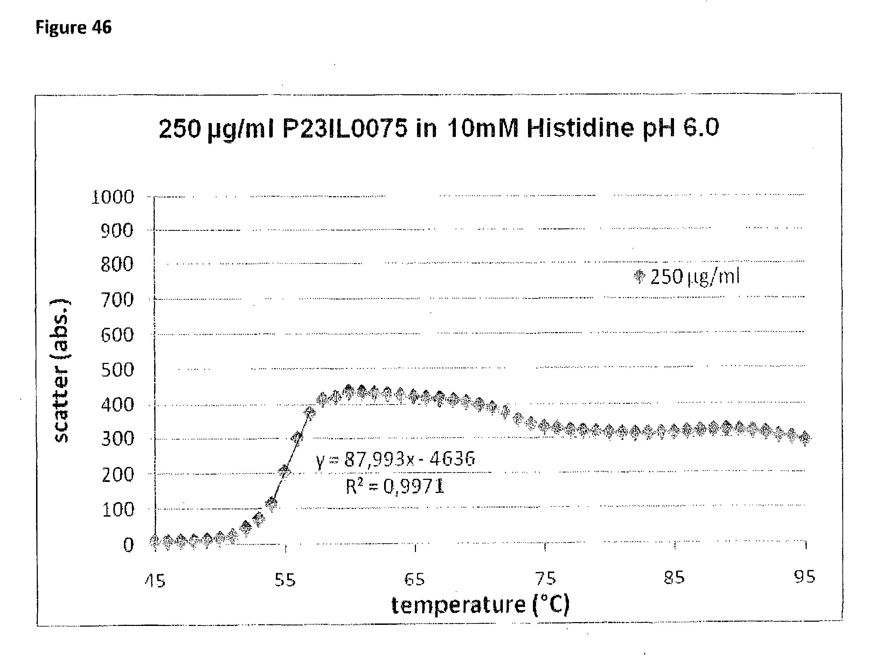

The experiment was repeated for the acetate and the histidine buffers, both at pH 6.0. The aggregation onset temperatures in the three buffers were very similar (Table 37). The main difference between the three buffers was the maximum scatter: it stayed within detector range (around 435 abs) for histidine while it went out of range in the phosphate as well as in the acetate buffers (Figure 44 and 45). In histidine the second transition was absent (Figure 46). As the scatter is proportional to the level of aggregates formed, this indicated that the 10 mM histidine would be a more optimal formulation buffer than 10 mM acetate and 10 mM phosphate pH 6.

### Example 3.7. Freeze/thaw and shear stress study on 23IL0075 in a histidine, acetate and phosphate formulation buffer with mannitol or a mixture of a mannitol and glycine as excipients, and a nonionic detergent as surfactant

The sensitivity of 23IL0075 to freeze/thawing and to shear or stirring has been investigated in different candidate formulation buffers (see Table 38). The freeze/thaw stress study consisted of 10 cycles: 100 µL sample in an eppendorf tube was frozen at -20°C until completely frozen, and thawed at room temperature for 30 minutes followed by gentle mixing. The shear stress test was conducted in a cold room (4-8°C) in small glass tubes with 150 µl of protein solution; the protein solution was stirred through a magnetic bar for 4 hours at a medium rotation speed. All samples were analyzed by RP-HPLC, SE-HPLC and OD500, some samples also by Biacore. On RP-HPLC, no influence of shear or freeze/thaw was detected.

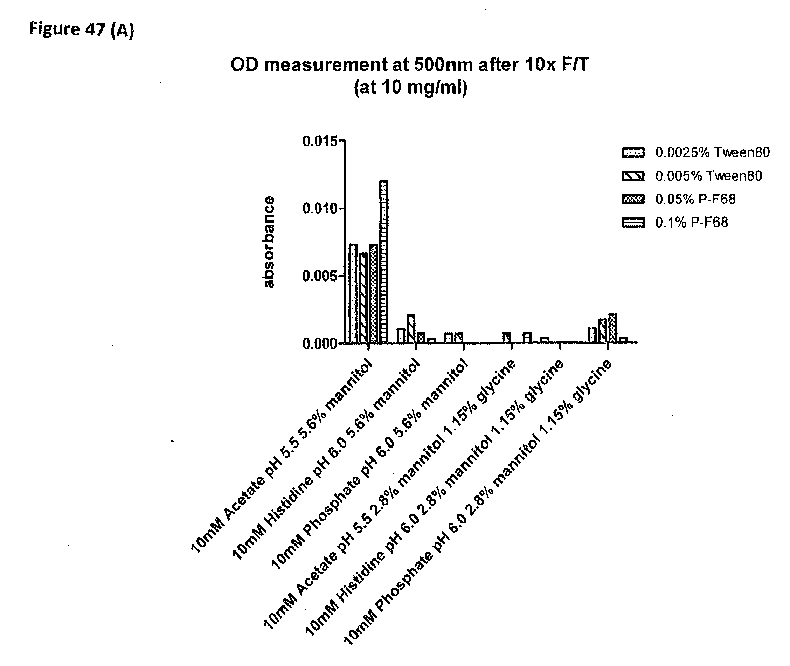

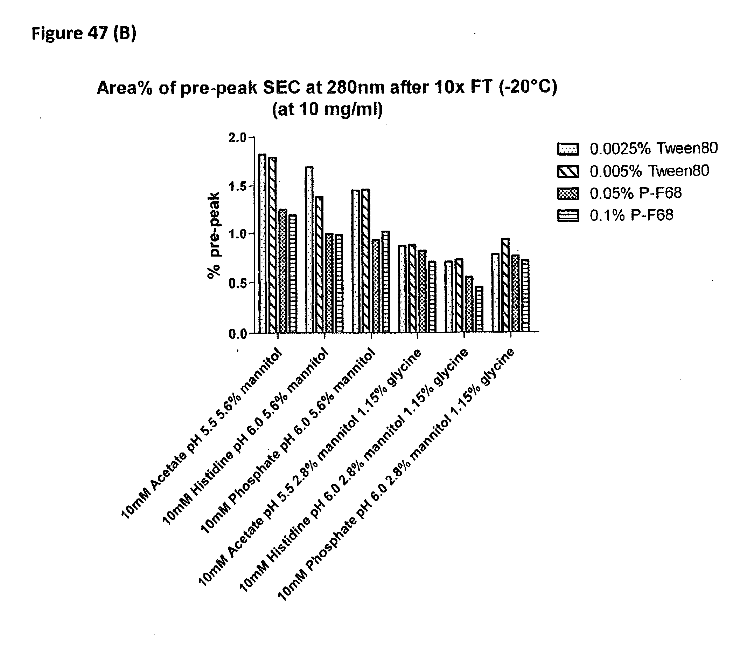

On SE-HPLC, dependent on the formulation buffer, freeze/thaw stress caused an increase in % pre-peak up to 2.5% (Figure 47 (B)). A mixture of mannitol and glycine protected better against freeze/thaw stress than only mannitol as excipient (Figure 47).

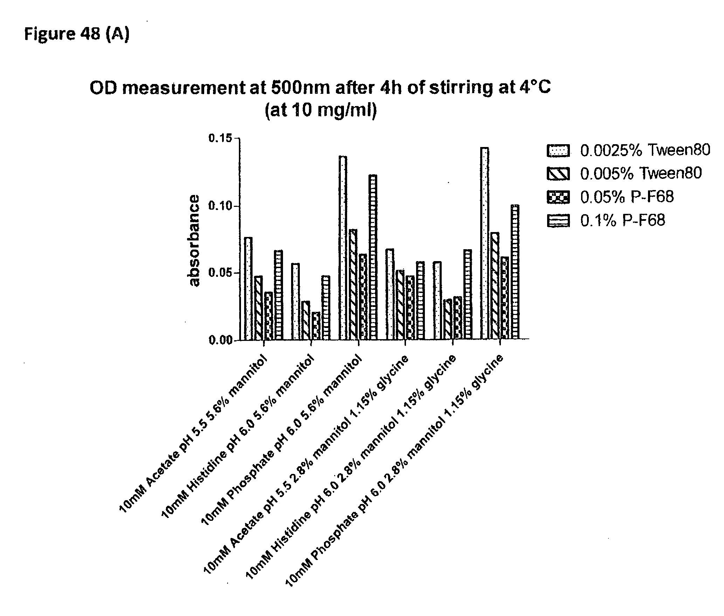

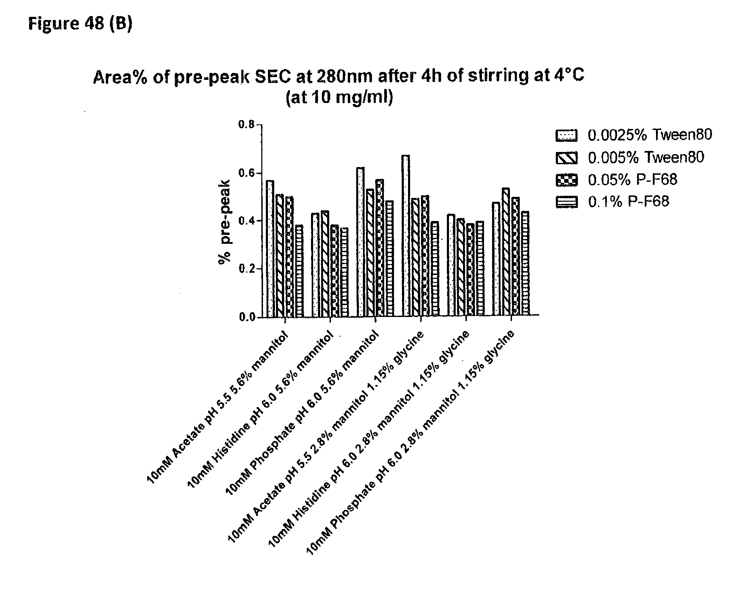

In the shear stressed samples hardly any increase in % pre-peak on SE-HPLC was detected but up to 10 times more opalescence (OD500) was measured than in the freeze/thaw samples. Optical density at 500 nm (OD500) increased in the stirred samples and correlated with the opalescence in the samples.We conclude that in the histidine buffer with 0.05% Poloxamer or with 0.005% Tween 80 the opalescence remained lowest (Figure 48).

### Example 3.8. Freeze/thaw, shear stress and temperature stress (37°C) stability study for 23IL0075 in a histidine formulation buffer with different combinations of excipients.

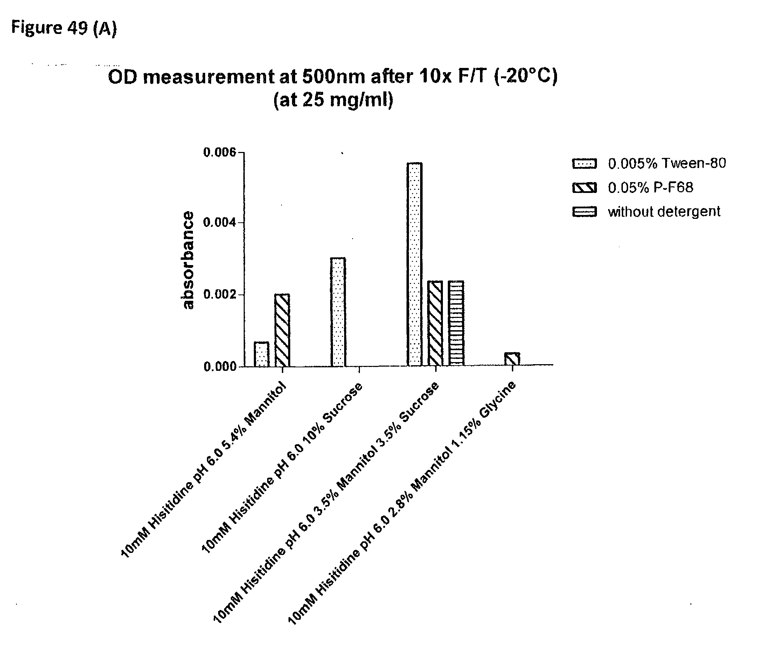

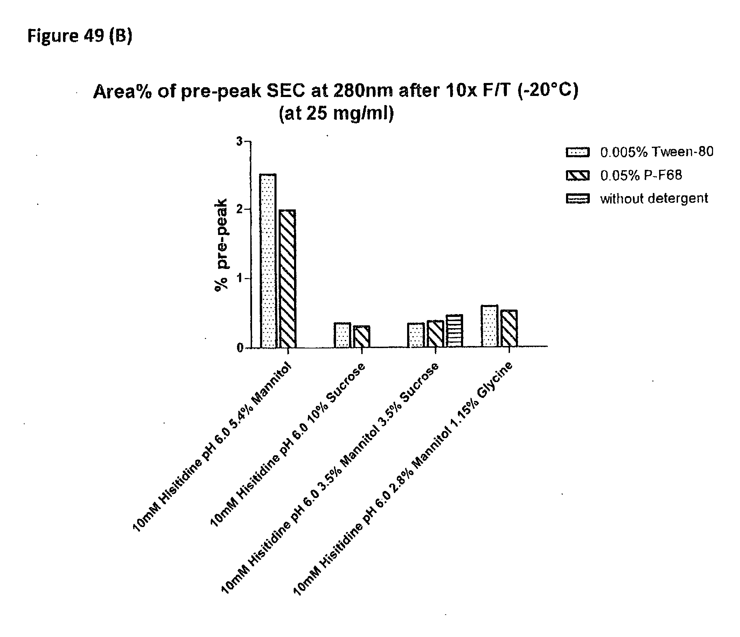

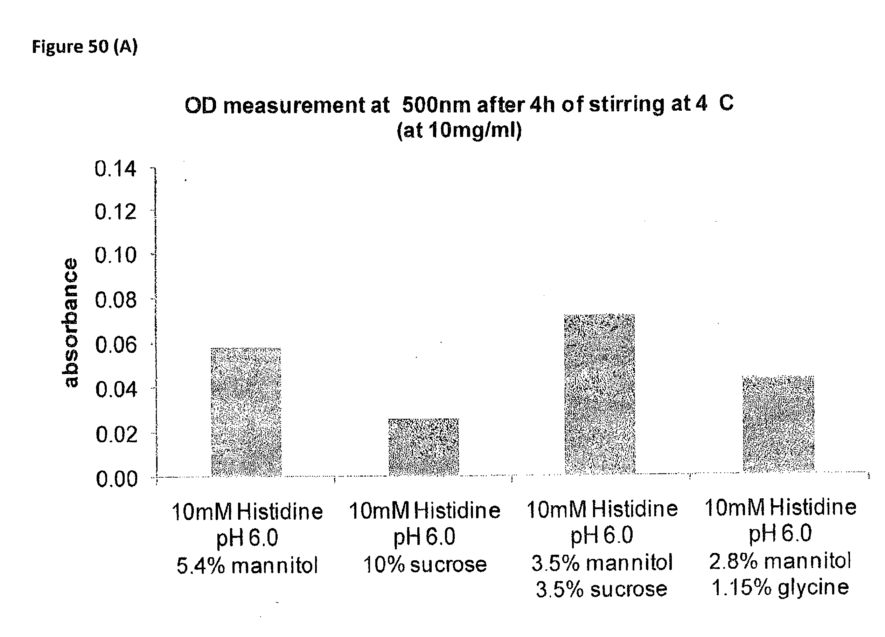

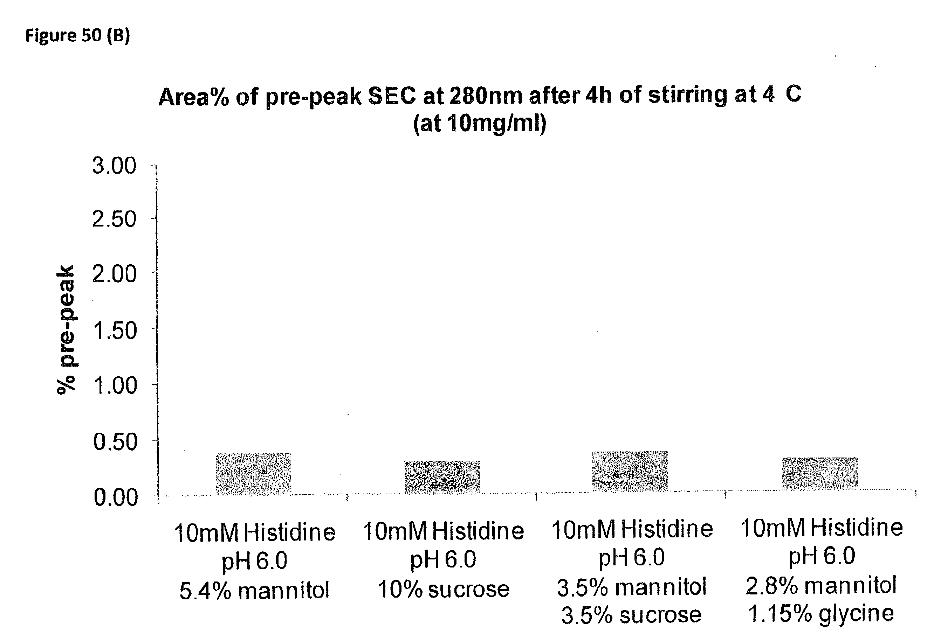

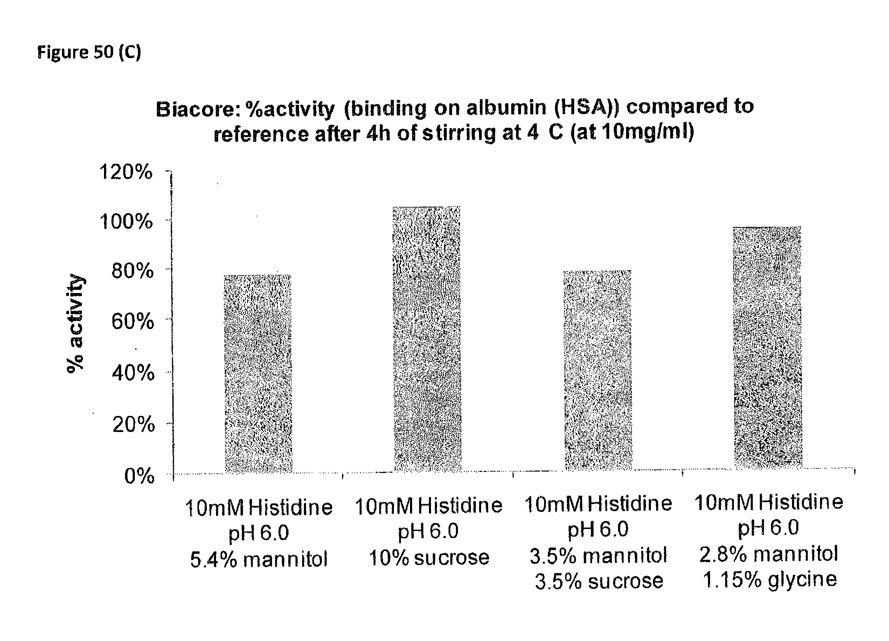

Based on the conclusions of Example 3.7, we further tested freeze/thaw, shear stress and temperature stress (37°C) stability in different histidine formulation buffers (Figure 49 and Figure 50). Different excipients were tested in a formulation with 25 mg/mL of protein. An overview of the formulation buffers tested in F/T and storage stability is presented in Table 39. In Figure 49 the results of OD500 measurements and SE-HPLC after freeze/thaw stress are presented. A negligible increase of OD500 was observed. In SE-HPLC, we saw an increase of oligomers only for the sample with 5.4% mannitol as excipient. Table 40 presents the tested buffers for shear stress. Here no detergents were included, to mimic the situation during the final concentration step of the DSP process. In Figure 50 the OD500, SE-HPLC and Biacore results obtained after 4 hours of stirring of the formulation are presented. Stirring of the sample caused increase in OD500 absorption, but no influence on the % soluble oligomers as measured by SE-HPLC. These samples were also tested for albumin binding on Biacore. We conclude that the protein was best protected against the shear stress by 10% sucrose, followed by a mixture of mannitol and glycine as excipients. By comparison of the values with the values of Example 3.7, we see that the results were reproducible, and that for the shear stress the addition of some detergent was beneficial.

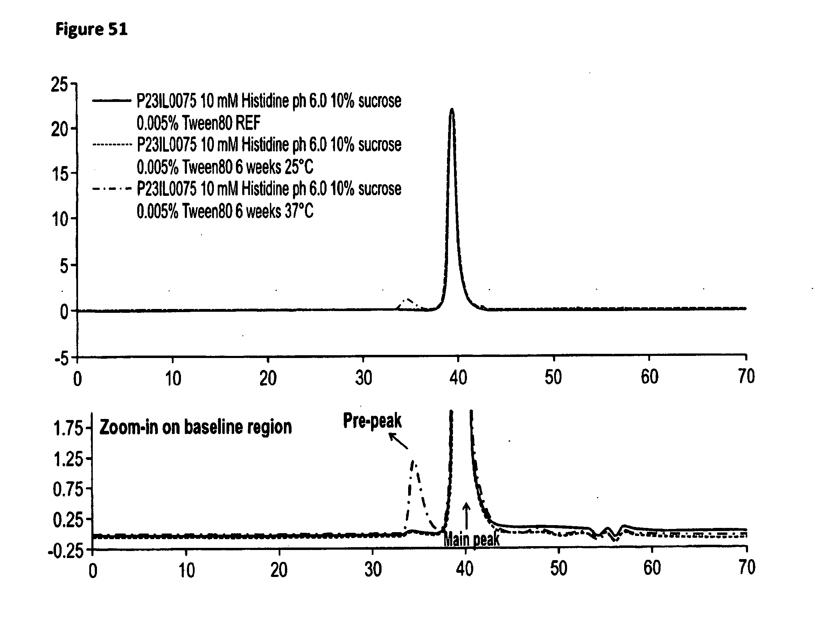

The accelerated stability samples at 25mg/mL in the different candidate formulation buffers were analyzed by OD500, SE-HPLC and RP-HPLC after 3 and 6 weeks storage. In SE-HPLC, the increase of oligomers was only seen at 37°C (Figure 51). In RP-HPLC the post-peak corresponding to the pyroglutamate increased from 3% to 4% after 6 weeks storage at 25°C, but to on average 9% after 6 weeks at 37°C. In Table 41 the RP-HPLC and SE-HPLC results after 3 and 6 weeks storage at 37°C and 6 weeks at 25°C are shown. The OD500 values remained for all buffers (except one outlier) below 0.01.

### TABLES

**Table 1. Overview of the different formulation buffers of RANKL008a used in stability testing.**

| **Buffer** | **Concentration RANKL008a (mg/mL)** | **Buffer** | **[NaCl] (mM)** | **Mannitol % (w:v)** |
|---|---|---|---|---|
| 1 | 60 | 10 mM NaH₂PO₄.2H₂O, pH 7 | 50 | 0 |
| 2 | 60 | 10 mM NaH₂PO₄.2H₂O, pH 7 | 100 | 0 |
| 3 | 60 | 10 mM NaH₂PO₄.2H₂O, pH 7 | 0 | 10 |
| 4 | 59 | 10 mM Na-acetate, pH 5.5 | 50 | 0 |
| 5 | 59 | 10 mM Na-acetate, pH 5.5 | 100 | 0 |
| 6 | 59 | 10 mM Na-acetate, pH 5.5 | 0 | 10 |
| 7 | 60 | 20 mM L-histidine, pH 5.5 | 50 | 0 |
| 8 | 60 | 20 mM L-histidine, pH 5.5 | 100 | 0 |
| 9 | 60 | 20 mM L-histidine, pH 5.5 | 0 | 10 |
| 10 | 58 | 20 mM L-histidine, pH 6 | 50 | 0 |
| 11 | 58 | 20 mM L-histidine, pH 6 | 100 | 0 |
| 12 | 58 | 20 mM L-histidine, pH 6 | 0 | 10 |
| 13 | 84.3 | 10 mM NaH₂PO₄.2H₂O, pH 7 | 100 | 0 |
| 14 | 70 | 10 mM NaH₂PO₄.2H₂O, pH 7 | 0 | 5 |

**Table 2. Relative potencies of HSA and RANKL binding moieties of RANKL008a after 10 F/T cycles as determined by the ELISA potency assays (inhibition and HSA binding).**

| **Buffer** | **Relative potency (relative to reference material)** | |
|---|---|---|
| | **RANKL** | **HSA** |
| **Phosphate + 50 mM NaCl, pH 7** | 0.904 | 0.767 |
| **Phosphate + 100 mM NaCl, pH 7** | 0.966 | 0.672 |
| **Phosphate + 10% Mannitol, pH 7** | 0.956 | 0.715 |
| **Acetate + 50 mM NaCl, pH 5.5** | 1.033 | 0.747 |
| **Acetate + 100 mM NaCl, pH 5.5** | 0.905 | 0.705 |
| **Acetate + 10% Mannitol, pH** 5.5 | 0.878 | 0.737 |
| **Histidine + 50 mM NaCl, pH 5.5** | 0.724 | 0.723 |
| **Histidine + 100 mM NaCl, pH 5.5** | 0.719 | 0.670 |
| **Histidine + 10% Mannitol, pH 5.5** | 0.692 | 0.572 |
| **Histidine + 50 mM NaCl, pH 6** | 0.927 | 0.768 |
| **Histidine + 100 mM NaCl, pH 6** | 0.923 | 0.680 |
| **Histidine + 10% Mannitol, pH 6** | 0.882 | 0.754 |

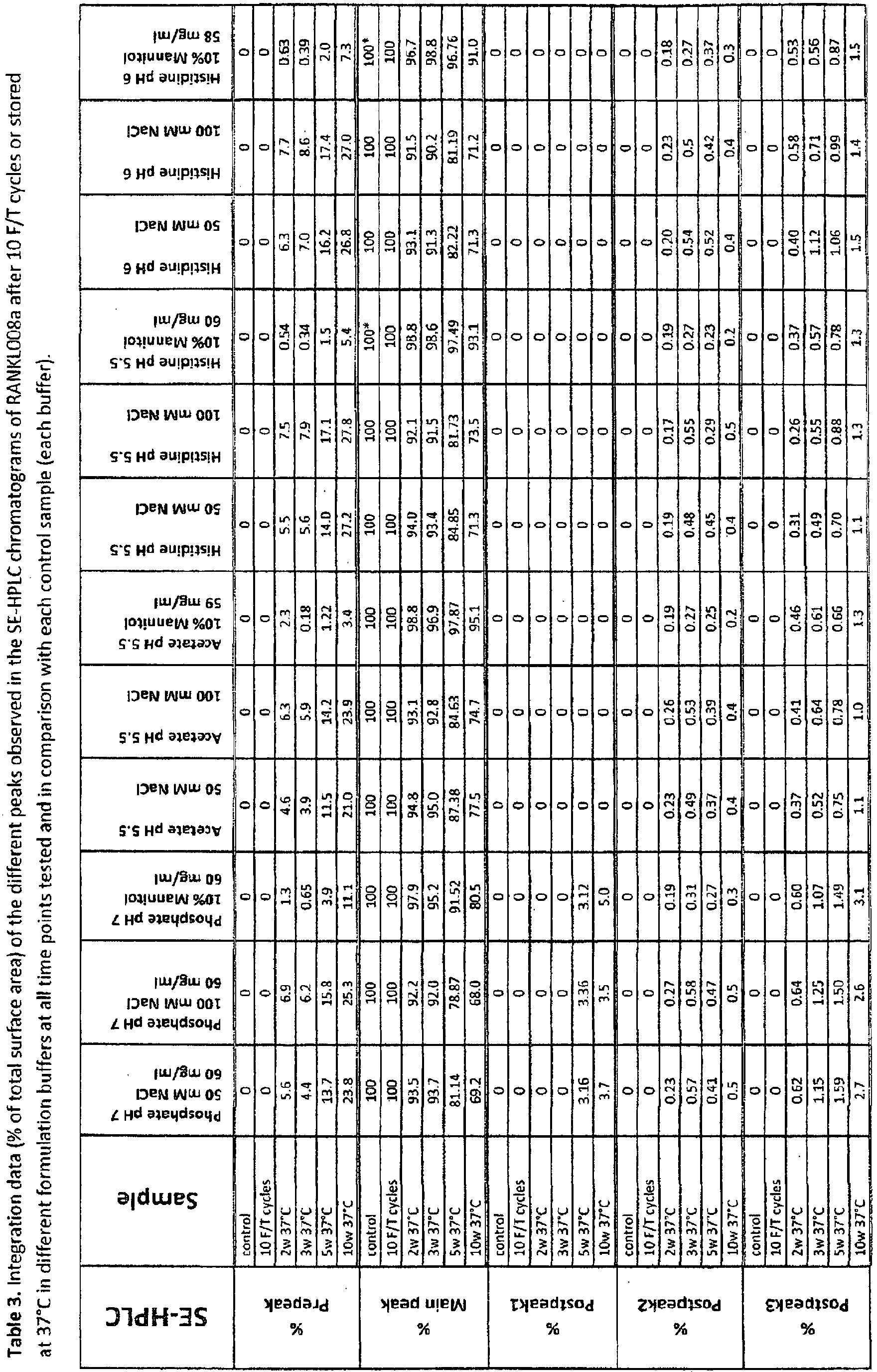

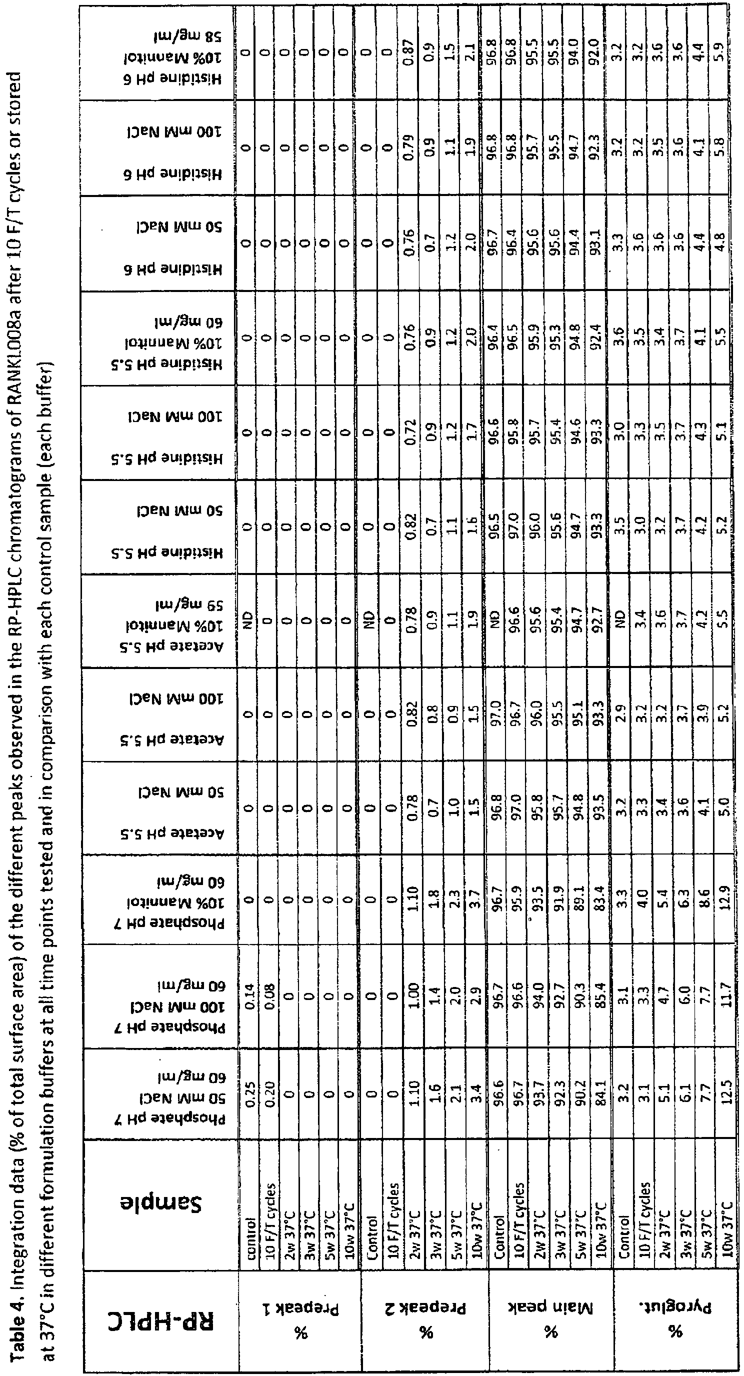

**Table 5. Integration data (% of total surface area) of the different peaks observed in the IEX-HPLC chromatograms of RANKL008a stored for 10 weeks at 37°C in different formulation buffers.**

| **Buffer** | **% Main peak** | **% Post peak 1** | **% Post peak 2** |
|---|---|---|---|
| **Phosphate + 50 mM NaCl, pH 7** | 69.6 | 4.4 | 26.0 |
| **Phosphate + 100 mM NaCl, pH 7** | 67.0 | 4.5 | 28.1 |
| **Phosphate + 10% Mannitol, pH 7** | 84.6 | 3.2 | 12.1 |
| **Acetate + 50 mM NaCl, pH 5.5** | 72.8 | 2.7 | 24.4 |
| **Acetate + 100 mM NaCl, pH 5.5** | 69.6 | 2.8 | 27.6 |
| **Acetate + 10% Mannitol, pH 5.5** | 95.4 | 0 | 4.6 |
| **Histidine + 50 mM NaCl, pH 5.5** | 66.0 | 2.8 | 31.2 |
| **Histidine + 100 mM NaCl, pH 5.5** | 68.1 | 2.8 | 29.0 |
| **Histidine + 10% Mannitol, pH 5.5** | 92.8 | 0 | 7.2 |
| **Histidine + 50 mM NaCl, pH 6** | 67.4 | 2.6 | 30.1 |
| **Histidine + 100 mM NaCl, pH 6** | 67.0 | 2.6 | 30.4 |
| **Histidine + 10% Mannitol, pH 6** | 88.8 | 2.6 | 9.0 |

**Table 6. Relative potencies of the HSA and RANKL binding moieties of RANKL008a after 10 weeks at 37°C as measured by Biacore analysis.**

| **Buffer** | **Relative potency** | |
|---|---|---|
| | **RANKL** | **HSA** |
| **Phosphate + 50 mM NaCl, pH 7** | 81.0 | 57.4 |
| **Phosphate + 100 mM NaCl, pH 7** | 78.6 | 56.6 |
| **Phosphate + 10% Mannitol, pH 7** | 76.3 | 66.8 |
| **Acetate + 50 mM NaCl, pH 5.5** | 80.1 | 63.0 |
| **Acetate + 100 mM NaCl, pH 5.5** | 78.0 | 59.0 |
| **Acetate + 10% Mannitol, pH 5.5** | 80.9 | 79.4 |
| **Histidine + 50 mM NaCl, pH 5.5** | 80.2 | 59.7 |
| **Histidine + 100 mM NaCl, pH 5.5** | 73.1 | 55.0 |
| **Histidine + 10% Mannitol, pH 5.5** | 75.2 | 73.6 |
| **Histidine + 50 mM NaCl, pH 6** | 79.1 | 59.3 |
| **Histidine + 100 mM NaCl, pH 6** | 78.3 | 57.5 |
| **Histidine + 10% Mannitol, pH 6** | 87.4 | 83.4 |

**Table 7. Visual inspection and content determination of RANKL008a after dilution to 0.28 mg/mL in different diluents and passage/storage in syringes (refer to Figure 9).**

| **Sample*** | **visual inspection** | **content (mg/mL) (95% confidence interval** |
|---|---|---|
| **0028 SALINE TUB** | small precipitates | 0.265 (0.261-0.270) |
| **0028 SALINE SYR S25/0** | small precipitates | 0.263 (0.261-0.265) |
| **0028 SALINE SYR S25/24** | small precipitates | 0.259 (0.256-0.262) |
| **0028 PLACEBO-TW TUB** | small precipitates | 0.272 (0.271-0.273) |
| **0028 PLACEBO-TW SYR S25/0** | small precipitates | 0.268 (0.267-0.269) |
| **0028 PLACEBO-TW SYR S25/24** | small precipitates | 0.268 (0.259-0.276) |
| **0028 PLACEBO+TW TUB** | clear | 0.281 (0.281-0.281) |
| **0028 PLACEBO+TW SYR S25/0** | slightly turbid | 0.280 (0.278-0.282) |
| **0028 PLACEBO+TW SYR S25/24** | slightly turbid | 0.279 (0.277-0.281) |

| | | |
|---|---|---|
| * S25/0: storage at 25°C for 0 minute S25/24: storage at 25°C for 24h -TW: buffer minus Tween 80 +TW: buffer + Tween 80 PLACEBO refers to the following buffer: 10 mM Na2HP04 pH 7.0 + 115 mM NaCl TUB: sample stored in a polystyrene tube | | |

**Table 8. Visual inspection, content (confidence interval) and turbidity of RANKL008a diluted to 0.28 mg/mL before (TUB) and after passage through syringes with different needle size as described in Example 1.8.**

| **Sample*** | **visual inspection** | **content (mg/mL) (95% confidence interval)** | **OD 320/278 ratio** | **OD 350/278 ratio** |
|---|---|---|---|---|
| **0028 PLACEBO+TW TUB** | clear | 0.288 (0.275-0.301) | 0.0010 | 0.0019 |
| **0028 PLACEBO+TW 18G/18G** | clear | 0.285 (0.284-0.286) | 0.0003 | 0.0000 |
| **0028 PLACEBO+TW 18G/23G** | clear | 0.288 (0.271-0.307) | 0.0000 | 0.0000 |
| **0028 PLACEBO+TW 18G/27G** | clear | 0.285 (0.279-0.290) | 0.0000 | 0.0002 |
| **0028 PLACEBO+TW 18G/30G** | clear | 0.286 (0.285-0.287) | 0.0005 | 0.0002 |
| **0028 PLACEBO+TW 23G/23G** | clear | 0.287 (0.285-0.289) | 0.0005 | 0.0007 |
| **0028 PLACEBO+TW 27G/27G** | clear | 0.285 (0.284-0.286) | 0.0001 | 0.0005 |
| **0028 PLACEBO+TW 30G/30G** | clear | 0.287 (0.280-0.294) | 0.0007 | 0.0019 |

| | | | | |
|---|---|---|---|---|
| * +TW: buffer + Tween 80 PLACEBO refers to the following buffer: 10 mM Na2HP04 pH 7.0 + 115 mM NaCl TUB: sample stored in a polystyrene tube 18G/18G: sample drawn up with a 18G needle and expelled through a 18G needle 18G/27G: drawn up with a 18G needle and expelled through a 27G needle All other coding is similar to the two examples given above | | | | |

**Table 9. Visual inspection, content (with 95% confidence interval) and turbidity of RANKL008a before (TUB) and after passage through syringes with different needle size as described in Example 1.7 at a concentration of 0.28 mg/mL or about 65 mg/mL.**

| **Sample*** | **visual inspection** | **content (mg/mL) (95% confidence interval)** | **OD 320/278 ratio** | **OD 350/278 ratio** |
|---|---|---|---|---|
| **0028 PLACEBO+TW TUB** | clear | 0.284 (0.283-0.285) | 0.0014 | 0.0010 |
| **0028 PLACEBO+TW 27G/27G (3x)** | clear | 0.284 (0.283-0.285) | 0.0031 | 0.0021 |
| **0028 PLACEBO+TW 29G**/**29G B** | clear | 0.282 (0.280-0.284) | 0.0024 | 0.0010 |
| **0028 PLACEBO+TW 29G/29G T** | clear | 0.283 (0.282-0.284) | 0.0041 | 0.0033 |
| **6500 PLACEBO+TW TUB** | clear | 63.5 (62.4-64.6) | 0.0019 | 0.0006 |
| **6500 PLACEBO+TW 27G**/**27G (3x)** | clear | 62.9 (62.7-63.1) | 0.0015 | 0.0008 |

| | | | | |
|---|---|---|---|---|
| * +TW: buffer + Tween 80 PLACEBO refers to the following buffer: 10 mM Na2HP04 pH 7.0 + 115 mM NaCl TUB: sample stored in a polystyrene tube 27G/27G: sample drawn up with a 27G needle and expelled through a 27G needle 29G/29G: drawn up with a 29G needle and expelled through a 29G needle T: Terumo needle, B Becton Dickinson needle 0028 refers to concentration at 0.28 mg/mL, 6500 to 65 mg/mL | | | | |

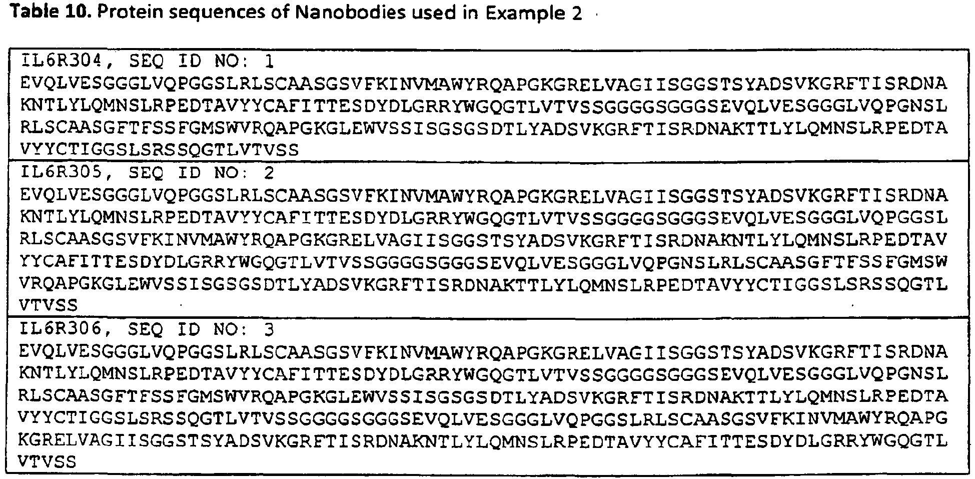

**Table 11. Overview of the IL6R batches used in the formulation and stability studies described in Example 2.**

| **Batch no.** | **Nanobody** | **Buffer** | **Concentration** |
|---|---|---|---|
| P#051108nr1 | IL6R304 | PBS | 4.58 mg/mL |
| P#051108nr2 | IL6R305 | PBS | 3.46 mg/mL |
| P#051108nr2 | IL6R306 | PBS | 5.65 mg/mL |
| BS#030309nr1.5-9 | IL6R304 | 25mM hepes, pH 7.5, 100 mM NaCl | 3.79 mg/mL |
| B5#060509nr1 | IL6R304 | 25mM hepes, pH 7.5, 100 mM NaCl | 4.6 mg/mL |

**Table 12. Reagents used in the formulation and stability study described in Example 2**

| **Reagent** | **Provider** | **Cat No.** |
|---|---|---|
| ACN, HPLC grade | Biosolve | Cat. No. 012007 |
| TFA | Biosolve | Cat. No. 20234131 |
| Isopropanol, HPLC grade | Biosolve | Cat. No. 162606 |
| MilliQ grade water | | |
| D-PBS | Gibco | Cat. No. 14190-094 |
| NaCl | Merck | Cat. No. 1.06404.1000 |
| Gel filtration standard | Bio-Rad | Cat. No. 151-1901 |
| HSA | Sigma | Cat. No. A3782 |
| L-histidine | Fluka | Cat. No. 53319 |
| D-Mannitol | Fiuka | Cat. No. 17311 |
| Sucrose | Fluka | Cat. No. 18219 |
| Glycine | Fluka | Cat. No. 50058 |
| Tween-80 | Merck | Cat. No. K351 65661 609 |
| L-histidine-HCl monohydrate | Sigma | Cat. No. 53369 |
| Succinic acid disodium hexahydrate | Fluka | Cat. No. 14158 |
| Trizma base | Sigma | Cat. No. T6066-5 |
| Sorbitol | Fluka | Cat. No. 85529 |
| Xylitol | Sigma | Cat. No. X3375-100g |
| Ribitol | Fluka | Cat. No. 02240 |
| L-Arginine | Fluka | Cat. No. 11009 |
| MES | Sigma | Cat. No. M3671 |
| Sodium dihydrogenphosphate | Merck | Cat. No. 1.06345.1000 |
| Disodium hydrogenphosphate | Merck | Cat. No. 1.06576.1000 |

**Table 13. Summary of the results from the TSA assay. The Tm values obtained in the different buffer are coded from white to dark grey, i.e. from higher to lower Tm values.**

| **Buffer/pH** | Histidine pH6.5 | Hepes pH8 | Phos. | Succinate | Histidine pH5.5 | Tris pH7 |
|---|---|---|---|---|---|---|
| | Hepes pH7 | Phos. pH6.7 | pH7.7 | pH6.2 | Succinate pH5.2 | PBS |
| **NaCl** | 0 | 100 | 200 | | 400 | 500 |
| **Mannitol** | 7.5% | 5% | 2.5% | 0 | | |
| **Sucrose** | 10% | 5% | | | | |
| **Glycine** | 200 mM | 100 mM | | | | |

**Table 14. Overview of the Tm values obtained by DSC using IL6R304.**

| **Buffer** | **Tm (°C) in 0 mM NaCl** | **Tm (°C) in 25 mM NaCl** | **Tm (°C) in 100 mM NaCl** | **Tm (°C) in 500 mM NaCl** |
|---|---|---|---|---|
| 25 mM citrate pH 3.5 | 50.21 | / | / | / |
| 25 mM acetate pH 5.5 | 61.30 | 61.21 | 60.19 | 58.59 |
| 25 mM MES pH 6.0 | 62.52 | 61.83 | 60.60 | 58.66 |
| 25 mM hepes pH 7.0 | 62.48 | 62.27 | 61.13 | 59.24 |
| 25 mM phosphate pH 7.0 | 60.70 | / | / | / |
| 25 mM Tris pH 7.5 | 61.82 | 61.82 | 60.91 | 59.43 |

**Table 15. Overview of the Tm results obtained by DSC and TSA using IL6R304 formulated in different buffers.**

| **Excipient(s)** | **DSC** | | **TSA** | |
|---|---|---|---|---|
| | **Tm (°C) in 15 mM L-histidine, pH 6.5** | **Tm (°C) 15 mM phosphate, pH 6.5** | **Tm (°C) in 15 mM L-histidine, pH 6.5** | **Tm (°C) 15 mM phosphate, pH 6.5** |
| / | ND | ND | 61.09* / 61.11** | 60.48 / 60.48 |
| 5% mannitol | 64.74 | 63.81 | 61.91 / 61.87 | 61.34 / 61.31 |
| 10% sucrose | **65.40** | 64.35 | 62.78 / 63.20 | 62.34 / 62.78 |
| 10% trehalose | 65.28 | **64.51** | **62.78 / 63.59** | **63.17 / 63.61** |
| 2.5% mannitol + 2.5% sucrose | 64.37 | 63.83 | 61.52 / 61.91 | 61.53 / 61.30 |
| 2.5% sorbitol + 2.5% trehalose | 64.65 | 63.85 | 62.34 / 62.34 | 62.77 / 62.77 |
| 2.5% sorbitol + 2.5% trehalose + 1.5 mM Glycine | 64.83 | 64.33 | 62.33 / 62.35 | 63.19 / 62.96 |
| 10% trehalose + 0.01% Tween-80 | Run failure | 63.77 | ND | ND |

| | | | | |
|---|---|---|---|---|
| * measurement 1, ** measurement 2 | | | | |

**Table 16. Visual appearance, UV spectroscopy and PAMAS data demonstrating a higher solubility for IL6R304 in the presence of Tween 80.**

| | **IL6R304 (2 mg/mL) stored for 4 days at 5°C** | | |
|---|---|---|---|
| | **PBS** | **PBS** + **0.1** % **Tween 80** | **PBS** + **0.2** % **Tween 80** |
| **Appearance** | Turbid, opaque | Clear, colorless | Clear, colorless |
| **A320/A280** | 0.014 | 0.012 | 0.009 |
| **Sub-visible particle counts/100 µl** | | | |
| > 1 µm | 157.557 | 42.243 | 52.157 |
| > 2 µm | 69.471 | 19.514 | 21.429 |
| > 3 µm | 43.371 | 12.043 | 12.743 |
| > 4 µm | 29.757 | 8.329 | 8.600 |
| > 5 µm | 18.300 | 4.971 | 4.800 |
| > 6 µm | 11.814 | 3.114 | 3.143 |
| > 7 µm | 8.300 | 2.300 | 2.186 |
| > 8 µm | 5.900 | 1.671 | 1.500 |
| > 9 µm | 4.543 | 1.214 | 1.086 |
| > 10 µm | 3.400 | 971 | 800 |
| > 15 µm | 800 | 271 | 300 |
| > 25 µm | 200 | 100 | 86 |
| > 50 µm | 114 | 29 | 14 |
| > 100 µm | 86 | 0 | 14 |
| > 150 µm | 71 | 0 | 14 |
| > 200 µm | 71 | 0 | 14 |

**Table 17. Overview of the different formulation buffers used in initial stability testing of IL6R304, IL6R305 and IL6R306.**

| **Condition** | **Buffer** | **[NaCl]** | **Mannitol** |
|---|---|---|---|
| 1 | PBS | 0 mM | 0 % |
| 2 | PBS | 0 mM | 5 % |
| 3 | 10 mM NaH₂PO₄.2H₂O, pH 7 | 100 mM | 0 % |
| 4 | 10 mM NaH₂PO₄.2H₂O, pH 7 | 100 mM | 5 % |
| 5 | 10 mM Na-acetate, pH 5.5 | 100 mM | 0 % |
| 6 | 10 mM Na-acetate, pH 5.5 | 100 m M | 5 % |
| 7 | 20 mM L-histidine, pH 6 | 100 mM | 0 % |
| 8 | 20 mM L-histidine, pH 6 | 100 mM | 5 % |

**Table 18. Overview of the different formulation buffers used in stability testing of IL6R304.**

| **Buffer** | **Concentration IL6R304** | **Buffer** | **% Tween 80** | % **Mannitol** | **% Sucrose** | **mM Glycine** |
|---|---|---|---|---|---|---|
| 1 | 10 mg/mL | 20 mM L-histidine | / | / | / | / |
| 2 | 10 mg/mL | 20 mM L-histidine | 0.01 | / | / | / |
| 3 | 10 mg/mL | 20 mM L-histidine | 0.05 | / | / | / |
| 4 | 10 mg/mL | 20 mM L-histidine | 0.05 | 5 | / | / |
| 5 | 10 mg/mL | 20 mM L-histidine | 0.05 | 5 | / | 200 |
| 6 | 10 mg/mL | 20 mM L-histidine | 0.05 | 2.5 | / | 100 |
| 7 | 10 mg/mL | 20 mM L-histidine | 0.05 | / | 10 | / |
| 8 | 10 mg/mL | 20 mM L-histidine | 0.05 | / | / | 200 |
| 9 | 10 mg/mL | 20 mM L-histidine | 0.05 | / | 5 | 100 |
| 10 | 10 mg/mL | 20 mM L-histidine | 0.05 | 2.5 | 5 | / |
| 11 | 10 mg/mL | 20 mM L-histidine | / | 2.5 | 5 | 100 |
| 12 | 10 mg/mL | 20 mM L-histidine | 0.05 | 2.5 | 5 | 100 |

**Table 19. Methods used for assessing the stability of IL6R304 at different time points (represented as x weeks or w) after storage at 5°C and 37°C.**

| **Method** | **Purpose** | **Ref. material** | **Stress condition** | |
|---|---|---|---|---|
| | | | **5°C** | **37°C** |
| **A280** | Content | 0w | 1, 2 and 5w | 1, 2, 3 and 5w |
| **Appearance** | Precipitation | 0w | 1, 2 and 5w | 1, 2, 3 and 5w |
| **RP-HPLC** | Purity/variants | 0w | 1, 2 and 5w | 1, 2, 3 and 5w |
| **SE-HPLC** | Purity/aggregation/ hydrolysis | 0w | 1, 2 and 5w | 1, 2, 3 and 5w |
| **Biacore** | Potency (HSA binding) | 0w | 5w | 5w |
| **Osmolality** | Characteristic | 0w | / | / |

**Table 20. Overview of the SE-HPLC integration results after storage for 6 months at 37°C.**

| **Buffer** | **% pre peak 1** | **% pre peak 2** | **% main peak** | **% post peak** |
|---|---|---|---|---|
| Ref | 0.52 | 0.17 | 99.3 | 0 |
| Buffer 1 | ND | ND | ND | ND |
| Buffer 2 | 20.4 | 2.1 | 73.4 | 4.1 |
| Buffer 3 | ND | ND | ND | ND |
| Buffer 4 | 18.1 | 1.7 | 76.0 | 4.2 |
| Buffer 5 | 22.2 | 2.0 | 71.4 | 4.4 |
| Buffer 6 | 21.4 | 1.7 | 72.7 | 4.2 |
| Buffer 7 | 15.1 | 0 | 80.5 | 4.4 |
| Buffer 8 | 21.1 | 2.4 | 72.0 | 4.5 |
| Buffer 9 | 16.7 | 2.7 | 76.3 | 4.3 |
| Buffer 10 | 15.8 | 1.9 | 77.9 | 4.4 |
| Buffer 11 | 17.5 | 2.0 | 76.4 | 4.2 |
| Buffer 12 | 16.8 | 3.3 | 75.7 | 4.2 |

**Table 21. Overview of the different formulation buffers tested in the stability study.**

| **Nr.** | **Conc.** | **Buffer** | **Mannitol** | **Sucrose** | **Trehalose** | **Glycine** | **Tween-80** |
|---|---|---|---|---|---|---|---|
| 1 | 10 mg/mL | 15 mM L-histidine, pH6.5 | 5% | | | | 0.01% |
| 2 | 10 mg/mL | 15 mM L-histidine, pH6.5 | | 10% | | | 0.01% |
| 3 | 10 mg/mL | 15 mM L-histidine, pH6.5 | | | 10% | | 0.01% |
| 4 | 10 mg/mL | 15 mM L-histidine, pH6.5 | | 7.5% | | 0.35% | 0.01% |
| 5 | 10 mg/mL | 15 mM L-histidine, pH6.5 | 2.5% | 5% | | | 0.01% |
| 6 | 10 mg/mL | 15 mM phosphate, pH6.5 | 5% | | | | 0.01% |
| 7 | 10 mg/mL | 15 mM phosphate, pH6.5 | | 10% | | | 0.01% |
| 8 | 10 mg/mL | 15 mM phosphate, pH6.5 | | | 10% | | 0.01% |
| 9 | 10 mg/mL | 15 mM phosphate, pH6.5 | 7.5% | 7.5% | | 0.35% | 0.01% |
| 10 | 10 mg/mL | 15 mM phosphate, pH6.5 | 2.5% | 5% | | | 0.01% |

**Table 22. cIEF integration data of IL6R304 stored for 8 weeks at 37°C in the different buffers**

| **Buffer** | **% prepeak (acidic variants)** | **% main peak** | **% postpeak (basic variants)** |
|---|---|---|---|
| 1 | 5.5 | 81.3 | 13.0 |
| 2 | 5.0 | 81.5 | 13.5 |
| 3 | 6.1 | 79.7 | 14.2 |
| 4 | 5.7 | 81.2 | 13.2 |
| 5 | 5.1 | 81.2 | 13.7 |
| 6 | 9.0 | 71.6 | 19.3 |
| 7 | 9.9 | 70.5 | 19.6 |
| 8 | 8.3 | 71.8 | 19.9 |
| 9 | 11.7 | 68.5 | 19.8 |
| 10 | 8.7 | 70.5 | 20.2 |

**Table 23. Relative potency of IL6R304 after 8 weeks at +37°C compared to B5#030309nr2.3-5.**

| **Buffer** | **HSA** | **IL-6R** |
|---|---|---|
| 1 | 1.080 (0.954-1.223) | 1.153 (0.957-1.389) |
| 2 | 0.975 (0.887-1.072) | 0.980 (0.760-1.263) |
| 3 | 1.038 (0.952-1.132) | 1.117 (0.910-1.372) |
| 4 | 1.182 (1.074-1.300) | 1.061 (0.908-1.240) |
| 5 | 1.080 (1.004-1.161) | 1.082 (0.925-1.266) |

**Table 24. Summary of the Biacore results for HSA binding of the stability samples stored for 8 weeks at 37°C, expressed as % activity compared to the equivalent sample stored at -70°C.**

| **Buffer** | **% activity compared to reference** |
|---|---|
| 1 | 97.5 |
| 2 | 93.2 |
| 3 | 92.5 |
| 4 | 83.9 |
| 5 | 101.9 |
| 6 | 92.2 |
| 7 | 89.4 |
| 8 | 99.0 |
| 9 | 84.3 |
| 10 | 89.6 |

**Table 25. Appearance of IL6R304 after 0, 2, 4 and 24 hours of stirring at 2-8°C.**

| **Buffer** | **0 hrs** | **2 hrs** | **4 hrs** | **24 hrs** |
|---|---|---|---|---|
| 1 | clear | clear | clear | clear/slightly opalescent |
| 2 | clear | clear | clear | clear/slightly opalescent |
| 3 | clear | clear | slightly opalescent | slightly opalescent |
| 4 | clear | clear | clear | clear/siightly opalescent |
| 5 | clear | clear | slightly opalescent | slightly opalescent |
| 6 | clear | clear | slightly opalescent | opalescent |
| 7 | clear | clear | slightly opalescent | opalescent |
| 8 | clear | clear | slightly opalescent | highly opalescent |
| 9 | clear | clear | clear | opalescent |
| 10 | clear | clear | opalescent | opalescent |

**Table 26. Reagents used in the formulation and stability study described in Examples 3.**

| **Reagent** | **Provider** | **Cat No.** |
|---|---|---|
| ACN, HPLC grade | Biosolve | Cat. No. 012007 |
| TFA | Biosolve | Cat. No. 20234131 |
| N-propanol, HPLC grade | Sigma-Aldrich | Cat. No. 34871 |
| MilliQ grade water | | |
| D-PBS | Invitrogen | Cat. No. 14190 |
| NaCl | Merck | Cat. No. 1.06404.1000 |
| Gel filtration standard | Bio-Rad | Cat. No. 151-1901 |
| HSA | Sigma | Cat. No. A3782 |
| L-histidine | Fluka | Cat. No. 53319 |
| D-Mannitol | Fluka | Cat. No. 17311 |
| Sucrose | Fluka | Cat. No. 18219 |
| Glycine | Fluka | Cat. No. 50058 |
| Tween 80 | Merck | Cat. No. K351 65661 609 |

**Table 27. Final concentrations obtained after concentration of 23IL0064 using Vivaspin filters. The filtration was stopped at the moment the final volume became limited (100 to 200 µL) and protein loss occurred. All samples were analyzed by SE-HPLC: the percent pre-peak (% of total surface area) represent aggregates in the sample.**

| **buffer condition** | **start conditions** | | **after ULTRAFILTRATION** | | |
|---|---|---|---|---|---|
| | **concentration** | **% pre-peak in SE-HPLC** | **concentration** | **% pre-peak in SE-HPLC** | **% recovery** |
| D-PBS | 3.8 mg/mL | 2,80% | 110 mg/mL | 6,30% | 42% ⁽¹⁾ |
| 50 mM NaCl, 10 mM Phosphate pH 7 | 3.4 mg/mL | 2,70% | 83 mg/mL | 2,80% | 66% ⁽¹⁾ |
| 50 mM NaCl, 40 mM Histidine pH 6 | 3.4 mg/mL | 2,70% | 150 mg/mL | 2,70% | 59% ⁽¹⁾ |

| | | | | | |
|---|---|---|---|---|---|
| ⁽¹⁾Low recoveries can be due to local concentration effects obtained during the dead-end ultrafiltration set-up. | | | | | |

**Table 28. Buffers tested in a thermal shift assay for 23IL0064.**

| **Buffer** | **pKa** | **pH** | **Concentration** (**mM**) | **mM NaCl** | **% mannitol** |
|---|---|---|---|---|---|
| Succinate | 5.64 (pK2) | 5.2 | 20 | 0 | 0 - 2.5 - 5 - 7.5 |
| | | 6.2 | | 50 - 150 - 300 - 500 | 0 |
| Histidine | 6.04 (pK2) | 5.5 | 20 | 0 | 0 - 2.5 - 5 - 7.5 |
| | | 6.5 | | 50 - 150 - 300 - 500 | 0 |
| Phosphate | 7.20 (pK2) | 6.7 | 20 | 0 | 0 - 2.5 - 5 - 7.5 |
| | | 7.7 | | 50 -150 - 300 - 500 | 0 |
| hepes | 7.48 | 7.0 | 20 | 0 | 0 - 2.5 - 5 - 7.5 |
| | | 8.0 | | 50 - 150 - 300 - 500 | 0 |

**Table 29. Concentrations measured by Nanodrop (average of 2 measurements) in the samples stressed at 37°C. The concentration was determined after a short high speed spin (1 min at 15000xg)**

| **P23IL0064 Samples (4mg/mL in D-PBS)** | **Concentration (mg/mL)** |
|---|---|
| Reference (-20°C) | 4.05 |
| 3 weeks 37°C (label 24w) | 3.99 |
| 4 weeks 37°C (label 4w) | 2.0 |
| 6 weeks 37°C (label 8w) | 2.30 |
| 6 weeks 37°C (label 12w) | 4.11 |
| 6 weeks 37°C (label 16w) | 2.33 |

**Table 30. Buffers tested in stressed stability for 23IL0064.**

| **Buffer** | **pH** | **Concentration*** | **Excipient/ surfactant** | **Time point analyzed** | **Storage Temp.** |
|---|---|---|---|---|---|
| Hepes 20mM | 8 | 5 mg/mL | | 2.5w | 37°C |
| His 20mM | 6.5 | 5 mg/mL | | 2.5w / 6 w | 4 - 25 - 37°C |
| His 20mM | 6.5 | 22 mg/mL** | | 2.5w / 6 w | 4 - 25 - 37°C |
| His 20mM | 6 | 5 mg/mL | | 2.5w / 6 w | 4 - 25 - 37°C |
| His 20mM | 6.5 | 5 mg/mL | 0.02% Tween 80 | 2.5w / 6 w | 4 - 25 - 37°C |
| His 20mM | 6.5 | 5 mg/mL | 8% mannitol | 2.5w / 6 w | 4 - 25 - 37°C |
| His 20mM | 6.5 | 5 mg/mL | 8% sucrose | 2.5w / 6 w | 4 - 25 - 37°C |
| His 20mM | 6.5 | 5 mg/mL | 1.5% glycine | 2.5w / 6 w | 4 - 25 - 37°C |

| | | | | | |
|---|---|---|---|---|---|
| *The exact concentrations used in the study ranged between 4.9 and 5.1 mg/mL **Labeled as 'HIGH CONC' in figures, actual conc. was 22.36 mg/mL. | | | | | |

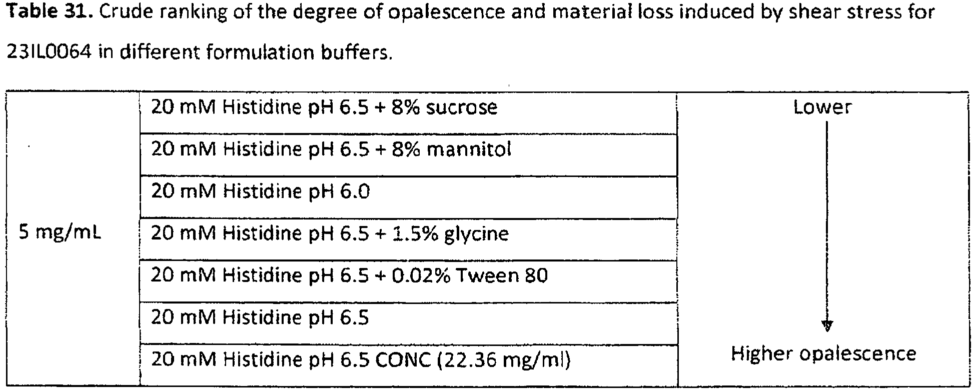

**Table 32. Integration data of the RP-HPLC analysis of the stability samples of 23IL0064 in different buffer conditions (comparison of 6 weeks 37°C, 6 weeks 25°C, 6 weeks 4°C and -80°C Ref).**

| **p23IL0064 in** | **Stress condition** | **pre 1 22.4** | **pre 2 23.3** | **main peak 23.9** | **post 1 27.5** | **post 2 29.0** | **Total peak area (*%*)*** |
|---|---|---|---|---|---|---|---|
| *20mM Histidine pH 6.5* | **-80°C Ref** | 68 | 195 | 1671 | 76 | 20 | 2029 |
| | **%** | 3% | 10% | 82% | 4% | 1% | 100% |
| | **6 weeks 4°C** | 77 | 201 | 1791 | 85 | 29 | 2182 |
| | **%** | 4% | 9% | 82% | 4% | 1% | **108%** |
| | **6 weeks 25°C** | 77 | 196 | 1696 | 125 | 33 | 2127 |
| | **%** | 4% | 9% | 80% | 6% | 2% | ***105%*** |
| | **6 weeks 37°C** | 130 | 186 | 1499 | 274 | 57 | 2146 |
| | **%** | 6% | 9% | 70% | 13% | 3% | ***106%*** |
| *20mM Histidine pH 6.0* | **-80°C Ref** | 68 | 199 | 1807 | 66 | 20 | 2159 |
| | **%** | 3% | 9% | 84% | 3% | 1% | 100% |
| | **6 weeks 4°C** | 69 | 195 | 1751 | 71 | 17 | 2104 |
| | **%** | 3% | 9% | 83% | 3% | 1% | **97%** |
| | **6 weeks 25°C** | 67 | 206 | 1696 | 90 | 22 | 2080 |
| | **%** | 3% | 10% | 82% | 4% | 1% | ***96%*** |
| | **6 weeks 37°C** | 117 | 200 | 1573 | 152 | 37 | 2079 |
| | **%** | 6% | 10% | 76% | 7% | 2% | ***96%*** |
| *20mM Histidine pH 6.5* + *0.02% Tween80* | **-80°C Ref** | 66 | 175 | 1564 | 49 | 14 | 1867 |
| | **%** | 4% | 9% | 84% | 3% | 1% | 100% |
| | **6 weeks 4°C** | 74 | 196 | 1749 | 85 | 29 | 2133 |
| | **%** | 3% | 9% | 82% | 4% | 1% | **114%** |
| | **6 weeks 25°C** | 86 | 196 | 1556 | 117 | 37 | 1992 |
| | **%** | 4% | 10% | 78% | 6% | 2% | ***107%*** |
| | **6 weeks 37°C** | 116 | 195 | 1258 | 245 | 66 | 1881 |
| | **%** | 6% | 10% | 67% | 13% | 4% | ***101%*** |
| *20mM Histidine pH 6.5* + *8% mannitol* | **-80°C** Ref | 70 | 201 | 1770 | 66 | 19 | 2128 |
| | **%** | 3% | 9% | 83% | 3% | 1% | 100% |
| | **6 weeks 4°C** | 68 | 193 | 1739 | 80 | 14 | 2093 |
| | **%** | 3% | 9% | 83% | 4% | 1% | **98%** |
| | **6 weeks 25°C** | 82 | 192 | 1665 | 123 | 32 | 2094 |
| | **%** | 4% | 9% | 80% | 6% | 2% | ***98%*** |
| | **6 weeks 37°C** | 95 | 173 | 1438 | 256 | 35 | 1998 |
| | **%** | 5% | 9% | 72% | 13% | 2% | ***94%*** |
| *20mM Histidine pH 6.5* + *8% sucrose* | **-80°C Ref** | 56 | 193 | 1685 | 70 | 16 | 2019 |
| | **%** | 3% | 10% | 83% | 3% | 1% | 100% |
| | **6 weeks 4°C** | 61 | 198 | 1691 | 62 | 17 | 2029 |
| | **%** | 3% | 10% | 83% | 3% | 1% | **100%** |
| | **6 weeks 25°C** | 80 | 202 | 1591 | 123 | 29 | 2026 |
| | **%** | 4% | 10% | 79% | 6% | 1% | ***100%*** |
| | **6 weeks 37°C** | 139 | 246 | 1292 | 316 | 73 | 2065 |
| | **%** | 7% | 12% | 63% | 15% | 4% | ***102%*** |
| *20mM Histidine pH 6.5* + *1.5% glycine* | **-80°C Ref** | 53 | 167 | 1413 | 51 | 15 | 1698 |
| | **%** | 3% | 10% | 83% | 3% | 1% | 100% |
| | **6 weeks 4°C** | 68 | 197 | 1669 | 67 | 18 | 2019 |
| | **%** | 3% | 10% | 83% | 3% | 1% | **119%** |
| | **6 weeks 25°C** | 74 | 189 | 1596 | 124 | 31 | 2014 |
| | **%** | 4% | 9% | 79% | 6% | 2% | ***119%*** |
| | **6 weeks 37°C** | 95 | 185 | 1359 | 278 | 52 | 1968 |
| | **%** | 5% | 9% | 69% | 14% | 3% | ***116%*** |
| *20mM Histidine pH 6.5 CONC* | **-80°C Ref** | 72 | 233 | 1984 | 81 | 15 | 2385 |
| | **%** | 3% | 10% | 83% | 3% | 1% | 100% |
| | **6 weeks 4°C** | 90 | 218 | 1880 | 88 | 21 | 2297 |
| | **%** | 4% | 9% | 82% | 4% | 1% | **96%** |
| | **6 weeks 25°C** | 96 | 235 | 1848 | 143 | 33 | 2355 |
| | **%** | 4% | 10% | 78% | 6% | 1% | ***99%*** |
| | **6 weeks 37°C** | 126 | 261 | 1725 | 332 | 62 | 2505 |
| | **%** | 5% | 10% | 69% | 13% | 2% | ***105%*** |

| | | | | | | | |
|---|---|---|---|---|---|---|---|
| %*: recovery calculated by using the total area compared to the -80°C Ref of the same condition. | | | | | | | |

**Table 33. Melting temperatures for 23IL0064 and 23IL0075 in different buffers as determined by differential scanning calorimetry (at 1mg/mL). Scanning was performed at 1°C/min, starting at 30°C.**

| **Buffer** | **23IL0064** | **23IL0075** |
|---|---|---|
| **25 mM acetate; pH 5.5; 50 mM NaCl** | 55,5 | 58,0 |
| **25 mM acetate; pH 5.5; 250 mM NaCl** | 52,9 | 55,5 |
| **25 mM MES; pH 6.0; 50 mM NaCl** | 56,2 | 58,8 |
| **25 mM MES; pH 6.0; 250 mM NaCl** | 53,2 | 55,7 |
| **25 mM hepes; pH 7.0; 50 mM NaCl** | 56,7 | 59,3 |
| **25 mM hepes; pH 7.0; 250 mM NaCl** | 53,7 | 56,2 |
| **25 mM Tris; pH 7.5; 50 mM NaCl** | 56,3 | 58,7 |
| **25 mM Tris; pH 7.5; 250 mM NaCl** | 53,5 | 56,1 |

**Table 34. Melting temperatures for 23IL0064 and 23IL0075 in buffers as determined by thermal shift assay (at 0.1mg/mL).**

| **Buffer** | **23IL0064** | **23IL0075*** | **23IL0075*** |
|---|---|---|---|
| **20mM Histidine pH6.5; 50mM NaCl** | 54.5 | 57.0 | 57.0 |
| **20mM Histidine pH6.5** | 56.6 | 59.2 | 59.3 |
| **20mM Histidine pH6.5; 7 5% mannitol** | 57.9 | 60.7 | 60.6 |
| **20mM hepes pH7; 50mM NaCl** | 54.8 | 57.4 | 57.5 |
| **20mM hepes pH7** | 56.8 | 59.8 | 59.9 |
| **20mM hepes pH7; 7.5% mannitol** | 58.2 | 61.1 | 61.2 |
| **20mM hepes pH8; 50mM NaCl** | 55.0 | 57.6 | 57.8 |
| **20mM hepes pH8** | 56.4 | 59.5 | 59.4 |
| **20mM hepes pH8; 7.5% mannitol** | 57.6 | 60.3 | 60.3 |

| | | | |
|---|---|---|---|
| *Measurements were performed on the 2 batches | | | |

**Table 35. Design-Expert Numerical Optimization of the model. The larger the Desirability coefficient the better the proposal of the optimum.**

| **Solutions for phosphate** | | | | | |
|---|---|---|---|---|---|
| **Number** | **Conc(log10) (mM)** | **PH** | **Buffer** | **Tm (°C)** | **Desirability** |
| 1 | 1.00 | 6.00 | Phosphate | 59.0012 | 0.895 |
| 2 | 1.04 | 6.00 | Phosphate | 58.9419 | 0.881 |
| | | | | | |

| **Solutions for Acetate** | | | | | |
|---|---|---|---|---|---|
| **Number** | **Conc(log10) (mM)** | **PH** | **Buffer** | **Tm (°C)** | **Desirability** |
| 1 | 1.00 | 6.00 | Acetate | 59.2759 | 0.959 |
| 2 | 1.01 | 6.08 | Acetate | 59.2615 | 0.956 |
| 3 | 1.11 | 5.88 | Acetate | 59.1489 | 0.929 |
| 4 | 1.10 | 5.80 | Acetate | 59.1345 | 0.926 |
| 5 | 1.15 | 5.72 | Acetate | 59.0452 | 0.905 |
| 6 | 1.16 | 5.68 | Acetate | 59.0036 | 0.895 |
| 7 | 1.32 | 6.09 | Acetate | 58.9476 | 0.882 |
| 8 | 1.33 | 6.15 | Acetate | 58.9406 | 0.881 |
| 9 | 1.35 | 6.21 | Acetate | 58.9317 | 0.879 |
| 10 | 1.19 | 5.56 | Acetate | 58.8937 | 0.870 |
| 11 | 1.32 | 5.89 | Acetate | 58.8726 | 0.865 |
| 12 | 1.00 | 5.00 | Acetate | 58.77 | 0.841 |
| 13 | 1.35 | 5.69 | Acetate | 58.7182 | 0.829 |
| 14 | 1.50 | 6.12 | Acetate | 58.7181 | 0.829 |
| 15 | 1.10 | 5.10 | Acetate | 58.682 | 0.820 |
| 16 | 1.16 | 5.19 | Acetate | 58.6346 | 0.809 |
| 17 | 1.57 | 6.08 | Acetate | 58.5857 | 0.798 |
| 18 | 1.44 | 5.71 | Acetate | 58.5814 | 0.797 |
| 19 | 1.35 | 5.50 | Acetate | 58.5692 | 0.794 |
| 20 | 1.55 | 5.96 | Acetate | 58.5672 | 0.793 |
| 21 | 1.6 | 6.18 | Acetate | 58.5354 | 0.786 |
| 22 | 1.70 | 6.00 | Acetate | 58.3294 | 0.738 |
| 23 | 1.62 | 5.68 | Acetate | 58.215 | 0.711 |
| 24 | 1.34 | 5.01 | Acetate | 58.0486 | 0.672 |
| 25 | 1.47 | 5.20 | Acetate | 57.9981 | 0.660 |
| 26 | 1.33 | 4.96 | Acetate | 57.9943 | 0.659 |
| 27 | 1.56 | 5.35 | Acetate | 57.9714 | 0.654 |
| 28 | 1.27 | 4.83 | Acetate | 57.9579 | 0.651 |
| 29 | 1.62 | 5.41 | Acetate | 57.9098 | 0.639 |
| 30 | 1.53 | 5.16 | Acetate | 57.8061 | 0.615 |
| 31 | 1.61 | 5.29 | Acetate | 57.7715 | 0.607 |
| 32 | 1.57 | 5.20 | Acetate | 57.7583 | 0.604 |
| 33 | 1.56 | 5.18 | Acetate | 57.7496 | 0.602 |
| 34 | 1.35 | 4.79 | Acetate | 57.7035 | 0.591 |
| 35 | 1.65 | 5.24 | Acetate | 57.61 | 0.569 |
| 36 | 1.65 | 5.17 | Acetate | 57.5141 | 0.547 |
| 37 | 1.70 | 5.00 | Acetate | 57.0985 | 0.449 |
| **37 Solutions found** | | | | | |

| **Solutions for Histidine** | | | | | |
|---|---|---|---|---|---|
| **Number** | **Conc(log10) (mM)** | **PH** | **Buffer** | **Tm (°C)** | **Desirability** |
| 1 | 1.01 | 6.19 | Histidine | 59.4631 | 1.000 |
| 2 | 1.00 | 6.18 | Histidine | 59.4654 | 1.000 |
| 3 | 1.02 | 6.20 | Histidine | 59.4617 | 1.000 |
| 4 | 1.01 | 6.20 | Histidine | 59.4767 | 1.000 |
| 5 | 1.01 | 6.20 | Histidine | 59.4702 | 1.000 |
| 6 | 1.01 | 6.18 | Histidine | 59.4525 | 1.000 |
| 7 | 1.01 | 6.18 | Histidine | 59.4554 | 1.000 |
| 8 | 1.00 | 6.19 | Histidine | 59.4736 | 1.000 |
| 9 | 1.02 | 6.19 | Histidine | 59.4524 | 1.000 |
| 10 | 1.02 | 6.19 | Histidine | 59.4537 | 1.000 |
| 11 | 1.01 | 6.20 | Histidine | 59.4644 | 1.000 |
| 12 | 1.02 | 6.20 | Histidine | 59.4559 | 1.000 |
| 13 | 1.01 | 6.18 | Histidine | 55.4608 | 1.000 |
| 14 | 1.02 | 6.19 | Histidine | 59.4556 | 1.000 |
| 15 | 1.00 | 6.21 | Histidine | 59.485 | 1.000 |
| 16 | 1.01 | 6.18 | Histidine | 59.4505 | 1.000 |
| 17 | 1.02 | 6.19 | Histidine | 59.4579 | 1.000 |
| 18 | 1.03 | 6.21 | Histidine | 59.4523 | 1.000 |
| 19 | 1.01 | 6.18 | Histidine | 59.4562 | 1.000 |
| 20 | 1.01 | 6.21 | Histidine | 59.4702 | 1.000 |
| 21 | 1.00 | 6.19 | Histidine | 59.4689 | 1.000 |
| 22 | 1.01 | 6.20 | Histidine | 59.4711 | 1.000 |
| 23 | 1.05 | 6.21 | Histidine | 59.4363 | 0.997 |
| **23 Solutions found** | | | | | |
| | | | | | |

| **Solutions for Succinate** | | | | | |
|---|---|---|---|---|---|
| **Number** | **Conc(log10) (mM)** | **PH** | **Buffer** | **Tm (°C)** | **Desirability** |
| 1 | 1.00 | 5.94 | Succinate | 57.8819 | 0.633 |
| 2 | 1.00 | 5.94 | Succinate | 57.8819 | 0.633 |
| 3 | 1.00 | 5.94 | Succinate | 57.8819 | 0.633 |
| 4 | 1.00 | 5.93 | Succinate | 57.8819 | 0.633 |
| 5 | 1.00 | 5.95 | Succinate | 57.8818 | 0.633 |
| 6 | 1.00 | 5.92 | Succinate | 57.8818 | 0.633 |
| 7 | 1.00 | 5.96 | Succinate | 57.8817 | 0.633 |
| **7 Solutions found** | | | | | |

**Table 36. Optimization results based on obtained melting temperatures in the screening of a wide range of excipients in a histidine, acetate or phosphate buffer for the formulation of 23IL0075. The results were ordered from high to low Tm value. The formulation composition has to be read combining the identity of the excipients in columns 2 to 4 and the amount of each excipient in columns 5 to 7.**

| **Combination number** | **Sugar/ polyol** | **Detergent** | **Amino acid** | **Buffer** | **% sugar/ polyol** | **% amino acid** | **% Detergent** | **Tm value (°C)** |
|---|---|---|---|---|---|---|---|---|
| 47 | Sucrose | Tween 20 | Glycine | HistidinePH6 | 10,5 | 0 | 0 | 62,97 |
| 44 | Sucrose | Tween 20 | Arg/Glu | HistidinePH6 | 10,5 | 0 | 0 | 62,5 |
| 5 | Mannitol | P-F68 | Glycine | HistidinePH6 | 5,6 | 0 | 0 | 62,22 |
| 41 | Sucrose | P-F68 | Glycine | HistidinePH6 | 10,5 | 0 | 0 | 62,22 |
| 11 | Mannitol | Tween 20 | Glycine | HistidinePH6 | 5,6 | 0 | 0 | 62,18 |
| 46 | Sucrose | Tween 20 | Glycine | AcetatePH5.5 | 10,5 | 0 | 0 | 62,16 |
| 42 | Sucrose | P-F68 | Glycine | PhosphatePH6 | 6,3 | 0,91 | 0 | 62,13 |
| 38 | Sucrose | P-F68 | Arg/Glu | HistidinePH66 | 10,5 | 0 | 0 | 61,9 |
| 6 | Mannitol | P-F68 | Glycine | PhosphatePH6 | 3,7 | 0,77 | 0 | 61,86 |
| 43 | Sucrose | Tween 20 | Arg/Glu | AcetatePH5.5 | 10,5 | 0 | 0 | 61,77 |
| 40 | Sucrose | P-F68 | Glycine | AcetatePH5.5 | 8,8 | 0,37 | 0 | 61,74 |
| 22 | Sorbitol | P-F68 | Glycine | AcetatePH5.5 | 3,9 | 0,69 | 0 | 61,7 |
| 48 | Sucrose | Tween 20 | Glycine | PhosphatePH6 | 9,2 | 0,28 | 0 | 61,67 |
| 4 | Mannitol | P-F68 | Glycine | AcetatePH5.5 | 4,7 | 0,37 | 0 | 61,58 |
| 53 | Sucrose | Tween 80 | Glycine | HistidinePH6 | 10,5 | 0 | 0 | 61,58 |
| 54 | Sucrose | Tween 80 | Glycine | PhosphatePH6 | 6,4 | 0,90 | 0 | 61,52 |
| 17 | Mannitol | Tween 80 | Glycine | HistidinePH6 | 5,6 | 0 | 0 | 61,44 |
| 37 | Sucrose | P-F68 | Arg/Glu | AcetatePH5.5 | 10,5 | 0 | 0 | 61,43 |
| 23 | Sorbitol | P-F68 | Glycine | HistidinePH6 | 5,6 | 0 | 0 | 61,39 |
| 12 | Mannitol | Tween 20 | Glycine | PhosphatePH6 | 5,6 | 0 | 0 | 61,33 |
| 10 | Mannitol | Tween 20 | Glycine | AcetatePH5.5 | 5,6 | 0 | 0 | 61,2 |
| 30 | Sorbitol | Tween 20 | Glycine | PhosphatePH6 | 0,0 | 2,3 | 0,0021 | 61,2 |
| 29 | Sorbitol | Tween 20 | Glycine | HistidinePH6 | 5,6 | 0 | 0 | 61,19 |
| 50 | Sucrose | Tween 80 | Arg/Glu | HistidinePH6 | 10,5 | 0 | 0 | 61,14 |
| 52 | Sucrose | Tween 80 | Glycine | AcetatePH5.5 | 8,4 | 0,46 | 0 | 61,1 |
| 2 | Mannitol | P-F68 | Arg/Glu | HistidinePH6 | 5,6 | 0 | 0 | 61,07 |
| 45 | Sucrose | Tween 20 | Arg/Glu | PhosphatePH6 | 10,5 | 0 | 0 | 61,01 |
| 18 | Mannitol | Tween 80 | Glycine | PhosphatePH6 | 5,6 | 0 | 0 | 60,93 |
| 8 | Mannitol | Tween 20 | Arg/Glu | HistidinePH6 | 5,6 | 0 | 0 | 60,88 |
| 28 | Sorbitol | Tween 20 | Glycine | AcetatePH5.5 | 5,6 | 0 | 0 | 60,86 |
| 24 | Sorbitol | P-F68 | Glycine | PhosphatePH6 | 3,2 | 1,00 | 0 | 60,84 |
| 16 | Mannitol | Tween 80 | Glycine | AcetatePH5.5 | 5,6 | 0 | 0 | 60,73 |
| 19 | Sorbitol | P-F68 | Arg/Glu | AcetatePh5.5 | 5,6 | 0 | 0,062 | 60,71 |
| 49 | Sucrose | Tween 80 | Arg/Glu | AcetatePH5.5 | 10,5 | 0 | 0 | 60,68 |
| 20 | Sorbitol | P-F68 | Arg/Glu | HistidinePH6 | 5,6 | 0 | 0,036 | 60,65 |
| 39 | Sucrose | P-F68 | Arg/Glu | PhosphatePH6 | 10,5 | 0 | 0 | 60,5 |
| 1 | Mannitol | P-F68 | Arg/Glu | AcetatePH5.5 | 5,6 | 0 | 0 | 60,43 |
| 34 | Sorbitol | Tween 80 | Glycine | AcetatePH5.5 | 3,9 | 0,69 | 0 | 60,29 |
| 26 | Sorbitol | Tween 20 | Arg/Glu | HistidinePH6 | 5,6 | 0 | 0,0004 | 60,27 |
| 35 | Sorbitol | Tween 80 | Glycine | HistidinePH6 | 5,6 | 0 | 0 | 60,18 |
| 14 | Mannitol | Tween 80 | Arg/Glu | HistidinePH6 | 5,6 | 0 | 0 | 60,17 |
| 3 | Mannitol | P-F68 | Arg/Glu | PhosphatePH6 | 5,5 | 0.06 + 0.05 | 0 | 60,15 |
| 25 | Sorbitol | Tween 20 | Arg/Glu | AcetatePHS.5 | 5,6 | 0 | 0,0017 | 60,04 |
| 7 | Mannitol | Tween 20 | Arg/Glu | AcetatePH5.5 | 5,6 | 0 | 0 | 59,98 |
| 51 | Sucrose | Tween 80 | Arg/Glu | PhosphatePH6 | 10,5 | 0 | 0 | 59,98 |
| 9 | Mannitol | Tween 20 | Arg/Glu | PhosphatePH6 | 5,6 | 0 | 0 | 59,86 |
| 21 | Sorbitol | P-F68 | Ang/Giu | PhosphatePH6 | 5,6 | 0 | 0,106 | 59,73 |
| 13 | Mannitol | Tween 80 | Arg/Glu | AcetatePH5.5 | 5,6 | 0 | 0 | 59,54 |
| 15 | Mannitol | Tween 80 | Arg/Glu | PhosphatePH6 | 5,6 | 0 | 0 | 59,49 |
| 36 | Sorbitol | Tween 80 | Glycine | PhosphatePH6 | 5,6 | 0 | 0 | 59,46 |
| 31 | Sorbitol | Tween 80 | Arg/Glu | AcetatePH5.5 | 5,6 | 0 | 0,0027 | 59,36 |
| 32 | Sorbitol | Tween 80 | Arg/Glu | HistidinePH6 | 4,9 | 0.34 + 0.29 | 0,0009 | 59,34 |
| 27 | Sorbitol | Tween 20 | Arg/Glu | PhosphatePH6 | 5,6 | 0 | 0,0039 | 59,17 |
| 33 | Sorbitol | Tween 80 | Arg/Glu | PhosphatePH6 | 5,6 | 0 | 0,0049 | 58,59 |

**Table 37. Comparison of the aggregation onset temperatures and maximum scatter reached for 23IL0075 in 3 buffers (25µg/mL), as measured by elastic light scattering. (Temperature interval: 45-95°C, temperature gradient: 2°C/min, data pitch: 1°C, wavelength (ex/em): 500nm, band width (ex/em): 3nm.)**

| | **Aggregation onset temperature** | **Maximum scatter reached** |
|---|---|---|
| **10 mM phosphate pH 6** | 52.1 °C | Out of scale |
| **10 mM acetate pH 6** | 51.0 °C | Out of scale |
| **10 mM histidine pH 6** | 52.7 °C | 435 abs |

**Table 38. List of buffers tested in freeze/thaw and stir stress study of 23IL0075 (10 mg/mL)**

| **No.** | **Buffer** | | |
|---|---|---|---|
| 1 | 10mM Acetate pH 5.5 | 5.6% mannitol | 0.0025% Tween 80 |
| 2 | 10mM Acetate pH 5.5 | 5.6% mannitol | 0.005% Tween 80 |
| 3 | 10mM Acetate pH 5.5 | 5.6% mannitol | 0.05% P-F68 |
| 4 | 10mM Acetate pH 5.5 | 5.6% mannitol | 0.1% P-F68 |
| 5 | 10mM Histidine pH 6.0 | 5.6% mannitol | 0.0025% Tween 80 |
| 6 | 10mM Histidine pH 6.0 | 5.6% mannitol | 0.005% Tween 80 |
| 7 | 10mM Histidine pH 6.0 | 5.6% mannitol | 0.05% P-F68 |
| 8 | 10mM Histidine pH 6.0 | 5.6% mannitol | 0.1% P-F68 |
| 9 | 10mM Phosphate pH 6.0 | 5.6% mannitol | 0.0025% Tween 80 |
| 10 | 10mM Phosphate pH 6.0 | 5.6% mannitol | 0.005% Tween 80 |
| 11 | 10mM Phosphate pH 6.0 | 5.6% mannitol | 0_05°/a P-F68 |
| 12 | 10mM Phosphate pH 6.0 | 5.6% mannitol | 0.1 iso P-F68 |
| 13 | 10mM Acetate pH 5.5 | 2.8% mannitol 1.15% glycine | 0.0025% Tween 80 |
| 14 | 10mM Acetate pH 5.5 | 2.8°/ mannitol 1.15% glycine | 0.005% Tween 80 |
| 15 | 10mM Acetate pH 5.5 | 2.8% mannitol 1.15% glycine | 0.05°lo P-F68 |
| 16 | 10mM Acetate pH 5.5 | 2.8% mannitol 1.15% glycine | 0.1% P-F68 |
| 17 | 10mM Histidine pH 6.0 | 2.8% mannitol 1.15% glycine | 0.0025% Tween 80 |
| 18 | 10mM Histidine pH 6.0 | 2.8% mannitol 1.15% glycine | 0.005% Tween 80 |
| 19 | 10mM Histidine pH 6.0 | 2.8% mannitol 1.15% glycine | 0.05% P-F68 |
| 20 | 10mM Histidine pH 6.0 | 2.8% mannitol 1.15% glycine | 0.1% P-F68 |
| 21 | 10mM Phosphate pH 6.0 | 2.8% mannitol 1.15% glycine | 0.0025% Tween 80 |
| 22 | 10mM Phosphate pH 6.0 | 2.8% mannitol 1.15% glycine | 0.005% Tween 80 |
| 23 | 10mM Phosphate pH 6.0 | 2.8% mannitol 1.15% glycine | 0.05% P-F68 |
| 24 | 10mM Phosphate pH 6.0 | 2.8% mannitol 1.15% glycine | 0.1% P-F68 |

**Table 39. Stability study of 23IL0075 in 10mM Histidine pH 6.0 with different excipients. The samples were stressed by 10Xfreeze/thaw, and were stored at different temperatures (-70°C, 5°C, 25°C and 37°C) for a stability study. The stressed and stability samples were analyzed using OD measurement, RP-HPLC and SE-HPLC.**

| **No.** | **Conc.** (mg/mL) | **Buffer** | **Mannitol** | **Sucrose** | **Glycine** | **Poloxamer 188** | **Tween-80** |
|---|---|---|---|---|---|---|---|
| 1 | 25 | 10 mM L-histidine, pH 6 | 5,4% | | | | 0,005% |
| 2 | 25 | 10 mM L-histidine, pH 6 | | 10,0% | | | 0,005% |
| 3 | 25 | 10 mM L-histidine, pH 6 | 3,5% | 3,5% | | | 0,005% |
| 4 | 25 | 10 mM L-histidine, pH 6 | 2,8% | | 1,15% | | 0,005% |
| 5 | 25 | 10 mM L-histidine, pH 6 | 5,4% | | | 0,05% | |
| 6 | 25 | 10 mM L-histidine, pH 6 | | 10,0% | | 0,05% | |
| 7 | 25 | 10 mM L-histidine, pH 6 | 3,7% | 3,5% | | 0,05% | |
| 8 | 25 | 10 mM L-histidine, pH 6 | 2,8% | | 1,15% | 0,05% | |
| 9 | 25 | 10 mM L-histidine, pH 6 | 3,5% | 3,5% | | | |

**Table 40. Samples subjected to shear stress by stirring. The samples were afterwards analyzed by OD measurements, RP-HPLC, SE-HPLC and Biacore.**

| **No.** | **Conc.** | **Buffer** | **Mannitol** | **Sucrose** | **Glycine** |
|---|---|---|---|---|---|
| 1 | 10 mg/mL | 10 mM L-histidine, pH 6 | 5,4% | | |
| 2 | 10 mg/mL | 10 mM L-histidine, pH 6 | | 10,0% | |
| 3 | 10 mg/mL | 10 mM L-histidine, pH 6 | 3,5% | 3,5% | |
| 4 | 10 mg/mL | 10 mM L-histidine, pH 6 | 2,8% | | 1,15% |

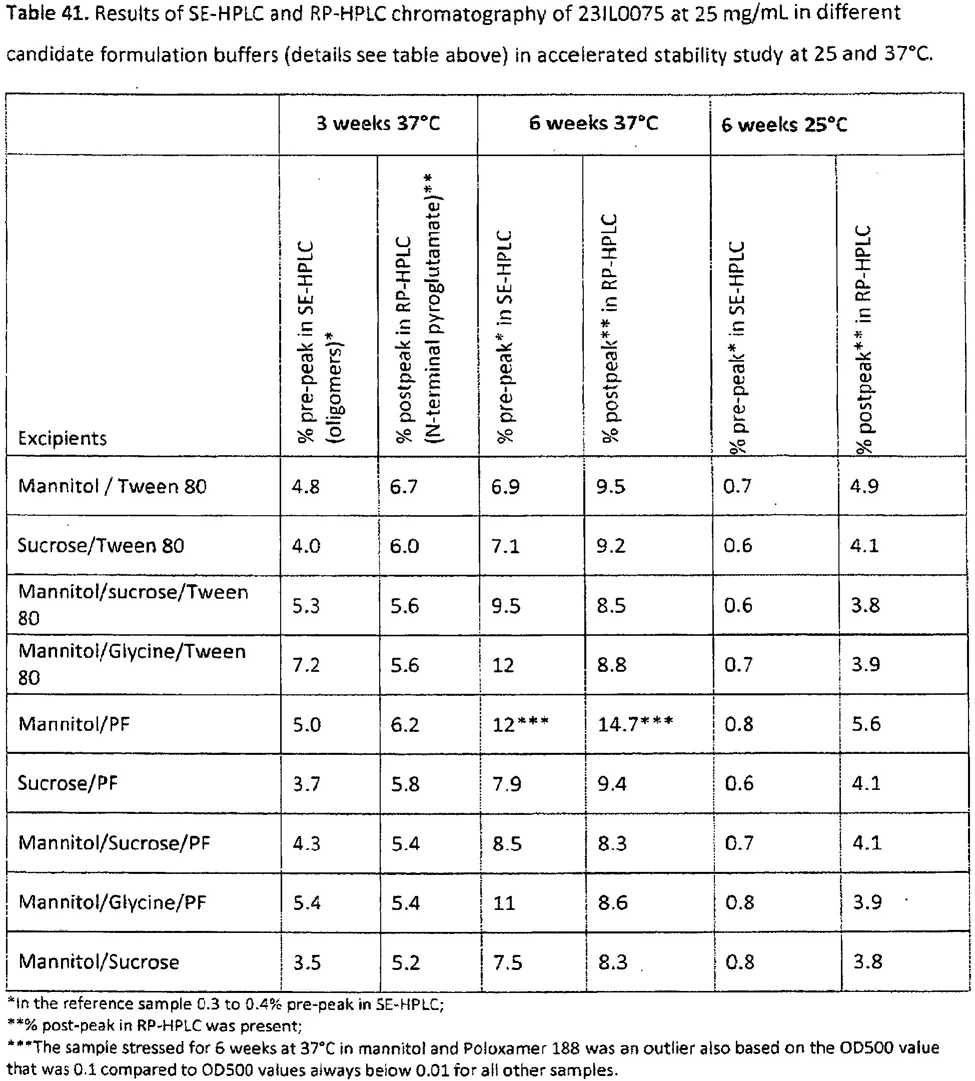

**Table 42. Stability data of IL6R304 batch CMC-D-0048, stored at -70°C.**

| | **Time point (months)** | | |
|---|---|---|---|
| **Test Method** | **Initial (0)** | **3** | **6** |
| Appearance | Clear, colorless solution | Clear, colorless solution | Clear, colorless solution |
| A280 | 10.52 mg/m | 10.38 mg/mL | mg/mL |
| SEC-HPLC | Purity = 99.20 % | Purity = 98.67% | Purity = 98.76 % |
| | Pre peaks = 0.80 % | Pre peaks = 1.33 % | Pre peaks = 1.24 % |
| | Post peaks = 0.00 % | Post peaks = 0.00 % | Post peaks = 0.00 % |
| cIEF | Purity = 100.00 % | Purity = 100.00 % | Purity = 99.30 % |
| | Post peak = 0.00 % | Post peak = 0.00 % | Post peak = 0.70 % |
| RP-HPLC | Purity = 93.90 % | Purity = 91.93 % | Purity = 92.8 % |
| | Pre peak 1 = 0.00 % | Pre peak 1 = 0.14 % | Pre peak 1 = 0.12 % |
| | Pre peak 2 = 3.20 % | Pre peak 2 = 3.41 % | Pre peak 2 = 3.00% |
| | Post peak 1 = 0.00 % | Post peak 1 = 0.00 % | Post peak 1 = 0.00 % |
| | Post peak 2 = 2.60 % | Post peak 2 = 4.10 % | Post peak 2 = 3.60 % |
| | Post peak 3 = 0.00 % | Post peak 3 = 0.00 % | Post peak 3 = 0.00 % |
| | Post peak 4 = 0.20 % | Post peak 4 = 0.29 % | Post peak 4 = 0.32 % |
| | Post peak 5 = 0.00 % | Post peak 5 = 0.14 % | Post peak 5 = 0.15 % |
| Potency (IL-6R inhibition) | 1.256 ± 0.084 | 0.973 ± 0.072 | 1.049 ± 0.090 |
| Potency (HSA binding) | 1.044 ± 0.094 | 0.955 ± 0.085 | 0.985 ± 0.069 |

**Table 43. Stability data of IL6R304 batch CMC-D-0048, stored at +5°C.**

| | **Time point (months)** | | |
|---|---|---|---|
| **Test Method** | **Initial (0)** | **3** | **6** |
| Appearance | Clear, colorless solution | Clear, colorless solution | Clear, colorless solution |
| A280 | 10.52 mg/mL | 10.29 mg/mL | 10.33 mg/mL |
| SEC-HPLC | Purity = 99.20 % | Purity = 98.50 % | Purity = 98.62% |
| | Pre peaks = 0.80 % | Pre peaks = 1.50 % | Pre peaks = 1.38 % |
| | Post peaks = 0.00 % | Post peaks = 0.00 % | Post peaks = 0.00 % |
| cIEF | Purity = 100.00 % | Purity = 100.00 % | Purity = 99.30 % |
| | Post peak = 0.00 % | Post peak = 0.00 % | Post peak = 0.70 % |
| RP-HPLC | Purity = 93.90 % | Purity = 91.71 % | = 92.30 % |
| | Pre peak 1 = 0.00 % | Pre peak 1 = 0.15 % | Pre peak 1=0.11% |
| | Pre peak 2 = 3.20 % | Pre peak 2 = 3.53 % | Pre peak 2 = 3.20 % |
| | Post peak 1 = 0.00 % | Post peak 1 = 0.00 % | Post peak 1 = 0.00 % |
| | Post peak 2 = 2.60 % | Post peak 2 = 4.16 % | Post peak 2 = 3.90 % |
| | Post peak 3 = 0.00 % | Post peak 3 = 0.00 % | Post peak 3 = 0.00 % |
| | Post peak 4 = 0.20 % | Post peak 4 = 0.29 % | Post peak 4 = 0.35 % |
| | Post peak 5 = 0.00 % | Post peak 5 = 0.15 % | Post peak 5 = 0.12 % |
| Potency (IL6R inhibition) | 1.256 ± 0.084 | 0.959 ± 0.061 | 1.015 ± 0.076 |
| Potency (HSA binding) | 1.044 ± 0.094 | 0.930 ± 0.103 | 0.983 ± 0.078 |

**Table 44. Stability data of IL6R304 batch CMC-D-0048, stored at +25°C.**

| | **Time point (months)** | | |
|---|---|---|---|
| **Test Method** | **Initial (0)** | **3** | **6** |
| Appearance | Clear, colorless solution | Clear, colorless solution | Clear, colorless solution |
| A280 | 10.52 mg/mL | 10.29 mg/mL | 10.48 mg/mL |
| SEC-HPLC | Purity = 99.20 % | Purity = 97.81 % | Purity = 97.13 % |
| | Pre peaks = 0.80 % | Pre peaks = 1.57 % | Pre peaks = 1.87 % |
| | Post peaks = 0.00 % | Post peaks = 0.62 % | Post peaks = 1.00 % |
| clEF | Purity = 100.00 % | Purity = 96.40 % | Purity = 92.30 % |
| | Post peak = 0.00 % | Post peak = 3.60 % | Post peak = 7.70 % |
| RP-HPLC | Purity = 93.90 % | Purity = 87.77 % | Purity = 82.30 % |
| | Pre peak 1 = 0.00 % | Pre peak 1 = 0.44 % | Pre peak 1 = 0.87 % |
| | Pre peak 2 = 3.20 % | Pre peak 2 = 4.56 % | Pre peak 2 = 6.40 % |
| | Post peak 1= 0.00 % | Post peak 1 = 0.00 % | Post peak 1 = 1.10 % |
| | Post peak 2 = 2.60 % | Post peak 2 = 6.26 % | Post peak 2 = 8.80 % |
| | Post peak 3 = 0.00 % | Post peak 3 = 0.54 % | Post peak 3 = 0.86 % |
| | Post peak 4 = 0.20 % | Post peak 4 = 0.30 % | Post peak 4 = 0.32 % |
| | Post peak 5 = 0.00 % | Post peak 5 = 0.13 % | Post peak 5 = 0.16 % |
| Potency (IL6R inhibition) | 1.256 ± 0.084 | 0.945 ± 0.065 | 0.949 ± 0.066 |
| Potency (HSA binding) | 1.044 ± 0.094 | 0.967 ± 0.095 | 0.926 ± 0.065 |

## Claims

1. A formulation comprising an aqueous carrier having a pH of 5.5 to 8.0 and a polypeptide comprising one or more single variable domains at a concentration of 1 mg/mL to 200 mg/mL, said formulation being formulated for administration to a human subject and said formulation further comprising
a buffer at a concentration of 10mM to 100mM selected from histidine pH 6.0-6.5,
wherein said formulation has an inorganic salt concentration of 150 mM or lower and wherein the polypeptide is selected from one of SEQ ID NO's: 1 to 6.

2. The formulation of claim 1, that does not contain any inorganic salt.

3. The formulation of any of claims 1 or 2, wherein the concentration of polypeptide is 5 to 100 mg/mL, more preferably 5 to 50 mg/mL, such as 5 mg/mL, 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, or even more.

4. The formulation of any of claims 1 to 3, wherein
- the polypeptide has a solubility of at least 20 mg/mL, preferably 50 mg/mL or more, more preferably 90 mg/mL, as determined by the PEG exclusion method or by a concentration experiment;
- less than 10% of the polypeptide forms pyroglutamate at the N-terminal glutamic acid during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more), the % of pyroglutamate as measured by RP-HPLC;
- less than 10% of the polypeptide forms dimers during storage at a temperature of 37±5°C up to at least 2 weeks (preferably at least 3 weeks, at least 5 weeks, at least 8 weeks, at least 10 weeks, at least 3 months, at least 6 months, at least 1 year, 1.5 year or even 2 years or more), the % of dimers as measured by SE-HPLC;
- at least 80% of the polypeptides retains their binding activity to at least one of their targets after storage at 37±5°C up to at least 2 weeks (preferably at least 3 weeks, at least 5 weeks, at least 2 months, at least 6 months, at least 1 year, 1.5 year or even 2 years or more) compared to the binding activity prior to storage, said binding activity as measured by ELISA and/or Biacore; and/or
- the polypeptide has an OD320/OD280 ratio of 0.05 or lower after mechanical stress selected from:
∘ shaking the formulation during 10s to 1 min;
∘ pushing the formulation through a needle (25G, preferably 26G, more preferably 27G, even more preferably 28G, most preferably 29G or more) with a syringe (the syringe used can be any commercially available syringe, such as e.g. a 1 mL, 2 mL, 3mL, 4 mL, 5 mL, 10 mL, 20 mL, 30 mL, 40 mL up to 50 mL syringe);
∘ rotating for two days at 10 rpm; and/or
∘ stirring for 1 hour at room temperature and/or 4-48 hours at 4°C at at least 10 rpm (such as 50 rpm, 100 rpm or more).

5. The formulation of any of claims 1 to 4, wherein the buffer is a histidine pH 6.5 or histidine pH 6.0 buffer.

6. The formulation of claim 5, wherein the histidine buffer has a concentration of 10 to 50 mM, more preferably 10 to 20 mM, such as 10 mM or 15 mM.

7. The formulation of any of claims 1 to 6, further comprising an excipient at a concentration of 1% to 20%.

8. The formulation of any of claims 1 to 7, wherein the excipient is a saccharide, a non-reducing sugar and/or polyol.

9. The formulation of claim 8, wherein the excipient is selected from mannitol, trehalose, sorbitol and sucrose.

10. The formulation of any of claims 8 or 9 , wherein the excipient has a concentration of 2.5% to 15%, more preferably 5% to 10%, such as about 5%, 7.5%, 8% and 10%.

11. The formulation of any of claims 1 to 10, further comprising a surfactant at a concentration of 0.001% to 1% selected from polysorbate 20, polysorbate 80 or a poloxamer.

12. The formulation of any of claims 1 to 11, wherein the surfactant is polysorbate 80.

13. The formulation of claim 12, wherein the surfactant has a concentration of 0.01% to 0.1%, preferably 0.01% to 0.05%, such as about 0.01% or 0.005%.

14. The formulation of any of claims 1 to 13, comprising:
a) A histidine pH 6.5 or pH 6.0 buffer at a concentration of 10mM to 100mM;
b) Sucrose at a concentration of 1% to 20%; and
c) Polysorbate 80 at a concentration of 0.001% to 1%.

15. The formulation of claim 14, comprising:
a) 15 mM histidine pH 6.5;
b) 8% sucrose; and
c) 0.01% polysorbate 80.

16. The formulation of claim 14, comprising:
a) 10 mM histidine pH 6.0;
b) 10% sucrose; and
c) 0.005% polysorbate 80.

17. A method for the preparation of a formulation of any of claims 1 to 16, at least comprising the step of concentrating the polypeptide and exchanging it with the selected buffer and/or excipient.

18. A sealed container containing a formulation according to any of claims 1 to 16.

19. A pharmaceutical unit dosage form suitable for parenteral administration to a human, comprising a formulation according to any of claims 1 to 16 in a suitable container.

20. A kit comprising one or more of the sealed containers according to claim 18 and/or pharmaceutical unit dosage forms according to claim 19, and instructions for use of the formulation.

21. The formulation, container, pharmaceutical unit dosage or kit according to any of the preceding claims for use in therapy.

22. The formulation, container, pharmaceutical unit dosage or kit according to claim 21 for treatment of osteoporosis, cancer induced bone loss, bone loss associated with autoimmunity and/or viral infection, rheumatoid arthritis, abnormal synovial cell growth, plasmocytosis induced Castleman's disease, tumor, muscle protein proteolysis, multiple sclerosis, systemic lupus erythematosus, inflammatory bowel disease, pancreatitis, psoriasis, angiogenesis, systemic-onset type juvenile rheumatoid arthritis, spinal cord injury, endothelial injury or destruction, mesothelioma, vasculitis, osteoarthritis, inner ear disorder, cancer, rejection after transplantation, pancreatic islet transplantation, myocardial infraction, prostate cancer, choroidal neovascularization, muscle regeneration, inflammatory myopathy, chronic rejection in cardiac transplant, delayed graft function, as bowel diseases (colitis, Crohn's disease, IBD), infectious diseases, psoriasis, cancer, carcoidis, transplant rejection, cystic fibrosis, asthma, chronic obstructive pulmonary disease, viral infection, common variable immunodeficiency.

## Patentansprüche

1. Formulierung, umfassend einen wässrigen Träger mit einem pH von 5,5 bis 8,0 und ein Polypeptid, das eine oder mehr einzelne variable Domänen umfasst, in einer Konzentration von 1 mg/mL bis 200 mg/mL, wobei die Formulierung für die Verabreichung an ein menschliches Subjekt formuliert ist und wobei die Formulierung ferner
einen Puffer in einer Konzentration von 10 mM bis 100 mM, der aus Histidin pH 6,0 bis 6,5 ausgewählt ist, umfasst, wobei die Formulierung eine Konzentration anorganischer Salze von 150 mM oder niedriger aufweist und wobei das Polypeptid ausgewählt ist aus einem von SEQ ID NO's: 1 bis 6.

2. Formulierung gemäß Anspruch 1, die kein anorganisches Salz enthält.

3. Formulierung gemäß einem der Anprüche 1 oder 2, wobei die Konzentration von Polypeptid 5 bis 100 mg/mL, mehr bevorzugt 5 bis 50 mg/mL, wie zum Beispiel 5 mg/mL, 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, oder noch mehr beträgt.

4. Formulierung gemäß einem der Ansprüche 1 bis 3, wobei
- das Polypeptid eine Löslichkeit von mindestens 20 mg/mL aufweist, bevorzugt 50 mg/mL oder mehr, mehr bevorzugt 90 mg/mL, wie durch die PEG-Ausschlussmethode oder durch ein Konzentrationsexperiment ermittelt;
- weniger als 10% des Polypeptids an der N-terminalen Glutaminsäure Pyroglutamat bildet während Lagerung bei einer Temperatur von 37±5°C bis zu mindestens 2 Wochen (bevorzugt mindestens 3 Wochen, mindestens 5 Wochen, mindestens 8 Wochen, mindestens 10 Wochen, mindestens 3 Monate, mindestens 6 Monate, mindestens 1 Jahr, 1,5 Jahre oder sogar 2 Jahre oder mehr), wobei das % von Pyroglutamat mittels RP-HPLC gemessen wird;
- weniger als 10% des Polypeptids Dimere bildet während Lagerung bei einer Temperatur von 37±5°C bis zu mindestens 2 Wochen (bevorzugt mindestens 3 Wochen, mindestens 5 Wochen, mindestens 8 Wochen, mindestens 10 Wochen, mindestens 3 Monate, mindestens 6 Monate, mindestens 1 Jahr, 1,5 Jahre oder sogar 2 Jahre oder mehr), wobei das % von Dimeren mittels SE-HPLC gemessen wird;
- mindestens 80% der Polypeptide ihre Bindungsaktivität gegenüber mindestens einem ihrer Ziele beibehalten nach Lagerung bei 37±5°C bis zu mindestens 2 Wochen (bevorzugt mindestens 3 Wochen, mindestens 5 Wochen, mindestens 2 Monate, mindestens 6 Monate, mindestens 1 Jahr, 1,5 Jahre oder sogar 2 Jahre oder mehr) verglichen mit der Bindungsaktivität vor Lagerung, wobei die Bindungsaktivität mittels ELISA und/oder Biacore gemessen wird; und/oder
- das Polypeptid ein OD320/OD280-Verhältnis von 0,05 oder geringer aufweist nach mechanischer Belastung, die ausgewählt ist aus:
∘ die Formulierung während 10s bis 1 min schütteln;
∘ die Formulierung durch eine Nadel (25G, bevorzugt 26G, mehr bevorzugt 27G, noch mehr bevorzugt 28G, am meisten bevorzugt 29G oder mehr) drücken mit einer Spritze (die verwendete Spritze kann jede kommerziell erhältliche Spritze sein, wie zum Beispiel eine 1 mL-, 2 mL- , 3 mL-, 4 mL-, 5 mL-, 10 mL-, 20 mL-, 30 mL-, 40 mL- bis zu 50 mL-Spritze);
∘ zwei Tage lang Rotieren bei 10 rpm; und/oder
∘ 1 Stunde lang Rühren bei Raumtemperatur und/oder 4 bis 48 Stunden bei 4°C bei mindestens 10 rpm (wie zum Beispiel 50 rpm, 100 rpm oder mehr).

5. Formulierung gemäß einem der Ansprüche 1 bis 4, wobei der Puffer ein Histidinpuffer mit pH 6,5 oder pH 6,0 ist.

6. Formulierung gemäß Anspruch 5, wobei der Histidinpuffer eine Konzentration von 10 bis 50 mM aufweist, mehr bevorzugt 10 bis 20 mM, wie zum Beispiel 10 mM oder 15 mM.

7. Formulierung gemäß einem der Ansprüche 1 bis 6, die ferner einen Exzipienten in einer Konzentration von 1% bis 20% umfasst.

8. Formulierung gemäß einem der Ansprüche 1 bis 7, wobei der Exzipient ein Saccharid, ein nicht-reduzierender Zucker und/oder ein Polyol ist.

9. Formulierung gemäß Anspruch 8, wobei der Exzipient ausgewählt ist aus Mannitol, Trehalose, Sorbitol und Sucrose.

10. Formulierung gemäß einem der Ansprüche 8 oder 9, wobei der Exzipient eine Konzentration von 2,5% bis 15% aufweist, mehr bevorzugt 5% bis 10%, wie zum Beispiel ungefähr 5%, 7,5%, 8% und 10%.

11. Formulierung gemäß einem der Ansprüche 1 bis 10, die ferner ein Tensid in einer Konzentration von 0,001% bis 1% umfasst, das ausgewählt ist aus Polysorbat 20, Polysorbat 80 oder einem Poloxamer.

12. Formulierung gemäß einem der Ansprüche 1 bis 11, wobei das Tensid Polysorbat 80 ist.

13. Formulierung gemäß Anspruch 12, wobei das Tensid eine Konzentration von 0,01% bis 0,1% aufweist, bevorzugt 0,01% bis 0,05%, wie zum Beispiel ungefähr 0,01% oder 0,005%.

14. Formulierung gemäß einem der Ansprüche 1 bis 13, umfassend:
a) einen Histidinpuffer mit pH 6,5 oder pH 6,0 in einer Konzentration von 10mM bis 100mM;
b) Sucrose in einer Konzentration von 1% bis 20%; und
c) Polysorbat 80 in einer Konzentration von 0,001% bis 1%.

15. Formulierung gemäß Anspruch 14, umfassend:
a) 15 mM Histidin pH 6,5;
b) 8% Sucrose; und
c) 0,01% Polysorbat 80.

16. Formulierung gemäß Anspruch 14, umfassend:
a) 10 mM Histidin pH 6,0;
b) 10% Sucrose; und
c) 0,005% Polysorbat 80.

17. Verfahren für die Herstellung einer Formulierung gemäß einem der Ansprüche 1 bis 16, das mindestens den Schritt des Konzentrierens des Polypeptids und des Austauschens desselben mit dem gewählten Puffer und/oder Exzipienten umfasst.

18. Versiegelter Behälter, der eine Formulierung gemäß einem der Ansprüche 1 bis 16 enthält.

19. Pharmazeutische Einheitsdosierungsform, die geeignet ist für die parenterale Verabreichung an einen Menschen, umfassend eine Formulierung gemäß einem der Ansprüche 1 bis 16 in einem geeigneten Behälter.

20. Kit, das einen oder mehr der versiegelten Behälter gemäß Anspruch 18 und/oder pharmazeutische Einheitsdosierungsformen gemäß Anspruch 19, und Anweisungen für die Verwendung der Formulierung, umfasst.

21. Formulierung, Behälter, pharmazeutische Einheitsdosierung oder Kit gemäß einem der vorhergehenden Ansprüche zur Verwendung in Therapie.

22. Formulierung, Behälter, pharmazeutische Einheitsdosierung oder Kit gemäß Anspruch 21 zur Behandlung von Osteoporose, krebsinduziertem Knochenverlust, mit Autoimmunität und/oder viraler Infektion assoziiertem Knochenverlust, rheumatoider Arthritis, abnormalem synovialem Zellwachstum, plasmozytoseinduzierter Castleman-Krankheit, Tumor, Muskelprotein-Proteolyse, Multipler Sklerose, systemischem Lupus erythematodes, chronischentzündlicher Darmerkrankung, Pankreatitis, Psoriasis, Angiogenese, systemisch einsetzender juveniler rheumatoider Arthritis, Wirbelsäulenverletzung, endothelialer Verletzung oder Zerstörung, Mesotheliom, Vaskulitis, Osteoarthritis, Innenohrstörung, Krebs, Abstoßung nach Transplantation, Transplantation von pankreatischen Inseln, Herzinfarkt, Prostatakrebs, choroidaler Neovaskularisation, Muskelregeneration, entzündlicher Myopathie, chronischer Abstoßung bei Herztransplantation, verzögerter Transplantatfunktion, als Darmkrankheiten (Colitis, Morbus Crohn, chronischentzündlicher Darmerkrankung), Infektionskrankheiten, Psoriasis, Krebs, Carcoidis, Transplantatabstoßung, zystischer Fibrose, Asthma, chronischer obstruktiver Lungenerkrankung, viraler Infektion, variablem Immundefektsyndrom.

## Revendications

1. Formulation comprenant un support aqueux ayant un pH de 5,5 à 8,0 et un polypeptide comprenant un ou plusieurs domaines variables simples à une concentration de 1 mg/ml à 200 mg/ml, ladite composition étant formulée pour une administration à un sujet humain et ladite formulation comprenant en outre
un tampon à une concentration de 10 mM à 100 mM choisi parmi l'histidine pH 6,0 à 6,5, dans laquelle ladite formulation a une concentration en sel minéral de 150 mM ou moins et dans laquelle le polypeptide est choisi parmi l'une de SEQ ID NOs : 1 à 6.

2. Formulation selon la revendication 1, qui ne contient pas de sel minéral.

3. Formulation selon l'une quelconque des revendications 1 ou 2, dans laquelle la concentration de polypeptide est 5 à 100 mg/ml, de préférence encore 5 à 50 mg/ml, tel que 5 mg/ml, 10 mg/ml, 20 mg/ml, 30 mg/ml, 40 mg/ml, 50 mg/ml, 60 mg/ml, 70 mg/ml, 80 mg/ml, 90 mg/ml, 100 mg/ml, ou même plus.

4. Formulation selon l'une quelconque des revendications 1 à 3, dans laquelle
- le polypeptide a une solubilité d'au moins 20 mg/ml, de préférence 50 mg/ml ou plus, de préférence encore 90 mg/ml, telle que déterminée par le procédé d'exclusion au PEG ou par une expérience de concentration ;
- moins de 10 % du polypeptide forme du pyroglutamate au niveau de l'acide glutamique N-terminal au cours du stockage à une température de 37 ± 5 °C jusqu'à au moins 2 semaines (de préférence au moins 3 semaines, au moins 5 semaines, au moins 8 semaines, au moins 10 semaines, au moins 3 mois, au moins 6 mois, au moins 1 an, 1,5 année ou même 2 ans ou plus), le % de pyroglutamate tel que mesuré par RP-HPLC ;
- moins de 10 % du polypeptide forme des dimères au cours du stockage à une température de 37 ± 5 °C jusqu'à au moins 2 semaines (de préférence au moins 3 semaines, au moins 5 semaines, au moins 8 semaines, au moins 10 semaines, au moins 3 mois, au moins 6 mois, au moins 1 an, 1,5 année ou même 2 ans ou plus), le % de dimères tel que mesuré par SE-HPLC ;
- au moins 80 % des polypeptides conservent leur activité de liaison à au moins l'une de leurs cibles après stockage à 37 ± 5 °C jusqu'à au moins 2 semaines (de préférence au moins 3 semaines, au moins 5 semaines, au moins 2 mois, au moins 6 mois, au moins 1 an, 1,5 année ou même 2 ans ou plus) par rapport à l'activité de liaison avant le stockage, ladite activité de liaison telle que mesurée par ELISA et/ou Biacore ; et/ou
- le polypeptide a un rapport DO320/DO280 de 0,05 ou moins après une contrainte mécanique choisie parmi :
∘ secouer la formulation pendant 10 s à 1 min ;
∘ pousser la formulation à travers une aiguille (25G, de préférence 26G, de préférence encore 27G, même plus préférablement 28G, mieux encore 29G ou plus) avec une seringue (la seringue utilisée peut être n'importe quelle seringue disponible dans le commerce, telle que par exemple une seringue de 1 ml, 2 ml, 3 ml, 4 ml, 5 ml, 10 ml, 20 ml, 30 ml, 40 ml jusqu'à 50 ml) ;
∘ faire tourner pendant deux jours à 10 tours par minute ; et/ou
∘ agiter pendant 1 heure à température ambiante et/ou 4 à 48 heures à 4 °C à au moins 10 tours par minute (tel que 50 tours par minute, 100 tours par minute ou plus).

5. Formulation selon l'une quelconque des revendications 1 à 4, dans laquelle le tampon est un tampon d'histidine à pH 6,5 ou d'histidine à pH 6,0.

6. Formulation selon la revendication 5, dans laquelle le tampon d'histidine a une concentration de 10 à 50 mM, de préférence encore 10 à 20 mM, tel que 10 mM ou 15 mM.

7. Formulation selon l'une quelconque des revendications 1 à 6, comprenant en outre un excipient à une concentration de 1 % à 20 %.

8. Formulation selon l'une quelconque des revendications 1 à 7, dans laquelle l'excipient est un saccharide, un sucre non réducteur et/ou un polyol.

9. Formulation selon la revendication 8, dans laquelle l'excipient est choisi parmi le mannitol, le tréhalose, le sorbitol et le saccharose.

10. Formulation selon l'une quelconque des revendications 8 ou 9, dans laquelle l'excipient a une concentration de 2,5 % à 15 %, de préférence encore 5 % à 10 %, tel qu'environ 5 %, 7,5 %, 8 % et 10 %.

11. Formulation selon l'une quelconque des revendications 1 à 10, comprenant en outre un tensioactif à une concentration de 0,001 % à 1 % choisi parmi le polysorbate 20, le polysorbate 80 ou un poloxamère.

12. Formulation selon l'une quelconque des revendications 1 à 11, dans laquelle le tensioactif est le polysorbate 80.

13. Formulation selon la revendication 12, dans laquelle le tensioactif a une concentration de 0,01 % à 0,1 %, de préférence 0,01 % à 0,05 %, tel qu'environ 0,01 % ou 0,005 %.

14. Formulation selon l'une quelconque des revendications 1 à 13, comprenant :
a) un tampon d'histidine à pH 6,5 ou pH 6,0 à une concentration de 10 mM à 100 mM ;
b) du saccharose à une concentration de 1 % à 20 % ; et
c) du polysorbate 80 à une concentration de 0,001 % à 1 %.

15. Formulation selon la revendication 14, comprenant :
a) 15 mM d'histidine à pH 6,5 ;
b) 8 % de saccharose ; et
c) 0,01 % de polysorbate 80.

16. Formulation selon la revendication 14, comprenant :
a) 10 mM d'histidine à pH 6,0 ;
b) 10 % de saccharose ; et
c) 0,005% de polysorbate 80.

17. Procédé de préparation d'une formulation selon l'une quelconque des revendications 1 à 16, comprenant au moins l'étape de concentration du polypeptide et son échange avec le tampon et/ou l'excipient choisi.

18. Récipient scellé contenant une formulation selon l'une quelconque des revendications 1 à 16.

19. Forme posologique unitaire pharmaceutique appropriée pour une administration parentérale à un humain, comprenant une formulation selon l'une quelconque des revendications 1 à 16 dans un récipient approprié.

20. Trousse comprenant un ou plusieurs des récipients scellés selon la revendication 18 et/ou des formes posologiques unitaires pharmaceutiques selon la revendication 19, et des instructions pour l'utilisation de la formulation.

21. Formulation, récipient, dose unitaire pharmaceutique ou trousse selon l'une quelconque des revendications précédentes, pour une utilisation en thérapie.

22. Formulation, récipient, dose unitaire pharmaceutique ou trousse selon la revendication 21 pour le traitement de l'ostéoporose, d'une perte osseuse induite par un cancer, d'une perte osseuse associée à l'auto-immunité et/ou une infection virale, de l'arthrite rhumatoïde, de la croissance anormale des cellules synoviales, de la maladie de Castleman induite par une plasmocytose, d'une tumeur, de la protéolyse des protéines musculaires, de la sclérose en plaques, du lupus érythémateux disséminé, de la maladie inflammatoire de l'intestin, de la pancréatite, du psoriasis, de l'angiogenèse, de l'arthrite rhumatoïde juvénile du type à apparition systémique, d'une lésion de la moelle épinière, d'une lésion ou d'une destruction endothéliale, d'un mésothéliome, d'une vascularite, de l'arthrose, des troubles de l'oreille interne, du cancer, du rejet après transplantation, de la transplantation d'îlots de Langerhans, de l'infarctus du myocarde, du cancer de la prostate, de la néovascularisation choroïdienne, de la régénération musculaire, de la myopathie inflammatoire, du rejet chronique dans une transplantation cardiaque, de la reprise retardée de fonction du greffon, comme les maladies de l'intestin (colite, maladie de Crohn, MII), les maladies infectieuses, le psoriasis, le cancer, carcoidis, le rejet de greffe, la fibrose kystique, l'asthme, la broncho-pneumopathie chronique obstructive, une infection virale, l'immunodéficience variable commune.
